# EUROPEAN PATENT APPLICATION

(11) **EP 3 872 180 A1**
(43) Date of publication of application: **01.09.2021**
(21) Application number: 21154405.1
(22) Date of filing: 20.10.2017
(51) Int. Cl.: C12N 15/12, C12N 15/86, C07K 14/47, C07K 14/705, A61K 38/17

(54) **SECRETABLE VARIANT IMMUNOMODULATORY PROTEINS AND ENGINEERED CELL THERAPY**

(30) Priority: 20.10.2016 US 201662410827 P; 22.03.2017 US 201762475210 P; 27.07.2017 US 201762537921 P
(62) Divisional of application: 17804986.2
(71) Applicant: Alpine Immune Sciences, Inc., Seattle, WA 98119 (US)
(72) Inventor: Swanson, Ryan, Seattle, WA 98119 (US); Kornacker, Michael, Seattle, WA 98119 (US)
(74) Representative: Chapman, Paul Gilmour

(57) **Abstract**

Provided are immunomodulatory proteins, nucleic acids encoding such immunomodulatory proteins, cells engineered to express the immunomodulatory proteins and infections agents containing nucleic acid encoding the immunomodulatory proteins. In some embodiments, the immunomodulatory proteins are secretable. In some embodiments, the immunomodulatory proteins are transmembrane proteins that are surface expressed. The immunomodulatory proteins, engineered cells and infectious agents provide therapeutic utility for a variety of immunological and oncological conditions. Compositions and methods for making and using such proteins are provided.

## Description

### Cross-Reference to Related Applications

This application claims priority from U.S. provisional application No. 62/410,827, filed October 20, 2016, entitled "Secretable Variant Immunomodulatory Proteins and Engineered Cell Therapy," U.S. provisional application No. 62/475,210 filed March 22, 2017, entitled "Secretable Variant Immunomodulatory Proteins and Engineered Cell Therapy, " and U.S. provisional application No. 62/537,921 filed July 27, 2017, entitled "Secretable Variant Immunomodulatory Proteins and Engineered Cell Therapy," the contents of each of which are incorporated by reference in their entirety.

### Incorporation by Reference of Sequence Listing

The present application is being filed along with a Sequence Listing in electronic format. The Sequence Listing is provided as a file entitled 761612001440SeqList.TXT, created October 19, 2017 which is 3,524,332 bytes in size. The information in the electronic format of the Sequence Listing is incorporated by reference in its entirety.

### Field

The present disclosure provides immunomodulatory proteins, nucleic acids encoding such immunomodulatory proteins, cells engineered to express the immunomodulatory proteins and infections agents containing nucleic acid encoding the immunomodulatory proteins. In some embodiments, the immunomodulatory proteins are secretable. In some embodiments, the immunomodulatory proteins are transmembrane proteins that are surface expressed. The immunomodulatory proteins, engineered cells and infectious agents provide therapeutic utility for a variety of immunological and oncological conditions. Compositions and methods for making and using such proteins are provided.

### Background

Modulation of the immune response by intervening in the processes that occur in the immunological synapse (IS) formed by and between antigen-presenting cells (APCs) or target cells and lymphocytes is of increasing medical interest. Currently, biologics used to enhance or suppress immune responses have generally been limited to immunoglobulins (e.g., anti-PD-1 mAbs) or soluble receptors (e.g., Fc-CTLA4). Soluble receptors, in some cases, suffer from a number of deficiencies. While useful for antagonizing interactions between proteins, soluble receptors often lack the ability to agonize such interactions. Antibodies have proven less limited in this regard and examples of both agonistic and antagonistic antibodies are known in the art. Nevertheless, both soluble receptors and antibodies lack important attributes that are critical to function in the IS. Mechanistically, cell surface proteins in the IS can involve the coordinated and often simultaneous interaction of multiple protein targets with a single protein to which they bind. IS interactions occur in close association with the junction of two cells, and a single protein in this structure can interact with both a protein on the same cell (cis) as well as a protein on the associated cell (trans), likely at the same time. Although some agents are known that can modulate the IS, improved therapeutics are needed. Provided are embodiments that meet such needs.

### Summary

In one aspect, there is provided an immunomodulatory protein comprising at least one non-immunoglobulin affinity-modified immunoglobulin superfamily (IgSF) domain comprising one or more amino acid substitutions in a wild-type IgSF domain, wherein the at least one affinity-modified IgSF domain specifically binds at least one cell surface cognate binding partner of the wild-type IgSF domain; the immunomodulatory protein does not comprise a transmembrane domain; and the immunomodulatory protein is not conjugated to a half-life extending moiety. In some embodiments, the half-life extending moiety is a multimerization domain. In some embodiments, the half-life extending moiety is an Fc domain.

In some embodiments, the immunomodulatory protein further comprises a signal peptide. In some embodiments, the signal peptide is a native signal peptide from the corresponding wild-type IgSF member. In some embodiments, the signal peptide is a non-native signal peptide. In some embodiments, the signal peptide is a signal peptide from an immunoglobulin antibody molecule (e.g. an IgG-kappa signal peptide), an IL-2 signal peptide, or a CD33 signal peptide or other signal peptide known or described. Exemplary signal peptides are set forth in any of SEQ ID NOS: 413-430.

In some embodiments of the immunomodulatory protein, the at least one cell surface cognate binding partner is expressed on a mammalian cell. In some embodiments, the mammalian cell is an antigen presenting cell (APC), a tumor cell, or a lymphocyte. In some embodiments, the mammalian cell is a T-cell. In some embodiments, the mammalian cell is a mouse, rat, cynomolgus monkey, or human cell.

In some embodiments of the immunomodulatory protein, at least one affinity modified IgSF domain has increased binding affinity to the at least one cell surface cognate binding partner compared with the wild-type IgSF domain.

In some embodiments of the immunomodulatory protein, specific binding of the immunomodulatory protein comprising the at least one affinity-modified IgSF domain modulates immunological activity of the mammalian cell compared to the wild-type IgSF domain. In some embodiments, specific binding of the immunomodulatory protein comprising the at least one affinity-modified IgSF domain increases immunological activity of the mammalian cell compared to the wild-type IgSF domain. In some embodiments, specific binding of the immunomodulatory protein attenuates immunological activity of the mammalian cell compared to the wild-type IgSF domain.

In some embodiments of the immunomodulatory protein, the wild-type IgSF domain is from an IgSF family member of a family selected from the group consisting of Signal-Regulatory Protein (SIRP) Family, Triggering Receptor Expressed On Myeloid Cells Like (TREML) Family, Carcinoembryonic Antigen-related Cell Adhesion Molecule (CEACAM) Family, Sialic Acid Binding Ig-Like Lectin (SIGLEC) Family, Butyrophilin Family, B7 family, CD28 family, V-set and Immunoglobulin Domain Containing (VSIG) family, V-set transmembrane Domain (VSTM) family, Major Histocompatibility Complex (MHC) family, Signaling lymphocytic activation molecule (SLAM) family, Leukocyte immunoglobulin-like receptor (LIR), Nectin (Nec) family, Nectin-like (NECL) family, Poliovirus receptor related (PVR) family, Natural cytotoxicity triggering receptor (NCR) family, T cell immunoglobulin and mucin (TIM) family, and Killer-cell immunoglobulin-like receptors (KIR) family. In some embodiments, the wild-type IgSF domain is from an IgSF member selected from the group consisting of CD80, CD86, PD-L1, PD-L2, ICOS Ligand, B7-H3, B7-H4, CD28, CTLA4, PD-1, ICOS, BTLA, CD4, CD8-alpha, CD8-beta, LAG3, TIM-3, CEACAM1, TIGIT, PVR, PVRL2, CD226, CD2, CD160, CD200, CD200R, NKp30, VISTA, VSIG3, and VSIG8. In some embodiments, the wild-type IgSF domain is a human IgSF domain. In some embodiments, at least one affinity modified IgSF domain has at least 90% sequence identity to a wild-type IgSF domain or a specific binding fragment thereof contained in the sequence of amino acids set forth in any of SEQ ID NOS: 1-27 and 408. In some embodiments, the immunomodulatory protein has at least 90% sequence identity to the amino acid sequence selected from any of SEQ ID NOS:28-54 and 410 or to a specific binding fragment thereof containing an IgSF domain. In some embodiments, the wild-type IgSF domain is a member of the B7 family. In some embodiments, the wild-type IgSF domain is a domain of CD80, CD86 or ICOSL.

In some embodiments of the immunomodulatory protein, the at least one cell surface cognate binding partner is a stimulatory receptor expressed on a T-cell, and the at least one affinity-modified IgSF domain has increased binding affinity to the stimulatory receptor compared to the binding affinity of the wild-type IgSF domain to the stimulatory receptor. In some embodiments, binding of the affinity-modified IgSF domain to the stimulatory receptor, such as when secreted from a cell, increases immunological activity of the T-cell. In some embodiments, binding of the affinity-modified IgSF domain to the stimulatory receptor, such as when secreted from a cell, decreases immunological activity of the T-cell. In some embodiments, the stimulatory receptor is CD28, ICOS, or CD226. In some embodiments, the at least one affinity-modified IgSF domain is an affinity-modified ICOSL IgSF domain that has increased binding affinity to at least one of: ICOS and CD28. In some embodiments, the at least one affinity-modified IgSF domain is an affinity modified ICOSL IgSF domain and the stimulatory receptor is ICOS. In some embodiments, the at least one affinity-modified IgSF domain is an affinity modified ICOSL IgSF domain and the stimulatory receptor is CD28. In some embodiments, the at least one affinity-modified IgSF domain is an affinity modified CD80 IgSF domain and the stimulatory receptor is CD28. In some embodiments, the affinity-modified IgSF domain does not substantially specifically bind to CTLA-4 or exhibits decreased binding affinity to CTLA-4 compared to the binding affinity of wild-type IgSF domain to CTLA-4.

In some embodiments of the immunomodulatory protein, the at least one cell surface cognate binding partner is an inhibitory receptor expressed on a T-cell, and the at least one affinity-modified IgSF domain has increased binding affinity to the inhibitor receptor compared to the binding affinity of the wild-type IgSF domain to the inhibitor receptor. In some embodiments, binding of the affinity-modified IgSF domain to the inhibitory receptor, such as when secreted from a cell, increases immunological activity of the T-cell. In some embodiments, binding of the affinity-modified IgSF domain to the inhibitor receptor, such as when secreted from a cell, decreases immunological activity of the T-cell. In some embodiments of the immunomodulatory protein, the inhibitory receptor comprises an ITIM signaling domain. In some embodiments, the inhibitory receptor is PD-1, CTLA-4, LAG3, TIGIT, TIM-3, BTLA VSIG3 or VSIG8 and the at least one affinity-modified IgSF domain is an affinity-modified IgSF domain of a ligand of PD-1, CTLA-4, LAG3, TIGIT, TIM-3, BTLA, VSIG3 or VSIG8, respectively. In some embodiments, the ligand of the inhibitory receptor is PD-L1, PD-L2, B7-1, B7-2, MHC class II, PVR, CEACAM-1, GAL9 or VISTA and the at least one affinity-modified IgSF domain is an affinity-modified IgSF domain of a ligand of PD-L1, PD-L2, B7-1, B7-2, MHC class II, PVR, CEACAM-1, GAL9 or VISTA, respectively. In some embodiments, the inhibitory receptor is PD-1 and the at least one affinity-modified IgSF domain is an affinity-modified IgSF of PD-L1 or an affinity-modified IgSF of PD-L2. In some embodiments, the inhibitory receptor is TIGIT and the at least one affinity-modified IgSF domain is an affinity-modified IgSF of CD112 or CD155.

In some embodiments of the immunomodulatory protein, the at least one affinity-modified IgSF domain specifically binds to no more than one cell surface cognate binding partner. In some embodiments, the immunomodulatory protein specifically binds to no more than one cell surface cognate binding partner.

In some embodiments of the immunomodulatory protein, the at least one affinity-modified domain specifically binds to at least two cell surface cognate binding partners. In some embodiments, the first cell surface cognate binding partner is a stimulatory receptor expressed on a T cell; and the second cell surface cognate binding partner is an inhibitory ligand of an inhibitory receptor, wherein the inhibitory receptor is expressed on a T-cell. In some embodiments, binding of the affinity-modified domain to the inhibitory ligand competitively inhibits binding of the inhibitory ligand to the inhibitory receptor. In some embodiments, the inhibitory receptor is PD-1, CTLA-4, LAG-3, TIGIT, CD96, CD112R, BTLA, CD160, TIM-3 VSIG3, or VSIG8; or the ligand of the inhibitory receptor is PD-L1, PD-L2, B7-1, B7-2, HVEM, MHC class II, PVR, CEACAM-1, GAL9 or VISTA. In some embodiments, the affinity modified IgSF domain is an affinity modified CD80 domain and the stimulatory receptor is CD28. In some embodiments, the inhibitory ligand is PD-L1 and the inhibitory receptor is PD-1. In some embodiments, the affinity-modified IgSF domain exhibits decreased binding affinity to CTLA-4 compared to the wild-type IgSF domain. In some embodiments, the affinity-modified IgSF domain does not substantially specifically bind to CTLA-4.

In some embodiments of the immunomodulatory protein, the affinity modified IgSF domain is an affinity modified CD155 IgSF domain or an affinity modified CD112 IgSF domain and the at least one cell surface cognate binding partner is CD226, TIGIT or CD112R. In some embodiments, the affinity-modified IgSF domain exhibits decreased binding affinity to CD226 compared to the binding affinity of the wild-type IgSF domain to CD226. In some embodiments, the affinity-modified IgSF domain retains or exhibits increased binding to TIGIT or CD112R compared to the binding affinity of the wild-type IgSF domain to TIGIT or CD112R.

In some embodiments of the immunomodulatory protein, the at least one affinity-modified IgSF domain specifically binds to a cell surface cognate binding partner that is a tumor specific antigen. In some embodiments, the tumor specific antigen is B7-H6. In some embodiments, the affinity-modified IgSF domain is an affinity modified NKp30 IgSF domain.

In some embodiments of the immunomodulatory protein, the at least one affinity-modified IgSF domain comprises a first affinity-modified IgSF domain and a second affinity-modified IgSF domain. In some embodiments, the first affinity-modified IgSF domain and the second affinity-modified IgSF domain are different. In some embodiments, the first affinity-modified IgSF domain and the second affinity-modified IgSF domain each comprise one or more different amino acid substitutions in the same wild-type IgSF domain. In some embodiments, the first affinity-modified IgSF domain and the second affinity-modified IgSF domain each comprise one or more amino acid substitutions in a different wild-type IgSF domain.

In some embodiments of the immunomodulatory protein, the wild-type IgSF domain is from an IgSF member that is a ligand of an inhibitory receptor in which the inhibitory receptor comprises an ITIM signaling domain. In some embodiments, the inhibitory receptor is PD-1, CTLA-4, LAG3, TIGIT, TIM-3, BTLA, VSIG3, or VSIG8 and the at least one affinity-modified IgSF domain is an affinity-modified IgSF domain of a ligand of PD-1, CTLA-4, LAG3, TIGIT, TIM-3, BTLA, VSIG3, or VSIG8, respectively. In some embodiments, the ligand of the inhibitory receptor is PD-L1, PD-L2, B7-1, B7-2, MHC class II, PVR, CEACAM-1, GAL9 or VISTA and the at least one affinity-modified IgSF domain is an affinity-modified IgSF domain of a ligand of PD-L1, PD-L2, B7-1, B7-2, MHC class II, PVR, CEACAM-1, GAL9 or VISTA, respectively. In some embodiments, the inhibitory receptor is PD-1 and the at least one affinity-modified IgSF domain is an affinity-modified IgSF of PD-L1 or PD-L2. In some embodiments, the inhibitory receptor is TIGIT and the at least one affinity-modified IgSF domain is an affinity-modified IgSF of CD112 or CD155.

In some embodiments of the immunomodulatory proteins, the affinity modified IgSF domain differs by no more than ten amino acid substitutions from the wildtype IgSF domain. In some embodiments, the affinity modified IgSF domain differs by no more than five amino acid substitutions from the wildtype IgSF domain.

In some embodiments of the immunomodulatory protein, the one or more affinity-modified IgSF domain is or comprises an affinity modified IgV domain, affinity modified IgC1 domain, or an affinity modified IgC2 domain, or is a specific binding fragment thereof comprising the one or more amino acid substitutions.

In some embodiments of the immunomodulatory protein, the immunomodulatory protein further comprises one or more non-affinity modified IgSF domains.

In some embodiments of the immunomodulatory protein, the immunomodulatory protein has been or is capable of being secreted from an engineered cell. In some embodiments, the engineered cell is an immune cell. In some embodiments, the engineered cell is a primary cell.

In another aspect, there is provided a recombinant nucleic acid encoding an immunomodulatory protein. In some embodiments, the nucleic acid molecule further comprises at least one promoter operably linked to control expression of the immunomodulatory protein. In some embodiments, the promoter is a constitutively active promoter. In some embodiments, the promoter is an inducible promoter. In some embodiments, the promoter is responsive to an element responsive to T-cell activation signaling. In some embodiments, the promoter comprises a binding site for NFAT or a binding site for NF-κB. In another aspect, there is provided a recombinant expression vector comprising the nucleic acid described.

In a further aspect, there is provided a recombinant expression vector comprising a nucleic acid encoding an immunomodulatory protein under the operable control of a signal sequence for secretion, wherein: the immunomodulatory protein comprises at least one non-immunoglobulin affinity-modified immunoglobulin superfamily (IgSF) domain comprising one or more amino acid substitutions in a wild-type IgSF domain, wherein the at least one affinity-modified IgSF domain specifically binds at least one cell surface cognate binding partner of the wild-type IgSF domain; and the encoded immunomodulatory protein is secreted when expressed from a cell. In some embodiments, the immunomodulatory protein does not comprise a transmembrane domain. In some embodiments, the immunomodulatory protein is not conjugated to a half-life extending moiety. In some embodiments, the half-life extending moiety is a multimerization domain. In some embodiments, the half-life extending moiety is an Fc domain. In some embodiments, the signal sequence for secretion encodes a secretory signal peptide. In some embodiments, the signal peptide is a native signal peptide from the corresponding wild-type IgSF member. In some embodiments, the signal peptide is a non-native signal peptide. In some embodiments, the signal peptide is an IgG-kappa signal peptide, an IL-2 signal peptide, or a CD33 signal peptide or other signal peptides known or described. Exemplary signal peptides are set forth in any of SEQ ID NOS: 413-430.

In some embodiments of the expression vector, the nucleic acid molecule further comprises at least one promoter operably linked to control expression of the immunomodulatory protein. In some embodiments, the promoter is a constitutively active promoter. In some embodiments, the promoter is an inducible promoter. In some embodiments, the promoter is responsive to an element responsive to T-cell activation signaling. In some embodiments, the promoter comprises a binding site for NFAT or a binding site for NF-κB.

In some embodiments of the expression vector, the vector is a viral vector. In some embodiments, the viral vector is a retroviral vector. In some embodiments, the viral vector is a lentiviral vector or a gammaretroviral vector.

In some embodiments of the expression vector, the at least one affinity-modified IgSF domain has increased binding affinity to the at least one cell surface cognate binding partner compared with the wild-type IgSF domain.

In some embodiments of the expression vector, the at least one cell surface cognate binding partner is expressed on a mammalian cell. In some embodiments, the mammalian cell is an antigen presenting cell (APC), a tumor cell, or a lymphocyte. In some embodiments, the mammalian cell is a T-cell. In some embodiments, the mammalian cell is a mouse, rat, cynomolgus monkey, or human cell.

In some embodiments of the expression vector, specific binding of the immunomodulatory protein comprising the at least one affinity-modified IgSF domain modulates immunological activity of the mammalian cell compared to the wild-type IgSF domain. In some embodiments, specific binding of the immunomodulatory protein comprising the at least one affinity-modified IgSF domain increases immunological activity of the mammalian cell compared to the wild-type IgSF domain. In some embodiments, specific binding of the immunomodulatory protein attenuates immunological activity of the mammalian cell compared to the wild-type IgSF domain.

In some embodiments of the expression vector, the wild-type IgSF domain is from an IgSF family member of a family selected from the group consisting of Signal-Regulatory Protein (SIRP) Family, Triggering Receptor Expressed On Myeloid Cells Like (TREML) Family, Carcinoembryonic Antigen-related Cell Adhesion Molecule (CEACAM) Family, Sialic Acid Binding Ig-Like Lectin (SIGLEC) Family, Butyrophilin Family, B7 family, CD28 family, V-set and Immunoglobulin Domain Containing (VSIG) family, V-set transmembrane Domain (VSTM) family, Major Histocompatibility Complex (MHC) family, Signaling lymphocytic activation molecule (SLAM) family, Leukocyte immunoglobulin-like receptor (LIR), Nectin (Nec) family, Nectin-like (NECL) family, Poliovirus receptor related (PVR) family, Natural cytotoxicity triggering receptor (NCR) family, T cell immunoglobulin and mucin (TIM) family, and Killer-cell immunoglobulin-like receptors (KIR) family. In some embodiments, the wild-type IgSF domain is from an IgSF member selected from the group consisting of CD80, CD86, PD-L1, PD-L2, ICOS Ligand, B7-H3, B7-H4, CD28, CTLA4, PD-1, ICOS, BTLA, CD4, CD8-alpha, CD8-beta, LAG3, TIM-3, CEACAM1, TIGIT, PVR, PVRL2, CD226, CD2, CD160, CD200, CD200R, NKp30, VISTA, VSIG3, and VSIG8. In some embodiments, the wild-type IgSF domain is a human IgSF domain. In some embodiments, the at least one affinity modified IgSF domain has at least 90% sequence identity to a wild-type IgSF domain or a specific binding fragment thereof contained in the sequence of amino acids set forth in any of SEQ ID NOS: 1-27 and 408. In some embodiments, the immunomodulatory protein has at least 90% sequence identity to the amino acid sequence selected from any of SEQ ID NOS: 28-54 and 410. In some embodiments, the wild-type IgSF domain is a member of the B7 family. In some embodiments, the wild-type IgSF domain is a domain of CD80, CD86 or ICOSL.

In some embodiments of the expression vector, the at least one cell surface cognate binding partner is a stimulatory receptor expressed on a T-cell and the at least one affinity-modified IgSF domain has increased binding affinity to the stimulatory receptor compared to the binding affinity of the wild-type IgSF domain to the stimulatory receptor. In some embodiments, binding of the affinity-modified IgSF domain to the stimulatory receptor, such as when expressed from a cell containing the expression vector, increases immunological activity of the T-cell. In some embodiments, binding of the affinity-modified IgSF domain to the stimulatory receptor, such as when expressed from a cell containing the expression vector, decreases immunological activity of the T-cell. In some embodiments, the stimulatory receptor is CD28, ICOS, or CD226. In some embodiments, the at least one affinity-modified IgSF domain is an affinity-modified ICOSL IgSF domain that has increased binding affinity to at least one of: ICOS and CD28. In some embodiments, the at least one affinity-modified IgSF domain is an affinity modified ICOSL IgSF domain and the stimulatory receptor is ICOS. In some embodiments, the at least one affinity-modified IgSF domain is an affinity modified ICOSL IgSF domain and the stimulatory receptor is CD28. In some embodiments, the at least one affinity-modified IgSF domain is an affinity modified CD80 IgSF domain and the stimulatory receptor is CD28. In some embodiments, the affinity-modified IgSF domain does not substantially specifically bind to CTLA-4 or exhibits decreased binding affinity to CTLA-4 compared to the binding affinity of wild-type IgSF domain to CTLA-4.

In some embodiments of the expression vector, the at least one affinity-modified IgSF domain specifically binds to no more than one cell surface cognate binding partner. In some embodiments, the immunomodulatory protein specifically binds to no more than one cell surface cognate binding partner.

In some embodiments of the expression vector, the at least one affinity-modified domain specifically binds to at least two cell surface cognate binding partners. In some embodiments, the first cell surface cognate binding partner is a stimulatory receptor expressed on a T cell; and the second cell surface cognate binding partner is an inhibitory ligand of an inhibitory receptor, wherein the inhibitory receptor is expressed on a T-cell. In some embodiments, binding of the affinity-modified IgSF domain to the inhibitory ligand competitively inhibits binding of the inhibitory ligand to the inhibitory receptor. In some embodiments, the inhibitory receptor is PD-1, CTLA-4, LAG-3, TIGIT, CD96, CD112R, BTLA, CD160, TIM-3, VSIG3, or VSIG8; or the ligand of the inhibitory receptor is PD-L1, PD-L2, B7-1, B7-2, HVEM, MHC class II, PVR, CEACAM-1, GAL9 or VISTA.

In some embodiments of the expression vector, the affinity modified IgSF domain is an affinity modified CD80 domain and the stimulatory receptor is CD28. In some embodiments, the affinity-modified IgSF domain exhibits decreased binding affinity to CTLA-4 compared to the wild-type IgSF domain. In some embodiments, the affinity-modified IgSF domain does not substantially specifically bind to CTLA-4. In some embodiments, the inhibitory ligand is PD-L1 and the inhibitory receptor is PD-1. In some embodiments, the inhibitory ligand is PD-L2 and the inhibitory receptor is PD-1.

In some embodiments of the expression vector, the affinity-modified IgSF domain is an affinity modified CD155 IgSF domain or an affinity modified CD112 IgSF domain and the at least one cell surface cognate binding partner is CD226, TIGIT or CD112R. In some embodiments, the affinity-modified IgSF domain exhibits decreased binding affinity to CD226 compared to the binding affinity of the wild-type IgSF domain to CD226. In some embodiments, the affinity-modified IgSF domain retains or exhibits increased binding to TIGIT (T-cell immunoreceptor with Ig and ITIM domains) or CD112R compared to the binding affinity of the wild-type IgSF domain for TIGIT or CD112R.

In some embodiments of the expression vector, the at least one affinity-modified IgSF domain specifically binds to a cell surface cognate binding partner that is a tumor specific antigen. In some embodiments, the tumor specific antigen is B7-H6. In some embodiments, the affinity-modified IgSF domain is an affinity modified NKp30 IgSF domain.

In some embodiments of the expression vector, the at least one affinity-modified IgSF domain comprises a first affinity-modified IgSF domain and a second affinity-modified IgSF domain. In some embodiments, the first affinity-modified IgSF domain and the second affinity-modified IgSF domain are different. In some embodiments, the first affinity-modified IgSF domain and the second affinity-modified IgSF domain each comprise one or more different amino acid substitutions in the same wild-type IgSF domain. In some embodiments, the first affinity-modified IgSF domain and the second affinity-modified IgSF domain each comprise one or more amino acid substitutions in a different wild-type IgSF domain.

In some embodiments of the expression vector, the wild-type IgSF domain is from an IgSF member that is a ligand of an inhibitory receptor in which the inhibitory receptor comprises an ITIM signaling domain. In some embodiments, the inhibitory receptor is PD-1, CTLA-4, LAG3, TIGIT, TIM-3, BTLA, VSIG3, or VSIG8 and the at least one affinity-modified IgSF domain is an affinity-modified IgSF domain of a ligand of PD-1, CTLA-4, LAG3, TIGIT, TIM-3, BTLA, VSIG3, or VSIG8, respectively. In some embodiments, the ligand of the inhibitory receptor is PD-L1, PD-L2, B7-1, B7-2, MHC class II, PVR, CEACAM-1, GAL9 or VISTA and the at least one affinity-modified IgSF domain is an affinity-modified IgSF domain of a ligand of PD-L1, PD-L2, B7-1, B7-2, MHC class II, PVR, CEACAM-1, GAL9 or VISTA, respectively. In some embodiments, the inhibitory receptor is PD-1 and the at least one affinity-modified IgSF domain is an affinity-modified IgSF of PD-L1 or PD-L2. In some embodiments, the affinity-modified IgSF domain has increased binding affinity for a trans surface cognate binding partner compared to the wildtype IgSF domain, whereby the increased binding affinity competitively inhibits binding of the trans surface cognate binding partner to the inhibitory receptor.

In some embodiments of the expression vector, the affinity modified IgSF domain differs by no more than ten amino acid substitutions from the wildtype IgSF domain. In some embodiments, the affinity modified IgSF domain differs by no more than five amino acid substitutions from the wildtype IgSF domain.

In some embodiments of the expression vector, the one or more affinity-modified IgSF domain is or comprises an affinity modified IgV domain, affinity modified IgC1 domain, or an affinity modified IgC2 domain, or is a specific binding fragment thereof, comprising the one or more amino acid substitutions.

In some embodiments of the expression vector, the immunomodulatory protein further comprises one or more non-affinity modified IgSF domains.

In another aspect, there is provided an engineered cell comprising any one or more of the above nucleic acid or the above expression vector. In another aspect, there is provided an engineered cell comprising any one or more of the immunomodulatory protein. In another aspect, there is provided an engineered cell that secretes any one or more of the immunomodulatory protein. In some embodiments, the engineered cell is an immune cell.

In another aspect, there is provided an engineered immune cell comprising a nucleic acid molecule that encodes an immunomodulatory protein, wherein the immunomodulatory protein comprises at least one non-immunoglobulin affinity-modified immunoglobulin superfamily (IgSF) domain comprising one or more amino acid substitutions in a wild-type IgSF domain, wherein the at least one affinity-modified IgSF domain specifically binds at least one cell surface cognate binding partner of the wild-type IgSF domain; and the engineered cell expresses and secretes the immunomodulatory protein. In some embodiments, the immunomodulatory protein does not comprise a transmembrane domain. In some embodiments, the immunomodulatory protein is not conjugated to a half-life extending moiety. In some embodiments, the half-life extending moiety is a multimerization domain. In some embodiments, the half-life extending moiety is an Fc domain.

In some embodiments of the engineered cell, the nucleic acid molecule comprises a sequence encoding a secretory signal peptide operably linked to the sequence encoding the immunomodulatory protein. In some embodiments, the signal peptide is the native signal peptide from the corresponding wild-type IgSF member. In some embodiments, the signal peptide is a non-native signal sequence. In some embodiments, the signal peptide is an IgG-kappa signal peptide, an IL-2 signal peptide, or a CD33 signal peptide or any other signal peptide known in the art. Exemplary signal peptides are set forth in any of SEQ ID NOS: 413-430.

In some embodiments of the engineered cell, the nucleic acid molecule further comprises at least one promoter operably linked to control expression of the immunomodulatory protein. In some embodiments, the promoter is a constitutively active promoter. In some embodiments, the promoter is an inducible promoter. In some embodiments, the promoter is responsive to an element responsive to T-cell activation signaling. In some embodiments, the promoter comprises a binding site for NFAT or a binding site for NF-κB. In some embodiments, the immunomodulatory protein is expressed and secreted by the engineered cell after the engineered cell is contacted with an inducing agent or after induction of T cell activation signaling, which optionally is induced upon binding of an antigen to a chimeric antigen receptor (CAR) or engineered T-cell receptor (TCR) expressed by the engineered cell. In some embodiments, the cell is a lymphocyte. In some embodiments, the lymphocyte is a T cell, a B cell or an NK cell. In some embodiments, the cell is a T cell. In some embodiments, the T cell is CD4+ or CD8+. In some embodiments, the cell is an antigen presenting cell. In some embodiments, the cell is a primary cell obtained from a subject. In some embodiments, the subject is a human subject.

In some embodiments of the engineered cell, the at least one affinity modified IgSF domain has increased binding affinity to the at least one cell surface cognate binding partner compared with the wild-type IgSF domain. In some embodiments, the at least one cell surface cognate binding partner is expressed on a mammalian cell. In some embodiments, the mammalian cell is an antigen presenting cell (APC), a tumor cell, or a lymphocyte. In some embodiments, the mammalian cell is a T-cell. In some embodiments, the mammalian cell is a mouse, rat, cynomolgus monkey, or human cell.

In some embodiments of the engineered cell, specific binding of the immunomodulatory protein comprising the at least one affinity-modified IgSF domain modulates immunological activity of the mammalian cell compared to the wild-type IgSF domain. In some embodiments, specific binding of the immunomodulatory protein comprising the at least one affinity-modified IgSF domain increases immunological activity of the mammalian cell compared to the wild-type IgSF domain. In some embodiments, specific binding of the immunomodulatory protein attenuates immunological activity of the mammalian cell compared to the wild-type IgSF domain.

In some embodiments of the engineered cell, the wild-type IgSF domain is from an IgSF family member of a family selected from the group consisting of Signal-Regulatory Protein (SIRP) Family, Triggering Receptor Expressed On Myeloid Cells Like (TREML) Family, Carcinoembryonic Antigen-related Cell Adhesion Molecule (CEACAM) Family, Sialic Acid Binding Ig-Like Lectin (SIGLEC) Family, Butyrophilin Family, B7 family, CD28 family, V-set and Immunoglobulin Domain Containing (VSIG) family, V-set transmembrane Domain (VSTM) family, Major Histocompatibility Complex (MHC) family, Signaling lymphocytic activation molecule (SLAM) family, Leukocyte immunoglobulin-like receptor (LIR), Nectin (Nec) family, Nectin-like (NECL) family, Poliovirus receptor related (PVR) family, Natural cytotoxicity triggering receptor (NCR) family, T cell immunoglobulin and mucin (TIM) family, and Killer-cell immunoglobulin-like receptors (KIR) family. In some embodiments, the wild-type IgSF domain is from an IgSF member selected from the group consisting of CD80, CD86, PD-L1, PD-L2, ICOS Ligand, B7-H3, B7-H4, CD28, CTLA4, PD-1, ICOS, BTLA, CD4, CD8-alpha, CD8-beta, LAG3, TIM-3, CEACAM1, TIGIT, PVR, PVRL2, CD226, CD2, CD160, CD200, CD200R, NKp30, VISTA, VSIG3, and VSIG8. In some embodiments, the wild-type IgSF domain is a human IgSF domain. In some embodiments, the at least one affinity modified IgSF domain has at least 90% sequence identity to a wild-type IgSF domain or a specific binding fragment thereof contained in the sequence of amino acids set forth in any of SEQ ID NOS: 1-27 and 408. In some embodiments, the immunomodulatory protein has at least 90% sequence identity to the amino acid sequence selected from any of SEQ ID NOS: 28-54 and 410.

In some embodiments of the engineered cell, the at least one cell surface cognate binding partner is a stimulatory receptor expressed on a T-cell and the at least one affinity-modified IgSF domain has increased binding affinity to the stimulatory receptor compared to the binding affinity of the wild-type IgSF domain to the stimulatory receptor. In some embodiments, binding of the affinity-modified IgSF domain to the stimulatory receptor, such as when secreted from the cell, increases immunological activity of the T-cell. In some embodiments, binding of the affinity-modified IgSF domain to the stimulatory receptor, such as when secreted from the cell, decreases immunological activity of the T-cell. In some embodiments, the stimulatory receptor is CD28, ICOS, or CD226. In some embodiments, the at least one affinity-modified IgSF domain is an affinity-modified ICOSL IgSF domain that has increased binding affinity to at least one of: ICOS and CD28. In some embodiments, the at least one affinity-modified IgSF domain is an affinity modified ICOSL IgSF domain and the stimulatory receptor is ICOS. In some embodiments, the at least one affinity-modified IgSF domain is an affinity modified ICOSL IgSF domain and the stimulatory receptor is CD28. In some embodiments, the at least one affinity-modified IgSF domain is an affinity modified CD80 IgSF domain and the stimulatory receptor is CD28. In some embodiments, the affinity-modified IgSF domain does not substantially specifically bind to CTLA-4 or exhibits decreased binding affinity to CTLA-4 compared to the binding affinity of wild-type IgSF domain to CTLA-4.

In some embodiments of the engineered cell, the at least one affinity-modified IgSF domain specifically binds to no more than one cell surface cognate binding partner. In some embodiments, the immunomodulatory protein specifically binds to no more than one cell surface cognate binding partner.

In some embodiments of the engineered cell, the at least one affinity-modified domain specifically binds to at least two cell surface cognate binding partners. In some embodiments, the first cell surface cognate binding partner is a stimulatory receptor expressed on a T cell; and the second cell surface cognate binding partner is an inhibitory ligand of an inhibitory receptor, wherein the inhibitory receptor is expressed on a T-cell. In some embodiments, binding of the affinity-modified domain to the inhibitory ligand competitively inhibits binding of the inhibitory ligand to the inhibitory receptor. In some embodiments, the inhibitory receptor is PD-1, CTLA-4, LAG-3, TIGIT, CD96, CD112R, BTLA, CD160, TIM-3, VSIG3, or VSIG8; or the ligand of the inhibitory receptor is PD-L1, PD-L2, B7-1, B7-2, HVEM, MHC class II, PVR, CEACAM-1, GAL9 or VISTA. In some embodiments, the affinity modified IgSF domain is an affinity modified CD80 domain and the stimulatory receptor is CD28. In some embodiments, the inhibitory ligand is PD-L1 and the inhibitory receptor is PD-1. In some embodiments, the inhibitory ligand is PD-L2 and the inhibitory receptor is PD-1. In some embodiments, the affinity-modified IgSF domain exhibits decreased binding affinity to CTLA-4 compared to the wild-type IgSF domain. In some embodiments, the affinity-modified IgSF domain does not substantially specifically bind to CTLA-4.

In some embodiments of the engineered cell, the affinity modified IgSF domain is an affinity modified CD155 IgSF domain or an affinity modified CD112 IgSF domain and the at least one cell surface cognate binding partner is CD226, TIGIT or CD112R. In some embodiments, the affinity-modified IgSF domain exhibits decreased binding affinity to CD226 compared to the binding affinity of the wild-type IgSF domain to CD226. In some embodiments, the affinity-modified IgSF domain retains or exhibits increased binding to TIGIT (T-cell immunoreceptor with Ig and ITIM domains) or CD112R compared to the binding affinity of the wild-type IgSF domain for TIGIT or CD112R.

In some embodiments of the engineered cell, the at least one affinity-modified IgSF domain specifically binds to a cell surface cognate binding partner that is a tumor specific antigen. In some embodiments, the tumor specific antigen is B7-H6. In some embodiments, the affinity-modified IgSF domain is an affinity modified NKp30 IgSF domain.

In some embodiments of the engineered cell, the at least one affinity-modified IgSF domain comprises a first affinity-modified IgSF domain and a second affinity-modified IgSF domain. In some embodiments, the first affinity-modified IgSF domain and the second affinity-modified IgSF domain are different. In some embodiments, the first affinity-modified IgSF domain and the second affinity-modified IgSF domain each comprises one or more different amino acid substitutions in the same wild-type IgSF domain. In some embodiments, the first affinity-modified IgSF domain and the second affinity-modified IgSF domain each comprise one or more amino acid substitutions in a different wild-type IgSF domain.

In some embodiments of the engineered cell, the wild-type IgSF domain is from an IgSF member that is a ligand of an inhibitory receptor in which the inhibitory receptor comprises an ITIM signaling domain. In some embodiments, the inhibitory receptor is PD-1, CTLA-4, LAG3, TIGIT, TIM-3, BTLA, VSIG3, or VSIG8 and the at least one affinity-modified IgSF domain is an affinity-modified IgSF domain of a ligand of PD-1, CTLA-4, LAG3, TIGIT, TIM-3, BTLA, VSIG3, or VSIG8, respectively. In some embodiments, the ligand of the inhibitory receptor is PD-L1, PD-L2, B7-1, B7-2, MHC class II, PVR, CEACAM-1, GAL9 or VISTA and the at least one affinity-modified IgSF domain is an affinity-modified IgSF domain of a ligand of PD-L1, PD-L2, B7-1, B7-2, MHC class II, PVR, CEACAM-1, GAL9 or VISTA, respectively. In some embodiments, the inhibitory receptor is PD-1 and the at least one affinity-modified IgSF domain is an affinity-modified IgSF of PD-L1 or PD-L2. In some embodiments, the inhibitory receptor is TIGIT and the affinity-modified IgSF domain is an affinity-modified IgSF domain of CD155 or CD112.

In some embodiments of the engineered cell, the affinity modified IgSF domain differs by no more than ten amino acid substitutions from the wildtype IgSF domain. In some embodiments, the affinity modified IgSF domain differs by no more than five amino acid substitutions from the wildtype IgSF domain.

In some embodiments of the engineered cell, the one or more affinity-modified IgSF domain is or comprises an affinity modified IgV domain, affinity modified IgC1 domain, or an affinity modified IgC2 domain, or is a specific binding fragment thereof comprising the one or more amino acid substitutions.

In some embodiments of the engineered cell, the immunomodulatory protein further comprises one or more non-affinity modified IgSF domains.

In some embodiments of the engineered cell further comprises a chimeric antigen receptor (CAR) or an engineered T-cell receptor (TCR).

In another aspect, there is provided a pharmaceutical composition comprising the above engineered cell and a pharmaceutically acceptable carrier. In some embodiments, the pharmaceutical composition is sterile.

In another aspect, there is provided a method of introducing an immunomodulatory protein into a subject, comprising administering any of the provided engineered cells or the pharmaceutical composition containing the engineered cells to the subject.

In another aspect, there is provided a method of modulating an immune response in a subject, comprising administering any of the provided engineered cells or a pharmaceutical composition containing any of the provided engineered cells to the subject. In some embodiments, modulating the immune response treats a disease or disorder in the subject. In some embodiments, the modulated immune response is increased. In some embodiments, the disease or disorder is a tumor. In some embodiments, the disease or disorder is a cancer. In some embodiments, the disease or disorder is melanoma, lung cancer, bladder cancer, or a hematological malignancy. In some embodiments, the modulated immune response is decreased. In some embodiments, the disease or disorder is an inflammatory disease or condition. In some embodiments, the disease or condition is Crohn's disease, ulcerative colitis, multiple sclerosis, asthma, rheumatoid arthritis, or psoriasis.

In some embodiments of the methods, the subject is human. In some embodiments, the cell is autologous to the subject. In some embodiments, the cell is allogenic to the subject. In some embodiments, the engineered cell expresses and secretes the immunomodulatory protein. In some embodiments, the immunomodulatory protein is constitutively expressed by the engineered cell. In some embodiments, the immunomodulatory protein is expressed and secreted by the engineered cell after the engineered cell is contacted with an inducing agent. In some embodiments, the immunomodulatory protein is expressed and secreted by the engineered cell upon T cell activation signaling. In some embodiments, the engineered cell expresses a chimeric antigen receptor (CAR) or an engineered T-cell receptor (TCR) and T cell activation signaling is induced upon binding of an antigen by the CAR or TCR.

Also provided are any of the infectious agents as described containing a nucleic acid molecule encoding a secretable immunomodulatory protein or a transmembrane immunomodulatory protein.

Provided are infectious agents containing a nucleic acid molecule encoding a transmembrane immunomodulatory protein (TIP) containing an ectodomain comprising at least one non-immunoglobulin affinity-modified immunoglobulin superfamily (IgSF) domain comprising one or more amino acid substitution(s) in a wild-type IgSF domain, wherein the at least one affinity-modified IgSF domain specifically binds at least one cell surface cognate binding partner of the wild-type IgSF domain; and a transmembrane domain.

In some embodiments, the at least one affinity modified IgSF domain has increased binding affinity to the at least one cell surface cognate binding partner compared with the reference wild-type IgSF domain.

In some of any such embodiments, the wild-type IgSF domain is from an IgSF family member of a family selected from Signal-Regulatory Protein (SIRP) Family, Triggering Receptor Expressed On Myeloid Cells Like (TREML) Family, Carcinoembryonic Antigen-related Cell Adhesion Molecule (CEACAM) Family, Sialic Acid Binding Ig-Like Lectin (SIGLEC) Family, Butyrophilin Family, B7 family, CD28 family, V-set and Immunoglobulin Domain Containing (VSIG) family, V-set transmembrane Domain (VSTM) family, Major Histocompatibility Complex (MHC) family, Signaling lymphocytic activation molecule (SLAM) family, Leukocyte immunoglobulin-like receptor (LIR), Nectin (Nec) family, Nectin-like (NECL) family, Poliovirus receptor related (PVR) family, Natural cytotoxicity triggering receptor (NCR) family, T cell immunoglobulin and mucin (TIM) family or Killer-cell immunoglobulin-like receptors (KIR) family. In some embodiments, the wild-type IgSF domain is from an IgSF member selected from CD80, CD86, PD-L1, PD-L2, ICOS Ligand, B7-H3, B7-H4, CD28, CTLA4, PD-1, ICOS, BTLA, CD4, CD8-alpha, CD8-beta, LAG3, TIM-3, CEACAM1, TIGIT, PVR, PVRL2, CD226, CD2, CD160, CD200, CD200R, NKp30, VISTA, VSIG3, and VSIG8.

In some of any such embodiments, the wild-type IgSF domain is a human IgSF member. In some embodiments, the transmembrane immunomodulatory protein has at least 90% sequence identity to the amino acid sequence selected from any of SEQ ID NOS: 381-407 and 409 or to a contiguous portion thereof containing the affinity-modified IgSF domain and a transmembrane domain.

In some embodiments, the transmembrane immunomodulatory protein is a chimeric receptor, wherein the endodomain is not the endodomain from the wild-type IgSF member comprising the wild-type IgSF domain. In some cases, the endodomain contains at least one ITAM (immunoreceptor tyrosine-based activation motif)-containing signaling domain. In some instances, the endodomain contains a CD3-zeta signaling domain. In some aspects, the endodomain further contains at least one of: a CD28 costimulatory domain, an ICOS signaling domain, an OX40 signaling domain, and a 41BB signaling domain.

In some of any such embodiments, the affinity modified IgSF domain differs by no more than ten amino acid substitutions or no more than five amino acid substitutions from the wildtype IgSF domain. In some embodiments, the affinity-modified IgSF domain is or contains an affinity modified IgV domain, affinity modified IgC1 domain or an affinity modified IgC2 domain or is a specific binding fragment thereof comprising the one or more amino acid substitutions.

In some of any such embodiments, the transmembrane domain is the native transmembrane domain from the corresponding wild-type IgSF member. In some cases, the transmembrane domain is not the native transmembrane domain from the corresponding wild-type IgSF member. In some examples, the transmembrane protein is a transmembrane protein derived from CD8.

In some of any such embodiments, the infectious agent is a bacteria or a virus. In some cases, the virus is an oncolytic virus. In some aspects, the oncolytic virus is an adenovirus, adeno-associated virus, herpes virus, Herpes Simplex Virus, Vesticular Stomatic virus, Reovirus, Newcastle Disease virus, parvovirus, measles virus, vesticular stomatitis virus (VSV), Coxsackie virus or a Vaccinia virus. In some cases, the virus specifically targets dendritic cells (DCs) and/or is dendritic cell-tropic. In some embodiments, the virus is a lentiviral vector that is pseudotyped with a modified Sindbis virus envelope product.

In some of any such embodiments, the infectious agent further contains a nucleic acid molecule encoding a further gene product that results in death of a target cell or that can augment or boost an immune response. In some cases, the further gene product is selected from an anticancer agent, anti-metastatic agent, an antiangiogenic agent, an immunomodulatory molecule, an immune checkpoint inhibitor, an antibody, a cytokine, a growth factor, an antigen, a cytotoxic gene product, a pro-apoptotic gene product, an anti-apoptotic gene product, a cell matrix degradative gene, genes for tissue regeneration or a reprogramming human somatic cells to pluripotency.

### Brief Description of the Drawings

**FIG. 1A** depicts results of a competition binding assay for binding of biotinylated recombinant CD28-Fc fusion protein (rCD28.Fc) to immobilized CD80 variant A91G ECD-Fc fusion molecule in the presence of unlabeled recombinant human PD-L1-His, human CTLA-4-His, or human-PD-L2-Fc fusion protein.
**FIG. 1B** depicts results of a competition binding assay for binding of biotinylated recombinant human PD-L1-his monomeric protein to immobilized CD80 variant A91G ECD-Fc fusion molecule in the presence of unlabeled recombinant human rCD28.Fc, human CTLA-4.Fc or human PD-L2.Fc
**FIG. 2A** **and** **2B** depicts the detection of PD-L2 SIP in supernatant of transduced CD19 CAR T cells and in HEK-293 cells, respectively.
**FIG. 3A** depicts the proliferation studies for T cells transduced with exemplary tested variant PD-L2 SIP.
**FIG. 3B** depicts levels of IFN-gamma in the supernatant released by T cells transduced with exemplary tested variant PD-L2 SIP as measured by ELISA on day 5 after re-stimulation.
**FIG. 3C** depicts proliferation of T cells co-transduced with a CAR and an exemplary variant PD-L2 SIP or a wild-type PD-L2 SIP following stimulation with target cells.

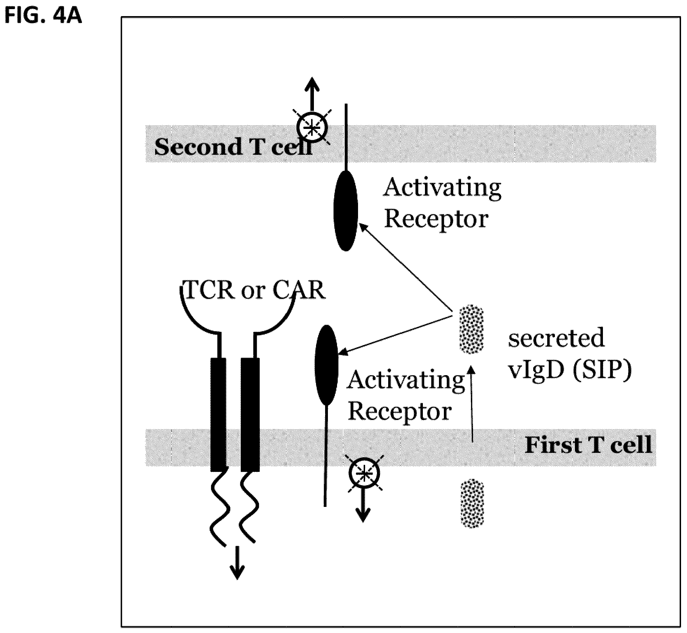

**FIG. 4A** depicts a secreted immunomodulatory protein (SIP) in which a variant IgSF domain (vIgD) is secreted from a cell, such as a first T cell (e.g. CAR T cell). In an exemplary embodiment, the cognate binding partner of the secreted vIgD is an activating receptor, which can be expressed on the first cell (e.g. T cell) and/or on a second cell (e.g. T cell; either endogenous or engineered, such as a CAR T cell). Upon binding of the SIP with its cognate binding partner, signaling via the activating receptor is blocked. In all cases, the vIgD can be a V-domain (IgV) only, the combination of the V-domain (IgV) and C-domain (IgC), including the entire extracellular domain (ECD), or any combination of Ig domains of the IgSF superfamily member.

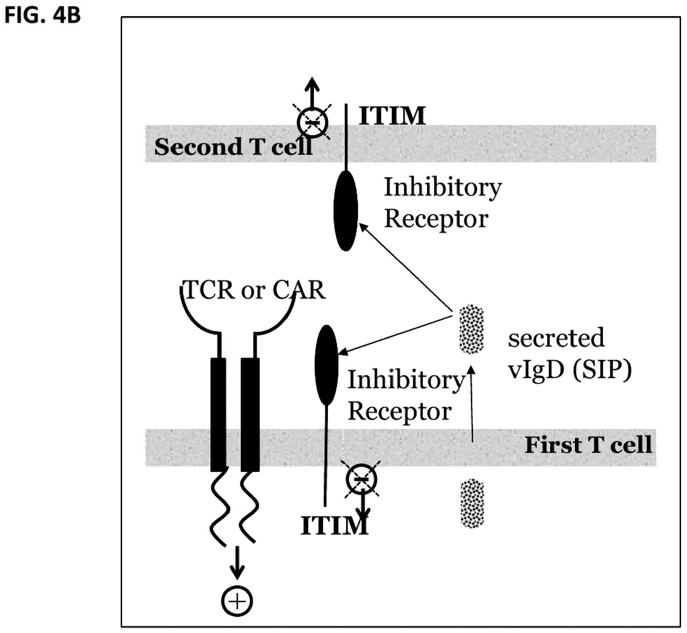

**FIG. 4B** depicts a secreted immunomodulatory protein (SIP) in which a variant IgSF domain (vIgD) is secreted from a cell, such as a first T cell (e.g. CAR T cell). In an exemplary embodiment, the cognate binding partner of the secreted vIgD is an inhibitory receptor, which can be expressed on the first cell (e.g. CAR T cell) and/or on a second cell (e.g. T cell; either endogenous or engineered, such as a CAR T cell). Upon binding of the SIP with its cognate binding partner, the SIP antagonizes or blocks the negative signaling via the inhibitory receptor, thereby resulting in an activated T cell or effector T cell is blocked. In all cases, the vIgD can be a V-domain (IgV) only, the combination of the V-domain (IgV) and C-domain (IgC), including the entire extracellular domain (ECD), or any combination of Ig domains of the IgSF superfamily member.
**FIG. 5** depicts proliferation studies for T cells transduced with exemplary tested variant PD-L1 SIP.
**FIG. 6** depicts results of a cell-based assay for detection of SIPs, including variant or wild-type PD-L1 and PD-L2 SIPS, in supernatant of transduced HEK-293 cells.

### Detailed Description

Provided herein are secretable immunomodulatory proteins that can be secreted when expressed in a cell, nucleic acids and vectors encoding the same, and cells, such as immune cells or infectious agents, engineered to express and secrete such immunomodulatory proteins. The immunomodulatory protein contains an affinity-modified IgSF domain comprising one or more amino acid substitutions in a wild-type IgSF domain, wherein at least one affinity-modified IgSF domain specifically binds at least one cell surface cognate binding partner of the wild-type IgSF domain. In some embodiments, the immunomodulatory protein does not include a transmembrane domain and/or a half-life extending moiety.

Among the provided embodiments are cells, such as immune cells (e.g. T cell), engineered to express and secrete the immunomodulatory proteins, for example by engineering the cells to include an expression vector that encodes the immunomodulatory protein. In some embodiments, the immunomodulatory protein encoded by the expression vector is under the operable control of a signal sequence for secretion, such that when the cell expresses the immunomodulatory protein, the immunomodulatory protein is secreted by the engineered cell. In some embodiments, expression of the immunomodulatory protein is under the operable control of a promotor. The promotor can be, for example, an inducible promoter. In some embodiments, the inducible promoter is responsive to an element responsive to T-cell activation signaling. In some embodiments, the engineered cell (e.g. T cell) also is engineered to express on its surface an antigen receptor, such as a T cell receptor (TCR) or chimeric antigen receptor (CAR), containing an intracellular signaling domain capable of or that does induce or mediate T-cell signaling upon antigen binding. In some aspects, the intracellular signaling domain comprises an immunoreceptor tyrosine-based activation motif (ITAM). Thus, in some embodiments, an engineered immune cell expresses and secretes an immunomodulatory protein only in response to activation of the immune cell, including, in some aspects, in response to recognition of antigen by an engineered antigen receptor.

Also provided herein are methods of introducing an immunomodulatory protein into a subject, the method comprising administering an engineered cell or infectious agent (or a pharmaceutical composition comprising an engineered cell or infectious agent) into the subject. In another aspect, there is provided a method of modulating an immune response in a subject, the method comprising administering an engineered cell (or a pharmaceutical composition comprising an engineered cell or infectious agent) into the subject. In another aspect, there is provided a method of treating a subject in need of treatment (such as a subject with a disease, such as cancer), the method comprising administering an effective amount of an engineered cell or infectious agent (or a pharmaceutical composition comprising an engineered cell or infectious agent) into the subject. The engineered cell can be a population of cells, such as a cell culture or a plurality of separately engineered cells.

In some embodiments, the provided engineered cells are administered to a subject and, in response to an induction signal (for example, immune cell activation or administration of an inducing agent) the engineered cells administered to the subject express and secrete the immunomodulatory protein. In some embodiments, the engineered cells constitutively express and secrete the immunomodulatory protein. In some embodiments, the engineered cells localize to a diseased cell or lesion, such as a cell present in a tumor microenvironment (e.g. tumor cell), which, in some aspects, is mediated by recognition of an antigen by an engineered antigen receptor expressed by the engineered cells, thereby targeting the secreted immunomodulatory proteins to the site of the disease or lesion, e.g. tumor microenvironment.

In some embodiments, the cognate binding partner of the immunomodulatory protein is a cell surface protein expressed by immune cells that engage with one or more other immune receptors (e.g. on lymphocytes) to induce inhibitory or activating signals. For example, the interaction of certain receptors on lymphocytes with their cell surface cognate binding partners to form an immunological synapse (IS) between antigen-presenting cells (APCs) or target cells and lymphocytes can provide costimulatory or inhibitory signals that can regulate the immune system. In some aspects, the immunomodulatory proteins provided herein (which can be expressed and secreted by an engineered cell) can alter the interaction of cell surface protein ligands with their receptors to modulate immune cells, such as T cell, activity. In some embodiments, the binding of the immunomodulatory protein to a ligand (cognate binding partner) modulates, e.g. induces, enhances or suppresses, immunological immune responses of a cell to which the immunomodulatory protein specifically binds. In some embodiments, the secretable immunomodulatory protein as provided, such as is secreted by the engineered cells, suppresses, such as inhibits or antagonizes, its cognate binding partner.

In some embodiments, under normal physiological conditions, the T cell-mediated immune response is initiated by antigen recognition by the T cell receptor (TCR) and is regulated by a balance of co-stimulatory and inhibitory signals (i.e., immune checkpoint proteins). The immune system relies on immune checkpoints to prevent autoimmunity (i.e., self-tolerance) and to protect tissues from excessive damage during an immune response, for example during an attack against a pathogenic infection. In some cases, however, these immunomodulatory proteins can be dysregulated in diseases and conditions, including tumors, as a mechanism for evading the immune system.

Thus, in some aspects, immunotherapy that alters immune cell activity, such as T cell activity, can treat certain diseases and conditions in which the immune response is dysregulated. Therapeutic approaches that seek to modulate interactions in the IS would benefit from the ability to bind multiple IS targets simultaneously and in a manner that is sensitive to temporal sequence and spatial orientation. Current therapeutic approaches fall short of this goal. Wild-type receptors and ligands possess low affinities for cognate binding partners, which can preclude their use as soluble therapeutics. Additionally, soluble receptors and antibodies, in some aspects, typically bind no more than a single target protein at a time and/or bind competitively to their targets (e.g., to no more than one target species at a time), and therefore lack the ability to simultaneously bind multiple targets. And while bispecific antibodies, as well as modalities comprising dual antigen binding regions, can bind to more than one target molecule simultaneously, the three-dimensional configuration typical of these modalities often precludes them intervening in key processes occurring in the IS in a manner consistent with their temporal and spatial requirements.

What is needed is an entirely new class of therapeutic molecules that have the specificity and affinity of antibodies, but also maintain the size, volume, and spatial orientation constraints required in the IS. Further, such therapeutics would have the ability to bind to their targets non-competitively as well as competitively. A molecule with these properties would therefore have novel function in the ability to integrate into multi-protein complexes at IS and generate the desired binding configuration and resulting biological activity.

To this end, emerging immuno-oncology therapeutic regimes need to safely break tumor-induced T cell tolerance. Current state-of-the-art immuno-therapeutics block PD-1 or CTLA4, central inhibitory molecules of the B7/CD28 family that are known to limit T cell effector function. While antagonistic antibodies against such single targets function to disrupt immune synapse checkpoint signaling complexes, they fall short of simultaneously activating T cells. Conversely, bispecific antibody approaches activate T cells, but fall short of simultaneously blocking inhibitory ligands that regulate the induced signal.

To address these shortcomings, provided are immunotherapies, such as cell therapies, that can modulate immune cell activities. In some embodiments, the provided immunotherapies can enhance immune cells signaling, such as T-cell activation signaling, and/or can block inhibitory regulation, which, in some cases, can occur simultaneously. In some embodiments, the provided immunotherapies relate to immunoglobulin superfamily (IgSF) components of the immune synapse that are known to have a dual role in both T-cell activation and blocking of inhibitory ligands. In some aspects, IgSF based-cell therapies engineered from immune system ligands, such as human immune system ligands themselves are more likely to retain their ability to normally assemble into key pathways of the immune synapse and maintain normal interactions and regulatory functions in ways that antibodies or next-generation bi-specific reagents cannot. This is due to the relatively large size of antibodies as well as from the fact they are not natural components of the immune synapse. These unique features of human immune system ligands, and cells engineered to express affinity-modified variants of such ligands, promise to provide a new level of immunotherapeutic efficacy and safety. In particular aspects, the provided engineered cells provide a secretable immunomodulatory platform using affinity-modified native immune ligands to generate immunotherapy biologics that bind with tunable affinities to one or more of their cognate immune receptors in the treatment of a variety of oncological and immunological indications.

All publications, including patents, patent applications scientific articles and databases, mentioned in this specification are herein incorporated by reference in their entirety for all purposes to the same extent as if each individual publication, including patent, patent application, scientific article or database, were specifically and individually indicated to be incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### I. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The terms used throughout this specification are defined as follows unless otherwise limited in specific instances. Unless defined otherwise, all technical and scientific terms, acronyms, and abbreviations used herein have the same meaning as commonly understood by one of ordinary skill in the art to which the invention pertains. Unless indicated otherwise, abbreviations and symbols for chemical and biochemical names are per IUPAC-IUB nomenclature. Unless indicated otherwise, all numerical ranges are inclusive of the values defining the range as well as all integer values in-between.

As used in the specification and the appended claims, the singular forms "a," "an," and "the" include plural referents unless the context clearly indicates otherwise.

The term "affinity-modified" as used in the context of an immunoglobulin superfamily domain, means a mammalian immunoglobulin superfamily (IgSF) domain having an altered amino acid sequence (relative to the corresponding wild-type parental or unmodified IgSF domain) such that it has an increased or decreased binding affinity or avidity to at least one of its cognate binding partners (alternatively "counter-structures") compared to the parental wild-type or unmodified (i.e., non-affinity modified) IgSF control domain. In some embodiments, the affinity-modified IgSF domain can contain 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more amino acid differences, such as amino acid substitutions, in a wild-type or unmodified IgSF domain. An increase or decrease in binding affinity or avidity can be determined using well known binding assays such as flow cytometry. Larsen et al., American Journal of Transplantation, Vol 5: 443-453 (2005). See also, Linsley et al., Immunity, 1: 7930801 (1994). An increase in a protein's binding affinity or avidity to its cognate binding partner(s) is to a value at least 10% greater than that of the wild-type IgSF domain control and in some embodiments, at least 20%, 30%, 40%, 50%, 100%, 200%, 300%, 500%, 1000%, 5000%, or 10000% greater than that of the wild-type IgSF domain control value. A decrease in a protein's binding affinity or avidity to at least one of its cognate binding partner is to a value no greater than 90% of the control but no less than 10% of the wild-type IgSF domain control value, and in some embodiments no greater than 80%, 70% 60%, 50%, 40%, 30%, or 20% but no less than 10% of the wild-type IgSF domain control value. An affinity-modified protein is altered in primary amino acid sequence by substitution, addition, or deletion of amino acid residues. The term "affinity-modified IgSF domain" is not be construed as imposing any condition for any particular starting composition or method by which the affinity-modified IgSF domain was created. Thus, the affinity-modified IgSF domains of the present invention are not limited to wild-type IgSF domains that are then transformed to an affinity-modified IgSF domain by any particular process of affinity modification. An affinity-modified IgSF domain polypeptide can, for example, be generated starting from wild-type mammalian IgSF domain sequence information, then modeled in silico for binding to its cognate binding partner, and finally recombinantly or chemically synthesized to yield the affinity-modified IgSF domain composition of matter. In but one alternative example, an affinity-modified IgSF domain can be created by site-directed mutagenesis of a wild-type IgSF domain. Thus, affinity modified IgSF domain denotes a product and not necessarily a product produced by any given process. A variety of techniques including recombinant methods, chemical synthesis, or combinations thereof, may be employed.

The term "allogeneic" as used herein means a cell or tissue that is removed from one organism and then infused or adoptively transferred into a genetically dissimilar organism of the same species.

The term "autologous" as used herein means a cell or tissue that is removed from the same organism to which it is later infused or adoptively transferred. An autologous cell or tissue can be altered by, for example, recombinant DNA methodologies, such that it is no longer genetically identical to the native cell or native tissue which is removed from the organism. For example, a native autologous T-cell can be genetically engineered by recombinant DNA techniques to become an autologous engineered cell expressing a immunomodulatory protein (which can be secreted from the engineered cell) and/or chimeric antigen receptor (CAR), which in some cases involves engineering a T-cell or TIL (tumor infiltrating lymphocyte). The engineered cell can then be infused into a patient from which the native T-cell was isolated. In some embodiments, the organism is human or murine.

The terms "binding affinity," and "binding avidity" as used herein means the specific binding affinity and specific binding avidity, respectively, of a protein for its cognate binding partner (i.e., its counter-structure) under specific binding conditions. In biochemical kinetics avidity refers to the accumulated strength of multiple affinities of individual non-covalent binding interactions, such as between an IgSF domain and its cognate binding partner (i.e., its counter-structure). As such, avidity is distinct from affinity, which describes the strength of a single interaction. An increase or attenuation in binding affinity of an affinity-modified IgSF domain to its cognate binding partner is determined relative to the binding affinity of the unmodified IgSF domain (e.g., the native or wild-type IgSF domain). Methods for determining binding affinity or avidity are known in art. See, for example, Larsen et al., American Journal of Transplantation, vol. 5: 443-453 (2005).The term "cell surface counter-structure" (alternatively "cognate cell surface binding partner") as used herein is a counter-structure (alternatively is a cognate binding partner) expressed on a mammalian cell. Typically, the cell surface cognate binding partner is a transmembrane protein. In some embodiments, the cell surface cognate binding partner is a receptor.

The term "chimeric antigen receptor" or "CAR" as used herein refers to an artificial (i.e., man-made) transmembrane protein expressed on a mammalian cell comprising at least an ectodomain, a transmembrane, and an endodomain. Optionally, the CAR protein includes a "spacer" which covalently links the ectodomain to the transmembrane domain. A spacer is often a polypeptide linking the ectodomain to the transmembrane domain via peptide bonds. The CAR is typically expressed on a mammalian lymphocyte. In some embodiments, the CAR is expressed on a mammalian cell such as a T-cell or a tumor infiltrating lymphocyte (TIL). A CAR expressed on a T-cell is referred to herein as a CAR T-cell or "CAR-T." In some embodiments the CAR-T is a T helper cell, a cytotoxic T-cell, a natural killer T-cell, a memory T-cell, a regulatory T-cell, or a gamma delta T-cell. When used clinically in, e.g. adoptive cell transfer, a CAR with antigen binding specificity to the patient's tumor is typically engineered to be expressed on a native lymphocyte obtained from the patient. The engineered lymphocyte expressing the CAR is then infused back into the patient. The lymphocyte is thus often an autologous T-cell although allogeneic T-cells are included within the scope of the invention. The ectodomain of a CAR comprises an antigen binding region, such as an antibody or antigen binding fragment thereof (e.g. scFv), that specifically binds under physiological conditions with an antigen, such as a tumor specific antigen. Upon specific binding a biochemical chain of events (i.e., signal transduction) results in modulation of the immunological activity of the cell on which the CAR is expressed. Thus, for example, upon specific binding by the antigen binding region of the CAR-T to its antigen can lead to changes in the immunological activity of the T-cell activity as reflected by changes in cytotoxicity, proliferation or cytokine production. Signal transduction upon CAR activation is achieved in some embodiments by the CD3-zeta chain ("CD3-z") which is involved in signal transduction in native mammalian T-cells. CARs can further comprise multiple signaling domains such as CD28, ICOS, 41BB or OX40, to further modulate immunomodulatory response of the T-cell. CD3-z comprises a conserved motif known as an immunoreceptor tyrosine-based activation motif (ITAM) which is involved in T-cell receptor signal transduction.

The terms "cognate binding partner" or "counter-structure" in reference to a protein, such as an IgSF domain or an affinity-modified IgSF domain, refers to at least one molecule (typically a native mammalian protein) to which the referenced protein specifically binds under specific binding conditions. In some aspects, an affinity-modified IgSF domain specifically binds to the cognate binding partner of the corresponding native or wild-type IgSF domain but with increased or attenuated affinity. A species of ligand recognized and specifically binding to its cognate receptor under specific binding conditions is an example of a counter-structure or cognate binding partner of that receptor. A receptor, to which a native ligand recognizes and specifically binds to under specific binding conditions, is an example of a cognate binding partner of that ligand. In turn, the native ligand is the cognate binding partner of the receptor. For example, ICOSL specifically binds to CD28 and ICOS and thus these proteins are cognate binding partners of ICOSL. In another example, a tumor specific antigen and an affinity-modified IgSF domain to which it specifically binds are each cognate binding partners of the other. In the present invention a "cell surface molecular species" is a cognate binding partner of ligands of the immunological synapse (IS), expressed on and by cells, such as mammalian cells, forming the immunological synapse, for example immune cells.

The term "competitive binding" as used herein means that a protein is capable of specifically binding to at least two cognate binding partners but that specific binding of one cognate binding partner inhibits, such as prevents or precludes, simultaneous binding of the second cognate binding partner. Thus, in some cases, it is not possible for a protein to bind the two cognate binding partners at the same time. Generally, competitive binders contain the same or overlapping binding site for binding but this is not a requirement. In some embodiments, competitive binding causes a measurable inhibition (partial or complete) of specific binding of a protein to one of its cognate binding partner due to specific binding of a second cognate binding partner. A variety of methods are known to quantify competitive binding such as ELISA (enzyme linked immunosorbent assay) or Forte-Bio Octet experimental systems.

The term "conservative amino acid substitution" as used herein means an amino acid substitution in which an amino acid residue is substituted by another amino acid residue having a side chain R group with similar chemical properties (e.g., charge or hydrophobicity). Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Conservative amino acids substitution groups are: valine-leucine-isoleucine, phenylalanine-tyrosine, lysine-arginine, alanine-valine, glutamate-aspartate, and asparagine-glutamine.

The term, "corresponding to" with reference to positions of a protein, such as recitation that nucleotides or amino acid positions "correspond to" nucleotides or amino acid positions in a disclosed sequence, such as set forth in the Sequence Listing, refers to nucleotides or amino acid positions identified upon alignment with the disclosed sequence based on structural sequence alignment or using a standard alignment algorithm, such as the GAP algorithm. By aligning the sequences, one skilled in the art can identify corresponding residues, for example, using conserved and identical amino acid residues as guides.

The term "cytokine" includes, e.g., but is not limited to, interleukins, interferons (IFN), chemokines, hematopoietic growth factors, tumor necrosis factors (TNF), and transforming growth factors. In general, these are small molecular weight proteins that regulate maturation, activation, proliferation, and differentiation of cells of the immune system.

The terms "derivatives" or "derivatized" refer to modification of an immunomodulatory protein by covalently linking it, directly or indirectly, so as to alter such characteristics as half-life, bioavailability, immunogenicity, solubility, toxicity, potency, or efficacy while retaining or enhancing its therapeutic benefit. Derivatives can be made by glycosylation, pegylation, lipidation, or Fc-fusion. In some embodiments, the immunomodulatory protein is not derivatized. In some embodiments, the immunomodulatory protein is not conjugated to a half-life extending moiety, such as an Fc domain.

As used herein, "domain" (typically a sequence of three or more, generally 5 or 7 or more amino acids, such as 10 to 200 amino acid residues) refers to a portion of a molecule, such as a protein or encoding nucleic acid, that is structurally and/or functionally distinct from other portions of the molecule and is identifiable. For example, domains include those portions of a polypeptide chain that can form an independently folded structure within a protein made up of one or more structural motifs and/or that is recognized by virtue of a functional activity, such as binding activity. A protein can have one, or more than one, distinct domains. For example, a domain can be identified, defined or distinguished by homology of the primary sequence or structure to related family members, such as homology to motifs. In another example, a domain can be distinguished by its function, such as an ability to interact with a biomolecule, such as a cognate binding partner. A domain independently can exhibit a biological function or activity such that the domain independently or fused to another molecule can perform an activity, such as, for example binding. A domain can be a linear sequence of amino acids or a non-linear sequence of amino acids. Many polypeptides contain a plurality of domains. Such domains are known, and can be identified by those of skill in the art. For exemplification herein, definitions are provided, but it is understood that it is well within the skill in the art to recognize particular domains by name. If needed appropriate software can be employed to identify domains. It is understood that reference to amino acids, including to a specific sequence set forth as a SEQ ID NO used to describe domain organization of an IgSF domain are for illustrative purposes and are not meant to limit the scope of the embodiments provided. It is understood that polypeptides and the description of domains thereof are theoretically derived based on homology analysis and alignments with similar molecules. Thus, the exact locus can vary, and is not necessarily the same for each protein. Hence, the specific IgSF domain, such as specific IgV domain or IgC domain, can be several amino acids (one, two, three or four) longer or shorter.

The term "ectodomain," "extracellular domain," or "ECD" as used herein refers to the region of a membrane protein, such as a transmembrane protein, that lies outside the vesicular membrane (e.g., the space outside of a cell). Ectodomains often interact with specific ligands or specific cell surface receptors, such as via a binding domain that specifically binds to the ligand or cell surface receptor.

The terms "effective amount" or "therapeutically effective amount" refer to a quantity and/or concentration of a therapeutic composition of the invention, such as composition containing engineered cells, that when administered ex vivo (by contact with a cell from a patient) or in vivo (by administration into a patient, such as by adoptive transfer) either alone (i.e., as a monotherapy) or in combination with additional therapeutic agents, yields a statistically significant inhibition of disease progression as, for example, by ameliorating or eliminating symptoms and/or the cause of the disease. An effective amount for treating an immune system disease or disorder may be an amount that relieves, lessens, or alleviates at least one symptom or biological response or effect associated with the disease or disorder, prevents progression of the disease or disorder, or improves physical functioning of the patient. In the case of cell therapy, the effective amount is an effective dose or number of cells administered to a patient. In some embodiments the patient is a human patient.

The term "endodomain" as used herein refers to the region found in some membrane proteins, such as transmembrane proteins, that extends into the interior space defined by the cell surface membrane. In mammalian cells, the endodomain is the cytoplasmic region of the membrane protein. In cells, the endodomain interacts with intracellular constituents and can be play a role in signal transduction and thus, in some cases, can be an intracellular signaling domain. The endodomain of a cellular transmembrane protein is alternately referred to as a cytoplasmic domain, which, in some cases, can be a cytoplasmic signaling domain.

The term "enhanced" or "increased" as used herein in the context of increasing immunological activity of a mammalian lymphocyte means to increase one or more activities of the lymphocyte. An increased activity can be one or more of an increase cell survival, cell proliferation, cytokine production, or T-cell cytotoxicity, such as by a statistically significant amount. In some embodiments, reference to increased immunological activity means to increase interferon gamma (IFN-gamma) production, such as by a statistically significant amount. Methods of assessing activities of lymphocytes are known in the art, including any assay as described herein. In some embodiments an enhancement can be an increase of at least 10%, 20%, 30%, 40%, 50%, 75%, 100%, 200%, 300%, 400%, or 500% greater than a non-zero control value.

The term "engineered cell" as used herein refers to a mammalian cell that has been genetically modified by human intervention such as by recombinant DNA methods or viral transduction. In some embodiments, the engineered cell is an immune cell, such as a lymphocyte (e.g. T cell, B cell, NK cell) or an antigen presenting cell (e.g. dendritic cell). The cell can be a primary cell from a patient or can be a cell line. In some embodiments, an engineered cell is capable of expressing and secreting an immunomodulatory protein as described herein. In some embodiments, the engineered cell further expresses or contains an engineered T-cell receptor (TCR) or chimeric antigen receptor (CAR).

The term "engineered T-cell" as used herein refers to a T-cell such as a T helper cell, cytotoxic T-cell (alternatively, cytotoxic T lymphocyte or CTL), natural killer T-cell, regulatory T-cell, memory T-cell, or gamma delta T-cell, that has been genetically modified by human intervention such as by recombinant DNA methods. In some embodiments, an engineered T-cell is capable of expressing and secreting an immunomodulatory protein as described herein.

The term "engineered T-cell receptor" or "engineered TCR" refers to a T-cell receptor (TCR) engineered to specifically bind with a desired affinity to a major histocompatibility complex (MHC)/peptide target antigen that is selected, cloned, and/or subsequently introduced into a population of T-cells, often used for adoptive immunotherapy. In contrast to engineered TCRs, CARs are engineered to bind target antigens in a MHC independent manner.

A protein "expressed on" a cell is used herein to reference to a protein expressed by a cell and in which at least a portion of the protein is present on the surface of the cell. Proteins expressed on a cell include transmembrane proteins or cell surface receptors. In some embodiments, the protein is conjugated to a small molecule moiety such as a drug or detectable label.

The term "half-life extending moiety" refers to a moiety of a polypeptide fusion or chemical conjugate that extends the half-life of a protein circulating in mammalian blood serum compared to the half-life of the protein that is not so conjugated to the moiety. In some embodiments, half-life is extended by greater than or greater than about 1.2-fold, 1.5-fold, 2.0-fold, 3.0-fold, 4.0-fold, 5.0-fold, or 6.0-fold. In some embodiments, half-life is extended by more than 6 hours, more than 12 hours, more than 24 hours, more than 48 hours, more than 72 hours, more than 96 hours or more than 1 week after *in vivo* administration compared to the protein without the half-life extending moiety. The half-life refers to the amount of time it takes for the protein to lose half of its concentration, amount, or activity. Half-life can be determined for example, by using an ELISA assay or an activity assay. Exemplary half-life extending moieties include an Fc domain, a multimerization domain, polyethylene glycol (PEG), hydroxyethyl starch (HES), XTEN (extended recombinant peptides; see, WO2013130683), human serum albumin (HSA), bovine serum albumin (BSA), lipids (acylation), and poly-Pro-Ala-Ser (PAS), and polyglutamic acid (glutamylation).

The term "host cell" refers to any cell that can be used to express a protein encoded by a recombinant expression vector. A host cell can be a prokaryote, for example, E. coli, or it can be a eukaryote, for example, a single-celled eukaryote (e.g., a yeast or other fungus), a plant cell (e.g., a tobacco or tomato plant cell), an animal cell (e.g., a human cell, a monkey cell, a hamster cell, a rat cell, a mouse cell, or an insect cell) or a hybridoma. Examples of host cells include Chinese hamster ovary (CHO) cells or their derivatives such as Veggie CHO and related cell lines which grow in serum-free media or CHO strain DX-B11, which is deficient in DHFR.

The term "immunological synapse" or "immune synapse" (abbreviated "IS") as used herein means the interface between a mammalian cell that expresses MHC I (major histocompatibility complex) or MHC II, such as an antigen-presenting cell or tumor cell, and a mammalian lymphocyte such as an effector T cell or Natural Killer (NK) cell.

The term "immunoglobulin" (abbreviated "Ig") as used herein is synonymous with the term "antibody" (abbreviated "Ab") and refers to a mammalian immunoglobulin protein including any of the five human classes: IgA (which includes subclasses IgA1 and IgA2), IgD, IgE, IgG (which includes subclasses IgG1, IgG2, IgG3, and IgG4), and IgM. The term is also inclusive of immunoglobulins that are less than full-length, whether wholly or partially synthetic (e.g., recombinant or chemical synthesis) or naturally produced, such as antigen binding fragment (Fab), variable fragment (Fv) containing VH and VL, the single chain variable fragment (scFv) containing VH and VL linked together in one chain, as well as other antibody V region fragments, such as Fab', F(ab)2, F(ab')2, dsFv diabody, Fc, and Fd polypeptide fragments. Bispecific antibodies, homobispecific and heterobispecific, are included within the meaning of the term.

An Fc (fragment crystallizable) region or domain of an immunoglobulin molecule (also termed an Fc polypeptide) corresponds largely to the constant region of the immunoglobulin heavy chain, and is responsible for various functions, including the antibody's effector function(s). An immunoglobulin Fc fusion ("Fc-fusion") is a molecule comprising one or more polypeptides (or one or more small molecules) operably linked to an Fc region of an immunoglobulin. An Fc-fusion may comprise, for example, the Fc region of an antibody (which, in some cases, facilitates effector functions and pharmacokinetics) and the IgSF domain of a wild-type or affinity-modified immunoglobulin superfamily domain ("IgSF"), or other protein or fragment thereof. In some embodiments, the Fc is a variant Fc that exhibits reduced (e.g. reduced greater than 30%, 40%, 50%, 60%, 70%, 80%, 90% or more) activity to facilitate an effector function. The IgSF domain mediates recognition of the cognate binding partner (comparable to that of antibody variable region of an antibody for an antigen). An immunoglobulin Fc region may be linked indirectly or directly to one or more polypeptides or small molecules (fusion partners). Various linkers are known in the art and can be used to link an Fc to a fusion partner to generate an Fc-fusion. An Fc-fusion protein can comprise an immunoglobulin Fc region covalently linked, directly or indirectly, to at least one affinity modified IgSF domain. Fc-fusions of identical species can be dimerized to form Fc-fusion homodimers, or using non-identical species to form Fc-fusion heterodimers. In some embodiments, the immunomodulatory protein is not conjugated to an Fc domain or any portion thereof.

The term "immunoglobulin superfamily" or "IgSF" as used herein means the group of cell surface and soluble proteins that are involved in the recognition, binding, or adhesion processes of cells. Molecules are categorized as members of this superfamily based on shared structural features with immunoglobulins (i.e., antibodies); they all possess a domain known as an immunoglobulin domain or fold. Members of the IgSF include cell surface antigen receptors, co-receptors and co-stimulatory molecules of the immune system, molecules involved in antigen presentation to lymphocytes, cell adhesion molecules, certain cytokine receptors and intracellular muscle proteins. They are commonly associated with roles in the immune system. Proteins in the immunological synapse are often members of the IgSF. IgSF can also be classified into "subfamilies" based on shared properties such as function. Such subfamilies typically consist of from 4 to 30 IgSF members.

The terms "IgSF domain" or "immunoglobulin domain" or "Ig domain" as used herein refers a structural domain of IgSF proteins. Ig domains are named after the immunoglobulin molecules. They contain about 70-110 amino acids and are categorized according to their size and function. Ig-domains possess a characteristic Ig-fold, which has a sandwich-like structure formed by two sheets of antiparallel beta strands. Interactions between hydrophobic amino acids on the inner side of the sandwich and highly conserved disulfide bonds formed between cysteine residues in the B and F strands, stabilize the Ig-fold. One end of the Ig domain has a section called the complementarity determining region that is important for the specificity of antibodies for their ligands. The Ig like domains can be classified (into classes) as: IgV, IgC1, IgC2, or IgI. Most Ig domains are either variable (IgV) or constant (IgC). IgV domains with 9 beta strands are generally longer than IgC domains with 7 beta strands. Ig domains of some members of the IgSF resemble IgV domains in the amino acid sequence, yet are similar in size to IgC domains. These are called IgC2 domains, while standard IgC domains are called IgC1 domains. T-cell receptor (TCR) chains contain two Ig domains in the extracellular portion; one IgV domain at the N-terminus and one IgC1 domain adjacent to the cell membrane.

The term "IgSF species" as used herein means an ensemble of IgSF member proteins with identical or substantially identical primary amino acid sequence. Each mammalian immunoglobulin superfamily (IgSF) member defines a unique identity of all IgSF species that belong to that IgSF member. Thus, each IgSF family member is unique from other IgSF family members and, accordingly, each species of a particular IgSF family member is unique from the species of another IgSF family member. Nevertheless, variation between molecules that are of the same IgSF species may occur owing to differences in post-translational modification such as glycosylation, phosphorylation, ubiquitination, nitrosylation, methylation, acetylation, and lipidation. Additionally, minor sequence differences within a single IgSF species owing to gene polymorphisms constitute another form of variation within a single IgSF species as do wild type truncated forms of IgSF species owing to, for example, proteolytic cleavage. A "cell surface IgSF species" is an IgSF species expressed on the surface of a cell, generally a mammalian cell.

The term "immunological activity" as used herein in the context of mammalian lymphocytes refers to one or more cell survival, cell proliferation, cytokine production (e.g. interferon-gamma), or T-cell cytotoxicity activities. Methods to assay the immunological activity of engineered cells, including to evaluate the activity of the immunomodulatory protein, are known in the art and include, but are not limited to, the ability to expand T cells following antigen stimulation, sustain T cell expansion in the absence of re- stimulation, and anti-cancer activities in appropriate animal models. Assays also include assays to assess cytotoxicity, including a standard 51Cr-release assay (see e.g. Milone et al., (2009) Molecular Therapy 17: 1453-1464) or flow based cytotoxicity assays, or an impedance based cytotoxicity assay (Peper et al. (2014) Journal of Immunological Methods, 405:192-198). Assays to assess immunological activity of engineered cells can be compared to control non-engineered cells or to cells containing one or more other engineered recombinant receptor (e.g. antigen receptor) with a known activity.

An "immunomodulatory protein" or "immunomodulatory polypeptide" is a protein that modulates immunological activity. By "modulation" or "modulating" an immune response is meant that immunological activity is either enhanced or suppressed. An immunomodulatory protein can be a single polypeptide chain or a multimer (dimers or higher order multimers) of at least two polypeptide chains covalently bonded to each other by, for example, interchain disulfide bonds. Thus, monomeric, dimeric, and higher order multimeric proteins are within the scope of the defined term. Multimeric proteins can be homomultimeric (of identical polypeptide chains) or heteromultimeric (of different polypeptide chains). Secretable immunomodulatory proteins are a type of immunomodulatory protein.

The term "increase" as used herein means to increase by a statistically significant amount. An increase can be at least 5%, 10%, 20%, 30%, 40%, 50%, 75%, 100%, or greater than a non-zero control value.

The term "lymphocyte" as used herein means any of three subtypes of white blood cell in a mammalian immune system. They include natural killer cells (NK cells) (which function in cell-mediated, cytotoxic innate immunity), T cells (for cell-mediated, cytotoxic adaptive immunity), and B cells (for humoral, antibody-driven adaptive immunity). T cells include T helper cells, cytotoxic T-cells, natural killer T-cells, memory T-cells, regulatory T-cells, or gamma delta T-cells. Innate lymphoid cells (ILC) are also included within the definition of lymphocyte.

An "inhibitory counter-structure" or "inhibitory cognate binding partner" is a cell membrane protein, often a receptor, which when proximally bound near a separate activating receptor leads to an attenuation in the frequency, duration, magnitude, or intensity of the activating signaling cascade and phenotype mediated by the activating receptor. Examples of inhibitory receptors include PD-1, CTLA-4, LAG-3, TIGIT, CD96, CD112R, BTLA, CD160, TIM-3, and VSIG8. The term "stimulatory counter-structure" or "stimulatory cognate binding partner" is a cell membrane protein, often a receptor, which when activated and signal transduction is thereby induced, leads to an increase in the frequency, duration, or intensity of the phenotype mediated by that receptor. Examples of stimulatory receptors include CD28, ICOS, and CD226.

The term "lymphocyte" as used herein means any of three subtypes of white blood cell in a mammalian immune system. They include natural killer cells (NK cells) (which function in cell-mediated, cytotoxic innate immunity), T cells (for cell-mediated, cytotoxic adaptive immunity), and B cells (for humoral, antibody-driven adaptive immunity). T cells include: T helper cells, cytotoxic T-cells, natural killer T-cells, memory T-cells, regulatory T-cells, or gamma delta T-cells. Innate lymphoid cells (ILC) are also included within the definition of lymphocyte.

The terms "mammal," "subject," or "patient" specifically includes reference to at least one of a: human, chimpanzee, rhesus monkey, cynomolgus monkey, dog, cat, mouse, or rat.

The term "membrane protein" as used herein means a protein that, under physiological conditions, is attached directly or indirectly to a lipid bilayer. A lipid bilayer that forms a membrane can be a biological membrane such as a eukaryotic (e.g., mammalian) cell membrane or an artificial (i.e., man-made) membrane such as that found on a liposome. Attachment of a membrane protein to the lipid bilayer can be by way of covalent attachment, or by way of non-covalent interactions such as hydrophobic or electrostatic interactions. A membrane protein can be an integral membrane protein or a peripheral membrane protein. Membrane proteins that are peripheral membrane proteins are non-covalently attached to the lipid bilayer or non-covalently attached to an integral membrane protein. A peripheral membrane protein forms a temporary attachment to the lipid bilayer such that under the range of conditions that are physiological in a mammal, peripheral membrane protein can associate and/or disassociate from the lipid bilayer. In contrast to peripheral membrane proteins, integral membrane proteins form a substantially permanent attachment to the membrane's lipid bilayer such that under the range of conditions that are physiological in a mammal, integral membrane proteins do not disassociate from their attachment to the lipid bilayer. A membrane protein can form an attachment to the membrane by way of one layer of the lipid bilayer (monotopic), or attached by way of both layers of the membrane (polytopic). An integral membrane protein that interacts with only one lipid bilayer is an "integral monotopic protein". An integral membrane protein that interacts with both lipid bilayers is an "integral polytopic protein" alternatively referred to herein as a "transmembrane protein".

The terms "modulating" or "modulate" as used herein in the context of an immune response, such as a mammalian immune response, refer to any alteration, such as an increase or decrease, of an existing or potential immune responses that occurs as a result of administration of an immunomodulatory protein or as a result of administration of engineered cells expressing an immunomodulatory protein, such as a secretable immunomodulatory protein of the present invention. Such modulation includes any induction, or alteration in degree or extent, or suppression of immunological activity of an immune cell. Immune cells include B cells, T cells, NK (natural killer) cells, NK T cells, professional antigen-presenting cells (APCs), and non-professional antigen-presenting cells, and inflammatory cells (neutrophils, macrophages, monocytes, eosinophils, and basophils). Modulation includes any change imparted on an existing immune response, a developing immune response, a potential immune response, or the capacity to induce, regulate, influence, or respond to an immune response. Modulation includes any alteration in the expression and/or function of genes, proteins and/or other molecules in immune cells as part of an immune response. Modulation of an immune response or modulation of immunological activity includes, for example, the following: elimination, deletion, or sequestration of immune cells; proliferation, induction, survival or generation of immune cells that can modulate the functional capacity of other cells such as autoreactive lymphocytes, antigen presenting cells, or inflammatory cells; induction of an unresponsive state in immune cells (i.e., anergy); enhancing or suppressing the activity or function of immune cells, including but not limited to altering the pattern of proteins expressed by these cells. Examples include altered production and/or secretion of certain classes of molecules such as cytokines, chemokines, perforins, granzymes, growth factors, transcription factors, kinases, costimulatory molecules, or other cell surface receptors or any combination of these modulatory events. Modulation can be assessed, for example, by an alteration of an immunological activity of engineered cells, such as an alteration in in cytotoxic activity of engineered cells or an alteration in cytokine secretion of engineered cells relative to cells engineered with the wild-type IgSF protein.

The term "molecular species" as used herein means an ensemble of proteins with identical or substantially identical primary amino acid sequence. Each mammalian immunoglobulin superfamily (IgSF) member defines a collection of identical or substantially identical molecular species. Thus, for example, human CD80 is an IgSF member and each human CD80 molecule is a species of CD80. Variation between molecules that are of the same molecular species may occur owing to differences in post-translational modification such as glycosylation, phosphorylation, ubiquitination, nitrosylation, methylation, acetylation, and lipidation. Additionally, minor sequence differences within a single molecular species owing to gene polymorphisms constitute another form of variation within a single molecular species as do wild type truncated forms of a single molecular species owing to, for example, proteolytic cleavage. A "cell surface molecular species" is a molecular species expressed on the surface of a mammalian cell. Two or more different species of protein, each of which is present exclusively on one or exclusively the other (but not both) of the two mammalian cells forming the IS, are said to be in "cis" or "cis configuration" with each other. Two different species of protein, the first of which is exclusively present on one of the two mammalian cells forming the IS and the second of which is present exclusively on the second of the two mammalian cells forming the IS, are said to be in "trans" or "trans configuration." Two different species of protein each of which is present on both of the two mammalian cells forming the IS are in both cis and trans configurations on these cells.

The term "non-competitive binding" as used herein means the ability of a protein to specifically bind simultaneously to at least two cognate binding partners. In some embodiments, the binding occurs under specific binding conditions. Thus, the protein is able to bind to at least two different cognate binding partners at the same time although the binding interaction need not be for the same duration such that, in some cases, the protein is specifically bound to only one of the cognate binding partners. In some embodiments, the simultaneous binding is such that binding of one cognate binding partner does not substantially inhibit simultaneous binding to a second cognate binding partner. In some embodiments, non-competitive binding means that binding a second cognate binding partner to its binding site on the protein does not displace the binding of a first cognate binding partner to its binding site on the protein. Methods of assessing non-competitive binding are well known in the art such as the method described in Perez de La Lastra et al., Immunology, 1999 Apr: 96(4): 663-670. In some cases, in non-competitive interactions, the first cognate binding partner specifically binds at an interaction site that does not overlap with the interaction site of the second cognate binding partner such that binding of the second cognate binding partner does not directly interfere with the binding of the first cognate binding partner. Thus, any effect on binding of the cognate binding partner by the binding of the second cognate binding partner is through a mechanism other than direct interference with the binding of the first cognate binding partner. For example, in the context of enzyme-substrate interactions, a non-competitive inhibitor binds to a site other than the active site of the enzyme. Non-competitive binding encompasses uncompetitive binding interactions in which a second cognate binding partner specifically binds at an interaction site that does not overlap with the binding of the first cognate binding partner but binds to the second interaction site only when the first interaction site is occupied by the first cognate binding partner.

The terms "nucleic acid" and "polynucleotide" are used interchangeably to refer to a polymer of nucleic acid residues (e.g., deoxyribonucleotides or ribonucleotides) in either single- or double-stranded form. Unless specifically limited, the terms encompass nucleic acids containing known analogues of natural nucleotides and that have similar binding properties to it and are metabolized in a manner similar to naturally-occurring nucleotides. Unless otherwise indicated, a particular nucleic acid sequence also implicitly encompasses conservatively modified variants thereof (e.g., degenerate codon substitutions) and complementary nucleotide sequences as well as the sequence explicitly indicated. Specifically, degenerate codon substitutions may be achieved by generating sequences in which the third position of one or more selected (or all) codons is substituted with mixed-base and/or deoxyinosine residues. The term nucleic acid or polynucleotide encompasses cDNA or mRNA encoded by a gene.

The term "pharmaceutical composition" refers to a composition suitable for pharmaceutical use in a mammalian subject, often a human. A pharmaceutical composition typically comprises an effective amount of an active agent (e.g., an immunomodulatory protein or engineered cells expressing and/or secreting an immunomodulatory protein of the present invention) and a carrier, excipient, or diluent. The carrier, excipient, or diluent is typically a pharmaceutically acceptable carrier, excipient or diluent, respectively.

The terms "polypeptide" and "protein" are used interchangeably herein and refer to a molecular chain of two or more amino acids linked through peptide bonds. The terms do not refer to a specific length of the product. Thus, "peptides," and "oligopeptides," are included within the definition of polypeptide. The terms include post-translational modifications of the polypeptide, for example, glycosylations, acetylations, phosphorylations and the like. The terms also include molecules in which one or more amino acid analogs or non-canonical or unnatural amino acids are included as can be synthesized, or expressed recombinantly using known protein engineering techniques. In addition, proteins can be derivatized as described herein by well-known organic chemistry techniques.

The term "primary T-cell assay" as used herein refers to an in vitro assay to measure interferon-gamma ("IFN-gamma") expression. A variety of such primary T-cell assays are known in the art such as that described in Example 6. In a preferred embodiment, the assay used is anti-CD3 coimmobilization assay. In this assay, primary T cells are stimulated by anti-CD3 immobilized with or without additional recombinant proteins. Culture supernatants are harvested at timepoints, usually 24-72 hours. In another embodiment, the assay used is a mixed lymphocyte reaction (MLR). In this assay, primary T cells are simulated with allogenic APC. Culture supernatants are harvested at timepoints, usually 24-72 hours. Human IFN-gamma levels are measured in culture supernatants by standard ELISA techniques. Commercial kits are available from vendors and the assay is performed according to manufacturer's recommendation.

The term "purified" as applied to nucleic acids, such as encoding a secretable immunomodulatory proteins, or proteins (e.g. immunomodulatory proteins) generally denotes a nucleic acid or polypeptide that is substantially free from other components as determined by analytical techniques well known in the art (e.g., a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid, or protein is at least about 50% pure, usually at least about 75%, 80%, 85%, 90%, 95%, 96%, 99% or more pure (e.g., percent by weight or on a molar basis).

The term "recombinant" indicates that the material (e.g., a nucleic acid or a polypeptide) has been artificially (i.e., non-naturally) altered by human intervention. The alteration can be performed on the material within, or removed from, its natural environment or state. For example, a "recombinant nucleic acid" is one that is made by recombining nucleic acids, e.g., during cloning, affinity modification, DNA shuffling or other well-known molecular biological procedures. A "recombinant DNA molecule," is comprised of segments of DNA joined together by means of such molecular biological techniques. The term "recombinant protein" or "recombinant polypeptide" as used herein refers to a protein molecule (e.g., an immunomodulatory protein) which is expressed using a recombinant DNA molecule. A "recombinant host cell" is a cell that contains and/or expresses a recombinant nucleic acid or that is otherwise altered by genetic engineering, such as by introducing into the cell a nucleic acid molecule encoding a recombinant protein, such as a immunomodulatory protein provided herein. Transcriptional control signals in eukaryotes comprise "promoter" and "enhancer" elements. Promoters and enhancers consist of short arrays of DNA sequences that interact specifically with cellular proteins involved in transcription. Promoter and enhancer elements have been isolated from a variety of eukaryotic sources including genes in yeast, insect and mammalian cells and viruses (analogous control elements, i.e., promoters, are also found in prokaryotes). The selection of a particular promoter and enhancer depends on what cell type is to be used to express the protein of interest. The terms "in operable combination," "in operable order" and "operably linked" as used herein refer to the linkage of nucleic acid sequences in such a manner or orientation that a nucleic acid molecule capable of directing the transcription of a given gene and/or the synthesis of a desired protein molecule is produced. The term also refers to the linkage of amino acid sequences in such a manner so that a functional protein is produced and/or transported.

The term "recombinant expression vector" as used herein refers to a DNA molecule containing a desired coding sequence (e.g., encoding an immunomodulatory protein) and appropriate nucleic acid sequences necessary for the expression of the operably linked coding sequence in a particular cell. Nucleic acid sequences necessary for expression in prokaryotes include a promoter, optionally an operator sequence, a ribosome binding site and possibly other sequences. Eukaryotic cells are known to utilize promoters, enhancers, and termination and polyadenylation signals. A secretory signal peptide sequence can also, optionally, be encoded by the recombinant expression vector, operably linked to the coding sequence so that the expressed protein can be secreted by the recombinant host cell, for more facile isolation of the fusion protein from the cell, if desired. The term includes the vector as a self-replicating nucleic acid structure as well as the vector incorporated into the genome of a host cell into which it has been introduced. Among the vectors are viral vectors, such as lentiviral vectors.

The term "selectivity" refers to the preference of a subject protein, or polypeptide, for specific binding of one substrate, such as one cognate binding partner, compared to specific binding for another substrate, such as a different cognate binding partner of the subject protein. Selectivity can be reflected as a ratio of the binding activity (e.g. binding affinity) of a subject protein and a first substrate, such as a first cognate binding partner, (e.g., K_{d1}) and the binding activity (e.g. binding affinity) of the same subject protein with a second cognate binding partner (e.g., K_{d2}).

The term "sequence identity" as used herein refers to the sequence identity between genes or proteins at the nucleotide or amino acid level, respectively. "Sequence identity" is a measure of identity between proteins at the amino acid level and a measure of identity between nucleic acids at nucleotide level. The protein sequence identity may be determined by comparing the amino acid sequence in a given position in each sequence when the sequences are aligned. Similarly, the nucleic acid sequence identity may be determined by comparing the nucleotide sequence in a given position in each sequence when the sequences are aligned. Methods for the alignment of sequences for comparison are well known in the art, such methods include GAP, BESTFIT, BLAST, FASTA and TFASTA. The BLAST algorithm calculates percent sequence identity and performs a statistical analysis of the similarity between the two sequences. The software for performing BLAST analysis is publicly available through the National Center for Biotechnology Information (NCBI) website.

The term "soluble" as used herein in reference to proteins, means that the protein is not a membrane protein. In general, a soluble protein contains only the extracellular domain of an IgSF family member receptor, or a portion thereof containing an IgSF domain or domains or specific-binding fragments thereof.

The term "species" as used herein in the context of a nucleic acid sequence or a polypeptide sequence refers to an identical collection of such sequences. Slightly truncated sequences that differ (or encode a difference) from the full length species at the amino-terminus or carboxy-terminus by no more than 1, 2, or 3 amino acid residues are considered to be of a single species. Such microheterogeneities are a common feature of manufactured proteins.

The term "specifically binds" as used herein means the ability of a protein, under specific binding conditions, to bind to a target protein such that its affinity or avidity is at least 10 times as great, but optionally 50, 100, 250 or 500 times as great, or even at least 1000 times as great as the average affinity or avidity of the same protein to a collection of random peptides or polypeptides of sufficient statistical size. A specifically binding protein need not bind exclusively to a single target molecule (e.g., its cognate binding partner) but may specifically bind to a non-target molecule due to similarity in structural conformation between the target and non-target (e.g., paralogs or orthologs). Those of skill will recognize that specific binding to a molecule having the same function in a different species of animal (i.e., ortholog) or to a non-target molecule having a substantially similar epitope as the target molecule (e.g., paralog) is possible and does not detract from the specificity of binding which is determined relative to a statistically valid collection of unique non-targets (e.g., random polypeptides). Thus, an affinity-modified polypeptide of the invention may specifically bind to more than one distinct species of target molecule due to cross-reactivity. Generally, such off-target specific binding is mitigated by reducing affinity or avidity for undesired targets. Solid-phase ELISA immunoassays or Biacore measurements can be used to determine specific binding between two proteins. Generally, interactions between two binding proteins have dissociation constants (Kd) less than about 1x10⁻⁵ M, and often as low as about 1 x 10⁻¹² M. In certain aspects of the present disclosure, interactions between two binding proteins have dissociation constants of less than about 1x10⁻⁶ M, 1x10⁻⁷ M, 1x10⁻⁸ M, 1x10⁻⁹ M, 1x10⁻¹⁰ M, or 1x10⁻¹¹ M.

The term "specific binding fragment" or "fragment" as used herein in reference to a mature (i.e., absent the signal peptide) wild-type IgSF domain, means a polypeptide that is shorter than the full-length mature IgSF domain and that specifically binds in vitro and/or in vivo to the wild-type IgSF domain's native cognate binding partner. In some embodiments, the specific binding fragment is at least 20%, 30%, 40%, 50%, 60%, 70%, 75%, 80%, 85%, 90%, 95%, 96%, 97%, 98%, or 99% the sequence length of the full-length mature wild-type sequence. The specific binding fragment can be altered in sequence to form an affinity modified IgSF domain of the invention. In some embodiments, the specific binding fragment modulates immunological activity of a lymphocyte. The terms "suppress" or "attenuate" or "decrease" as used herein means to decrease by a statistically significant amount. In some embodiments suppression can be a decrease of at least 10%, and up to 20%, 30%, 40%, 50%, 60%, 70%, 80%, or 90%.

As used herein, "synthetic," with reference to, for example, a synthetic nucleic acid molecule or a synthetic gene or a synthetic peptide refers to a nucleic acid molecule or polypeptide molecule that is produced by recombinant methods and/or by chemical synthesis methods.

The term "transmembrane protein" as used herein means a membrane protein that substantially or completely spans a lipid bilayer such as those lipid bilayers found in a biological membrane such as a mammalian cell, or in an artificial construct such as a liposome. The transmembrane protein comprises a transmembrane domain ("transmembrane domain") by which it is integrated into the lipid bilayer and by which the integration is thermodynamically stable under physiological conditions. Transmembrane domains are generally predictable from their amino acid sequence via any number of commercially available bioinformatics software applications on the basis of their elevated hydrophobicity relative to regions of the protein that interact with aqueous environments (e.g., cytosol, extracellular fluid). A transmembrane domain is often a hydrophobic alpha helix that spans the membrane. A transmembrane protein can pass through the both layers of the lipid bilayer once or multiple times.

The terms "treating," "treatment," or "therapy" of a disease or disorder as used herein mean slowing, stopping or reversing the disease or disorders progression, as evidenced by decreasing, cessation or elimination of either clinical or diagnostic symptoms, by administration of an immunomodulatory protein or engineered cells expressing a transmembrane immunomodulatory protein of the present invention either alone or in combination with another compound as described herein. "Treating," "treatment," or "therapy" also means a decrease in the severity of symptoms in an acute or chronic disease or disorder or a decrease in the relapse rate as for example in the case of a relapsing or remitting autoimmune disease course or a decrease in inflammation in the case of an inflammatory aspect of an autoimmune disease. As used herein in the context of cancer, the terms "treatment" or, "inhibit," "inhibiting" or "inhibition" of cancer refers to at least one of: a statistically significant decrease in the rate of tumor growth, a cessation of tumor growth, or a reduction in the size, mass, metabolic activity, or volume of the tumor, as measured by standard criteria such as, but not limited to, the Response Evaluation Criteria for Solid Tumors (RECIST), or a statistically significant increase in progression free survival (PFS) or overall survival (OS). "Preventing," "prophylaxis," or "prevention" of a disease or disorder as used in the context of this invention refers to the administration of an immunomodulatory protein or engineered cells expressing an immunomodulatory protein of the present invention, either alone or in combination with another compound, to prevent the occurrence or onset of a disease or disorder or some or all of the symptoms of a disease or disorder or to lessen the likelihood of the onset of a disease or disorder.

The term "tumor specific antigen" or "TSA" as used herein refers to a an antigen that is present primarily on tumor cells of a mammalian subject but generally not found on normal cells of the mammalian subject. In some cases, a tumor specific antigen is a counter structure or cognate binding partner of an IgSF member. A tumor specific antigen need not be exclusive to tumor cells but the percentage of cells of a particular mammal that have the tumor specific antigen is sufficiently high or the levels of the tumor specific antigen on the surface of the tumor are sufficiently high such that it can be targeted by anti-tumor therapeutics and provide prevention or treatment of the mammal from the effects of the tumor. In some embodiments, in a random statistical sample of cells from a mammal with a tumor, at least 50% of the cells displaying a TSA are cancerous. In other embodiments, at least 60%, 70%, 80%, 85%, 90%, 95%, or 99% of the cells displaying a TSA are cancerous.

The term "wild-type" or "natural" or "native" as used herein is used in connection with biological materials such as nucleic acid molecules, proteins, IgSF members, host cells, and the like, refers to those which are found in nature and not modified by human intervention.

### II. IMMUNOMODULATORY PROTEINS CONTAINING AFFINITY-MODIFIED DOMAINS

In some embodiments, the immunomodulatory protein, including secretable immunomodulatory proteins (SIPs) or transmembrane immunomodulatory proteins (TIPs), includes at least one affinity-modified IgSF domain compared to an IgSF domain of a wild-type mammalian IgSF member. The wild-type mammalian IgSF member excludes antibodies (i.e., immunoglobulins) such as those that are mammalian or may be of mammalian origin. Thus, the present invention relates to IgSF domains that are non-immunoglobulin (i.e., non-antibody) IgSF domains. Wild-type mammalian IgSF family members that are not immunoglobulins (i.e. antibodies) are known in the art as are their nucleic and amino acid sequences. Affinity-modified IgSF domains of a wild-type IgSF domain of all non-immunoglobulin mammalian IgSF family members are included within the scope of the invention.

In some embodiments, immunomodulatory proteins as provided in their various embodiments comprise at least one affinity-modified mammalian IgSF domain, such as at least one affinity modified non-immunoglobulin mammalian IgSF domain. In some embodiments, the non-immunoglobulin IgSF family members, and the corresponding IgSF domains present therein, are of mouse, rat, cynomolgus monkey, or human origin. In some embodiments, the IgSF family members are members from at least or exactly one, two, three, four, five, or more IgSF subfamilies such as: Signal-Regulatory Protein (SIRP) Family, Triggering Receptor Expressed On Myeloid Cells Like (TREML) Family, Carcinoembryonic Antigen-related Cell Adhesion Molecule (CEACAM) Family, Sialic Acid Binding Ig-Like Lectin (SIGLEC) Family, Butyrophilin Family, B7 family, CD28 family, V-set and Immunoglobulin Domain Containing (VSIG) family, V-set transmembrane Domain (VSTM) family, Major Histocompatibility Complex (MHC) family, Signaling lymphocytic activation molecule (SLAM) family, Leukocyte immunoglobulin-like receptor (LIR), Nectin (Nec) family, Nectin-like (NECL) family, Poliovirus receptor related (PVR) family, Natural cytotoxicity triggering receptor (NCR) family, or Killer-cell immunoglobulin-like receptors (KIR) family. In some embodiments, the at least one IgSF domain is derived from an IgSF protein that is any of CD80(B7-1), CD86(B7-2), CD274 (PD-L1, B7-H1), PDCD1LG2(PD-L2, CD273), ICOSLG(B7RP1, CD275, ICOSL, B7-H2), CD276(B7-H3), VTCN1(B7-H4), CD28, CTLA4, PDCD1(PD-1), ICOS, BTLA(CD272), CD4, CD8A(CD8-alpha), CD8B(CD8-beta), LAG3, HAVCR2(TIM-3), CEACAM1, TIGIT, PVR(CD155), PVRL2(CD112), CD226, CD2, CD160, CD200, CD200R1(CD200R), NC R3 (NKp30), VISTA, VSIG3, and VSIG8.

In some embodiments, the immunomodulatory protein contains at least one affinity-modified IgSF domain. In some embodiments, the at least one affinity-modified IgSF domain is affinity-modified compared to a corresponding IgSF domain of a non-immunoglobulin IgSF family member that is a mammalian IgSF member. In some embodiments, the mammalian IgSF member is one of the IgSF members or comprises an IgSF domain from one of the IgSF members as indicated in Table 1 including any mammalian orthologs thereof. Orthologs are genes in different species that evolved from a common ancestral gene by speciation. Normally, orthologs retain the same function in the course of evolution. In some embodiments, the affinity modified IgSF domain is an affinity modified IgV or IgC domain, including IgC1 or IgC2 domain.

In some embodiments, the immunomodulatory protein of the present invention comprises the sequence of the extracellular domain of a wild-type mammalian non-immunoglobulin (i.e., non-antibody) IgSF family member but wherein at least one IgSF domain therein is affinity modified. Solely by way of example, in some embodiments, a wild-type mammalian non-immunoglobulin IgSF family member comprises a first IgSF domain and a second IgSF domain, and an immunomodulatory protein comprises the first IgSF domain and the second IgSF domain of the wild-type IgSF family member, except at least the first IgSF domain or the second IgSF domain is affinity-modified. Immunomodulatory proteins comprising the sequence of the extracellular domain of a wild-type mammalian immunoglobulin (i.e., non-antibody) IgSF family member, but wherein at least one IgSF domain is affinity modified can be referred to as "Type I" immunomodulatory proteins. Additional domains present within the IgSF family can be affinity modified, such as at least two, three, four, or five IgSF domains and, in some embodiments, exactly two, three, four, or five IgSF domains. In some embodiments of an immunomodulatory protein of the invention, the mammalian IgSF member will be one of the IgSF members as indicated in Table 1 including any mammalian orthologs thereof

The first column of Table 1 provides the name and, optionally, the name of some possible synonyms for that particular IgSF member. The second column provides the protein identifier of the UniProtKB database, a publicly available database accessible via the internet at uniprot.org. The Universal Protein Resource (UniProt) is a comprehensive resource for protein sequence and annotation data. The UniProt databases include the UniProt Knowledgebase (UniProtKB). UniProt is a collaboration between the European Bioinformatics Institute (EMBL-EBI), the SIB Swiss Institute of Bioinformatics and the Protein Information Resource (PIR) and supported mainly by a grant from the U.S. National Institutes of Health (NIH). The third column provides the region where the indicated IgSF domain is located. The region is specified as a range where the domain is inclusive of the residues defining the range. Column 3 also indicates the IgSF domain class for the specified IgSF region. Column 4 provides the region where the indicated additional domains are located (signal peptide, S; extracellular domain, E; transmembrane domain, T; cytoplasmic domain, C). Column 5 indicates for some of the listed IgSF members, some of its cognate cell surface binding partners.

Typically, the affinity-modified IgSF domain of the immunomodulatory protein of the provided embodiments is a human or murine affinity modified IgSF domain.

| **TABLE 1. IgSF members according to the present disclosure.** | | | | | | | |
|---|---|---|---|---|---|---|---|
| **IgSF Member (Synonyms)** | **NCBI Protein Accession Number/ UniProtKB Protein Identifier** | **IgSF Region & Domain Class** | **Other Domains** | **Cognate Cell Surface Binding Partners** | **IgSF Member Amino Acid Sequence (SEQ ID NO)** | | |
| | | | | | **Precursor (mature residues)** | **Mature** | **ECD** |
| CD80 (B7-1) | NP_005182. 1 P33681 | 35-135, 35-138,35-141 or 37-138 IgV, 145-230 or 154-232 IgC | S: 1-34, E: 35-242, T: 243-263, C: 264-288 | CD28, CTLA4, PD-L1 | SEQ ID NO: 1 (35-288) | SEQ ID NO: 381 | SEQ ID NO: 28 |
| CD86 (B7-2) | P42081.2 | 33-131 IgV, 150-225 IgC2 | S: 1-23, E: 24-247, T: 248-268, C: 269-329 | CD28, CTLA4 | SEQ ID NO: 2 (24-329) | SEQ ID NO: 382 | SEQ ID NO: 29 |
| CD274 (PD-L1, B7-H1) | Q9NZQ7.1 | 24-130 IgV, 133-225 IgC2 | S: 1-18, E: 19-238, T: 239-259, C: 260-290 | PD-1, B7-1 | SEQ ID NO: 3 (19-290) | SEQ ID NO: 383 | SEQ ID NO: 30 |
| PDCD1LG2 (PD-L2, CD273) | Q9BQ51.2 | 21-118 IgV, 122-203 IgC2 | S: 1-19, E: 20-220, T: 221-241, C: 242-273 | PD-1, RGMb | SEQ ID NO: 4 (20-273) | SEQ ID NO: 384 | SEQ ID NO: 31 |
| ICOSLG (B7RP1, CD275, ICOSL, B7-H2) | 075144.2 | 19-129 IgV, 141-227 IgC2 | S: 1-18, E: 19-256, T: 257-277, C: 278-302 | ICOS, CD28, CTLA4 | SEQ ID NO: 5 (19-302) | SEQ ID NO: 385 | SEQ ID NO: 32 |
| CD276 (B7-H3) | Q5ZPR3.1 | 29-139 IgV, 145-238 IgC2, 243-357 IgV2, 363-456, 367-453 IgC2 | S: 1-28, E: 29-466, T: 467-487, C: 488-534 | | SEQ ID NO: 6 (29-534) | SEQ ID NO: 386 | SEQ ID NO: 33 |
| VTCN1 (B7-H4) | Q7Z7D3.1 | 35-146 IgV, 153-241 IgV | S: 1-24, E: 25-259, T: 260-280, C: 281-282 | | SEQ ID NO: 7 (25-282) | SEQ ID NO: 387 | SEQ ID NO: 34 |
| CD28 | P10747.1 | 28-137 IgV | S: 1-18, E: 19-152, T: 153-179, C: 180-220 | B7-1, B7-2, B7RP1 | SEQ ID NO: 8 (19-220) | SEQ ID NO: 388 | SEQ ID NO: 35 |
| CTLA4 | P16410.3 | 39-140 IgV | S: 1-35, E: 36-161, T: 162-182, C: 183-223 | B7-1, B7-2, B7RP1 | SEQ ID NO: 9 (36-223) | SEQ ID NO: 389 | SEQ ID NO: 36 |
| PDCD1 (PD-1) | Q15116.3 | 35-145 IgV | S: 1-20, E: 21-170, T: 171-191, C: 192-288 | PD-L1, PD-L2 | SEQ ID NO: 10 (21-288) | SEQ ID NO: 390 | SEQ ID NO: 37 |
| ICOS | Q9Y6W8.1 | 30-132 IgV | S: 1-20, E: 21-140, T: 141-161, C: 162-199 | B7RP1 | SEQ ID NO: 11 (21-199) | SEQ ID NO: 391 | SEQ ID NO: 38 |
| BTLA (CD272) | Q7Z6A9.3 | 31-132 IgV | S: 1-30, E: 31-157, T: 158-178, C: 179-289 | HVEM | SEQ ID NO: 12 (31-289) | SEQ ID NO: 392 | SEQ ID NO: 39 |
| CD4 | P01730.1 | 26-125 IgV, 126-203 IgC2, 204-317 IgC2, 317-389, 318-374 IgC2 | S: 1-25, E: 26-396, T: 397-418, C: 419-458 | MHC class II | SEQ ID NO: 13 (26-458) | SEQ ID NO: 393 | SEQ ID NO: 40 |
| CD8A (CD8-alpha) | P01732.1 | 22-135 IgV | S: 1-21, E: 22-182, T: 183-203, C: 204-235 | MHC class I | SEQ ID NO: 14 (22-235) | SEQ ID NO: 394 | SEQ ID NO: 41 |
| CD8B (CD8-beta) | P10966.1 | 22-132 IgV | S: 1-21, E: 22-170, T: 171-191, C: 192-210 | MHC class I | SEQ ID NO: 15 (22-210) | SEQ ID NO: 395 | SEQ ID NO: 42 |
| LAG3 | P18627.5 | 37-167 IgV, 168-252 IgC2, 265-343 IgC2, 349-419 IgC2 | S: 1-28, E: 29-450, T: 451-471, C: 472-525 | MHC class II | SEQ ID NO: 16 (29-525) | SEQ ID NO: 396 | SEQ ID NO: 43 |
| HAVCR2 (TIM-3) | Q8TDQ0.3 | 22-124 IgV | S: 1-21, E: 22-202, T: 203-223, C: 224-301 | CEACAM-1, phosphatidyl serine, Galectin-9, HMGB1 | SEQ IDNO: 17 (22-301) | SEQ ID NO: 397 | SEQ ID NO: 44 |
| CEACAM1 | P13688.2 | 35-142 IgV, 145-232 IgC2, 237-317 IgC2, 323-413 IgC2 | S: 1-34, E: 35-428, T: 429-452, C: 453-526 | TIM-3 | SEQ ID NO: 18 (35-526) | SEQ ID NO: 398 | SEQ ID NO: 45 |
| TIGIT | Q495A1.1 | 22-124 IgV | S: 1-21, E: 22-141, T: 142-162, C: 163-244 | CD155, CD112 | SEQ ID NO: 19 (22-244) | SEQ ID NO: 399 | SEQ ID NO: 46 |
| PVR (CD155) | P15151.2 | 24-139 IgV, 145-237 IgC2, 244 - 328 IgC2 | S: 1-20, E: 21-343, T: 344-367, C: 368-417 | TIGIT, CD226, CD96, poliovirus | SEQ ID NO: 20 (21-417) | SEQ ID NO: 400 | SEQ ID NO: 47 |
| PVRL2 (CD112) | Q92692.1 | 32-156 IgV, 162-256 IgC2, 261-345 IgC2 | S: 1-31, E: 32-360, T: 361-381, C: 382-538 | TIGIT, CD226, CD112R | SEQ ID NO: 21 (32-538) | SEQ ID NO: 401 | SEQ ID NO: 48 |
| CD226 | Q15762.2 | 19-126 IgC2, 135-239 IgC2 | S: 1-18, E: 19-254, T: 255-275, C: 276-336 | CD155, CD112 | SEQ ID NO: 22 (19-336) | SEQ ID NO: 402 | SEQ ID NO: 49 |
| CD2 | P06729.2 | 25-128 IgV, 129-209 IgC2 | S: 1-24, E: 25-209, T: 210-235, C: 236-351 | CD58 | SEQ ID NO: 23 (25-351) | SEQ ID NO: 403 | SEQ ID NO: 50 |
| CD 160 | 095971.1 | 27-122 IgV | N/A | HVEM, MHC family of proteins | SEQ ID NO: 24 (27-159) | SEQ ID NO: 404 | SEQ ID NO: 51 |
| CD200 | P41217.4 | 31-141 IgV, 142-232 IgC2 | S: 1-30, E: 31-232, T: 233-259, C: 260-278 | CD200R | SEQ ID NO: 25 (31-278) | SEQ ID NO: 405 | SEQ ID NO: 52 |
| CD200R1 (CD200R) | Q8TD46.2 | 53-139 IgV, 140-228 IgC2 | S: 1-28, E: 29-243, T: 244-264, C: 265-325 | CD200 | SEQ ID NO: 26 (29-325) | SEQ ID NO: 406 | SEQ ID NO: 53 |
| NCR3 (NKp30) | 014931.1 | 19-126 IgC-like | S: 1-18, E: 19-135, T: 136-156, C: 157-201 | B7-H6 | SEQ ID NO:27 (19-201) | SEQ ID NO: 407 | SEQ ID NO: 54 |
| VSIG8 | Q5VU13 | 22-141 IgV1, 146-257 IgV2 | S: 1-21 E: 22-263 T: 264-284 C: 285-414 | VISTA | SEQ ID NO: 408 (22-414) | SEQ ID NO: 409 | SEQ ID NO: 410 |

In some embodiments, the immunomodulatory protein contains at least one affinity-modified domain and at least one non-affinity modified IgSF domain (e.g. unmodified or wild-type IgSF domain). In some embodiments, the immunomodulatory protein contains at least two affinity modified domains. In some embodiments, the immunomodulatory protein contains a plurality of non-affinity modified IgSF domains and/or affinity-modified IgSF domains, such as 1, 2, 3, 4, 5, or 6 non-affinity modified IgSF and/or affinity modified IgSF domains.

In some embodiments, the immunomodulatory protein comprises a combination (a "non-wild-type combination") and/or arrangement (a "non-wild type arrangement" or "non-wild-type permutation") of an affinity-modified and/or non-affinity modified IgSF domain sequences that are not found in wild-type IgSF family members ("Type II" immunomodulatory proteins). The sequences of the IgSF domains which are non-affinity modified (e.g., wild-type) or have been affinity modified can be mammalian, such as from mouse, rat, cynomolgus monkey, or human origin, or combinations thereof. In some embodiments, the sequence of the non-affinity modified domain is any IgSF domain set forth in Table 1. The number of such non-affinity modified or affinity modified IgSF domains present in these embodiments of a Type II immunomodulatory protein (whether non-wild type combinations or non-wild type arrangements) is at least 2, 3, 4, or 5 and in some embodiments exactly 2, 3, 4, or 5 IgSF domains.

In some embodiments, at least two of the affinity modified IgSF domains are identical affinity modified IgSF domains. In some embodiments, the affinity modified IgSF domains are non-identical (i.e., different) IgSF domains. Non-identical affinity modified IgSF domains specifically bind, under specific binding conditions, different cognate binding partners and are "non-identical" irrespective of whether or not the wild-type IgSF domains from which they are designed was the same. Thus, for example, a combination of at least two non-identical IgSF domains in the immunomodulatory protein of the present invention can comprise at least one IgSF domain sequence whose origin is from and unique to one IgSF family member and at least one of a second IgSF domain sequence whose origin is from and unique to another IgSF family member wherein the IgSF domains of the immunomodulatory protein are in affinity modified form. However, in alternative embodiments, the two non-identical IgSF domains originate from the same IgSF domain sequence but are affinity modified differently such that they specifically bind to different cognate binding partners. In some embodiments, the number of non-identical affinity modified IgSF domains present in the immunomodulatory protein of the invention is at least 2, 3, 4, or 5 and in some embodiments exactly 2, 3, 4, or 5 non-identical affinity modified IgSF domains. In some embodiments, the non-identical IgSF domains are combinations from at least two IgSF members indicated in Table 1, and in some embodiments at least three or four IgSF members of Table 1.

In other embodiments an immunomodulatory protein provided herein comprises at least two IgSF domains from a single IgSF member but in a non-wild-type arrangement. One illustrative example of a non-wild type arrangement or permutation is an immunomodulatory protein of the present invention comprising a non-wild type order of affinity modified IgSF domain sequences relative to those found in the wild-type mammalian IgSF family member whose IgSF domain sequences served as the source of the affinity modified IgSF domains. The mammalian wild-type IgSF member in the preceding embodiment specifically includes those listed in Table 1. Thus, in one example, if the wild-type family member comprises an IgC1 domain proximal to the N-terminus of the protein and an IgV domain distal to the N-terminus, then the immunomodulatory protein provided herein can comprise an IgV proximal and an IgC1 distal to the N-terminus, albeit in affinity modified form. The presence, in an immunomodulatory protein, of both non-wild type combinations and non-wild type arrangements of affinity modified IgSF domains is also within the scope of the present invention. A plurality of affinity-modified IgSF domains in an immunomodulatory protein's polypeptide chain need not be covalently linked directly to one another. In some embodiments, an intervening span of one or more amino acid residues indirectly covalently bonds the affinity-modified IgSF domains to each other. Such "peptide linkers" can be a single amino acid residue or greater in length.

In some embodiments, the affinity modified IgSF domain can be affinity modified to specifically bind to a single (e.g., 1) or multiple (e.g., 2, 3, 4, or more) counter-structures (also called a "cognate binding partner") expressed on a mammalian cell. Typically, the cognate binding partner is a native cognate binding partner of the wild-type IgSF domain that has been affinity modified. In some embodiments, the cognate binding partner is an IgSF member. In some embodiments the cognate binding partner e is a non-IgSF family member. For example, in some embodiments the cognate binding partner of an affinity-modified IgSF domain such as BTLA (B- and T-lymphocyte attenuation) is the non-IgSF member cognate binding partner HVEM (herpes virus entry mediator). BTLA-HVEM complexes negatively regulate T-cell immune responses. Each IgSF domain present in an immunomodulatory protein can be affinity modified to independently increase or attenuate specific binding affinity or avidity to each of the single or multiple cognate binding partners to which it binds. By this method, specific binding to each of multiple cognate binding partners is independently tuned to a particular affinity or avidity.

In some embodiments, the cognate binding partner of an IgSF domain is at least one, and sometimes at least two or three of the counter-structures (cognate binding partners) of the wild-type IgSF domain, such as those listed in Table 1.

The sequence of the IgSF domain, such as mammalian IgSF domain, is affinity-modified by altering its sequence with at least one substitution, addition, or deletion. Alteration of the sequence can occur at the binding site for the cognate binding partner or at an allosteric site. In some embodiments, a nucleic acid encoding an IgSF domain, such as a mammalian IgSF domain, is affinity modified by substitution, addition, deletion, or combinations thereof, of specific and pre-determined nucleotide sites to yield a nucleic acid encoding an immunomodulatory protein of the invention. In some contrasting embodiments, a nucleic acid encoding an IgSF domain, such as a mammalian IgSF domain, is affinity modified by substitution, addition, deletion, or combinations thereof, at random sites within the nucleic acid. In some embodiments, a combination of the two approaches (pre-determined and random) is utilized. In some embodiments, design of the affinity modified IgSF domains is performed in silico.

In some embodiments, the affinity-modified IgSF domain of the immunomodulatory protein contains one or more amino acid substitutions (alternatively, "mutations" or "replacements") relative to a wild-type or unmodified polypeptide or a portion thereof containing an immunoglobulin superfamily (IgSF) domain. In some embodiments, the IgSF domain is an IgV domain or an IgC domain or specific binding fragment of the IgV domain or the IgC domain. In some embodiments, the immunomodulatory protein comprises an affinity modified IgSF domain that contains an IgV domain or an IgC domain or specific binding fragments thereof in which the at least one of the amino acid substitutions is in the IgV domain or IgC domain or a specific binding fragment thereof. In some embodiments, by virtue of the altered binding activity or affinity, the IgV domain or IgC domain is an affinity-modified IgSF domain.

In some embodiments, the IgSF domain, such as a mammalian IgSF domain, is affinity-modified in sequence with no more than a total of 1, 2, 3, 4, 5, 6, 7, 8, 9, or 10 amino acid substitutions, additions, deletions, or combinations thereof. In some embodiments, the IgSF domain, such as a mammalian IgSF domain, is affinity-modified in sequence with at least 1, 2, 3, 4, 5, 6, 7, 8, 9, or 10 amino acid substitutions, additions, deletions, or combinations thereof. In some embodiments, the IgSF domain, such as a mammalian IgSF domain, is affinity-modified in sequence with between 1 (or 2, 3, 4, 5, 6, 7, 8, or 9) and 10 (or 9, 8, 7, 6, 5, 4, 3, or 2) amino acid substitutions, additions, deletions, or combinations thereof. In some embodiments, the IgSF domain, such as a mammalian IgSF domain, is affinity-modified in sequence with no more than a total of 1, 2, 3, 4, 5, 6, 7, 8, 9, or 10 amino acid substitutions. In some embodiments, the IgSF domain, such as a mammalian IgSF domain, is affinity-modified in sequence with at least 1, 2, 3, 4, 5, 6, 7, 8, 9, or 10 amino acid substitutions. In some embodiments, the IgSF domain, such as a mammalian IgSF domain, is affinity-modified in sequence with between 1 (or 2, 3, 4, 5, 6, 7, 8, or 9) and 10 (or 9, 8, 7, 6, 5, 4, 3, or 2) amino acid substitutions. In some embodiments, the substitutions are conservative substitutions. In some embodiments, the substitutions are non-conservative. In some embodiments, the substitutions are a combination of conservative and non-conservative substitutions. In some embodiments, the modification in sequence is made at the binding site of the IgSF domain for its cognate binding partner.

In some embodiments, the wild-type or unmodified IgSF domain is a mammalian IgSF domain. In some embodiments, the wild-type or unmodified IgSF domain can be an IgSF domain that includes, but is not limited to, human, mouse, cynomolgus monkey, or rat. In some embodiments, the wild-type or unmodified IgSF domain is human.

In some embodiments, the wild-type or unmodified IgSF domain is or comprises an extracellular domain of an IgSF family member or a portion thereof containing an IgSF domain (e.g. IgV domain or IgC domain). In some cases, the extracellular domain of an unmodified or wild-type IgSF domain can comprise more than one IgSF domain, for example, an IgV domain and an IgC domain. However, the affinity modified IgSF domain need not comprise both the IgV domain and the IgC domain. In some embodiments, the affinity modified IgSF domain comprises or consists essentially of the IgV domain or a specific binding fragment thereof. In some embodiments, the affinity modified IgSF domain comprises or consists essentially of the IgC domain or a specific binding fragment thereof. In some embodiments, the affinity modified IgSF domain comprises the IgV domain or a specific binding fragment thereof, and the IgC domain or a specific binding fragment thereof.

In some embodiments, the one or more amino acid substitutions of the affinity modified IgSF domain can be located in any one or more of the IgSF polypeptide domains. For example, in some embodiments, one or more amino acid substitutions are located in the extracellular domain of the IgSF polypeptide. In some embodiments, one or more amino acid substitutions are located in the IgV domain or specific binding fragment of the IgV domain. In some embodiments, one or more amino acid substitutions are located in the IgC domain or specific binding fragment of the IgC domain.

In some embodiments, the wild-type or unmodified IgSF domain is an IgSF domain or specific binding fragment thereof contained in the sequence of amino acids set forth in any of SEQ ID NOS:1-27 and 408 or contained in a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 1-27 and 408. In some embodiments, the IgSF domain is an IgV domain or IgC domain contained therein or specific binding fragments thereof. Table 1 identifies the IgSF domains contained in each of SEQ ID NOS: 1-27 and 408.

In some embodiments, the unmodified or wild-type IgSF domain comprises the extracellular domain (ECD) or a portion comprising an IgSF domain (e.g. IgV domain or IgC domain) of an IgSF member, such as a mammalian IgSF member. In some embodiments, the unmodified or wild-type IgSF domain comprises (i) the sequence of amino acids set forth in any of SEQ ID NOS:28-54 and 410, (ii) a sequence of amino acids that has at least about 85%, 86%, 87%, 88%, 89%, 90%, 91%, 92%, 93%, 94%, 95%, 96%, 97%, 98%, 99% sequence identity to any of SEQ ID NOS: 28-54 and 410, or (iii) is a specific binding fragment of (i) or (ii) comprising an IgV domain or an IgC domain.

In some embodiments, at least one IgSF domain, such as at least one mammalian IgSF domain, of an immunomodulatory protein of the present invention is independently affinity modified in sequence to have at least 99%, 98%, 97%, 96%, 95%, 94%, 93%, 92%, 91%, 90%, 89%, 88%, 87%, 86% 85%, or 80% sequence identity with the corresponding wild-type IgSF domain or specific binding fragment thereof contained in a wild-type or unmodified IgSF protein, such as, but not limited to, those disclosed in Table 1 as SEQ ID NOS: 1-27 and 408.

In some embodiments, the affinity-modified IgSF domain of an immunomodulatory protein provided herein is a specific binding fragment of a wild-type or unmodified IgSF domain contained in a wild-type or unmodified IgSF protein, such as but not limited to, those disclosed in Table 1 in SEQ ID NOS: 1-27 and 408. In some embodiments, the specific binding fragment can have an amino acid length of at least 50 amino acids, such as at least 60, 70, 80, 90, 100, or 110 amino acids. In some embodiments, the specific binding fragment of the IgV domain contains an amino acid sequence that is at least about 85%, 86%, 87%, 88%, 89%, 90%, 91%, 92%, 93%, 94%, 95%, 96%, 97%, 98%, 99% of the length of the wild-type or unmodified IgV domain. In some embodiments, the specific binding fragment of the IgC domain comprises an amino acid sequence that is at least about 85%, 86%, 87%, 88%, 89%, 90%, 91%, 92%, 93%, 94%, 95%, 96%, 97%, 98%, 99% of the length of the wild-type or unmodified IgC domain. In some embodiments, the specific binding fragment modulates immunological activity. In more specific embodiments, the specific binding fragment of an IgSF domain increases immunological activity. In alternative embodiments, the specific binding fragment decreases immunological activity.

In some embodiments, to determine the percent identity of two nucleic acid sequences or of two amino acids, the sequences are aligned for optimal comparison purposes (e.g., gaps may be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity = # of identical positions/total # of positions (e.g., overlapping positions) x 100). In one embodiment the two sequences are the same length. One may manually align the sequences and count the number of identical nucleic acids or amino acids. Alternatively, alignment of two sequences for the determination of percent identity may be accomplished using a mathematical algorithm. Such an algorithm is incorporated into the NBLAST and XBLAST programs. BLAST nucleotide searches may be performed with the NBLAST program, score = 100, wordlength = 12, to obtain nucleotide sequences homologous to a nucleic acid molecules of the invention. BLAST protein searches may be performed with the XBLAST program, score = 50, wordlength = 3 to obtain amino acid sequences homologous to a protein molecule of the invention. To obtain gapped alignments for comparison purposes, Gapped BLAST may be utilized. Alternatively, PSI-Blast may be used to perform an iterated search which detects distant relationships between molecules. When utilizing the NBLAST, XBLAST, and Gapped BLAST programs, the default parameters of the respective programs may be used such as those available on the NCBI website. Alternatively, sequence identity may be calculated after the sequences have been aligned e.g. by the BLAST program in the NCBI database. Generally, the default settings with respect to e.g. "scoring matrix" and "gap penalty" may be used for alignment. In the context of the present invention, the BLASTN and PSI BLAST NCBI default settings may be employed.

The means by which the affinity-modified IgSF domains of the immunomodulatory proteins are designed or created is not limited to any particular method. In some embodiments, however, wild-type IgSF domains are mutagenized (site specific, random, or combinations thereof) from wild-type IgSF genetic material and screened for altered binding according to the methods disclosed in the Examples. Methods or mutagenizing nucleic acids is known to those of skill in the art. In some embodiments, the affinity modified IgSF domains are synthesized de novo utilizing protein or nucleic acid sequences available at any number of publicly available databases and then subsequently screened. The National Center for Biotechnology Information provides such information and its website is publicly accessible via the internet as is the UniProtKB database as discussed previously.

In some embodiments, at least one non-affinity modified IgSF domain and/or one affinity modified IgSF domain present in the immunomodulatory protein provided herein specifically binds to at least one cell surface molecular species expressed on mammalian cells forming the immunological synapse (IS). In some embodiments, an immunomodulatory protein provided herein can comprise a plurality of non-affinity modified IgSF domains and/or affinity modified IgSF domains such as 1, 2, 3, 4, 5, or 6 non-affinity modified IgSF and/or affinity modified IgSF domains. One or more of these non-affinity modified IgSF domains and/or affinity modified IgSF domains can independently specifically bind to either one or both of the mammalian cells forming the IS.

Often, the cell surface molecular species to which the affinity- modified IgSF domain of the immunomodulatory protein specifically binds will be the cognate binding partner of the wild type IgSF family member or wild type IgSF domain that has been affinity modified. In some embodiments, the cell surface molecular species is a mammalian IgSF member. In some embodiments, the cell surface molecular species is a human IgSF member. In some embodiments, the cell surface molecular species will be the cell surface cognate binding partners as indicated in Table 1. In some embodiments, the cell surface molecular species will be a viral protein, such as a poliovirus protein, on the cell surface of a mammalian cell such as a human cell.

In some embodiments, at least one non-affinity modified and/or affinity modified IgSF domain of the immunomodulatory protein provided herein binds to at least two or three cell surface molecular species present on mammalian cells forming the IS. The cell surface molecular species to which the non-affinity modified IgSF domains and/or the affinity modified IgSF domains of the immunomodulatory protein specifically bind to can exclusively be on one or the other of the two mammalian cells (i.e. in cis configuration) forming the IS or, alternatively, the cell surface molecular species can be present on both.

In some embodiments, the affinity modified IgSF domain specifically binds to at least two cell surface molecular species wherein one of the molecular species is present on one of the two mammalian cells forming the IS and the other molecular species is present on the second of the two mammalian cells forming the IS. In such embodiments, the cell surface molecular species is not necessarily present solely on one or the other of the two mammalian cells forming the IS (i.e., in a trans configuration) although in some embodiments it is. Thus, embodiments provided herein include those wherein each cell surface molecular species is exclusively on one or the other of the mammalian cells forming the IS (cis configuration) as well as those where the cell surface molecular species to which each affinity modified IgSF binds is present on both of the mammalian cells forming the IS (i.e., cis and trans configuration).

Those of skill will recognize that antigen presenting cells (APCs) and tumor cells form an immunological synapse with lymphocytes. Thus, in some embodiments at least one non-affinity modified IgSF domain and/or at least one affinity modified IgSF domain of the immunomodulatory protein specifically binds to only cell surface molecular species present on a cancer cell, wherein the cancer cell in conjunction with a lymphocyte forms the IS. In other embodiments, at least one non-affinity modified IgSF domain and/or at least one affinity modified IgSF domain of the immunomodulatory protein specifically binds to only cell surface molecular species present on a lymphocyte, wherein the lymphocyte in conjunction with an APC or tumor cell forms the IS. In some embodiments, the non-affinity modified IgSF domain and/or affinity modified IgSF domain bind to cell surface molecular species present on both the target cell (or APC) and the lymphocyte forming the IS.

Embodiments of the invention include those in which an immunomodulatory protein provided herein comprises at least one affinity modified IgSF domain with an amino acid sequence that differs from a wild-type or unmodified IgSF domain (e.g. a mammalian IgSF domain) such that the binding affinity (or avidity if in a multimeric or other relevant structure) of the affinity-modified IgSF domain, under specific binding conditions, to at least one of its cognate binding partners is either increased or decreased relative to the unaltered wild-type or unmodified IgSF domain control. In some embodiments, an affinity modified IgSF domain has a binding affinity for a cognate binding partner that differs from that of a wild-type or unmodified IgSF control sequence as determined by, for example, solid-phase ELISA immunoassays, flow cytometry or Biacore assays. In some embodiments, the affinity modified IgSF domain has an increased binding affinity for one or more cognate binding partners, relative to a wild-type or unmodified IgSF domain. In some embodiments, the affinity modified IgSF domain has a decreased binding affinity for one or more cognate binding partners, relative to a wild-type or unmodified IgSF domain. In some embodiments, the cognate binding partner can be a mammalian protein, such as a human protein or a murine protein.

Binding affinities for each of the cognate binding partners are independent; that is, in some embodiments, an affinity modified IgSF domain has an increased binding affinity for one, two or three different cognate binding partners, and a decreased binding affinity for one, two or three of different cognate binding partners, relative to a wild-type or unmodified polypeptide.

In some embodiments, the immunomodulatory protein provided herein comprises at least one affinity modified domain in which the binding affinity or avidity of the affinity modified IgSF domain is increased at least 10%, 20%, 30%, 40%, 50%, 100%, 200%, 300%, 400%, 500%, 1000%, 5000%, or 10,000% relative to the wild type or unmodified control IgSF domain. In some embodiments, the increase in binding affinity relative to the wild-type or unmodified IgSF domain is more than 1.2-fold, 1.5-fold, 2-fold, 3-fold, 4-fold, 5-fold, 6-fold, 7-fold, 8-fold, 9-fold, 10-fold, 20-fold, 30-fold 40-fold or 50-fold.

In some embodiments, the immunomodulatory protein provided herein comprises at least one affinity modified domain in which the binding affinity or avidity of the affinity modified IgSF domain is decreased at least 10%, and up to 20%, 30%, 40%, 50%, 60%, 70%, 80% or up to 90% relative to the wild-type or unmodified control IgSF domain. In some embodiments, the decrease in binding affinity relative to the wild-type or unmodified IgSF domain is more than 1.2-fold, 1.5-fold, 2-fold, 3-fold, 4-fold, 5-fold, 6-fold, 7-fold, 8-fold, 9-fold, 10-fold, 20-fold, 30-fold 40-fold or 50-fold.

In some embodiments, the immunomodulatory protein provided herein comprises at least one affinity modified domain in which the selectivity of the affinity modified IgSF domain for a particular cognate binding partner is increased compared to the wild-type or unmodified control IgSF domain. In some embodiments, the selectivity is represented as a ratio for binding of the particular cognate binding partner compared to one or more other cognate binding partner. In some embodiments, the selectivity ratio for binding a particular cognate binding partner is greater than or greater than about 1.2-fold, 1.5-fold, 2-fold, 3-fold, 4-fold, 5-fold, 6-fold, 7-fold, 8-fold, 9-fold, 10-fold, 20-fold, 30-fold 40-fold or 50-fold. In some embodiments, the selectivity of the affinity modified IgSF domain is increased at least 10%, 20%, 30%, 40%, 50%, 100%, 200%, 300%, 400%, 500%, 1000%, 5000%, or 10,000% relative to the wild type or unmodified control IgSF domain.

In some embodiments, the immunomodulatory protein provided herein comprises at least one affinity modified domain in which its specific binding affinity to a cognate binding partner can be less than or less than about 1x10⁻⁵ M, 1x10⁻⁶ M, 1x10⁻⁷ M, 1x10⁻⁸ M, 1x10⁻⁹ M, 1x10⁻¹⁰ M or 1x10⁻¹¹ M, or 1x10⁻¹² M.

In some embodiments, the immunomodulatory protein provided comprises at least two IgSF domains in which at least one of the IgSF domain is affinity modified while in some embodiments both are affinity modified, and wherein at least one of the affinity modified IgSF domains has increased affinity (or avidity) to its cognate binding partner and at least one affinity modified IgSF domain has a decreased affinity (or avidity) to its cognate binding partner. Functionally, and irrespective of whether specific binding to its cognate binding partner is increased or decreased, the immunomodulatory protein comprising one or more affinity-modified IgSF domains acts to enhance or suppress immunological activity of engineered immune cells, such as lymphocytes or antigen presenting cells, relative to engineered immune cells expressing the wild-type, parental molecule under the appropriate assay controls. In some embodiments, an immunomodulatory protein comprising an at least two affinity modified IgSF domains is one in which at least one of the affinity-modified IgSF domains agonizes an activating receptor and at least one affinity modified IgSF domain acts to antagonize an inhibitory receptor. In some embodiments, an enhancement of immunological activity can be an increase of at least 10%, 20%, 30%, 40%, 50%, 75%, 100%, 200%, 300%, 400%, or 500% greater than a non-zero control value such as in a cytotoxic activity assay, an assay for assessing cellular cytokines or a cell proliferation assay. In some embodiments, suppression of immunological activity can be a decrease of at least 10%, and up to 20%, 30%, 40%, 50%, 60%, 70%, 80%, or 90%.

The affinity modified IgSF domains of the immunomodulatory proteins of the invention can in some embodiments specifically bind competitively to its cognate binding partner. In other embodiments the affinity modified IgSF domains of the present invention specifically bind non-competitively to its cognate binding partner.

In some embodiments, the immunomodulatory protein provided herein contains an IgSF domain that otherwise binds to multiple cell surface molecular species but is affinity modified such that it substantially no longer specifically binds to one of its cognate cell surface molecular species. Thus, in these embodiments the specific binding to one of its cognate cell surface molecular species is reduced to specific binding of no more than 90% of the wild type level, such as no more than 80%, 70%, 60%, 50%, 40%, 30%, 20% or less. In some embodiments, the specific binding to one of its cognate cell surface molecular species is reduced to specific binding of no more than 10% of the wild type level and often no more than 7%, 5%, 3%, 1%, or no detectable or statistically significant specific binding.

In some embodiments, a specific binding site on a mammalian IgSF domain is inactivated or substantially inactivated with respect to at least one of the cell surface molecular species. Thus, for example, if a wild type IgSF domain specifically binds to exactly two cell surface molecular species then in some embodiments it is affinity modified to specifically bind to exactly one cell surface molecular species. And, if a wild type IgSF domain specifically binds to exactly three cell surface molecular species then in some embodiments it is affinity modified to specifically bind to exactly two cell surface molecular species. The IgSF domain that is affinity modified to substantially no longer specifically bind to one of its cognate cell surface molecular species can be an IgSF domain that otherwise specifically binds competitively or non-competitively to its cell surface molecular species. An illustrative example concerns native CD80 (B7-1) which specifically binds cognate binding partners: CD28, PD-L1, and CTLA4. In some embodiments, CD80 can be IgSF affinity modified to increase or attenuate its specific binding to CD28 and/or PD-L1 but not to specifically bind to any physiologically significant extent to CTLA4. The IgSF domain that is affinity modified to substantially no longer specifically bind to one of its cell surface cognate binding partners can be an IgSF domain that otherwise specifically binds competitively or non-competitively to its cognate binding partner. Those of skill will appreciate that a wild-type IgSF domain that competitively binds to two cognate binding partners can nonetheless be inactivated with respect to exactly one of them if, for example, their binding sites are not precisely coextensive but merely overlap such that specific binding of one inhibits binding of the other cognate binding partner and yet both competitive binding sites are distinct.

The non-affinity modified IgSF domains and/or affinity modified IgSF domains of the immunomodulatory proteins provided herein can, in some embodiments, specifically bind competitively to its cognate cell surface molecular species. In other embodiments the non-affinity-modified IgSF domain(s) and/or affinity-modified IgSF domain(s) of the immunomodulatory protein provided herein specifically bind non-competitively to its cognate cell surface molecular species. Any number of the non-affinity modified IgSF domains and/or affinity modified IgSF domains present in the immunomodulatory protein provided herein can specifically bind competitively or non-competitively.

In some embodiments, the immunomodulatory protein provided herein comprises at least two non-affinity modified IgSF domains, or at least one non-affinity modified IgSF domain and at least one affinity modified IgSF domain, or at least two affinity modified IgSF domains wherein one IgSF domain specifically binds competitively and a second IgSF domain binds non-competitively to its cognate cell surface molecular species. More generally, the immunomodulatory protein provided herein can comprise 1, 2, 3, 4, 5, or 6 competitive or 1, 2, 3, 4, 5, or 6 non-competitive binding non-affinity modified IgSF and/or affinity modified IgSF domains or any combination thereof. Thus, the immunomodulatory protein provided herein can have the number of non-competitive and competitive binding IgSF domains, respectively, of: 0 and 1, 0 and 2, 0 and 3, 0 and 4, 1 and 0, 1 and 1, 1 and 2, 1 and 3, 2 and 0, 2 and 1, 2 and 2, 2 and 3, 3 and 0, 3 and 1, 3 and 2, 3 and 3, 4 and 0, 4 and 1, and, 4 and 2.

In some embodiments in which the immunomodulatory protein contains a plurality of IgSF domains, the plurality of non-affinity modified and/or affinity modified IgSF domains of the immunomodulatory protein provided herein need not be covalently linked directly to one another. In some embodiments, an intervening span of one or more amino acid residues indirectly covalently bonds the non-affinity modified and/or affinity modified IgSF domains to each other. The linkage can be via the N-terminal to C-terminal residues.

In some embodiments, the linkage can be made via side chains of amino acid residues that are not located at the N-terminus or C-terminus of the non-affinity modified or affinity-modified IgSF domain. Thus, linkages can be made via terminal or internal amino acid residues or combinations thereof.

The "peptide linkers" that link the non-affinity modified and/or affinity modified IgSF domains can be a single amino acid residue or greater in length. In some embodiments, the peptide linker has at least one amino acid residue but is no more than 20, 19, 18, 17, 16, 15, 14, 13, 12, 11, 10, 9, 8, 7, 6, 5, 4, 3, 2, or 1 amino acid residues in length. In some embodiments, the linker is three alanines (AAA). In some embodiments, the linker is (in one-letter amino acid code): GGGGS ("4GS"; SEQ ID NO: 1870) or multimers of the 4GS linker, such as repeats of 2, 3, 4, or 5 4GS linkers, such as set forth in SEQ ID NO: 229 (2xGGGGS) or SEQ ID NO: 228 (3xGGGGS). In some embodiments, the linker (in one-letter amino acid code) is GSGGGGS (SEQ ID NO:1869).

### III. EXEMPLARY AFFINITY-MODIFIED DOMAINS AND IMMUNOMODULATORY PROTEINS

In some embodiments, the immunomodulatory protein contains an affinity-modified IgSF domain that has one or more amino acid substitutions in an IgSF domain of a wild-type or unmodified IgSF protein, such as set forth in Table 1 above. In some embodiments, the one or more amino acid substitutions are in the IgV domain or specific binding fragment thereof. In some embodiments, the one or more amino acid substitutions are in the IgC domain or specific binding fragment thereof. In some embodiments, one or more amino acid substitutions are in the IgV domain or a specific binding fragment thereof, and some of the one or more amino acid substitutions are in the IgC domain or a specific binding fragment thereof.

The wild-type or unmodified IgSF domain sequence does not necessarily have to be used as a starting composition to generate variant IgSF domain polypeptides described herein. Therefore, use of the term "modification", such as "substitution" does not imply that the present embodiments are limited to a particular method of making variant IgSF protein. Variant IgSF polypeptides can be made, for example, by *de novo* peptide synthesis and thus does not necessarily require a modification, such as a "substitution" in the sense of altering a codon to encode for the modification, e.g. substitution. This principle also extends to the terms "addition" and "deletion" of an amino acid residue which likewise do not imply a particular method of making. The means by which the variant IgSF polypeptides are designed or created is not limited to any particular method. In some embodiments, however, a wild-type or unmodified IgSF polypeptide encoding nucleic acid is mutagenized from wild-type or unmodified genetic material and screened for desired specific binding affinity and/or induction of IFN-gamma expression or other functional activity. In some embodiments, a variant IgSF polypeptide is synthesized *de novo* utilizing protein or nucleic acid sequences available at any number of publicly available databases and then subsequently screened. The National Center for Biotechnology Information provides such information and its website is publicly accessible via the internet as is the UniProtKB database as discussed previously.

Unless stated otherwise, as indicated throughout the present disclosure, the amino acid substitution(s) are designated by amino acid position number corresponding to the numbering of positions of the unmodified ECD sequences set forth in Table 1.

It is within the level of a skilled artisan to identify the corresponding position of a modification, e.g. amino acid substitution, in an affinity-modified IgSF domain, including portion thereof containing an IgSF domain (e.g. IgV) thereof, such as by alignment of a reference sequence. In the listing of modifications throughout this disclosure, the amino acid position is indicated in the middle, with the corresponding unmodified (e.g. wild-type) amino acid listed before the number and the identified variant amino acid substitution listed after the number. If the modification is a deletion of the position a "del" is indicated and if the modification is an insertion at the position an "ins" is indicated. In some cases, an insertion is listed with the amino acid position indicated in the middle, with the corresponding unmodified (e.g. wild-type) amino acid listed before and after the number and the identified variant amino acid insertion listed after the unmodified (e.g. wild-type) amino acid.

In some embodiments, the affinity-modified IgSF domain has up to 1, 2, 3, 4, 5, 6, 7, 8, 9, 10, 11, 12, 13, 14, 15, 16, 17, 18, 19, or 20 amino acid substitutions. The substitutions can be in the IgV domain or the IgC domain. In some embodiments, the affinity modified IgSF domain has up to 1, 2, 3, 4, 5, 6, 7, 8, 9, 10, 11, 12, 13, 14, 15, 16, 17, 18, 19, or 20 amino acid substitutions in the IgV domain or specific binding fragment thereof. In some embodiments, the affinity modified IgSF domain has up to 1, 2, 3, 4, 5, 6, 7, 8, 9, 10, 11, 12, 13, 14, 15, 16, 17, 18, 19, or 20 amino acid substitutions in the IgC domain or specific binding fragment thereof. In some embodiments, the affinity modified IgSF domain has at least about 85%, 86%, 86%, 88%, 89%, 90%, 91%, 92%, 93%, 94%, 95%, 96%, 97%, 98%, or 99% sequence identity with the wild-type or unmodified IgSF domain or specific binding fragment thereof, such as an IgSF domain contained in the IgSF protein set forth in any of SEQ ID NOS: 1-27 and 408.

In some embodiments, the immunomodulatory protein contains at least one affinity-modified IgSF domain containing one or more amino acid substitutions in a wild-type or unmodified IgSF domain of a B7 IgSF family member. In some embodiments, the B7 IgSF family member is CD80, CD86 or ICOS Ligand (ICOSL). In some embodiments, the affinity modified IgSF domain of the immunomodulatory protein has at least about 85%, 86%, 86%, 88%, 89%, 90%, 91%, 92%, 93%, 94%, 95%, 96%, 97%, 98%, or 99% sequence identity with the wild-type or unmodified IgSF domain or specific binding fragment thereof of CD80, CD86 or ICOS Ligand (ICOSL), such as the IgSF domain (e.g. IgV or IgC) contained in the IgSF protein set forth in any of SEQ ID NOS: 1, 2 or 5. Exemplary affinity modified IgSF domains of CD80 are set forth in Table 2 and Table 3. In some embodiments, the affinity modified IgSF domain of CD80 exhibits increased binding affinity or increased binding selectivity to CD28. In some embodiments, the affinity modified IgSF domain of CD80 exhibits increased binding affinity or increased binding selectivity to CTLA-4. Exemplary affinity modified IgSF domains of ICOSL are set forth in Table 4. In some embodiments, the affinity modified IgSF domain of ICOSL exhibits increased binding affinity or increased binding selectivity to CD28 or ICOS. Exemplary affinity modified IgSF domains of CD86 are set forth in Table 5.

| **TABLE 2: Exemplary variant CD80 polypeptides** | | |
|---|---|---|
| **Mutation(s)** | **ECD SEQ ID NO** | **IgV SEQ ID NO** |
| Wild-type | 28 | 152, 504, 578 |
| L70Q/A91G | 55 | 153 |
| L70Q/A91G/T130A | 56 | |
| L70Q/A91G/I118A/T120S/T130A | 57 | |
| V4M/L70Q/A91G/T120S/T130A | 58 | 154 |
| L70Q/A91G/T120S/T130A | 59 | |
| V20L/L70Q/A91S/T120S/T130A | 60 | 155 |
| S44P/L70Q/A91G/T130A | 61 | 156 |
| L70Q/A91G/E117G/T120S/T130A | 62 | |
| A91G/T120S/T130A | 63 | 157 |
| L70R/A91G/T120S/T130A | 64 | 158 |
| L70Q/E81A/A91G/T120S/I127T/T130A | 65 | 159 |
| L70Q/Y87N/A91G/T130A | 66 | 160 |
| T28S/L70Q/A91G/E95K/T120S/T130A | 67 | 161 |
| N63S/L70Q/A91G/T120S/T130A | 68 | 162 |
| K36E/I67T/L70Q/A91G/T120S/T130A/N152T | 69 | 163 |
| E52G/L70Q/A91G/T120S/T130A | 70 | 164 |
| K37E/F59S/L70Q/A91G/T120S/T130A | 71 | 165 |
| A91G/S103P | 72 | |
| K89E/T130A | 73 | 166 |
| A91G | 74 | |
| D60V/A91G/T120S/T130A | 75 | 167 |
| K54M/A91G/T120S | 76 | 168 |
| M38T/L70Q/E77G/A91G/T120S/T130A/N152T | 77 | 169 |
| R29H/E52GAL70R/E88G/A91G/T130A | 78 | 170 |
| Y31H/T41G/L70Q/A91G/T120S/T130A | 79 | 171 |
| V68A/T110A | 80 | 172 |
| S66H/D90G/T1 10A/F 116L | 81 | 173 |
| R29H/E52G/T120S/T130A | 82 | 174 |
| A91G/L102S | 83 | |
| I67T/L70Q/A91G/T120S | 84 | 175 |
| L70Q/A91G/T110A/T120S/T130A | 85 | |
| M38V/T41D/M43I/W50G/D76G/V83A/K89E/T120S/T130A | 86 | 176 |
| V22A/L70Q/S121P | 87 | 177 |
| A12V/S15F/Y31H/T41G/T130AJP137L/N152T | 88 | 178 |
| I67F/L70R/E88G/A91G/T120S/T130A | 89 | 179 |
| E24G/L25P/L70Q/T120S | 90 | 180 |
| A91G/F92L/F108L/T120S | 91 | 181 |
| R29D/Y31L/Q33H/K36G/M38I/T41A/M43R/M47T/E81V/L85R/K89 N/A91T/F92P/K93V/R94L/I118T/N149S | 92 | 182 |
| R29D/Y31L/Q33H/K36G/M38I/T41A/M43R/M47T/E81V/L85R/K89 N/A91T/F92P/K93V/R94L/N144S/N149S | 93 | |
| R29D/Y31L/Q33H/K36G/M38I/T41A/M42T/M43R/M47T/E81V/L85 | 94 | 183 |
| R/K89N/A91T/F92P/K93V/R94L/L148S/N149S | | |
| E24G/R29D/Y31L/Q33H/K36G/M38I/T41A/M43R/M47T/F59L/E81V/L85R/K89N/A91T/F92P/K93V/R94L/H96R/N149S/C182S | 95 | 184 |
| R29D/Y31L/Q33H/K36G/M38I/T41A/M43R/M47T/E81V/L85R/K89N/A91T/F92P/K93V/R94L/N149S | 96 | |
| R29V/M43Q/E81R/L85I/K89R/D90L/A91E/F92N/K93Q/R94G | 97 | 185 |
| T41I/A91G | 98 | 186 |
| K89R/D90K/A91G/F92Y/K93R/N122S/N177S | 99 | 187 |
| K89R/D90K/ A91 G/F92Y/K93R | 100 | |
| K36G/K37Q/M38I/F59L/E81V/L85R/K89N/A91T/F92P/K93V/R94L/E99G/T130A/N149S | 101 | 188 |
| E88D/K89R/D90K/A91G/F92Y/K93R | 102 | 189 |
| K3 6G/K3 7Q/M3 8I/L40M | 103 | 190 |
| K36G | 104 | 191 |
| R29H/Y31H/T41G/Y87N/E88G/K89E/D90N/A91G/P109S | 105 | 192 |
| A12T/H18L/M43V/F59L/E77K/P109S/I118T | 106 | 193 |
| R29V/Y31F/K36G/M38L/M43Q/E81R/V83I/L85I/K89R/D90L/A91E/F92N/K93Q/R94G | 107 | 194 |
| V68M/L70P/L72P/K86E | 108 | 195 |

| **TABLE 3: Exemplary variant CD80 polypeptides** | | |
|---|---|---|
| **Mutation(s)** | **ECD SEQ ID NO** | **IgV SEQ ID NO** |
| Wild-type | 28 | 152, 504, 578 |
| L70P | 431 | 505, 579 |
| I30F/L70P | 432 | 506, 580 |
| Q27H/T41S/A71D | 433 | 507, 581 |
| I30T/L70R | 434 | 508, 582 |
| T13R/C16R/L70Q/A71D | 435 | 509, 583 |
| T57I | 436 | 510, 584 |
| M43I/C82R | 437 | 511, 585 |
| V22L/M38V/M47T/A71D/L85M | 438 | 512, 586 |
| I30V/T57I/L70P/A71D/A91T | 439 | 513, 587 |
| V22I/L70M/A71D | 440 | 514, 588 |
| N55D/L70P/E77G | 441 | 515, 589 |
| T57A/I69T | 442 | 516, 590 |
| N55D/K86M | 443 | 517, 591 |
| L72P/T79I | 444 | 518, 592 |
| L70P/F92S | 445 | 519, 593 |
| T79P | 446 | 520, 594 |
| E35D/M47I/L65P/D90N | 447 | 521, 595 |
| L25S/E35D/M47I/D90N | 448 | 522, 596 |
| A71D | 450 | 524, 598 |
| E81K/A91S | 452 | 526, 600 |
| A12V/M47V/L70M | 453 | 527, 601 |
| K34E/T41A/L72V | 454 | 528, 602 |
| T41S/A71D/V84A | 455 | 529, 603 |
| E35D/A71D | 456 | 530, 604 |
| E35D/M47I | 457 | 531, 605 |
| K36R/G78A | 458 | 532, 606 |
| Q33E/T41A | 459 | 533, 607 |
| M47V/N48H | 460 | 534, 608 |
| M47L/V68A | 461 | 535, 609 |
| S44P/A71D | 462 | 536, 610 |
| Q27H/M43I/A71D/R73S | 463 | 537, 611 |
| E35D/T57I/L70Q/A71D | 465 | 539, 613 |
| M47I/E88D | 466 | 540, 614 |
| M42I/I61V/A71D | 467 | 541, 615 |
| P51A/A71D | 468 | 542, 616 |
| H18Y/M47I/T57I/A71G | 469 | 543, 617 |
| V20I/M47V/T57I/V84I | 470 | 544, 618 |
| V20I/M47V/A71D | 471 | 545, 619 |
| A71D/L72V/E95K | 472 | 546, 620 |
| V22L/E35G/A71D/L72P | 473 | 547, 621 |
| E35D/A71D | 474 | 548, 622 |
| E35D/I67L/A71D | 475 | 549, 623 |
| Q27H/E35G/A71D/L72P/T79I | 476 | 550, 624 |
| T13R/M42V/M47I/A71D | 477 | 551, 625 |
| E35D | 478 | 552, 626 |
| E35D/M47I/L70M | 479 | 553, 627 |
| E35D/A71D/L72V | 480 | 554, 628 |
| E35D/M43L/L70M | 481 | 555, 629 |
| A26P/E35D/M43I/L85Q/E88D | 482 | 556, 630 |
| E35D/D46V/L85Q | 483 | 557, 631 |
| Q27L/E35D/M47I/T57I/L70Q/E88D | 484 | 558, 632 |
| M47V/I69F/A71D/V83I | 485 | 559, 633 |
| E35D/T57A/A71D/L85Q | 486 | 560, 634 |
| H18Y/A26T/E35D/A71D/L85Q | 487 | 561, 635 |
| E35D/M47L | 488 | 562, 636 |
| E23D/M42V/M43I/I58V/L70R | 489 | 563, 637 |
| V68M/L70M/A71D/E95K | 490 | 564, 638 |
| N55I/T57I/I69F | 491 | 565, 639 |
| E35D/M43I/A71D | 492 | 566, 640 |
| T41S/T57I/L70R | 493 | 567, 641 |
| H18Y/A71D/L72P/E88V | 494 | 568, 642 |
| V20I/A71D | 495 | 569, 643 |
| E23G/A26S/E35D/T62N/A71D/L72V/L85M | 496 | 570, 644 |
| A12T/E24D/E35D/D46V/I61V/L72P/E95V | 497 | 571, 645 |
| V22L/E35D/M43L/A71G/D76H | 498 | 572, 646 |
| E35G/K54E/A71D/L72P | 499 | 573, 647 |
| L70Q/A71D | 500 | 574, 648 |
| A26E/E35D/M47L/L85Q | 501 | 575, 649 |
| D46E/A71D | 502 | 576, 650 |
| Y31H/E35D/T41S/V68L/K93R/R94W | 503 | 577, 651 |

| **TABLE 4: Exemplary variant ICOSL polypeptides** | | |
|---|---|---|
| **Mutation(s)** | **ECD SEQ ID NO** | **IgV SEQ ID NO** |
| Wild-type | 32 | 196 |
| N52S | 109 | 197 |
| N52H | 110 | 198 |
| N52D | 111 | 199 |
| N52Y/N57Y/F 138L/L203P | 112 | 200 |
| N52H/N57Y/Q100P | 113 | 201 |
| N52S/Y146C/Y152C | 114 | |
| N52H/C198R | 115 | |
| N52H/C140D/T225A | 116 | |
| N52H/C198R/T225A | 117 | |
| N52H/K92R | 118 | 202 |
| N52H/S99G | 119 | 203 |
| N52Y | 120 | 204 |
| N57Y | 121 | 205 |
| N57Y/Q100P | 122 | 206 |
| N52S/S130G/Y152C | 123 | |
| N52S/Y152C | 124 | |
| N52S/C198R | 125 | |
| N52Y/N57Y/Y152C | 126 | |
| N52Y/N57Y/129P/C198R | 127 | |
| N52H/L161P/C198R | 128 | |
| N52S/T113E | 129 | |
| S54A | 130 | 207 |
| N52D/S54P | 131 | 208 |
| N52K/L208P | 132 | 209 |
| N52S/Y152H | 133 | |
| N52D/V151A | 134 | |
| N52H/I143T | 135 | |
| N52S/L80P | 136 | 210 |
| F120S/Y152H/N201S | 137 | |
| N52S/R75Q/L203P | 138 | 211 |
| N52S/D158G | 139 | |
| N52D/Q133H | 140 | |
| N52S/N57Y/H94D/L96F/L98F/Q100R | 141 | 212 |
| N52S/N57Y/H94D/L96F/L98F/Q100R/G103E/F120S | 142 | 213 |
| N52S/G103E | 239 | 240 |
| N52H/C140delta/T225A | 1563 | |
| N52S/S54P | 1564 | 1565 |

| **TABLE 5: Exemplary variant CD86 polypeptides** | | |
|---|---|---|
| **Mutation(s)** | **ECD SEQ ID NO** | **IgV SEQ ID NO** |
| Wild-type | 29 | 220 |
| Q35H/H90L/Q102H | 148 | 221 |
| Q35H | 149 | 222 |
| H90L | 150 | 223 |
| Q102H | 151 | 224 |

In some embodiments, the affinity-modified IgSF domain binds, in some cases with higher binding affinity or selectivity, to an inhibitory receptor. In some embodiments, the affinity-modified IgSF domain contains one or more amino acid modifications (e.g. substitutions, deletions or additions) in a wild-type or unmodified IgSF domain (e.g. IgV) of an IgSF family member that binds to an inhibitory receptor. Exemplary of such inhibitory receptors are PD-1, CTLA-4, LAG3, TIGIT, TIM-3, or BTLA.

In some embodiments, the immunomodulatory protein contains at least one affinity modified IgSF domain containing one or more amino acid substitutions in a wild-type or unmodified IgSF domain of a poliovirus receptor IgSF family member. In some embodiments, the poliovirus IgSF family member is CD155 (PVR) or CD122 (PRR-2). In some embodiments, the affinity modified IgSF domain of the immunomodulatory protein has at least about 85%, 86%, 86%, 88%, 89%, 90%, 91%, 92%, 93%, 94%, 95%, 96%, 97%, 98%, or 99% sequence identity with the wild-type or unmodified IgSF domain or specific binding fragment thereof of CD155 or CD112, such as an IgSF domain (e.g. IgV or IgC) contained in the IgSF protein set forth in any of SEQ ID NO:20 or 21. Exemplary affinity modified IgSF domains of CD155 are set forth in Table 6. Exemplary affinity modified IgSF domains of CD112 are set forth in Table 7. In some embodiments, the affinity modified IgSF domain of CD155 or CD122 exhibits increased binding affinity or increased binding selectivity to TIGIT.

| **TABLE 6: Exemplary variant CD155 polypeptides** | | |
|---|---|---|
| **Mutation(s)** | **ECD SEQ ID NO** | **IgV SEQ ID NO** |
| Wild-type | 47 | 241, 652 |
| P18S, P64S, F91S | 242 | 264, 653 |
| P18S, F91S, L104P | 243 | 265, 654 |
| L44P | 244 | 266, 655 |
| A56V | 245 | 267, 656 |
| P18L, L79V, F91S | 246 | 268, 657 |
| P18S, F91S | 247 | 269, 658 |
| P18T, F91S | 248 | 270, 659 |
| P18T, S42P, F91S | 249 | 271, 660 |
| G7E, P18T, Y30C, F91S | 250 | 272, 661 |
| P18T, F91S, G111D | 251 | 273, 662 |
| P18S, F91P | 252 | 274, 663 |
| P18T, F91S, F108L | 253 | 275, 664 |
| P18T, T45A, F91S | 255 | 277, 665 |
| P18T, F91S, R94H | 256 | 278, 666 |
| P18S, Y30C, F91S | 257 | 279, 667 |
| A81V, L83P | 258 | 280, 668 |
| L88P | 259 | 281, 669 |
| R94H | 260 | 282, 670 |
| A13E, P18S, A56V, F91S | 261 | 283, 671 |
| P18T, F91S, V115A | 262 | 284, 672 |
| P18T, Q60K | 263 | 285, 673 |
| S52M | 674 | 771, 868 |
| T45Q, S52L, L104E, G111R | 675 | 772, 869 |
| S42G | 676 | 773, 870 |
| Q62F | 677 | 774, 871 |
| S52Q | 678 | 775, 872 |
| S42A, L104Q, G111R | 679 | 776, 873 |
| S42A, S52Q, L104Q, G111R | 680 | 777, 874 |
| S52W, L104E | 681 | 778, 875 |
| S42C | 682 | 779, 876 |
| S52W | 683 | 780, 877 |
| S52M, L104Q | 684 | 781, 878 |
| S42L, S52L, Q62F, L104Q | 685 | 782, 879 |
| S42W | 686 | 783, 880 |
| S42Q | 687 | 784, 881 |
| S52L | 688 | 785, 882 |
| S52R | 689 | 786, 883 |
| L104E | 690 | 787, 884 |
| G111R | 691 | 788, 885 |
| S52E | 692 | 789, 886 |
| Q62Y | 693 | 790, 887 |
| T45Q, S52M, L104E | 694 | 791, 888 |
| S42N, L104Q, G111R | 695 | 792, 889 |
| S52M, V57L | 696 | 793, 890 |
| S42N, S52Q, Q62F | 697 | 794, 891 |
| S42A, S52L, L104E, G111R | 698 | 795, 892 |
| S42W, S52Q, V57L, Q62Y | 699 | 796, 893 |
| L104Q | 700 | 797, 894 |
| S42L, S52Q, L104E | 701 | 798, 895 |
| S42C, S52L | 702 | 799, 896 |
| S42W, S52R, Q62Y, L104Q | 703 | 800, 897 |
| T45Q, S52R, L104E | 704 | 801, 898 |
| S52R, Q62F, L104Q, G111R | 705 | 802, 899 |
| T45Q, S52L, V57L, L104E | 706 | 803, 900 |
| S52M, Q62Y | 707 | 804, 901 |
| Q62F, L104E, G111R | 708 | 805, 902 |
| T45Q, S52Q | 709 | 806, 903 |
| S52L, L104E | 710 | 807, 904 |
| S42V, S52E | 711 | 808, 905 |
| T45Q, S52R, G111R | 712 | 809, 906 |
| S42G, S52Q, L104E, G111R | 713 | 810, 907 |
| S42N, S52E, V57L, L104E | 714 | 811, 908 |
| S42C, S52M, Q62F | 715 | 812, 909 |
| S42L | 716 | 813, 910 |
| S42A | 717 | 814, 911 |
| S42G, S52L, Q62F, L104Q | 718 | 815, 912 |
| S42N | 719 | 816, 913 |
| P18T, S65A, S67V, F91S | 720 | 817, 914 |
| P18F, T39A, T45Q, T61R, S65N, S67L, E73G, R78G | 721 | 818, 915 |
| P18T, T45Q, T61R, S65N, S67L | 722 | 819, 916 |
| P18F, S65A, S67V, F91S | 723 | 820, 917 |
| P18F, T45Q, T61R, S65N, S67L, F91S, L104P | 724 | 821, 918 |
| P18S, L79P, L104M | 725 | 822, 919 |
| P18S, L104M | 726 | 823, 920 |
| L79P, L104M | 727 | 824, 921 |
| P18T, T45Q, L79P | 728 | 825, 922 |
| P18T, T45Q, T61R, S65H, S67H | 729 | 826, 923 |
| P18T, A81E | 730 | 827, 924 |
| P18S, D23Y, E37P, S52G, Q62M, G80S, A81P, G99Y, S112N | 731 | 828, 925 |
| A13R, D23Y, E37P, S42P, Q62Y, A81E | 732 | 829, 926 |
| A13R, D23Y, E37P, G99Y, S112N | 733 | 830, 927 |
| A13R, D23Y, E37P, Q62M, A77V, G80S, A81P, G99Y | 734 | 831, 928 |
| P18L, E37S, Q62M, G80S, A81P, G99Y, S112N | 735 | 832, 929 |
| P18S, L104T | 736 | 833, 930 |
| P18S, Q62H, L79Q, F91S | 737 | 834, 931 |
| T45Q, S52K, Q62F, L104Q, G111R | 738 | 835, 932 |
| T45Q, S52Q, Q62Y, L104Q, G111R | 739 | 836, 933 |
| T45Q, S52Q, Q62Y, L104E, G111R | 740 | 837, 934 |
| V57A, T61M, S65W, S67A, E96D, L104T | 741 | 838, 935 |
| P18L, V57T, T61S, S65Y, S67A, L104T | 742 | 839, 936 |
| P18T, T45Q | 743 | 840, 937 |
| P18L, V57A, T61M, S65W, S67A, L104T | 744 | 841, 938 |
| T61M, S65W, S67A, L104T | 745 | 842, 939 |
| P18S, V41A, S42G, T45G, L104N | 746 | 843, 940 |
| P18H, S42G, T45I, S52T, G53R, S54H, V57L, H59E, T61S, S65D, E68G, L104N | 747 | 844, 941 |
| P18S, S42G, T45V, F58L, S67W, L104N | 748 | 845, 942 |
| P18S, T45I, L104N | 749 | 846, 943 |
| P18S, S42G, T45G, L104N, V106A | 750 | 847, 944 |
| P18H, H40R, S42G, T45I, S52T, G53R, S54H, V57L, H59E, T61S, S65D, E68G, L104Y, V106L, F108H | 751 | 848, 945 |
| E37V, S42G, T45G, L104N | 752 | 849, 946 |
| P18S, T45Q, L79P, L104T | 753 | 850, 947 |
| P18L, Q62R | 754 | 851, 948 |
| A13R, D23Y, E37P, S42L, S52G, Q62Y, A81E | 755 | 852, 949 |
| P18L, H49R, L104T, D116N | 756 | 853, 950 |
| A13R, D23Y, E37P, Q62M, G80S, A81P, L104T | 757 | 854, 951 |
| S65T, L104T | 758 | 855, 952 |
| A13R, D23Y, E37P, S52G, V57A, Q62M, K70E, L104T | 759 | 856, 953 |
| P18L, A47V, Q62Y, E73D, L104T | 760 | 857, 954 |
| H40T, V41M, A47V, S52Q, Q62L, S65T, E73R, D97G, E98S, L104T, D116N | 761 | 858, 955 |
| P18L, S42P, T45Q, T61G, S65H, S67E, L104T, D116N | 762 | 859, 956 |
| P18S, H40T, V41M, A47V, S52Q, Q62L, S65T, E73R, L104M, V106A | 763 | 860, 957 |
| H40T, V41M, A47V, S52Q, Q62L, S65T, E68G, E73R, D97G, E98S, L104T | 764 | 861, 958 |
| T45Q, S52E, L104E | 765 | 862, 959 |
| T45Q, S52E, Q62F, L104E | 766 | 863, 960 |
| P18F, T26M, L44V, Q62K, L79P, F91S, L104M, G111D | 767 | 864, 961 |
| P18S, T45S, T61K, S65W, S67A, F91S, G111R | 768 | 865, 962 |
| P18S, L79P, L104M, T107M | 769 | 866, 963 |
| P18S, S65W, S67A, M90V, V95A, L104Q, G111R | 770 | 867, 964 |
| P18S, A47G, L79P, F91S, L104M, T107A, R113W | 1701 | 1655, 1678 |
| P18T, D23G, S24A, N35D, H49L, L79P, F91S, L104M, G111R | 1702 | 1656, 1679 |
| V9L, P18S, Q60R, V75L, L79P, R89K, F91S, L104E, G111R | 1703 | 1657, 1680 |
| P18S, H49R, E73D, L79P, N85D, F91S, V95A, L104M, G111R | 1704 | 1658, 1681 |
| V11A, P18S, L79P, F91S, L104M, G111R | 1705 | 1659, 1682 |
| V11A, P18S, S54R, Q60P, Q62K, L79P, N85D, F91S, T107M | 1706 | 1660, 1683 |
| P18T, S52P, S65A, S67V, L79P, F91S, L104M, G111R | 1707 | 1661, 1684 |
| P18T, M36T, L79P, F91S, G111R | 1708 | 1662, 1685 |
| D8G, P18S, M36I, V38A, H49Q, A76E, F91S, L104M, T107A, R113W | 1709 | 1663, 1686 |
| P18S, S52P, S65A, S67V, L79P, F91S, L104M, T107S, R113W | 1710 | 1664, 1687 |
| T15I, P18T, L79P, F91S, L104M, G111R | 1711 | 1665, 1688 |
| P18F, T26M, L44V, Q62K, L79P, E82D, F91S, L104M, G111D | 1712 | 1666, 1689 |
| P18T, E37G, G53R, Q62K, L79P, F91S, E98D, L104M, T107M | 1713 | 1667, 1690 |
| P18L, K70E, L79P, F91S, V95A, G111R | 1714 | 1668, 1691 |
| V9I, Q12K, P18F, S65A, S67V, L79P, L104T, G111R, S112I | 1715 | 1669, 1692 |
| P18F, S65A, S67V, F91S, L104M, G111R | 1716 | 1670, 1693 |
| V9I, V10I, P18S, F20S, T45A, L79P, F91S, L104M, F108Y, G111R, S112V | 1717 | 1671, 1694 |
| V9L, P18L, L79P, M90I, F91S, T102S, L104M, G111R | 1718 | 1672, 1695 |
| P18C, T26M, L44V, M55I, Q62K, L79P, F91S, L104M, T107M | 1719 | 1673, 1696 |
| V9I, P18T, D23G, L79P, F91S, G111R | 1720 | 1674, 1697 |
| P18F, L79P, M90L, F91S, V95A, L104M, G111R | 1721 | 1675, 1698 |
| P18T, M36T, S65A, S67E, L79Q, A81T, F91S, G111R | 1722 | 1676, 1699 |
| V9L, P18T, Q62R, L79P, F91S, L104M, G111R | 1723 | 1677, 1700 |
| P18S, S65W, S67A, L104Q, G111R | 1724 | 1725,1726 |
| P18T, G19D, M36T, S54N, L79P, L83Q, F91S, T107M, F108Y | 1727 | 1773, 1819 |
| V9L, P18L, M55V, S69L, L79P, A81E, F91S, T107M | 1728 | 1774, 1820 |
| P18F, H40Q, T61K, Q62K, L79P, F91S, L104M, T107V | 1729 | 1775, 1821 |
| P18S, Q32R, Q62K, R78G, L79P, F91S, T107A, R113W | 1730 | 1776, 1822 |
| Q12H, P18T, L21S, G22S, V57A, Q62R, L79P, F91S, T107M | 1731 | 1777, 1823 |
| V9I, P18S, S24P, H49Q, F58Y, Q60R, Q62K, L79P, F91S, T107M | 1732 | 1778, 1824 |
| P18T, W46C, H49R, S65A, S67V, A76T, L79P, S87T, L104M | 1733 | 1779, 1825 |
| P18S, S42T, E51G, L79P, F91S, G92W, T107M | 1734 | 1780, 1826 |
| V10F, T15S, P18L, R48Q, L79P, F91S, T107M, V115M | 1735 | 1781, 1827 |
| P18S, L21M, Y30F, N35D, R84W, F91S, T107M, D116G | 1736 | 1782, 1828 |
| P18F, E51V, S54G, Q60R, L79Q, E82G, S87T, M90I, F91S, G92R, T107M | 1737 | 1783, 1829 |
| Q16H, P18F, F91S, T107M | 1738 | 1784, 1830 |
| P18T, D23G, Q60R, S67L, L79P, F91S, T107M, V115A | 1739 | 1785, 1831 |
| D8G, V9I, V11A, P18T, T26M, S52P, L79P, F91S, G92A, T107L, V115A | 1740 | 1786, 1832 |
| V9I, P18F, A47E, G50S, E68G, L79P, F91S, T107M | 1741 | 1787, 1833 |
| P18S, M55I, Q62K, S69P, L79P, F91S, T107M | 1742 | 1788, 1834 |
| P18T, T39S, S52P, S54R, L79P, F91S, T107M | 1743 | 1789, 1835 |
| P18S, D23N, L79P, F91S, T107M, S114N | 1744 | 1790, 1836 |
| P18S, P34S, E51V, L79P, F91S, G111R | 1745 | 1791, 1837 |
| P18S, H59N, V75A, L79P, A81T, F91S, L104M, T107M | 1746 | 1792, 1838 |
| P18S, W46R, E68D, L79P, F91S, T107M, R113G | 1747 | 1793, 1839 |
| V9L, P18F, T45A, S65A, S67V, R78K, L79V, F91S, T107M, S114T | 1748 | 1794, 1840 |
| P18T, M55L, T61R, L79P, F91S, V106I, T107M | 1749 | 1795, 1841 |
| T15I, P18S, V33M, N35F, T39S, M55L, R78S, L79P, F91S, T107M | 1750 | 1796, 1842 |
| P18S, Q62K, K70E, L79P, F91S, G92E, R113W | 1751 | 1797, 1843 |
| P18F, F20I, T26M, A47V, E51K, L79P, F91S | 1752 | 1798, 1844 |
| P18T, D23A, Q60H, L79P, M90V, F91S, T107M | 1753 | 1799, 1845 |
| P18S, D23G, C29R, N35D, E37G, M55I, Q62K, S65A, S67G, R78G, L79P, F91S, L104M, T107M, Q110R | 1754 | 1800, 1846 |
| A13E, P18S, M36R, Q62K, S67T, L79P, N85D, F91S, T107M | 1755 | 1801, 1847 |
| V9I, P18T, H49R, L79P, N85D, F91S, L104T, T107M | 1756 | 1802, 1848 |
| V9A, P18F, T61S, Q62L, L79P, F91S, G111R | 1757 | 1803, 1849 |
| D8E, P18T, T61A, L79P, F91S, T107M | 1758 | 1804, 1850 |
| P18S, V41A, H49R, S54C, L79S, N85Y, L88P, F91S, L104M, T107M | 1759 | 1805, 1851 |
| V11E, P18H, F20Y, V25E, N35S, H49R, L79P, F91S, T107M, G111R | 1760 | 1806, 1852 |
| V11A, P18F, D23A, L79P, G80D, V95A, T107M | 1761 | 1807, 1853 |
| P18S, K70R, L79P, F91S, G111R | 1762 | 1808, 1854 |
| V9L, V11M, P18S, N35S, S54G, Q62K, L79P, L104M, T107M, V115M | 1763 | 1809, 1855 |
| V9L, P18Y, V25A, V38G, M55V, A77T, L79P, M90I, F91S, L104M | 1764 | 1810, 1856 |
| V10G, P18T, L72Q, L79P, F91S, T107M | 1765 | 1811, 1857 |
| P18S, H59R, A76G, R78S, L79P | 1766 | 1812, 1858 |
| V9A, P18S, M36T, S65G, L79P, F91S, L104T, G111R, S112I | 1767 | 1813, 1859 |
| P18T, S52A, V57A, Q60R, Q62K, S65C, L79P, F91T, N100Y, T107M | 1768 | 1814, 1860 |
| V11A, P18F, N35D, A47E, Q62K, L79P, F91S, G99D, T107M, S114N | 1769 | 1815, 1861 |
| V11A, P18T, N35S, L79P, S87T, F91S | 1770 | 1816, 1862 |
| V9D, V11M, Q12L, P18S, E37V, M55I, Q60R, K70Q, L79P, F91S, L104M, T107M | 1771 | 1817, 1863 |
| T15S, P18S, Y30H, Q32L, Q62R, L79P, F91S, T107M | 1772 | 1818, 1864 |

| **TABLE 7: Exemplary variant CD112 polypeptides** | | |
|---|---|---|
| **Mutation(s)** | **ECD SEQ ID NO** | **IgV SEQ ID NO** |
| Wild-type | 48 | 286, 965 |
| Y33H, A112V, G117D | 287 | 334, 966 |
| V19A, Y33H, S64G, S80G, G98S, N106Y, A112V | 288 | 335, 967 |
| L32P, A112V | 289 | 336, 968 |
| A95V, A112I | 290 | 337, 969 |
| P28S, A112V | 291 | 338, 970 |
| P27A, T38N, V101A, A112V | 292 | 339, 971 |
| S118F | 293 | 340, 972 |
| R12W, H48Y, F54S, S118F | 294 | 341, 973 |
| R12W, Q79R, S118F | 295 | 342, 974 |
| T113S, S118Y | 296 | 343, 975 |
| S118Y | 297 | 344, 976 |
| N106I, S118Y | 298 | 345, 977 |
| N106I, S118F | 299 | 346, 978 |
| A95T, L96P, S118Y | 300 | 347, 979 |
| Y33H, P67S, N106Y, A112V | 301 | 348, 980 |
| N106Y, A112V | 302 | 349, 981 |
| T18S, Y33H, A112V | 303 | 350, 982 |
| P9S, Y33H, N47S, A112V | 304 | 351, 983 |
| P42S, P67H, A112V | 305 | 352, 984 |
| P27L, L32P, P42S, A112V | 306 | 353, 985 |
| G98D, A112V | 307 | 354, 986 |
| Y33H, S35P, N106Y, A112V | 308 | 355, 987 |
| L32P, P42S, T100A, A112V | 309 | 356, 988 |
| P27S, P45S, N106I, A112V | 310 | 357, 989 |
| Y33H, N47K, A112V | 311 | 358, 990 |
| Y33H, N106Y, A112V | 312 | 359, 991 |
| K78R, D84G, A112V, F114S | 313 | 360, 992 |
| Y33H, N47K, F54L, A112V | 314 | 361, 993 |
| Y33H, A112V | 315 | 362, 994 |
| A95V, A112V | 316 | 363, 995 |
| R12W, A112V | 317 | 364, 996 |
| R12W, P27S, A112V | 318 | 365, 997 |
| Y33H, V51M, A112V | 319 | 366, 998 |
| Y33H, A112V, S118T | 320 | 367, 999 |
| Y33H, V101A, A112V, P115S | 321 | 368, 1000 |
| H24R, T38N, D43G, A112V | 322 | 369, 1001 |
| A112V | 323 | 370, 1002 |
| P27A, A112V | 324 | 371, 1003 |
| A112V, S118T | 325 | 372, 1004 |
| R12W, A112V, M122I | 326 | 373, 1005 |
| Q83K, N106Y, A112V | 327 | 374, 1006 |
| R12W, P27S, A112V, S118T | 328 | 375, 1007 |
| P28S, Y33H, A112V | 329 | 376, 1008 |
| P27S, Q90R, A112V | 330 | 377, 1009 |
| L15V, P27A, A112V, S118T | 331 | 378, 1010 |
| Y33H, N106Y, T108I, A112V | 332 | 379, 1011 |
| Y33H, P56L, V75M, V101M, A112V | 333 | 380, 1012 |
| N47K, Q79R, S118F | 1013 | 1054, 1095 |
| Q40R, P60T, A112V, S118T | 1014 | 1055, 1096 |
| F114Y, S118F | 1015 | 1056, 1097 |
| Y33H, K78R, S118Y | 1016 | 1057, 1098 |
| R12W, A46T, K66M, Q79R, N106I, T113A, S118F | 1017 | 1058, 1099 |
| Y33H, A112V, S118F | 1018 | 1059, 1100 |
| R12W, Y33H, N106I, S118F | 1019 | 1060, 1101 |
| L15V, Q90R, S118F | 1020 | 1061, 1102 |
| N47K, D84G, N106I, S118Y | 1021 | 1062, 1103 |
| L32P, S118F | 1022 | 1063, 1104 |
| Y33H, Q79R, A112V, S118Y | 1023 | 1064, 1105 |
| T18A, N106I, S118T | 1024 | 1065, 1106 |
| L15V, Y33H, N106Y, A112V, S118F | 1025 | 1066, 1107 |
| V37M, S118F | 1026 | 1067, 1108 |
| N47K, A112V, S118Y | 1027 | 1068, 1109 |
| A46T, A112V | 1028 | 1069, 1110 |
| P28S, Y33H, N106I, S118Y | 1029 | 1070, 1111 |
| P30S, Y33H, N47K, V75M, Q79R, N106I, S118Y | 1030 | 1071, 1112 |
| V19A, N47K, N106Y, K116E, S118Y | 1031 | 1072, 1113 |
| Q79R, T85A, A112V, S118Y | 1032 | 1073, 1114 |
| V101M, N106I, S118Y | 1033 | 1074, 1115 |
| Y33H, Q79R, N106I, A112V, S118T | 1034 | 1075, 1116 |
| Q79R, A112V | 1035 | 1076, 1117 |
| Y33H, A46T, Q79R, N106I, S118F | 1036 | 1077, 1118 |
| A112V, G121S | 1037 | 1078, 1119 |
| Y33H, Q79R, N106I, S118Y | 1038 | 1079, 1120 |
| Y33H, N106I, A112V | 1039 | 1080, 1121 |
| Y33H, A46T, V101M, A112V, S118T | 1040 | 1081, 1122 |
| L32P, L99M, N106I, S118F | 1041 | 1082, 1123 |
| L32P, T108A, S118F | 1042 | 1083, 1124 |
| R12W, Q79R, A112V | 1043 | 1084, 1125 |
| Y33H, N106Y, E110G, A112V | 1044 | 1085, 1126 |
| Y33H, N106I, S118Y | 1045 | 1086, 1127 |
| Q79R, S118F | 1046 | 1087, 1128 |
| Y33H, Q79R, G98D, V101M, A112V | 1047 | 1088, 1129 |
| N47K, T81S, V101M, A112V, S118F | 1048 | 1089, 1130 |
| G82S, S118Y | 1049 | 1090, 1131 |
| Y33H, A112V, S118Y | 1050 | 1091, 1132 |
| Y33H, N47K, Q79R, N106Y, A112V | 1051 | 1092, 1133 |
| Y33H, S118T | 1052 | 1093, 1134 |
| R12W, Y33H, Q79R, V101M, A112V | 1053 | 1094, 1135 |
| Y33H, Q83K, A112V, S118T | 1583 | 1607, 1631 |
| V29M, Y33H, N106I, S118F | 1584 | 1608, 1632 |
| Y33H, A46T, A112V | 1585 | 1609, 1633 |
| Y33H, Q79R, S118F | 1586 | 1610, 1634 |
| Y33H, N47K, F74L, S118F | 1587 | 1611, 1635 |
| R12W, V101M, N106I, S118Y | 1588 | 1612, 1636 |
| A46T, V101A, N106I, S118Y | 1589 | 1613, 1637 |
| N106Y, A112V, S118T | 1590 | 1614, 1638 |
| S76P, T81I, V101M, N106Y, A112V, S118F | 1591 | 1615, 1639 |
| P9R, L21V, P22L, I34M, S69F, F74L, A87V, A112V, L125A | 1592 | 1616, 1640 |
| Y33H, V101M, A112V | 1593 | 1617, 1641 |
| V29A, L32P, S118F | 1594 | 1618, 1642 |
| Y33H, V101M, N106I, A112V | 1595 | 1619, 1643 |
| R12W, Y33H, N47K, Q79R, S118Y | 1596 | 1620, 1644 |
| Y33H, A46T, A112V, S118T | 1597 | 1621, 1645 |
| Y33H, A112V, F114L, S118T | 1598 | 1622, 1646 |
| Y33H, T38A, A46T, V101M, A112V | 1599 | 1623, 1647 |
| P28S, Y33H, S69P, N106I, A112V, S118Y | 1600 | 1624, 1648 |
| Y33H, P42L, N47K, V101M, A112V | 1601 | 1625, 1649 |
| Y33H, N47K, F74S, Q83K, N106I, F111L, A112V, S118T | 1602 | 1626, 1650 |
| Y33H, A112V, S118T, V119A | 1603 | 1627, 1651 |
| Y33H, N106I, A112V, S118F | 1604 | 1628, 1652 |
| Y33H, K66M, S118F, W124L | 1605 | 1629, 1653 |
| N106I, A112V | 1606 | 1630, 1654 |

In some embodiments, the immunomodulatory protein contains at least one affinity modified IgSF domain containing one or more amino acid substitutions in a wild-type or unmodified IgSF domain of PD-L1 or PD-L2. In some embodiments, the affinity modified IgSF domain of the immunomodulatory protein has at least about 85%, 86%, 86%, 88%, 89%, 90%, 91%, 92%, 93%, 94%, 95%, 96%, 97%, 98%, or 99% sequence identity with the wild-type or unmodified IgSF domain or specific binding fragment thereof of PD-L1 or PD-L2, such as an IgSF domain (e.g. IgV or IgC) contained in the IgSF protein set forth in SEQ ID NO:3 or 4. Exemplary affinity modified IgSF domains of PD-L1 are set forth in Table 8. Exemplary affinity modified IgSF domains of PD-L2 are set forth in Table 9. In some embodiments, the affinity modified IgSF domain of PD-L1 or PD-L2 exhibits increased binding affinity or increased binding selectivity to PD-1.

| **TABLE 8: Exemplary variant PD-L1 polypeptides** | | |
|---|---|---|
| **Mutation(s)** | **ECD SEQ ID NO** | **IgV SEQ ID NO** |
| Wild-type | 30, 1874 | 1136, 1332 |
| K28N/M41V/N45T/H51N/K57E | 1137, 2087 | 1202, 1267 |
| I20L/I36T/N45D/I47T | 1138, 2088 | 1203, 1268 |
| I20L/M41K/K44E | 1139, 2089 | 1204, 1269 |
| P6S/N45T/N78I/I83T | 1140, 2090 | 1205, 1270 |
| N78I | 1141, 2091 | 1206, 1271 |
| M41K/N78I | 1142, 2092 | 1207, 1272 |
| N45T/N78I | 1143, 2093 | 1208, 1273 |
| I20L/N45T | 1144, 2094 | 1209, 1274 |
| N45T | 1145, 2095 | 1210, 1275 |
| M41K | 1146, 2096 | 1211, 1276 |
| I20L/I36T/N45D | 1147, 2097 | 1212, 1277 |
| N17D/N45T/V50A/D72G | 1148, 2098 | 1213, 1278 |
| I20L/F49S | 1149, 2099 | 1214, 1279 |
| N45T/V50A | 1150, 2100 | 1215, 1280 |
| I20L/N45T/N78I | 1151, 2101 | 1216, 1281 |
| I20L/N45T/V50A | 1152, 2102 | 1217, 1282 |
| M41V/N45T | 1153, 2103 | 1218, 1283 |
| M41K/N45T | 1154, 2104 | 1219, 1284 |
| A33D/S75P/D85E | 1155, 2105 | 1220, 1285 |
| M18I/M41K/D43G/H51R/N78I | 1156, 2106 | 1221, 1286 |
| VI 1E/I20L/I36T/N45D/H60R/S75P | 1157, 2107 | 1222, 1287 |
| A33D/V50A | 1158, 2108 | 1223, 1288 |
| S16G/A33D/K71E/S75P | 1159, 2109 | 1224, 1289 |
| E27G/N45T/M97I | 1160, 2110 | 1225, 1290 |
| E27G/N45T/K57R | 1161, 2111 | 1226, 1291 |
| A33D/E53V | 1162, 2112 | 1227, 1292 |
| D43G/N45D/V58A | 1163, 2113 | 1228, 1293 |
| E40G/D43V/N45T/V50A | 1164, 2114 | 1229, 1294 |
| Y14S/K28E/N45T | 1165, 2115 | 1230, 1295 |
| A33D/N78S | 1166, 2116 | 1231, 1296 |
| A33D/N78I | 1167, 2117 | 1232, 1297 |
| A33D/N45T | 1168, 2118 | 1233, 1298 |
| A33D/N45T/N78I | 1169, 2119 | 1234, 1299 |
| E27G/N45T/V50A | 1170, 2120 | 1235, 1300 |
| N45T/V50A/N78S | 1171, 2121 | 1236, 1301 |
| I20L/N45T/V110M | 1172, 2122 | 1237, 1302 |
| I20L/I36T/N45T/V50A | 1173, 2123 | 1238, 1303 |
| N45T/L74P/S75P | 1174, 2124 | 1239, 1304 |
| N45T/S75P | 1175, 2125 | 1240, 1305 |
| S75P/K106R | 1176, 2126 | 1241, 1306 |
| S75P | 1177, 2127 | 1242, 1307 |
| A33D/S75P | 1178, 2128 | 1243, 1308 |
| A33D/S75P/D104G | 1179, 2129 | 1244, 1309 |
| A33D/S75P | 1180, 2130 | 1245, 1310 |
| I20L/E27G/N45T/V50A | 1181, 2131 | 1246, 1311 |
| I20L/E27G/D43 G/N45D/V 5 8A/N78I | 1182, 2132 | 1247, 1312 |
| I20L/D43 G/N45D/V58A/N78I | 1183,2133 | 1248, 1313 |
| I20L/A33D/D43G/N45D/V58A/N78I | 1184, 2134 | 1249, 1314 |
| I20L/D43G/N45D/N78I | 1185, 2135 | 1250, 1315 |
| E27G/N45 T/V50A/N78I | 1186, 2136 | 1251, 1316 |
| N45T/V50A/N78I | 1187, 2137 | 1252, 1317 |
| V11A/I20LÆ27G/D43G/N45D/H51Y/S99G | 1188, 2138 | 1253, 1318 |
| I20L/E27G/D43G/N45T/V50A | 1189, 2139 | 1254, 1319 |
| I20L/K28E/D43G/N45D/V58A/Q89R/G 101 G-ins (G101GG) | 1190, 2140 | 1255, 1320 |
| I20L/I36T/N45D | 1191, 2141 | 1256, 1321 |
| I20L/K28E/D43G/N45D/E53G/V58A/N78I | 1192, 2142 | 1257, 1322 |
| A33D/D43G/N45D/V58A/S75P | 1193, 2143 | 1258, 1323 |
| K23R/D43G/N45D | 1194, 2144 | 1259, 1324 |
| I20L/D43G/N45D/V58A/N78I/D90G/G101D | 1195, 2145 | 1260, 1325 |
| D43G/N45D/L56Q/V58A/G101G-ins(G101GG) | 1196, 2146 | 1261, 1326 |
| I20L/K23E/D43 G/N45D/V 5 8A/N78I | 1197, 2147 | 1262, 1327 |
| I20L/K23E/D43G/N45D/V50A/N78I | 1198, 2148 | 1263, 1328 |
| T19I/E27G/N45I/V50A/N78I/M97K | 1199, 2149 | 1264, 1329 |
| I20L/M41K/D43G/N45D | 1200, 2150 | 1265, 1330 |
| K23R/N45T/N78I | 1201, 2151 | 1266, 1331 |
| I20L/K28E/D43G/N45D/V58A/Q89R/G 101 G-ins (G101GG) | 1871, 2152 | 1754, 1755 |
| K57R/S99G | 1875, 1965 | 2054, 2069 |
| K57R/S99G/F189L | 1876, 1966 | |
| M18V/M97L/F193S/R195G/E200K/H202Q | 1877, 1967 | |
| I36S/M41K/M97L/K144Q/R195G/E200K/H202Q/L206F | 1878, 1968 | |
| C22R/Q65L/L124S/K144Q/R195G/E200N/H202Q/T221L | 1879 | |
| M18V/I98L/L124S/P198T/L206F | 1880, 1969 | |
| S99G/N117S/I148V/K171R/R180S | 1881, 1970 | |
| I36T/M97L/A103V/Q155H | 1882, 1971 | |
| K28I/S99G | 1883, 1972 | 2055, 2070 |
| R195S | 1884, 1973 | |
| A79T/S99G/T185A/R195G/E200K/H202Q/L206F | 1885, 1974 | |
| K57R/S99G/L124S/K144Q | 1886, 1975 | |
| K57R/S99G/R195G | 1887, 1976 | |
| D55V/M97L/S99G | 1888, 1977 | 2056, 2071 |
| E27G/I36T/D55N/M97L/K111E | 1889, 1978 | 2057, 2072 |
| E54G/M97L/S99G | 1890, 1979 | 2058, 2073 |
| G15A/I36T/M97L/K111E/H202Q | 1891, 1980 | |
| G15A/I36T/V129D | 1892, 1981 | |
| G15A/I36T/V129D/R195G | 1893, 1982 | |
| G15A/V129D | 1894, 1983 | |
| I36S/M97L | 1895, 1984 | 2059, 2074 |
| I36T/D55N/M97L/K111E/A204T | 1896, 1985 | |
| I36T/D55N/M97L/K111E/V129A/F173L | 1897, 1986 | |
| I36T/D55S/M97L/K111E/I148V/R180S | 1898, 1987 | |
| I36T/G52R/M97L/V112A/K144E/V175A/P198T | 1899, 1988 | |
| I36T/I46V/D55G/M97L/K106E/K144E/T185A/R195G | 1900, 1989 | |
| I36T/I83T/M97L/K144E/P198T | 1901, 1990 | |
| I36T/M97L/K111E | 1902, 1991 | 2060, 2075 |
| I36T/M97L/K144E/P198T | 1903, 1992 | |
| 13 6T/M97L/Q155H/F193S/N201 Y | 1904, 1993 | |
| I36T/M97L/V129D | 1905, 1994 | |
| L35P/I36S/M97L/K111E | 1906, 1995 | 2061, 2076 |
| M18I/I36T/E53G/M97L/K144E/E199G/V207A | 1907, 1996 | |
| M18T/I36T/D55N/M97L/K1 1 IE | 1908, 1997 | 2062, 2077 |
| M18V/M97L/T176N/R195G | 1909, 1998 | |
| M97L/S99G | 1910, 1999 | 2063, 2078 |
| N17D/M97L/S99G | 1911, 2000 | 2064, 2079 |
| S99G/T185A/R195G/P198T | 1912, 2001 | |
| V129D/H202Q | 1913, 2002 | |
| V129D/P198T | 1914, 2003 | |
| V129D/T150A | 1915, 2004 | |
| V93E/V129D | 1916, 2005 | |
| Y10F/M18V/S99G/Q138R/T203A | 1917, 2006 | |
| N45D | 1918, 2007 | 2065, 2080 |
| K160M/R195G | 1919, 2008 | |
| N45D/K144E | 1920, 2009 | |
| N45D/P198S | 1921, 2010 | |
| N45D/P198T | 1922, 2011 | |
| N45D/R195G | 1923, 2012 | |
| N45D/R195S | 1924, 2013 | |
| N45D/S131F | 1925, 2014 | |
| N45D/V58D | 1926, 2015 | 2066, 2081 |
| V129D/R195S | 1927, 2016 | |
| I98T/F173Y/L196S | 1928, 2017 | |
| N45D/E134G/L213P | 1929, 2018 | |
| N45D/F173I/S177C | 1930, 2019 | |
| N45D/I148V/R195G | 1931, 2020 | |
| N45D/K111T/R195G | 1932, 2021 | |
| N45D/N113Y/R195S | 1933, 2022 | |
| N45D/N165Y/E170G | 1934, 2023 | |
| N45D/Q89R/I98V | 1935, 2024 | 2067, 2082 |
| N45D/S131F/P198S | 1936, 2025 | |
| N45D/S75P/P198S | 1937, 2026 | |
| N45D/V50A/R195T | 1938, 2027 | |
| E27D/N45D/T183A/I188V | 1939, 2028 | |
| F173Y/T183I/L196S/T203A | 1940, 2029 | |
| K23N/N45D/S75P/N120S | 1941, 2030 | |
| N45D/G102D/R194W/R195G | 1942, 2031 | |
| N45D/G52V/Q121L/P198S | 1943, 2032 | |
| N45D/I148V/R195G/N201D | 1944, 2033 | |
| N45D/K111T/T183A/I188V | 1945, 2034 | |
| N45D/Q89R/F189S/P198S | 1946, 2035 | |
| N45D/S99G/C137R/V207A | 1947, 2036 | |
| N45D/T163I/K167R/R195G | 1948, 2037 | |
| N45D/T183A/T192S/R194G | 1949, 2038 | |
| N45D/V50A/I119T/K144E | 1950, 2039 | |
| T19A/N45D/K144E/R195G | 1951, 2040 | |
| V11E/N45D/T130A/P198T | 1952, 2041 | |
| V26A/N45D/T163I/T185A | 1953, 2042 | |
| K23N/N45D/L124S/K167T/R195G | 1954, 2043 | |
| K23N/N45D/Q73R/T163I | 1955, 2044 | |
| K28E/N45D/W149R/S158G/P198T | 1956, 2045 | |
| K28R/N45D/K57E/I98V/R195S | 1957, 2046 | |
| K28R/N45D/V129D/T163N/R195T | 1958, 2047 | |
| M41K/D43G/N45D/R64S/R195G | 1959, 2048 | |
| M41K/D43G/N45D/R64S/S99G | 1960, 2049 | 2068, 2083 |
| N45D/R68L/F173L/D197G/P198S | 1961, 2050 | |
| N45D/V50A/I148V/R195G/N20 1D | 1962, 2051 | |
| M41K/D43G/K44E/N45D/R195G/N201D | 1963, 2052 | |
| N45D/V50A/L124S/K144E/L179P/R195G | 1964, 2053 | |

| **TABLE 9: Exemplary variant PD-L2 polypeptides** | | |
|---|---|---|
| **Mutation(s)** | **ECD SEQ ID NO** | **IgV SEQ ID NO** |
| Wild-type | 31 | 1333, 1393 |
| H15Q | 1334 | 1411, 1487 |
| N24D | 1335 | 1412, 1488 |
| E44D | 1336 | 1413, 1489 |
| V89D | 1337 | 1414, 1490 |
| Q82R/V89D | 1338 | 1415, 1491 |
| E59G/Q82R | 1339 | 1416, 1492 |
| S39I/V89D | 1340 | 1417, 1493 |
| S67L/V89D | 1341 | 1418, 1494 |
| S67L/I85F | 1342 | 1419, 1495 |
| S67L/I86T | 1343 | 1420, 1496 |
| H15Q/K65R | 1344 | 1421, 1497 |
| H15Q/Q72H/V89D | 1345 | 1422, 1498 |
| H15Q/S67L/R76G | 1346 | 1423, 1499 |
| H15Q/R76G/I85F | 1347 | 1424, 1500 |
| H15Q/T47A/Q82R | 1348 | 1425, 1501 |
| H15Q/Q82R/V89D | 1349 | 1426, 1502 |
| H15Q/C23 S/I86T | 1350 | 1427, 1503 |
| H15Q/S39I/I86T | 1351 | 1428, 1504 |
| H15Q/R76G/I85F | 1352 | 1429, 1505 |
| E44D/V89D/W91R | 1353 | 1430, 1506 |
| I13V/S67L/V89D | 1354 | 1431, 1507 |
| H15Q/S67L/I86T | 1355 | 1432, 1508 |
| I13V/H15Q/S67L/I86T | 1356 | 1433, 1509 |
| I13V/H15Q/E44D/V89D | 1357 | 1434, 1510 |
| I13V/S39I/E44D/Q82R/V89D | 1358 | 1435, 1511 |
| I13V/E44D/Q82R/V89D | 1359 | 1436, 1512 |
| I13V/Q72H/R76G/I86T | 1360 | 1437, 1513 |
| I13V/H15Q/R76G/I85F | 1361 | 1438, 1514 |
| H15Q/S39I/R76G/V89D | 1362 | 1439, 1515 |
| H15Q/S67L/R76G/I85F | 1363 | 1440, 1516 |
| H15Q/T47A/Q72H/R76G/I86T | 1364 | 1441, 1517 |
| H15Q/T47A/Q72H/R76G | 1365 | 1442, 1518 |
| I13V/H15Q/T47A/Q72H/R76G | 1366 | 1443, 1519 |
| H15Q/E44D/R76G/I85F | 1367 | 1444, 1520 |
| H15Q/S39I/S67L/V89D | 1368 | 1445, 1521 |
| H15Q/N32D/S67L/V89D | 1369 | 1446, 1522 |
| N32D/S67L/V89D | 1370 | 1447, 1523 |
| H15Q/S67L/Q72H/R76G/V89D | 1371 | 1448, 1524 |
| H15Q/Q72H/Q74R/R76G/I86T | 1372 | 1449, 1525 |
| G28V/Q72H/R76G/I86T | 1373 | 1450, 1526 |
| I13V/H15Q/S39I/E44D/S67L | 1374 | 1451, 1527 |
| E44D/S67L/Q72H/Q82R/V89D | 1375 | 1452, 1528 |
| H15Q/V89D | 1376 | 1453, 1529 |
| H15Q/T47A | 1377 | 1454, 1530 |
| I13V/H15Q/Q82R | 1378 | 1455, 1531 |
| I13V/H15Q/V89D | 1379 | 1456, 1532 |
| I13V/S67L/Q82R/V89D | 1380 | 1457, 1533 |
| I13V/H15Q/Q82R/V89D | 1381 | 1458, 1534 |
| H15Q/V31M/S67L/Q82R/V89D | 1382 | 1459, 1535 |
| I13V/H15Q/T47A/Q82R | 1383 | 1460, 1536 |
| I13V/H15Q/V31A/N45S/Q82R/V89D | 1384 | 1461, 1537 |
| I13V/T20A/T47A/K65X/Q82R/V89D | 1385 | 1462, 1538 |
| H1 5Q/T47A/H69L/Q82R/V89D | 1386 | 1463, 1539 |
| I13V/H15Q/T47A/H69L/R76G/V89D | 1387 | 1464, 1540 |
| I12V/I13V/H15Q/T47A/Q82R/V89D | 1388 | 1465, 1541 |
| I13V/H15Q/R76G/D77N/Q82R/V89D | 1389 | 1466, 1542 |
| I13V/H15Q/T47A/R76G/V89D | 1390 | 1467, 1543 |
| I13V/H15Q/T47A/Q82R/V89D | 1391 | 1468, 1544 |
| I13V/H15Q/N24D/Q82R/V89D | 1392 | 1469, 1545 |
| I13V/H15Q/I36V/T47A/S67L/V89D | 1394 | 1470, 1546 |
| H15Q/T47A/K65R/S67L/Q82R/V89D | 1395 | 1471, 1547 |
| H15Q/L33P/T47A/S67L/P71 S/V89D | 1396 | 1472, 1548 |
| I13V/H15Q/Q72H/R76G/I86T | 1397 | 1473, 1549 |
| H15Q/T47A/S67L/Q82R/V89D | 1398 | 1474, 1550 |
| F2L/H15Q/D46E/T47A/Q72H/R76G/Q82R/V89D | 1399 | 1475, 1551 |
| I13V/H15Q/L33F/T47A/Q82R/V89D | 1400 | 1476, 1552 |
| I13V/H15Q/T47A/E58G/S67L/Q82R/V89D | 1401 | 1477, 1553 |
| H15Q/N24S/T47A/Q72H/R76G/V89D | 1402 | 1478, 1554 |
| I13V/H15Q/E44V/T47A/Q82R/V89D | 1403 | 1479, 1555 |
| H15Q/N18D/T47A/Q72H/V73A/R76G/I86T/V89D | 1404 | 1480, 1556 |
| I13V/H15Q/T37A/E44D/S48C/S67L/Q82R/V89D | 1405 | 1481, 1557 |
| H15Q/L33H/S67L/R76G/Q82R/V89D | 1406 | 1482, 1558 |
| I13V/H15Q/T47A/Q72H/R76G/I86T | 1407 | 1483, 1559 |
| H15Q/S39I/E44D/Q72H/V75G/R76G/Q82R/V89D | 1408 | 1484, 1560 |
| H15Q/T47A/S67L/R76G/Q82R/V89D | 1409 | 1485, 1561 |
| I13V/H15Q/T47A/S67L/Q72H/R76G/Q82R/V89D | 1410 | 1486, 1562 |

In some embodiments, the immunomodulatory protein contains an affinity modified IgSF domain containing one or more amino acid substitutions in a wild-type or unmodified IgSF domain of an NKp30 family member. In some embodiments, the affinity modified IgSF domain has at least about 85%, 86%, 86%, 88%, 89%, 90%, 91%, 92%, 93%, 94%, 95%, 96%, 97%, 98%, or 99% sequence identity with the wild-type or unmodified IgSF domain or specific binding fragment thereof of an NKp30 family member, such as the IgSF domain (e.g. IgC) contained in the IgSF protein set forth in SEQ ID NO: 27. Table 10 provides exemplary affinity modified NKp30 IgSF domains.

| **TABLE 10: Exemplary variant NKp30 polypeptides** | | |
|---|---|---|
| **Mutation(s)** | **ECD SEQ ID NO** | **IgC-like domain SEQ ID NO** |
| Wild-type | 54 | 214 |
| L30V/A60V/S64P/S86G | 143 | 215 |
| L30V | 144 | 216 |
| A60V | 145 | 217 |
| S64P | 146 | 218 |
| S86G | 147 | 219 |

### IV. SECRETABLE IMMUNOMODULATORY PROTEINS AND ENGINEERED CELLS

Provided herein are immunomodulatory proteins, such as any described above, which can be expressed and secreted by engineered cells. Secretable immunomodulatory proteins (SIPs), and engineered cells expressing and secreting such secretable immunomodulatory proteins, can have therapeutic utility by modulating immunological activity in a mammal with a disease or disorder in which modulation of the immune system response is beneficial.

In some embodiments, the immunomodulatory protein comprises at least one non-immunoglobulin affinity-modified immunoglobulin superfamily (IgSF) domain comprising one or more amino acid substitutions in a wild-type IgSF domain, wherein the at least one affinity-modified IgSF domain specifically binds at least one cell surface cognate binding partner of the wild-type IgSF domain. In some embodiments, the affinity-modified IgSF domain can include an affinity-modified IgSF domain of an Ig super family member, such as any described herein. In some embodiments, the affinity-modified IgSF domain is in an IgSF family member of the B7 family of proteins. In some embodiments, the affinity-modified IgSF domain is in an IgSF family member that binds an inhibitory receptor. In some embodiments, the affinity-modified IgSF domain comprises any one or more amino acid substitution in an IgSF domain, such as any one or more amino acid substitution(s) set forth in any of Tables 2-10.

In some embodiments, the immunomodulatory protein does not comprise a transmembrane domain. In some embodiments, the immunomodulatory protein is not conjugated to a half-life extending moiety (such as an Fc domain or a multimerization domain). In some embodiments, the immunomodulatory protein comprises a signal peptide, e.g. an antibody signal peptide or other efficient signal sequence to get domains outside of cell. When the immunomodulatory protein comprises a signal peptide and is expressed by an engineered cell, the signal peptide causes the immunomodulatory protein to be secreted by the engineered cell. Generally, the signal peptide, or a portion of the signal peptide, is cleaved from the immunomodulatory protein with secretion. The immunomodulatory protein can be encoded by a nucleic acid (which can be part of an expression vector). In some embodiments, the immunomodulatory protein is expressed and secreted by a cell (such as an immune cell, for example a primary immune cell).

### A. Signal Peptide

In some embodiments, the immunomodulatory proteins provided herein further comprises a signal peptide. In some embodiments, provided herein is a nucleic acid molecule encoding the immunomodulatory protein operably connected to a secretion sequence encoding the signal peptide.

A signal peptide is a sequence on the N-terminus of an immunomodulatory protein that signals secretion of the immunomodulatory protein from a cell. In some embodiments, the signal peptide is about 5 to about 40 amino acids in length (such as about 5 to about 7, about 7 to about 10, about 10 to about 15, about 15 to about 20, about 20 to about 25, or about 25 to about 30, about 30 to about 35, or about 35 to about 40 amino acids in length).

In some embodiments, the signal peptide is a native signal peptide from the corresponding wild-type IgSF family member. For example, in some embodiments, the immunomodulatory protein comprises at least one non-immunoglobulin affinity modified immunoglobulin superfamily (IgSF) domain comprising one or more amino acid substitutions in a wild-type IgSF domain from a wild-type IgSF family member, and a signal peptide from the wild-type IgSF family member. Exemplary signal peptides from IgSF family members are identified in Table 1.

In some embodiments, the signal peptide is a non-native signal peptide. For example, in some embodiments, the non-native signal peptide is a mutant native signal peptide from the corresponding wild-type IgSF family member, and can include one or more (such as 2, 3, 4, 5, 6, 7, 8, 9, or 10 or more) substitutions insertions or deletions. In some embodiments, the non-native signal peptide is a signal peptide or mutant thereof of a family member from the same IgSF family as the wild-type IgSF family member. In some embodiments, the non-native signal peptide is a signal peptide or mutant thereof from an IgSF family member from a different IgSF family that the wild-type IgSF family member. In some embodiments, the signal peptide is a signal peptide or mutant thereof from a non-IgSF protein family, such as a signal peptide from an immunoglobulin (such as IgG heavy chain or IgG-kappa light chain), a cytokine (such as interleukin-2 (IL-2), or CD33), a serum albumin protein (e.g. HSA or albumin), a human azurocidin preprotein signal sequence, a luciferase, a trypsinogen (e.g. chymotrypsinogen or trypsinogen) or other signal peptide able to efficiently secrete a protein from a cell. Exemplary signal peptides include any described in the Table 11.

| **TABLE 11. Exemplary Signal Peptides** | | |
|---|---|---|
| **SEQ ID NO** | **Signal Peptide** | **Peptide Sequence** |
| SEQ ID NO: 413 | HSA signal peptide | MKWVTFISLLFLFSSAYS |
| SEQ ID NO: 414 | Ig kappa light chain | MDMRAPAGIFGFLLVLFPGYRS |
| SEQ ID NO: 415 | human azurocidin preprotein signal sequence | MTRLTVLALLAGLLAS SRA |
| SEQ ID NO: 416 | IgG heavy chain signal peptide | MELGLSWIFLLAILKGVQC |
| SEQ ID NO: 417 | IgG heavy chain signal peptide | MELGLRWVFLVAILEGVQC |
| SEQ ID NO: 418 | IgG heavy chain signal peptide | MKHLWFFLLLVAAPRWVLS |
| SEQ ID NO: 419 | IgG heavy chain signal peptide | MDWTWRILFLVAAATGAHS |
| SEQ ID NO: 420 | IgG heavy chain signal peptide | MDWTWRFLFVVAAATGVQS |
| SEQ ID NO: 421 | IgG heavy chain signal peptide | MEFGLSWLFLVAILKGVQC |
| SEQ ID NO: 422 | IgG heavy chain signal peptide | MEFGLSWVFLVALFRGVQC |
| SEQ ID NO: 423 | IgG heavy chain signal peptide | MDLLHKNMKHLWFFLLLVAA PRWVLS |
| SEQ ID NO: 424 | IgG Kappa light chain signal sequences: | MDMRVPAQLLGLLLLWLSGA RC |
| SEQ ID NO: 425 | IgG Kappa light chain signal sequences: | MKYLLPTAAAGLLLLAAQPAM A |
| SEQ ID NO: 426 | Gaussia luciferase | MGVKVLFALICIAVAEA |
| SEQ ID NO: 427 | Human albumin | MKWVTFISLLFLF S SAYS |
| SEQ ID NO: 428 | Human chymotrypsinogen | MAFLWLLSCWALLGTTFG |
| SEQ ID NO: 429 | Human interleukin-2 | MQLLSCIALILALV |
| SEQ ID NO: 430 | Human trypsinogen-2 | MNLLLILTFVAAAVA |

In some embodiments, the immunomodulatory protein comprises a signal peptide when expressed, and the signal peptide (or a portion thereof) is cleaved from the immunomodulatory protein upon secretion.

### B. Nucleic Acid Molecules and Expression Vectors

Provided herein are isolated or recombinant nucleic acids collectively referred to as "nucleic acids" which encode any of the various provided embodiments of the immunomodulatory polypeptides of the invention. In one aspect, there is provided a nucleic acid encoding an immunomodulatory protein comprising at least one non-immunoglobulin affinity-modified immunoglobulin superfamily (IgSF) domain comprising one or more amino acid substitutions in a wild-type IgSF domain, wherein the at least one affinity-modified IgSF domain specifically binds at least one cell surface cognate binding partner of the wild-type IgSF domain. In some embodiments, the immunomodulatory protein encoded by the nucleic acid molecule does not comprise a transmembrane domain. In some embodiments, the immunomodulatory protein encoded by the nucleic acid molecule does not comprise a half-life extending moiety (such as an Fc domain or a multimerization domain). In some embodiments, the immunomodulatory protein encoded by the nucleic acid molecule comprises a signal peptide. In some embodiments the nucleic acid molecule further comprises at least one promoter operably linked to control expression of the immunomodulatory protein.

Nucleic acids provided herein, including all described below, are useful in recombinant expression of the immunomodulatory proteins, including for engineering cells. The nucleic acids provided herein can be in the form of RNA or in the form of DNA, and include mRNA, cRNA, recombinant or synthetic RNA and DNA, and cDNA. The nucleic acids of the invention are typically DNA molecules, and usually double-stranded DNA molecules. However, single-stranded DNA, single-stranded RNA, double-stranded RNA, and hybrid DNA/RNA nucleic acids or combinations thereof comprising any of the nucleotide sequences of the invention also are provided.

Also provided herein are expression vectors useful in engineering cells to express the immunomodulatory proteins of the present invention. In one aspect, there is provided a recombinant expression vector comprising a nucleic acid encoding an immunomodulatory protein under the operable control of a signal sequence for secretion, wherein the immunomodulatory protein comprises at least one non-immunoglobulin affinity-modified immunoglobulin superfamily (IgSF) domain comprising one or more amino acid substitutions in a wild-type IgSF domain, wherein the at least one affinity-modified IgSF domain specifically binds at least one cell surface cognate binding partner of the wild-type IgSF domain. In some embodiments, the encoded immunomodulatory protein is secreted when expressed from a cell. In some embodiments, the immunomodulatory protein encoded by the nucleic acid molecule does not comprise a transmembrane domain. In some embodiments, the immunomodulatory protein encoded by the nucleic acid molecule does not comprise a half-life extending moiety (such as an Fc domain or a multimerization domain). In some embodiments, the immunomodulatory protein encoded by the nucleic acid molecule comprises a signal peptide.

The nucleic acids encoding the immunomodulatory polypeptides provided herein can be introduced into cells using recombinant DNA and cloning techniques. To do so, a recombinant DNA molecule encoding a immunomodulatory polypeptide is prepared. Methods of preparing such DNA molecules are well known in the art. For instance, sequences coding for the peptides could be excised from DNA using suitable restriction enzymes. Alternatively, the DNA molecule could be synthesized using chemical synthesis techniques, such as the phosphoramidite method. Also, a combination of these techniques could be used. In some instances, a recombinant or synthetic nucleic acid may be generated through polymerase chain reaction (PCR).

In some embodiments, a DNA insert can be generated encoding one or more affinity-modified IgSF domains and, in some embodiments, a signal peptide. This DNA insert can be cloned into an appropriate transduction/transfection vector as is known to those of skill in the art. Also provided are expression vectors containing the nucleic acid molecules.

In some embodiments, the expression vectors are capable of expressing the immunomodulatory proteins in an appropriate cell under conditions suited to expression of the protein. In some aspects, nucleic acid molecule or an expression vector comprises the DNA molecule that encodes the immunomodulatory protein operatively linked to appropriate expression control sequences. Methods of affecting this operative linking, either before or after the DNA molecule is inserted into the vector, are well known. Expression control sequences include promoters, activators, enhancers, operators, ribosomal binding sites, start signals, stop signals, cap signals, polyadenylation signals, and other signals involved with the control of transcription or translation. In some embodiments, a nucleic acid of the invention further comprises nucleotide sequence that encodes a secretory or signal peptide operably linked to the nucleic acid encoding the immunomodulatory protein, thereby allowing for secretion of the immunomodulatory protein.

In some embodiments, expression of the immunomodulatory protein is controlled by a promoter to enhance to control or regulate expression. The promoter is operably linked to the portion of the nucleic acid molecule encoding the immunomodulatory protein. In some embodiments, the promotor is a constitutively active promotor (such as a tissue-specific constitutively active promotor or other constitutive promotor). In some embodiments, the promotor is an inducible promotor, which may be responsive to an inducing agent (such as a T cell activation signal).

In some embodiments, a constitutive promoter is operatively linked to the nucleic acid molecule encoding the immunomodulatory protein. Exemplary constitutive promoters include the Simian vacuolating virus 40 (SV40) promoter, the cytomegalovirus (CMV) promoter, the ubiquitin C (UbC) promoter, and the EF-1 alpha (EF1a) promoter. In some embodiments, the constitutive promoter is tissue specific. For example, in some embodiments, the promoter allows for constitutive expression of the immunomodulatory protein in specific tissues, such as immune cells, lymphocytes, or T cells. Exemplary tissue-specific promoters are described in U.S. Patent No. 5,998,205, including, for example, a fetoprotein, DF3, tyrosinase, CEA, surfactant protein, and ErbB2 promoters.

In some embodiments, an inducible promoter is operatively linked to the nucleic acid molecule encoding the immunomodulatory protein such that expression of the nucleic acid is controllable by controlling the presence or absence of the appropriate inducer of transcription. For example, the promoter can be a regulated promoter and transcription factor expression system, such as the published tetracycline-regulated systems or other regulatable systems (see, e.g. published International PCT Appl. No. WO 01/30843), to allow regulated expression of the encoded polypeptide. An exemplary regulatable promoter system is the Tet-On (and Tet-Off) system available, for example, from Clontech (Palo Alto, CA). This promoter system allows the regulated expression of the transgene controlled by tetracycline or tetracycline derivatives, such as doxycycline. Other regulatable promoter systems are known (see e.g., published U.S. Application No. 2002-0168714, entitled "Regulation of Gene Expression Using Single-Chain, Monomeric, Ligand Dependent Polypeptide Switches," which describes gene switches that contain ligand binding domains and transcriptional regulating domains, such as those from hormone receptors).

In some embodiments, the promotor is responsive to an element responsive to T-cell activation signaling. Solely by way of example, in some embodiments, an engineered T cell comprises an expression vector encoding the immunomodulatory protein and a promotor operatively linked to control expression of the immunomodulatory protein. The engineered T cell can be activated, for example by signaling through an engineered T cell receptor (TCR) or a chimeric antigen rector (CAR), and thereby triggering expression and secretion of the immunomodulatory protein through the responsive promotor.

In some embodiments, an inducible promoter is operatively linked to the nucleic acid molecule encoding the immunomodulatory protein such that the immunomodulatory protein is expressed in response to a nuclear factor of activated T-cells (NFAT) or nuclear factor kappa-light-chain enhancer of activated B cells (NF-κB). For example, in some embodiments, the inducible promoter comprises a binding site for NFAT or NF-κB For example, in some embodiments, the promoter is an NFAT or NF-κB promoter or a functional variant thereof. Thus, in some embodiments, the nucleic acids make it possible to control the expression of immunomodulatory protein while also reducing or eliminating the toxicity of the immunomodulatory protein. In particular, engineered immune cells comprising the nucleic acids of the invention express and secrete the immunomodulatory protein only when the cell (e.g., a T-cell receptor (TCR) or a chimeric antigen receptor (CAR) expressed by the cell) is specifically stimulated by an antigen and/or the cell (e.g., the calcium signaling pathway of the cell) is non-specifically stimulated by, e.g., phorbol myristate acetate (PMA)/Ionomycin. Accordingly, the expression and secretion of immunomodulatory protein can be controlled to occur only when and where it is needed (e.g., in the presence of an infectious disease-causing agent, cancer, or at a tumor site), which can decrease or avoid undesired immunomodulatory protein interactions.

In some embodiments, the nucleic acid encoding an immunomodulatory protein described herein comprises a suitable nucleotide sequence that encodes a NFAT promoter, NF-κB promoter, or a functional variant thereof. "NFAT promoter" as used herein means one or more NFAT responsive elements linked to a minimal promoter. "NF-κB promoter" refers to one or more NF-κB responsive elements linked to a minimal promoter. In some embodiments, the minimal promoter of a gene is a minimal human IL-2 promoter or a CMV promoter.
The NFAT responsive elements may comprise, e.g., NFAT1, NFAT2, NFAT3, and/or NFAT4 responsive elements. The NFAT promoter, NF-κB promoter, or a functional variant thereof may comprise any number of binding motifs, e.g., at least two, at least three, at least four, at least five, or at least six, at least seven, at least eight, at least nine, at least ten, at least eleven, or up to twelve binding motifs.

In some embodiments, the resulting expression vector having the DNA molecule thereon is used to transform, such as transduce, an appropriate cell. The introduction can be performed using methods well known in the art. Exemplary methods include those for transfer of nucleic acids encoding the receptors, including via viral, e.g., retroviral or lentiviral, transduction, transposons, and electroporation. In some embodiments, the expression vector is a viral vector. In some embodiments, the nucleic acid is transferred into cells by lentiviral or retroviral transduction methods.

### C. Exemplary Immunomodulatory Proteins and Encoding Nucleic Acid Molecules

Provided herein is a immunomodulatory protein, e.g. secretable immunomodulatory protein, in accord with the above description that comprises a sequence of amino acids that exhibits at least 85%, 86%, 87%, 88%, 89%, 90%, 91%, 92%, 93%, 94%, 95%, 96%, 97%, 98% or 99% sequence identity to any of SEQ ID NOS: 28-54 and 410 or to a specific binding fragment thereof containing an IgSF domain (e.g. IgV domain or IgC domain), and that contains at least one affinity-modified IgSF domain as described containing one or more amino acid modifications, e.g. amino acid substitutions, in an IgSF domain. In some embodiments, the immunomodulatory protein e.g. secretable immunomodulatory protein, comprises a sequence of amino acids that comprises the IgV domain or a specific fragment thereof contained within the sequence of amino acids set forth in any of SEQ ID NOS: 28-54 and 410 (see e.g. Table 1) but in which is contained therein one or more amino acid modifications, e.g. amino acid substitutions, such that the IgV domain exhibits at least 85%, 86%, 87%, 88%, 89%, 90%, 91%, 92%, 93%, 94%, 95%, 96%, 97%, 98% or 99% sequence identity to the corresponding IgV domain contained in any of SEQ ID NOS: 28-54 and 410. In some embodiments, the immunomodulatory protein contains 1, 2, 3, 4, 5, 6, 7, 8, 9, 10, 11, 12, 13, 14, 15, 16, 17, 18, 19, 20 or more amino acid modifications, e.g. amino acid substitutions. In some embodiments, the immunomodulatory protein further comprises a signal peptide as described. In some embodiments, the signal peptide is the native signal peptide of the corresponding wild-type IgSF member (see e.g. Table 1). In some embodiments, the signal peptide is a non-native signal peptide, such as any as described, e.g. Table 11.

Also provided herein is a nucleic acid molecule encoding an immunomodulatory protein comprising a nucleotide sequence that encodes a sequence of amino acids that exhibits at least 85%, 86%, 87%, 88%, 89%, 90%, 91%, 92%, 93%, 94%, 95%, 96%, 97%, 98% or 99% sequence identity to any of SEQ ID NOS: 28-54 and 410 or to a specific binding fragment thereof containing an IgSF domain (e.g. IgV domain or IgC domain) and that contains at least one affinity-modified IgSF domain as described containing one or more amino acid modifications, e.g. amino acid substitutions. In some embodiments, the immunomodulatory protein e.g. secretable immunomodulatory protein, comprises a nucleotide sequence that encodes a sequence of amino acids that comprises the IgV domain or a specific fragment thereof contained within the sequence of amino acids set forth in any of SEQ ID NOS: 28-54 and 410 (see e.g. Table 1) but in which is contained therein one or more amino acid modifications, e.g. amino acid substitutions, such that the IgV domain exhibits at least 85%, 86%, 87%, 88%, 89%, 90%, 91%, 92%, 93%, 94%, 95%, 96%, 97%, 98% or 99% sequence identity to the corresponding IgV domain contained in any of SEQ ID NOS: 28-54 and 410. In some embodiments, the nucleic acid molecule further comprises a sequence of nucleotides encoding a signal peptide. In some embodiments, the signal peptide is the native signal peptide of the corresponding wild-type IgSF member (see e.g. Table 1). In some embodiments, the signal peptide is a non-native signal peptide, such as any as described, e.g. Table 11.

In some embodiments, the immunomodulatory protein has the sequence of amino acids set forth in any of the SEQ ID NOS of an ECD or an IgV set forth in any of Tables 2-10, or a sequence of amino acids that exhibits at least 85%, 86%, 87%, 88%, 89%, 90%, 91%, 92%, 93%, 94%, 95%, 96%, 97%, 98% or 99% to any of the SEQ ID NOS of an ECD or an IgV set forth in any of Tables 2-10 and that contains the recited amino acid modifications (e.g. amino acid substitutions). In some embodiments, the immunomodulatory protein further comprises a signal peptide as described. In some embodiments, the signal peptide is the native signal peptide of the corresponding wild-type IgSF member (see e.g. Table 1). In some embodiments, the signal peptide is a non-native signal peptide, such as any as described, e.g. Table 11.

In some embodiments, the nucleic acid molecule encodes an immunomodulatory protein that has the sequence of amino acids set forth in any of the SEQ ID NOS of an ECD or an IgV set forth in any of Tables 2-10, or a sequence of amino acids that exhibits at least 85%, 86%, 87%, 88%, 89%, 90%, 91%, 92%, 93%, 94%, 95%, 96%, 97%, 98% or 99% to any of the SEQ ID NOS of an ECD or an IgV set forth in any of Tables 2-10 and that contains the recited amino acid modifications (e.g. amino acid substitutions). In some embodiments, the nucleic acid molecule further comprises a sequence of nucleotides encoding a signal peptide. In some embodiments, the signal peptide is the native signal peptide of the corresponding wild-type IgSF member (see e.g. Table 1). In some embodiments, the signal peptide is a non-native signal peptide, such as any as described, e.g. Table 11.

### V. TRANSMEMBRANE IMMUNOMODULATORY PROTEIN

Provided herein are immunomodulatory proteins that are transmembrane proteins ("transmembrane immunomodulatory proteins"). Transmembrane immunomodulatory proteins, and engineered cells expressing such transmembrane immunomodulatory proteins, generally have therapeutic utility by modulating immunological activity in a mammal with a disease or disorder in which modulation of the immune system response is beneficial. A transmembrane immunomodulatory protein of the present invention comprises an ectodomain, a transmembrane, and in some embodiments, an endodomain, such as a cytoplasmic signaling domain.

### A. Ectodomain

In some embodiments, the provided transmembrane immunomodulatory proteins include an ectodomain comprising at least one affinity modified IgSF domain compared to an IgSF domain of a wild-type mammalian IgSF member. In some embodiments, the immunomodulatory protein comprises at least one non-immunoglobulin affinity-modified immunoglobulin superfamily (IgSF) domain comprising one or more amino acid substitutions in a wild-type IgSF domain, wherein the at least one affinity-modified IgSF domain specifically binds at least one cell surface cognate binding partner of the wild-type IgSF domain. In some embodiments, the affinity-modified IgSF domain can include an affinity-modified IgSF domain of an Ig super family member, such as any described herein In some embodiments, the affinity-modified IgSF domain is in an IgSF family member of the B7 family of proteins. In some embodiments, the affinity-modified IgSF domain is in an IgSF family member that binds an inhibitory receptor. In some embodiments, the affinity-modified IgSF domain comprises any one or more amino acid substitution in an IgSF domain described herein, such as any one or more amino acid substitution(s) set forth in any of Tables 2-10.

### B. Transmembrane Domain

The transmembrane immunomodulatory proteins provided herein further contain a transmembrane domain linked to the ectodomain. In some embodiments, the transmembrane domain results in an encoded protein for cell surface expression on a cell. In some embodiments, the transmembrane domain is linked directly to the ectodomain. In some embodiments, the transmembrane domain is linked indirectly to the ectodomain via one or more linkers or spacers. In some embodiments, the transmembrane domain contains predominantly hydrophobic amino acid residues, such as leucine and valine.

In some embodiments, a full length transmembrane anchor domain can be used to ensure that the TIPs will be expressed on the surface of the engineered cell, such as engineered T cell. Conveniently, this could be from a particular native protein that is being affinity modified (e.g. CD80 or ICOSL or other native IgSF protein), and simply fused to the sequence of the first membrane proximal domain in a similar fashion as the native IgSF protein (e.g. CD80 or ICOSL). In some embodiments, the transmembrane immunomodulatory protein comprises a transmembrane domain of the corresponding wild-type or unmodified IgSF member, such as a transmembrane domain contained in the sequence of amino acids set forth in any of SEQ ID NOs: 1-27 and 408 (see Table 1).

In some embodiments, the transmembrane domain is a non-native transmembrane domain that is not the transmembrane domain of the wild-type IgSF member. In some embodiments, the transmembrane domain is derived from a transmembrane domain from another non-IgSF family member polypeptide that is a membrane-bound or is a transmembrane protein. In some embodiments, a transmembrane anchor domain from another protein on T cells can be used. In some embodiments, the transmembrane domain is derived from CD8. In some embodiments, the transmembrane domain can further contain an extracellular portion of CD8 that serves as a spacer domain. An exemplary CD8 derived transmembrane domain is set forth in SEQ ID NO: 1574 or a portion thereof containing the CD8 transmembrane domain. In some embodiments, the transmembrane domain is a synthetic transmembrane domain.

### C. Endodomain

In some embodiments, the transmembrane immunomodulatory protein further contains an endodomain, such as a cytoplasmic signaling domain, linked to the transmembrane domain. In some embodiments, the cytoplasmic signaling domain induces cell signaling. In some embodiments, the endodomain of the transmembrane immunomodulatory protein comprises the cytoplasmic domain of the corresponding wild-type or unmodified polypeptide, such as a cytoplasmic domain contained in the sequence of amino acids set forth in any of SEQ ID NOS:1-27 and 408 (see Table 1).

In some embodiments, provided are CAR-related transmembrane immunomodulatory proteins in which the endodomain of a transmembrane immunomodulatory protein comprises a cytoplasmic signaling domain that comprises at least one ITAM (immunoreceptor tyrosine-based activation motif)-containing signaling domain. ITAM is a conserved motif found in a number of protein signaling domains involved in signal transduction of immune cells, including in the CD3-zeta chain ("CD3-z") involved in T-cell receptor signal transduction. In some embodiments, the endodomain comprises at CD3-zeta signaling domain. In some embodiments, the CD3-zeta signaling domain comprises the sequence of amino acids set forth in SEQ ID NO: 1575 or a sequence of amino acids that exhibits at least 85%, 86%, 87%, 88%, 89%, 90%, 91%, 92%, 93%, 94%, 95%, 96%, 97%, 98% or 99% to SEQ ID NO: 1575 and retains the activity of T cell signaling. In some embodiments, the endodomain of a CAR-related transmembrane immunomodulatory protein can further comprise a costimulatory signaling domain to further modulate immunomodulatory responses of the T-cell. In some embodiments, the costimulatory signaling domain is CD28, ICOS, 41BB or OX40. In some embodiments, the provided CAR-related transmembrane immunomodulatory proteins have features of CARs to stimulate T cell signaling upon binding of an affinity modified IgSF domain to a cognate binding partner or counter structure. In some embodiments, upon specific binding by the affinity-modified IgSF domain to its counter structure can lead to changes in the immunological activity of the T-cell activity as reflected by changes in cytotoxicity, proliferation or cytokine production.

In some embodiments, a CAR-related transmembrane immunomodulatory protein comprises an antigen binding region that is engineered to specifically bind to a desired counter-structure. In some embodiments, an affinity modified IgSF domain specifically binds its native counter-structure. In some embodiments the counter-structure is an IgSF family member. In some embodiments, provided is a CAR-related transmembrane immunomodulatory protein that specifically binds to a tumor specific IgSF counter-structure. In some embodiments, the antigen binding region (ectodomain) is an affinity modified IgSF domain NKp30. In some embodiments, the affinity modified IgSF domain specifically binds the tumor specific antigen NKp30 ligand B7-H6 (see, Levin et al., The Journal of Immunology, 2009, 182, 134.20). In examples of such embodiments, the endodomain comprises at least one ITAM (immunoreceptor tyrosine-based activation motif) containing signaling domain, such as a CD3-zeta signaling domain. In some embodiments, the endodomain can further comprises at least one of: a CD28 costimulatory domain, an OX40 signaling domain, and a 41BB signaling domain.

In some embodiments, the transmembrane immunomodulatory protein does not contain an endodomain capable of mediating cytoplasmic signaling. In some embodiments, the transmembrane immunomodulatory protein lacks the signal transduction mechanism of the wild-type or unmodified polypeptide and therefore does not itself induce cell signaling. In some embodiments, the transmembrane immunomodulatory protein lacks an intracellular (cytoplasmic) domain or a portion of the intracellular domain of the corresponding wild-type or unmodified polypeptide, such as a cytoplasmic signaling domain contained in the sequence of amino acids set forth in any of SEQ ID NOS: 1-27 and 408 (see Table 1). In some embodiments, the transmembrane immunomodulatory protein does not contain an ITIM (immunoreceptor tyrosine-based inhibition motif), such as contained in certain inhibitory receptors, including inhibitory receptors of the IgSF family (e.g. PD-1 or TIGIT). Thus, in some embodiments, the transmembrane immunomodulatory protein only contains the ectodomain and the transmembrane domain, such as any as described.

### D. Nucleic Acid Molecules and Vectors

Provided herein are isolated or recombinant nucleic acids collectively referred to as "nucleic acids" which encode any of the various provided embodiments of the transmembrane immunomodulatory polypeptides of the invention. Nucleic acids provided herein, including all described below, are useful in recombinant expression of the transmembrane immunomodulatory proteins, including for engineering cells. The nucleic acids provided herein can be in the form of RNA or in the form of DNA, and include mRNA, cRNA, recombinant or synthetic RNA and DNA, and cDNA. The nucleic acids of the invention are typically DNA molecules, and usually double-stranded DNA molecules. However, single-stranded DNA, single-stranded RNA, double-stranded RNA, and hybrid DNA/RNA nucleic acids or combinations thereof comprising any of the nucleotide sequences of the invention also are provided.

In some embodiments, expression of the transmembrane immunomodulatory protein is controlled by a promoter to enhancer to control or regulate expression, such as described herein for the alternative secretable immunomodulatory proteins. The promoter is operably linked to the portion of the nucleic acid molecule encoding the immunomodulatory protein. In some embodiments, the promotor is a constitutively active promotor (such as a tissue-specific constitutively active promotor or other constitutive promotor). In some embodiments, the promotor is an inducible promotor, which may be responsive to an inducing agent (such as a T cell activation signal). Any promoter or element for enhancing, controlling or regulating expression, either constitutively or inducibly, can be employed, include any described herein. In particular aspects, the promoter is a promoter that is responsive to an element responsive to T-cell activation signaling, for example, by signaling through an engineered T cell receptor (TCR) or a chimeric antigen receptor (CAR). Exemplary of such promoters are those containing a binding site for NFAT or NF-κB as described. For example, in some embodiments, the promoter is an NFAT or NF-κB promoter or a functional variant thereof.

Also provided herein are expression vectors useful in engineering cells to express the transmembrane immunomodulatory proteins of the present invention. The immunomodulatory polypeptides provided herein can be introduced into cells using recombinant DNA techniques. To do so, a recombinant DNA molecule encoding a transmembrane immunomodulatory polypeptide is prepared. Methods of preparing such DNA molecules are well known in the art. For instance, sequences coding for the peptides could be excised from DNA using suitable restriction enzymes. Alternatively, the DNA molecule could be synthesized using chemical synthesis techniques, such as the phosphoramidite method. Also, a combination of these techniques could be used. In some instances, a recombinant or synthetic nucleic acid may be generated through polymerase chain reaction (PCR).

In some embodiments, a full length DNA insert can be generated comprising an optional endodomain (i.e., cytoplasmic domain), a transmembrane anchor domain, an optional spacer domain, an optional epitope tag, and finally one or more extracellular affinity modified IgSF domains. This DNA insert can be cloned into an appropriate T cell transduction/transfection vector as is known to those of skill in the art. Also provided are vectors containing the nucleic acid molecules.

In some embodiments, the expression vectors are capable of expressing the transmembrane immunomodulatory proteins in an appropriate cell under conditions suited to expression of the protein. In some aspects, an expression vector comprises the DNA molecule that codes for the transmembrane immunomodulatory protein operatively linked to appropriate expression control sequences. Methods of affecting this operative linking, either before or after the DNA molecule is inserted into the vector, are well known. Expression control sequences include promoters, activators, enhancers, operators, ribosomal binding sites, start signals, stop signals, cap signals, polyadenylation signals, and other signals involved with the control of transcription or translation. In some embodiments, a nucleic acid of the invention further comprises nucleotide sequence that encodes a secretory or signal peptide operably linked to the nucleic acid encoding the transmembrane immunomodulatory protein.

In some embodiments, the resulting expression vector having the DNA molecule thereon is used to transform, such as transduce, an appropriate cell. The introduction can be performed using methods well known in the art. Exemplary methods include those for transfer of nucleic acids encoding the receptors, including via viral, e.g., retroviral or lentiviral, transduction, transposons, and electroporation. In some embodiments, the expression vector is a viral vector. In some embodiments, the nucleic acid is transferred into cells by lentiviral or retroviral transduction methods.

### E. Exemplary Transmembrane Immunomodulatory Proteins and Encoding Nucleic Acid Molecules

Provided herein is a transmembrane immunomodulatory protein in accord with the above description that comprises a sequence of amino acids that exhibits at least 85%, 86%, 87%, 88%, 89%, 90%, 91%, 92%, 93%, 94%, 95%, 96%, 97%, 98% or 99% sequence identity to any of SEQ ID NOS: 381-407 and 409 or to a portion thereof that contains an ectodomain comprising at least one affinity-modified IgSF domain as described and a transmembrane domain. In some embodiments, the transmembrane immunomodulatory protein has an ecotodomain containing the sequence of amino acids set forth in any of the SEQ ID NOS of an ECD or an IgV set forth in any of Tables 2-10, or a sequence of amino acids that exhibits at least 85%, 86%, 87%, 88%, 89%, 90%, 91%, 92%, 93%, 94%, 95%, 96%, 97%, 98% or 99% to any of the SEQ ID NOS of an ECD or an IgV set forth in any of Tables 2-10 and that contains the recited amino acid modifications (e.g. amino acid substitutions). In some embodiments, the encoded immunomodulatory protein contains 1, 2, 3, 4, 5, 6, 7, 8, 9, 10, 11, 12, 13, 14, 15, 16, 17, 18, 19, 20 or more amino acid modifications, e.g. amino acid substitutions. In some embodiments, the transmembrane immunomodulatory protein can further comprise a cytoplasmic domain as described, such as a cytoplasmic domain of any of SEQ ID NOS: 381-407 and 409 as set forth in Table 1. In some embodiments, the transmembrane immunomodulatory protein can further contain a signal peptide. In some embodiments, the signal peptide is the native signal peptide of the corresponding wild-type IgSF member (see e.g. Table 1). In some embodiments, the signal peptide is a non-native signal peptide, such as any as described, e.g. Table 11.

Also provided herein is a nucleic acid molecule encoding a transmembrane immunomodulatory protein comprising a nucleotide sequence that encodes a sequence of amino acids that exhibits at least 85%, 86%, 87%, 88%, 89%, 90%, 91%, 92%, 93%, 94%, 95%, 96%, 97%, 98% or 99% sequence identity to any of SEQ ID NOS: 381-407 and 409 or to a portion thereof that contains an ectodomain comprising at least one affinity-modified IgSF domain as described, a transmembrane domain and, optionally, a cytoplasmic domain. In some embodiments, the nucleic acid encodes an immunomodulatory protein that has the sequence of amino acids set forth in any of the SEQ ID NOS of an ECD or an IgV set forth in any of Tables 2-10, or a sequence of amino acids that exhibits at least 85%, 86%, 87%, 88%, 89%, 90%, 91%, 92%, 93%, 94%, 95%, 96%, 97%, 98% or 99% to any of the SEQ ID NOS of an ECD or an IgV set forth in any of Tables 2-10 and that contains the recited amino acid modifications (e.g. amino acid substitutions). In some embodiments, the encoded immunomodulatory protein contains 1, 2, 3, 4, 5, 6, 7, 8, 9, 10, 11, 12, 13, 14, 15, 16, 17, 18, 19, 20 or more amino acid modifications, e.g. amino acid substitutions. In some embodiments, the nucleic acid molecule can further comprise a sequence of nucleotides encoding a signal peptide. In some embodiments, the signal peptide is the native signal peptide of the corresponding wild-type IgSF member (see e.g. Table 1). In some embodiments, the signal peptide is a non-native signal peptide, such as any as described, e.g. Table 11.

### VI. ENGINEERED CELLS

Provided herein are engineered cells that comprise an immunomodulatory protein or a nucleic acid (such as an expression vector) that encodes an immunomodulatory protein as described herein.

In some embodiments, the engineered cells contain a secretable immunomodulatory protein or a nucleic acid molecule encoding a secretable immunomodulatory protein, such as any as described. In some embodiments, the engineered cell expresses and secretes the immunomodulatory protein. In some embodiments, the engineered cells express and are capable of or are able to secrete the immunomodulatory protein from the cells under conditions suitable for secretion of the protein.

In one aspect, there is provided an engineered immune cell comprising a nucleic acid molecule that encodes an immunomodulatory protein, wherein the immunomodulatory protein comprises at least one non-immunoglobulin affinity-modified immunoglobulin superfamily (IgSF) domain comprising one or more amino acid modifications (e.g., substitutions) in a wild-type IgSF domain, wherein the at least one affinity-modified IgSF domain specifically binds at least one cell surface cognate binding partner of the wild-type IgSF domain; and the engineered cell is configured to express and secrete the immunomodulatory protein. In some embodiments, the immunomodulatory protein does not comprise a transmembrane domain. In some embodiments, the immunomodulatory protein is not conjugated to half-life extending moiety (such as a multimerization domain or an Fc domain). In some embodiments, the nucleic acid molecule comprises a sequence encoding a secretory signal peptide operably linked to the sequence encoding the immunomodulatory protein.

In some embodiments, the engineered cells contain a transmembrane immunomodulatory protein or a nucleic acid molecule encoding a transmembrane immunomodulatory protein, such as any as described. In some embodiments, the engineered cells express the immunomodulatory protein on its surface.

In some embodiments, the engineered cell is an immune cell, such as a lymphocyte (for example, a tumor infiltrating lymphocyte (TIL), T-cell or NK cell) or on a myeloid cell. In some embodiments, the engineered cell is an antigen presenting cell (APC). In some embodiments, the engineered cells are engineered mammalian T cells or engineered mammalian antigen presenting cells (APCs). In some embodiments, the engineered immune cells or APCs are human or murine cells.

In some embodiments, engineered T cells include, but are not limited to, T helper cell, cytotoxic T-cell (alternatively, cytotoxic T lymphocyte or CTL), natural killer T-cell, regulatory T-cell, memory T-cell, or gamma delta T-cell. In some embodiments, the engineered T cells are CD4+ or CD8+. In addition to the signal of the MHC, engineered T-cells also require a co-stimulatory signal which in some embodiments is provided by the immunomodulatory proteins as discussed herein.

In some embodiments, the engineered APCs include, for example, MHC II expressing APCs such as macrophages, B cells, and dendritic cells, as well as artificial APCs (aAPCs) including both cellular and acellular (e.g., biodegradable polymeric microparticles) aAPCs. Artificial APCs (aAPCs) are synthetic versions of APCs that can act in a similar manner to APCs in that they present antigens to T cells as well as activate them. Antigen presentation is performed by the MHC (Class I or Class II). In some aspects, in engineered APCs such as aAPCs, the antigen that is loaded onto the MHC is, in some embodiments, a tumor specific antigen or a tumor associated antigen. The antigen loaded onto the MHC is recognized by a T-cell receptor (TCR) of a T cell, which, in some cases, can express one or more cognate binding partners or other molecule recognized by the affinity modified IgSF domain of the immunomodulatory proteins provided herein.

In some embodiments a cellular aAPC can be engineered to contain a SIP or TIP and TCR agonist which is used in adoptive cellular therapy. In some embodiments, a cellular aAPC can be engineered to contain a SIP or TIP and TCR agonist which is used in ex vivo expansion of human T cells, such as prior to administration, e.g., for reintroduction into the patient. In some aspects, the aAPC may include expression of at least one anti-CD3 antibody clone, e.g. such as, for example, OKT3 and/or UCHT1. In some aspects, the aAPCs may be inactivated (e.g. irradiated). In some embodiment, the SIP or TIP can include any variant or affinity-modified IgSF domain that exhibits binding affinity for a cognate binding partner on a T cell.

In some embodiments, a immunomodulatory protein provided herein, such as a transmembrane immunomodulatory protein or a secretable immunomodulatory protein, is co-expressed or engineered into a cell that expresses an antigen-binding receptor, such as a recombinant receptor, such as a chimeric antigen receptor (CAR) or T cell receptor (TCR). In some embodiments, the engineered cell, such as an engineered T cell, recognizes a desired antigen associated with cancer, inflammatory and autoimmune disorders, or a viral infection. In specific embodiments, the antigen-binding receptor contains an antigen-binding moiety that specifically binds a tumor specific antigen or a tumor associated antigen. In some embodiments, the engineered T-cell is a CAR (chimeric antigen receptor) T-cell that contains an antigen-binding domain (e.g. scFv) that specifically binds to an antigen, such as a tumor specific antigen or tumor associated antigen. In another embodiment, the engineered T-cell possesses a TCR, including a recombinant or engineered TCR. In some embodiments, the TCR can be a native TCR. Those of skill in the art will recognize that generally native mammalian T-cell receptors comprise an alpha and a beta chain (or a gamma and a delta chain) involved in antigen specific recognition and binding. In some embodiments, the TCR is an engineered TCR that is modified. In some embodiments, the TCR of an engineered T-cell specifically binds to a tumor associated or tumor specific antigen presented by an APC. Thus, in some embodiments, the immunomodulatory protein is expressed and secreted in an engineered T-cell receptor cell or and engineered chimeric antigen receptor cell. In such embodiments, the engineered cell co-expresses the immunomodulatory protein and the CAR or TCR. In some embodiments, the SIP protein is expressed in an engineered T-cell receptor cell or an engineered chimeric antigen receptor cell. In such embodiments, the engineered cell co-expresses the SIP and the CAR or TCR.

Chimeric antigen receptors (CARs) are recombinant receptors that include an antigen-binding domain (ectodomain), a transmembrane domain and an intracellular signaling region (endodomain) that is capable of inducing or mediating an activation signal to the T cell after the antigen is bound. In some examples, CAR-expressing cells are engineered to express an extracellular single chain variable fragment (scFv) with specificity for a particular tumor antigen linked to an intracellular signaling part comprising an activating domain and, in some cases, a costimulatory domain. The costimulatory domain can be derived from, e.g., CD28, OX-40, 4-1BB/CD137, inducible T cell costimulator (ICOS). The activating domain can be derived from, e.g., CD3, such as CD3 zeta, epsilon, delta, gamma, or the like. In certain embodiments, the CAR is designed to have two, three, four, or more costimulatory domains. The CAR scFv can be designed to target an antigen expressed on a cell associated with a disease or condition, e.g. a tumor antigen, such as, for example, CD19, which is a transmembrane protein expressed by cells in the B cell lineage, including all normal B cells and B cell malignances, including but not limited to NHL, CLL, and non-T cell ALL. Example CAR+ T cell therapies and constructs are described in U.S. Patent Publication Nos. 2013/0287748, 2014/0227237, 2014/0099309, and 2014/0050708, and these references are incorporated by reference in their entirety.

In some aspects, the antigen-binding domain is an antibody or antigen-binding fragment thereof, such as a single chain fragment (scFv). In some embodiments, the antigen is expressed on a tumor or cancer cell. Exemplary of an antigen is CD19. Exemplary of a CAR is an anti-CD19 CAR, such as a CAR containing an anti-CD19 scFv set forth in SEQ ID NO:1576 or SEQ ID NO:1577.

In some embodiments, the CAR further contains a spacer, a transmembrane domain, and an intracellular signaling domain or region comprising an ITAM signaling domain, such as a CD3zeta signaling domain.

In some embodiments, the CAR further includes a costimulatory signaling domain. In some embodiments, the spacer and transmembrane domain are the hinge and transmembrane domain derived from CD8, such as having an exemplary sequence set forth in SEQ ID NO: 1574, 1578, 2158 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 1574, 1578, 2158. In some embodiments, the endodomain comprises at CD3-zeta signaling domain. In some embodiments, the CD3-zeta signaling domain comprises the sequence of amino acids set forth in SEQ ID NO: 1575 or a sequence of amino acids that exhibits at least 85%, 86%, 87%, 88%, 89%, 90%, 91%, 92%, 93%, 94%, 95%, 96%, 97%, 98% or 99% or more sequence identity to SEQ ID NO: 1575 and retains the activity of T cell signaling. In some embodiments, the endodomain of a CAR can further comprise a costimulatory signaling domain or region to further modulate immunomodulatory responses of the T-cell. In some embodiments, the costimulatory signaling domain is CD28, ICOSL, 41BB or OX40. In some embodiments, the costimulatory signaling domain is a derived from CD28 or 4-1BB and comprises the sequence of amino acids set forth in any of SEQ ID NOS:1579-1582 or a sequence of amino acids that exhibits at least 85%, 86%, 87%, 88%, 89%, 90%, 91%, 92%, 93%, 94%, 95%, 96%, 97%, 98% or 99% or more sequence identity to SEQ ID NO: 1579-1582 and retains the activity of T cell costimulatory signaling.

In some embodiments, the construct encoding the CAR further encodes a second protein, such as a marker, e.g. detectable protein, separated from the CAR by a self-cleaving peptide sequence. In some embodiments, the self-cleaving peptide sequence is an F2A, T2A, E2A or P2A self-cleaving peptide. Exemplary sequences of a T2A self-cleaving peptide are set for the in any one of SEQ ID NOS: 2165, 2169, 2177 or a sequence of amino acids that exhibits at least 85%, 86%, 87%, 88%, 89%, 90%, 91%, 92%, 93%, 94%, 95%, 96%, 97%, 98% or 99% or more sequence identity to any of SEQ ID NOS: 2165, 2169, 2177. In some embodiments, the T2A is encoded by the sequence of nucleotides set forth in SEQ ID NO:2176 or a sequence that exhibits at least 85%, 86%, 87%, 88%, 89%, 90%, 91%, 92%, 93%, 94%, 95%, 96%, 97%, 98% or 99% or more sequence identity to any of SEQ ID NO: 2176. An exemplary sequence of a P2A self-cleaving peptide is set in SEQ ID NO: 2178 or a sequence of amino acids that exhibits at least 85%, 86%, 87%, 88%, 89%, 90%, 91%, 92%, 93%, 94%, 95%, 96%, 97%, 98% or 99% or more sequence identity to SEQ ID NOS: 2178.

In some embodiments, the marker is a detectable protein, such as a fluorescent protein, e.g. a green fluorescent protein (GFP) or blue fluorescent protein (BFP). Exemplary sequences of a fluorescent protein marker are set forth in SEQ ID NO: 2164, 2170, 2179, 2180, or a sequence of amino acids that exhibits at least 85%, 86%, 87%, 88%, 89%, 90%, 91%, 92%, 93%, 94%, 95%, 96%, 97%, 98% or 99% or more sequence identity to SEQ ID NO: 2164 or 2170.

In some embodiments, the CAR has the sequence of amino acids set forth in any of SEQ ID NOS: 2166, 2171, 2172, 2173, 2159, 2160, 2162, or 2163 or a sequence of amino acids that exhibits at least 85%, 86%, 87%, 88%, 89%, 90%, 91%, 92%, 93%, 94%, 95%, 96%, 97%, 98% or 99% or more sequence identity to any one of SEQ ID NOS: 2166, 2171, 2172, 2173, 2159, 2160, 2162 or 2163. In some embodiments, the CAR is encoded by a sequence of nucleotides set forth in SEQ ID NO: 2175 or 2161 or a sequence of amino acids that exhibits at least 85%, 86%, 87%, 88%, 89%, 90%, 91%, 92%, 93%, 94%, 95%, 96%, 97%, 98% or 99% or more sequence identity to any one of SEQ ID NO: 2175 or 2161.

In another embodiment, the engineered T-cell possesses a TCR, including a recombinant or engineered TCR. In some embodiments, the TCR can be a native TCR. Those of skill in the art will recognize that generally native mammalian T-cell receptors comprise an alpha and a beta chain (or a gamma and a delta chain) involved in antigen specific recognition and binding. In some embodiments, the TCR is an engineered TCR that is modified. In some embodiments, the TCR of an engineered T-cell specifically binds to a tumor associated or tumor specific antigen presented by an APC.

In some embodiments, nucleic acids encoding the immunomodulatory protein, such as transmembrane immunomodulatory polypeptides or secretable immunomodulatory polypeptides, are incorporated into engineered cells, such as engineered T cells or engineered APCs, by a variety of strategies such as those employed for recombinant host cells. A variety of methods to introduce a DNA construct into primary T cells are known in the art. In some embodiments, viral transduction or plasmid electroporation are employed. In typical embodiments, the nucleic acid molecule encoding the immunomodulatory protein, or the expression vector, comprises a signal peptide that causes the expressed immunomodulatory proteins to be secreted from the cell. In some embodiments, a nucleic acid encoding an immunomodulatory protein of the invention is sub-cloned into a viral vector, such as a retroviral vector, which allows expression in the host mammalian cell. The expression vector can be introduced into a mammalian host cell and, under host cell culture conditions, the immunomodulatory protein is expressed and secreted.

In an exemplary example, primary T cells can be purified *ex vivo* (CD4 cells or CD8 cells or both) and stimulated with an activation protocol consisting of various TCR/CD28 agonists, such as anti-CD3/anti-CD28 coated beads. After a 2 or 3 day activation process, the DNA vector encoding the immunomodulatory protein of the present invention can be stably introduced into the primary T cells through art standard lentiviral or retroviral transduction protocols or plasmid electroporation strategies. Cells can be monitored for immunomodulatory protein expression and secretion by, for example, using anti-epitope tag or antibodies that cross-react with native parental molecule and affinity modified variant.

Upon immunomodulatory protein expression and secretion, the engineered T cell can be assayed for improved function by a variety of means. The engineered CAR or TCR co-expression can be validated to show that this part of the engineered T cell was not significantly impacted by the expression and secretion of the immunomodulatory construct. Once validated, standard in vitro cytotoxicity, proliferation, or cytokine assays can be used to assess the function of the engineered cells. Exemplary standard endpoints are percent lysis of the tumor line, proliferation of the engineered T-cell, or IFN-gamma protein expression in culture supernatants. An engineered construct which results in statistically significant increased lysis of tumor line, increased proliferation of the engineered T-cell, or increased IFN-gamma expression over the control construct can be selected for. Additionally, non-engineered cells, such as native primary or endogenous T-cells, could also be incorporated into the same in vitro assay to measure the ability of the immunomodulatory protein construct expressed and secreted by the engineered cells, such as engineered T-cells, to modulate activity, including, in some cases, to activate and generate effector function in bystander, native T-cells. Increased expression of activation markers such as CD69, CD44, or CD62L could be monitored on endogenous T cells, and increased proliferation and/or cytokine production could indicate desired activity of the immunomodulatory protein expressed and secreted by the engineered T cells.

In some embodiments, the similar assays can be used to compare the function of engineered T cells containing the CAR or TCR alone to those containing the CAR or TCR and a expressing and secreting an immunomodulatory protein. Typically, these in vitro assays are performed by plating various ratios of the engineered T cell and a "tumor" cell line containing the cognate CAR or TCR antigen together in culture. Standard endpoints are percent lysis of the tumor line, proliferation of the engineered T cell, or IFN-gamma production in culture supernatants. An engineered cell which resulted in statistically significant increased lysis of tumor line, increased proliferation of the engineered T cell, or increased IFN-gamma production over the same TCR or CAR construct alone can be selected for. Engineered human T cells can be analyzed in immunocompromised mice, like the NSG strain, which lacks mouse T, NK, and B cells. Engineered human T cells in which the CAR or TCR binds a target cognate binding partner on the xenograft and is co-expressed with the immunomodulatory protein containing an affinity-modified IgSF domain (which is also secreted) can be adoptively transferred in vivo at different cell numbers and ratios compared to the xenograft. For example, engraftment of CD19+ leukemia tumor lines containing a luciferase/GFP vector can be monitored through bioluminescence or ex vivo by flow cytometry. In a common embodiment, the xenograft is introduced into the murine model, followed by the engineered T cells several days later. Engineered T cells that express and secrete an immunomodulatory protein can be assayed for increased survival, tumor clearance, or expanded engineered T cells numbers relative to engineered T cells containing the CAR or TCR alone. As in the in vitro assay, endogenous, native (i.e., non-engineered) human T cells could be co-adoptively transferred to look for successful epitope spreading in that population, resulting in better survival or tumor clearance.

### A. Exemplary Functional Activities and Features

In some aspects, engineered cells, such as engineered lymphocytes (e.g. tumor infiltrating lymphocytes, T cells or NK cells) or myeloid cells (e.g. antigen presenting cells), exhibit one or more desirable features or activities.

In some embodiments, when expressed on or from cells, the affinity-modified IgSF domain of the immunomodulatory protein specifically binds to at least one cognate binding partner expressed on a mammalian cell. In some embodiments, the mammalian cell is an autologous or allogeneic mouse, rat, cynomolgus monkey, or human cell. In some aspects, the mammalian cell can include such embodiments as an antigen presenting cell (APC), a tumor cell, or a T-cell. In some embodiments, the tumor cell is a mouse, rat, cynomolgus monkey, or human tumor cell.

An immunomodulatory protein can comprise one or multiple (e.g., 2, 3, or 4) affinity modified IgSF domains. Thus, in some embodiments the immunomodulatory protein binds to no more than one cognate binding partner on the mammalian cell. In some embodiments, an affinity-modified IgSF domain of an immunomodulatory protein specifically binds to no more than one cognate binding partner on the mammalian cell. Alternatively, in some embodiments, an immunomodulatory protein specifically binds to at least two, three or four, or exactly two, three, or four, cognate binding partners expressed on a mammalian cell. In some embodiments, the immunomodulatory protein specifically binds to no more than one cell surface cognate binding partners. Specific binding of the immunomodulatory protein containing one or more affinity-modified IgSF domains to the cognate binding partner on a mammalian cell modulates immunological activity of the mammalian cell. Specific binding by and between an affinity-modified IgSF domain and a mammalian cell cognate binding partner can be specific binding in cis arrangement (i.e., specific binding on the same cell) or in trans arrangement (i.e., specific binding on different cells) or in both cis and trans arrangement. Immunological activity of the cell can be increased as evidenced by increased, e.g., cell survival, cell proliferation, cytokine production, or T-cell cytotoxicity. In alternative embodiments, the immunological activity of the cell is attenuated as evidenced by a decrease of cell survival, cell proliferation, cytokine production, or T-cell cytotoxicity.

In some embodiments, at least one affinity-modified IgSF domain present in an immunomodulatory protein specifically binds to at least one cell surface cognate binding partner expressed on a mammalian cell and in which modulation of immunological activity is desired. In some embodiments, the cognate binding partner to which the affinity modified IgSF domain specifically binds is the native cognate binding partner of the wild-type IgSF family member or wild-type IgSF domain that has been affinity modified. In some embodiments, the specific binding increases and/or attenuates activity a mammalian cell expressing a cognate binding partner to which the affinity modified IgSF domain exhibits improved binding . Thus, by increasing specific binding affinity, the provided immunomodulatory proteins can either increase or attenuate immunological activity of a mammalian cell. In some embodiments, the specific binding modulates, such as increases, immunological activity of the engineered cell with the immunomodulatory protein.

In some embodiments, the cognate binding partner expressed on the mammalian cell is a mammalian IgSF member. The mammalian cell is, in some embodiments, an antigen presenting cell (APC), a lymphocyte, or a tumor cell. In some embodiments, the lymphocyte is a tumor infiltrating lymphocyte (TIL), an engineered or native T-cell, or an engineered or native NK cell. In some embodiments, the cognate binding partner of the affinity modified IgSF domain is a native human IgSF member. In some embodiments, the cognate binding partner is a "cell surface cognate binding partner" as indicated in Table 1.

In some embodiments, an immunomodulatory protein comprising an affinity-modified IgSF when expressed and secreted by an immune cell (e.g. a lymphocyte such as a T-cell) can specifically bind at least one cognate binding partner expressed on a second immune cells, e.g. a lymphocyte such as a T-cell. The cognate binding partner on the second immune cells, e.g. second T-cell, can be an inhibitory cognate binding partner or a stimulatory cognate binding partner. Exemplary cognate binding partners include cell surface receptors or ligands. Examples of inhibitory receptors/ligands include PD-1/PD-L1, PD-L2, CTLA-4/B7-1/B7-2, BTLA/HVEM, LAG3/MHC class II, TIGIT/PVR, TIM-3/CEACAM-1/GAL9 and VSIG8/VISTA. Examples of stimulatory receptors/ligands include CD28/B7-1/B7-2, ICOS/ICOSL, and CD226/PVR.

In a particular embodiment, the immunomodulatory protein is expressed and secreted by a T cell and comprises an affinity modified IgSF domain that specifically binds to a cognate binding partner expressed on a T-cell. In some embodiments the first and second T-cells are separate T-cells and in this embodiment the immunomodulatory protein and cognate binding partner are in trans to each other. In some embodiments, the immunomodulatory protein and cognate binding partner are expressed on the same T-cell (wherein the immunomodulatory protein is secreted) and are cis to each other. In some embodiments, at least one of the T-cells is a native T-cell or an engineered T-cell. In some embodiments, the engineered T-cell is a chimeric antigen receptor (CAR) T-cell or a T-cell receptor (TCR) engineered T-cell.

In some embodiments, a immunomodulatory protein comprises an affinity modified IgSF domain with increased affinity to a cell surface receptor to stimulate an increase in receptor signal transduction. Stimulating an increase in receptor signaling can in some embodiments increase immunological activity of that cell if, for example, the receptor is a stimulatory receptor that works to mediate those effects. In some cases, the inflammatory activity of the cell in which receptor signaling is stimulated is increased. In some embodiments, the immunomodulatory protein increases the activity of a stimulatory receptor. In such examples, an IgSF domain of an immunomodulatory protein can be affinity modified to increase the specific binding affinity to the native cognate binding partner on a mammalian cell, which, in some cases, is a stimulatory receptor. In some embodiments, the stimulatory receptor is expressed on T cells. In certain embodiments, the affinity modified IgSF domain of the immunomodulatory protein, such as is expressed and secreted by an engineered cell (e.g. a first T cell), specifically binds to a stimulatory cognate binding partner expressed on a T cell (e.g. a second T cell) with increased affinity (relative to the non-affinity modified IgSF domain as a control). In certain embodiments, the affinity modified IgSF domain of the immunomodulatory protein specifically binds to a stimulatory cognate binding partner expressed on a T cell (e.g. second T cell) and increases immunomodulatory activity of the T-cell. In some embodiments, the affinity modified IgSF domain of an immunomodulatory protein binds to a stimulatory cognate binding partner on the T cell (e.g. second T-cell) with increased affinity and increases immunomodulatory activity of the T-cell.

In some embodiments, the stimulatory receptor is CD28, ICOS or CD226 and the immunomodulatory protein containing an affinity-modified IgSF domain that exhibits increased binding affinity to one of CD28, ICOS or CD226 compared to a protein containing a wild-type IgSF domain. In some embodiments, the affinity modified IgSF domain is an affinity modified domain of CD80 (B7-1). In some embodiments, an affinity modified CD80 (B7-1) IgSF domain of an immunomodulatory protein of the present invention is expressed on a first T-cell and is affinity modified to bind with increased affinity to the stimulatory cognate binding partner CD28 on the second T-cell. In some embodiments, the affinity modified IgSF domain is an affinity modified domain of ICOSL. In specific embodiments, the affinity modified IgSF domain is an affinity modified ICOSL (inducible costimulator ligand) domain and the stimulatory cognate binding partner is at least one of: ICOS (inducible costimulator) or CD28. In some embodiments, the ICOSL domain is affinity-modified to specifically bind to both ICOS and CD28. In some embodiments, ICOSL is affinity modified to specifically bind to either ICOS or to CD28 but not both. In some embodiments, binding affinity to one of ICOS or CD28 is increased while binding affinity to the other is attenuated. In some embodiments, the affinity modified IgSF domain is an affinity modified CD155 and the activating cognate ligand is CD226. In some embodiment, the affinity modified IgSF domain is an affinity modified CD112 and the activating cognate ligand is CD226.

In some methods of the present invention, the immunomodulatory protein attenuates the activity of an inhibitory receptor. In some cases, the increased binding affinity of the immunomodulatory protein to a cognate cell surface molecule results in inhibition of specific binding between native cognate binding partners on mammalian cells. The greater affinity for that native cognate binding partner (relative to the competing affinity of the native IgSF member) attenuates specific binding affinity of native molecule to its cognate binding partner. Those of skill in the art will appreciate that antagonizing an inhibitory receptor signaling can in some embodiments attenuate immunological activity of that cell if, for example, the receptor is an inhibitory receptor that serves to cause those cellular effects. In some embodiments, one or more activities between the inhibitory receptor and its ligand from among CD155/TIGIT, CD112/TIGIT, CD80/CTLA-4, ICOSL/CTLA-4. PD-L1/PD-1 or PD-:2/PD-lis blocked by the immunomodulatory protein.

Thus, in some embodiments, an immunomodulatory protein can be used to stimulate a cell for which the immunomodulatory protein is not expressed and secreted (i.e., the trans cell) while attenuating inhibition of the cell by which the immunomodulatory protein is expressed and secreted (the cis cell). For example, in some embodiments, the immunomodulatory protein comprises at least one affinity-modified IgSF domain, and in some cases at least two affinity modified domains, that results in increased binding affinity to at least two cell surface cognate binding partners. In some embodiments, a first cognate binding partner is a stimulatory receptor and the second cell surface cognate binding partner is an inhibitory ligand of an inhibitory receptor. In some embodiments, binding of the affinity-modified domain to the inhibitory ligand competitively inhibits binding of the inhibitory ligand to the inhibitory receptor. In some embodiments, the stimulatory receptor and inhibitory receptor can independently be expressed on immune cells, such as T cells or antigen presenting cells. In some embodiments the stimulatory receptor is expressed on lymphocytes, such as T cells. In some embodiments, the stimulatory receptor is CD28, ICOS, or CD226 and/or the ligand of the stimulatory receptor is ICOSL. In some embodiments, the inhibitory receptor is expressed on the engineered cells, such as an engineered T-cell. In some embodiments, the inhibitory receptor is PD-1, CTLA-4, LAG-3, TIGIT, CD96, CD112R, BTLA, CD160, TIM-3, VSIG3, or VSIG8 and/or the ligand of the inhibitory receptor is PD-L1, PD-L2, B7-1, B7-2, CD112, CD155, HVEM, MHC class II, PVR, CEACAM-1,GAL9 or VISTA (see e.g. Table 1). In some embodiments, the inhibitory cognate binding partner is PD-L1 or PD-L2.

In some embodiments, an immunomodulatory protein can be used to attenuate inhibition of the cell that expresses and secretes the immunomodulatory protein, such as a T cell that expresses and secretes the immunomodulatory protein. For example, an immunomodulatory protein expressed and secreted by a T cell (e.g. first T cell) can comprise an affinity-modified IgSF domain that inhibits specific binding between a cognate binding partner on a second T cell. In some embodiments, a ligand of an inhibitory receptor is affinity-modified and, when secreted from an engineered cell, antagonizes or inhibits its inhibitory receptor. In some embodiments, the affinity-modified ligand of the inhibitory receptor is PD-L1, PD-L2, B7-1, B7-2, HVEM, MHC class II, PVR (e.g. CD112 or CD155), CEACAM-1, GAL9 or VISTA (see e.g. Table 1). In some embodiments, the affinity-modified domain that is a ligand of the inhibitory receptor retains or exhibits increased binding for another receptor, such as a stimulatory receptor. For example, the affinity-modified domain can be CD155 or CD112 that is affinity-modified to exhibit reduced binding to CD226 but that retains or exhibits increased binding to TIGIT, and, in some cases, CD112R. In such embodiments, the affinity-modified CD155 or CD112 can antagonize TIGIT when expressed and secreted from a cell.

In some cases, this embodiment can be used independently or in conjunction with embodiments wherein an affinity-modified IgSF domain of the invention is expressed and secreted by a first T cell and specifically binds at least one stimulatory cognate binding partner expressed on a second T cell and increases immunological activity in the second T cell. By this mechanism, an increased immunomodulatory response is generated in the second T cell by specific binding of the immunomodulatory protein expressed on the first T cell to a stimulatory cognate binding partner on the second cell; and the second T cell is inhibited from attenuating the immunomodulatory activity of the first T cell by specific binding of an affinity modified IgSF domain expressed on the first T cell that inhibits specific binding by and between a cognate binding partner on the second T cell and an inhibitory cognate binding partner expressed on the first T cell. The T cells used in this and the preceding embodiments are generally murine or human T-cells although other mammalian T-cells can be employed. Often, cytotoxic T-cells (CTL) are used.

As previously noted, in some embodiments an immunomodulatory protein of the present invention is expressed on a first T-cell and comprises an affinity-modified IgSF domain that specifically binds to a stimulatory cognate binding partner (e.g. stimulatory receptor) on a second T cell while also inhibiting specific binding between a native cognate binding partner (e.g. inhibitory ligand) on the second T-cell to its inhibitory native cognate binding partner (e.g. inhibitory receptor) on the first T-cell. Inhibition of specific binding between the cognate binding partner on the second T-cell to the cognate binding partner on the first T-cell can be achieved by competitive binding of an affinity-modified IgSF domain with at least one of the two native cognate binding partners such that their mutual binding is interfered with. Typically, the IgSF domain is affinity modified to have a higher binding affinity to its cognate binding partner than the native cognate binding partners have to each other. In some embodiments of this design, an immunomodulatory protein can comprise an affinity-modified IgSF domain that binds to both the inhibitory and stimulatory cognate binding partners. Thus, in this embodiment the affinity-modified IgSF has dual binding capability. In some embodiments, an immunomodulatory protein comprises a first affinity-modified IgSF domain that binds a cognate binding partner on the first T-cell and a second affinity-modified IgSF domain that inhibits specific binding by and between the cognate binding partners on the first and second T-cells.

In yet another embodiment, the affinity-modified IgSF domain that binds to the stimulatory cognate binding partner on the first T-cell is on a first immunomodulatory protein and the affinity-modified IgSF domain that inhibits specific binding by and between the cognate binding partners on the first and second T cells is on a second immunomodulatory protein. In this embodiment, the first and second immunomodulatory proteins are different polypeptide chains. In some embodiments, the first affinity-modified IgSF domain and the second affinity-modified IgSF domain are the identical affinity-modified IgSF domain. For example, in a specific embodiment the ICOSL (inducible costimulator ligand) IgSF domain (e.g. affinity modified IgV domain) is affinity modified to specifically bind with increased affinity to both ICOS and CD28. In some embodiments, the affinity modified IgSF domain is an affinity modified ICOSL IgSF domain (e.g. affinity modified IgV domain) with increased affinity to both ICOS and CD28, or decreased affinity to one of or both of: ICOS and CD28.

In some embodiments, the immunomodulatory protein results in inhibition of specific binding by and between native cognate binding partners. In some embodiments, this can be achieved by an affinity-modified IgSF domain having greater affinity for one or both native cognate binding partners thereby competitively inhibiting the specific binding by and between these cognate binding partners.

In some embodiments, the immunomodulatory protein comprises an affinity-modified IgSF domain that is an affinity-modified CD155 IgSF domain with increased affinity to CD226 and attenuated affinity to TIGIT (T-cell immunoreceptor with Ig and ITIM domains).

In some embodiments, the immunomodulatory protein (e.g. expressed on a first T cell) comprises an affinity-modified CD80 (B7-1) IgSF domain that is affinity modified to bind with increased affinity to the stimulatory cognate binding partner CD28 (e.g. on a second T-cell). Additionally, in this embodiment, the affinity-modified CD80 (B7-1) IgSF domain can bind with increased affinity to PD-L1 (e.g. expressed on the second T-cell) and inhibit specific binding to its cognate binding partner PD-1 (e.g. expressed on the first T-cell). In yet a further addition either of the preceding embodiments, the affinity-modified CD80 (B7-1) IgSF domain can be affinity modified such that it does not substantially specifically bind to CTLA-4 or binds with attenuated affinity and therefore is not significantly inhibited in its specific binding to the stimulatory cognate binding partner CD28 by CTLA-4.

In some embodiments, an immunomodulatory protein is used as a decoy cognate binding partner to inhibit specific binding by and between native cognate binding partners, at least one of which comprises an IgSF family member. In some cases, specific binding of an immunomodulatory protein comprising an affinity-modified IgSF domain with one of the native cognate binding partners inhibits mutual specific binding by and between the native cognate binding partners (e.g. native receptor and ligand pairs). Thus, in some embodiments, immunomodulatory proteins can attenuate specific binding by means of competitive or non-competitive binding. In some embodiments, the native cognate binding partner is a cell surface receptor, which can be a stimulatory receptor or an inhibitory receptor. Embodiments wherein specific binding of the affinity modified IgSF domain of a cognate binding partner increases or attenuates immunological activity of the T-cell are included within the scope of the invention.

In some embodiments, a native cognate binding partner is an inhibitory cognate binding partner that acts to attenuate immunological activity when specifically bound by its native cognate binding partner. For example, a native cell surface cognate binding partner expressed on an antigen presenting cell (APC) or a mammalian tumor cell can specifically bind a native inhibitory cognate binding partner on an NK cell or a lymphocyte such as a T-cell. Specific binding to the inhibitory cognate binding partner acts to attenuate immunomodulatory activity of the NK cell or lymphocyte on which the inhibitory cognate binding partner is expressed.

In some embodiments, an inhibitory cognate binding partner is an inhibitory receptor. In some embodiments, the inhibitory cognate binding partner is an ITIM (immunoreceptor tyrosine-based inhibition motif) containing inhibitory cognate binding partner. The ITIM motif is found in the endodomain of many inhibitory receptors of the immune system (Cell Signal, 16 (4): 435-456, 2004). In some embodiments, the affinity-modified IgSF domain is an affinity-modified form of a wild-type inhibitory receptor IgSF domain that results in greater affinity of the affinity-modified IgSF domain of the immunomodulatory protein for its native cognate binding partner than the wild-type inhibitory receptor for the native cognate binding partner. Thus, in these embodiments a immunomodulatory protein can attenuate the inhibitory response of ITIM motif receptors by specific binding of the immunomodulatory protein affinity-modified IgSF domain to its native IgSF domain cognate binding partner, such as specific binding of the immunomodulatory protein affinity-modified IgSF domain to the ITIM containing inhibitory receptor. As an example, an ITIM containing cognate binding partner is PD-1. Typically, PD-1 is the inhibitory receptor that is specifically bound to the inhibitory ligand PD-1. Upon specific binding of PD-L1 to PD-1, PD-1 is involved in inhibiting T-cell activation via signal transduction from the ITIM domain.

In some embodiments, the inhibitory receptor cognate binding partner is PD-1, CTLA-4, LAG3, TIGIT, TIM-3, BTLA, VSIG3, or VSIG8. In some embodiments, the immunomodulatory protein contains an affinity-modified IgSF domain that is an affinity-modified IgSF domain of PD-1, CTLA-4, LAG3, TIGIT, TIM-3, or BTLA that binds with greater affinity to the native inhibitory ligand of the inhibitory receptor than the wild-type inhibitory receptor (see Table 1 for ligand binding partners of exemplary inhibitory receptors). In some embodiments, an immunomodulatory protein can comprise an affinity-modified PD-1 IgSF domain that binds with greater affinity to PD-L1 than wild-type PD-1. Specific binding can be achieved by competitive or non-competitive binding and are specific embodiments of the invention. Competitive binding by and between the affinity-modified IgSF domain and the cognate binding partner (i.e. inhibitory receptor, e.g. PD-1) inhibits its binding to its native ligand cognate binding partner (e.g., PD-L1). In some embodiments, the immunomodulatory protein of this embodiment substantially lacks the signal transduction mechanism of the wild-type inhibitory receptor and therefore does not itself induce an inhibitory response.

### VII. INFECTIOUS AGENTS

Also provided are infectious agents that contain nucleic acids encoding any of the immunomodulatory proteins containing an affinity-modified IgSF domain, including secretable or transmembrane immunomodulatory proteins described herein. In some embodiments, such infectious agents can deliver the nucleic acids encoding the immunomodulatory polypeptides described herein to a target cell in a subject, e.g., immune cell and/or antigen-presenting cell (APC) or tumor cell in a subject. Also provided are nucleic acids contained in such infectious agents, and/or nucleic acids for generation or modification of such infectious agents, such as vectors and/or plasmids, and compositions containing such infectious agents.

In some embodiments, the infectious agent is a microorganism or a microbe. In some embodiments, the infectious agent is a virus or a bacterium. In some embodiments, the infectious agent is a virus. In some embodiments, the infectious agent is a bacterium. In some embodiments, such infectious agents can deliver nucleic acid sequences encoding any of the immunomodulatory proteins, including secretable or transmembrane immunomodulatory proteins, described herein. Thus, in some embodiments, the cell in a subject that is infected or contacted by the infectious agents can be rendered to express on the cell surface or secrete, the immunomodulatory polypeptides. In some embodiments, the infectious agent can also deliver one or more other therapeutics or nucleic acids encoding other therapeutics to the cell and/or to an environment within the subject. In some embodiments, other therapeutics that can be delivered by the infectious agents include cytokines or other immunomodulatory molecules.

In some embodiments, the infectious agent, e.g., virus or bacteria, contains nucleic acid sequences that encode any of the immunomodulatory proteins, including secretable or transmembrane immunomodulatory proteins, described herein, and by virtue of contact and/or infection of a cell in the subject, the cell expresses the immunomodulatory proteins, including secretable or transmembrane immunomodulatory proteins, encoded by the nucleic acid sequences contained in the infectious agent. In some embodiments, the infectious agent can be administered to the subject. In some embodiments, the infectious agent can be contacted with cells from the subject *ex vivo.*

In some embodiments, the immunomodulatory protein is a transmembrane immunomodulatory proteins that is expressed by the cell infected by the infectious agent and is surface expressed. In some embodiments, the immunomodulatory protein is a secretable immunomodulatory protein that is expressed by the cell infected by the infectious agent and is expressed and secreted from the cell. The transmembrane immunomodulatory protein or secreted immunomodulatory protein can be any described herein.

In some embodiments, the cells in the subject that are targeted by the infectious agent include a tumor cell, an immune cell, and/or an antigen-presenting cell (APC). In some embodiments, the infectious agent targets a cell in the tumor microenvironment (TME). In some embodiments, the infectious agent delivers the nucleic acids encoding the immunomodulatory protein, including secretable or transmembrane immunomodulatory proteins, to an appropriate cell (for example, an APC, such as a cell that displays a peptide/MHC complex on its cell surface, such as a dendritic cell) or tissue (e.g., lymphoid tissue) that will induce and/or augment the desired effect, e.g., immunomodulation and/or a specific cell-medicated immune response, e.g., CD4 and/or CD8 T cell response, which CD8 T cell response may include a cytotoxic T cell (CTL) response. In some embodiments, the infectious agent targets an APC, such as a dendritic cell (DC). In some embodiments, the nucleic acid molecule delivered by the infectious agents described herein include appropriate nucleic acid sequences necessary for the expression of the operably linked coding sequences encoding the variant immunomodulatory polypeptides, in a particular target cell, e.g., regulatory elements such as promoters.

In some embodiments, the infectious agent that contains nucleic acid sequences encoding the immunomodulatory polypeptides can also contain nucleic acid sequences that encode one or more additional gene products, e.g., cytokines, prodrug converting enzymes, cytotoxins and/or detectable gene products. For example, in some embodiments, the infectious agent is an oncolytic virus and the virus can include nucleic acid sequences encoding additional therapeutic gene products (see, e.g., Kirn et al., (2009) Nat Rev Cancer 9:64-71; Garcia-Aragoncillo et al., (2010) Curr Opin Mol Ther 12:403-411; see U.S. Pat. Nos. 7,588,767, 7,588,771, 7,662,398 and 7,754,221 and U.S. Pat. Publ. Nos. 2007/0202572, 2007/0212727, 2010/0062016, 2009/0098529, 2009/0053244, 2009/0155287, 2009/0117034, 2010/0233078, 2009/0162288, 2010/0196325, 2009/0136917 and 2011/0064650. In some embodiments, the additional gene product can be a therapeutic gene product that can result in death of the target cell (e.g., tumor cell) or gene products that can augment or boost or regulate an immune response (e.g., cytokine). Exemplary gene products also include among an anticancer agent, an anti-metastatic agent, an antiangiogenic agent, an immunomodulatory molecule, an immune checkpoint inhibitor, an antibody, a cytokine, a growth factor, an antigen, a cytotoxic gene product, a pro-apoptotic gene product, an anti-apoptotic gene product, a cell matrix degradative gene, genes for tissue regeneration and reprogramming human somatic cells to pluripotency, and other genes described herein or known to one of skill in the art. In some embodiments, the additional gene product is Granulocyte-macrophage colony-stimulating factor (GM-CSF).

### A. Viruses

In some embodiments, the infectious agent is a virus. In some embodiments, the infectious agent is an oncolytic virus, or a virus that targets particular cells, e.g., immune cells. In some embodiments, the infectious agent targets a tumor cell and/or cancer cell in the subject. In some embodiments, the infectious agent targets an immune cell or an antigen-presenting cell (APC).

In some embodiments, the infectious agent is an oncolytic virus. Oncolytic viruses are viruses that accumulate in tumor cells and replicate in tumor cells. By virtue of replication in the cells, and optional delivery of nucleic acids encoding immunomodulatory polypeptides described herein, tumor cells are lysed, and the tumor shrinks and can be eliminated. Oncolytic viruses can also have a broad host and cell type range. For example, oncolytic viruses can accumulate in immunoprivileged cells or immunoprivileged tissues, including tumors and/or metastases, and also including wounded tissues and cells, thus allowing the delivery and expression of nucleic acids encoding the immunomodulatory polypeptides described herein in a broad range of cell types. Oncolytic viruses can also replicate in a tumor cell specific manner, resulting in tumor cell lysis and efficient tumor regression.

Exemplary oncolytic viruses include adenoviruses, adeno-associated viruses, herpes viruses, Herpes Simplex Virus, Vesticular Stomatic virus, Reovirus, Newcastle Disease virus, parvovirus, measles virus, vesticular stomatitis virus (VSV), Coxsackie virus and Vaccinia virus. In some embodiments, oncolytic viruses can specifically colonize solid tumors, while not infecting other organs, and can be used as an infectious agent to deliver the nucleic acids encoding the immunomodulatory polypeptides described herein to such solid tumors.

Oncolytic viruses for use in delivering the nucleic acids encoding variant ICOSL polypeptides or immunomodulatory polypeptides described herein, can be any of those known to one of skill in the art and include, for example, vesicular stomatitis virus, see, e.g., U.S. Pat. Nos. 7,731,974, 7,153,510, 6,653,103 and U.S. Pat. Pub. Nos. 2010/0178684, 2010/0172877, 2010/0113567, 2007/0098743, 20050260601, 20050220818 and EP Pat. Nos. 1385466, 1606411 and 1520175; herpes simplex virus, see, e.g., U.S. Pat. Nos. 7,897,146, 7,731,952, 7,550,296, 7,537,924, 6,723,316, 6,428,968 and U.S. Pat. Pub. Nos., 2014/0154216, 2011/0177032, 2011/0158948, 2010/0092515, 2009/0274728, 2009/0285860, 2009/0215147, 2009/0010889, 2007/0110720, 2006/0039894, 2004/0009604, 2004/0063094, International Patent Pub. Nos., WO 2007/052029, WO 1999/038955; retroviruses, see, e.g., U.S. Pat. Nos. 6,689,871, 6,635,472, 5,851,529, 5,716,826, 5,716,613 and U.S. Pat. Pub. No. 20110212530; vaccinia viruses, see, e.g., 2016/0339066, and adeno-associated viruses, see, e.g., U.S. Pat. Nos. 8,007,780, 7,968,340, 7,943,374, 7,906,111, 7,927,585, 7,811,814, 7,662,627, 7,241,447, 7,238,526, 7,172,893, 7,033,826, 7,001,765, 6,897,045, and 6,632,670.

Oncolytic viruses also include viruses that have been genetically altered to attenuate their virulence, to improve their safety profile, enhance their tumor specificity, and they have also been equipped with additional genes, for example cytotoxins, cytokines, prodrug converting enzymes to improve the overall efficacy of the viruses (see, e.g., Kirn et al., (2009) Nat Rev Cancer 9:64-71; Garcia-Aragoncillo et al., (2010) Curr Opin Mol Ther 12:403-411; see U.S. Pat. Nos. 7,588,767, 7,588,771, 7,662,398 and 7,754,221 and U.S. Pat. Publ. Nos. 2007/0202572, 2007/0212727, 2010/0062016, 2009/0098529, 2009/0053244, 2009/0155287, 2009/0117034, 2010/0233078, 2009/0162288, 2010/0196325, 2009/0136917 and 2011/0064650). In some embodiments, the oncolytic viruses can be those that have been modified so that they selectively replicate in cancerous cells, and, thus, are oncolytic. For example, the oncolytic virus is an adenovirus that has been engineered to have modified tropism for tumor therapy and also as gene therapy vectors. Exemplary of such is ONYX-015, H101 and Ad5ΔCR (Hallden and Portella (2012) Expert Opin Ther Targets, 16:945-58) and TNFerade (McLoughlin et al. (2005) Ann. Surg. Oncol., 12:825-30), or a conditionally replicative adenovirus Oncorine®.

In some embodiments, the infectious agent is a modified herpes simplex virus. In some embodiments, the infectious agent is a modified version of Talimogene laherparepvec (also known as T-Vec, Imlygic or OncoVex GM-CSF), that is modified to contain nucleic acids encoding any of the immunomodulatory polypeptides described herein. In some embodiments, the infectious agent is a modified herpes simplex virus that is described, e.g., in WO 2007/052029, WO 1999/038955, US 2004/0063094, US 2014/0154216, or, variants thereof.

In some embodiments, the infectious agent is a virus that targets a particular type of cells in a subject that is administered the virus, e.g., a virus that targets immune cells or antigen-presenting cells (APCs). Dendritic cells (DCs) are essential APCs for the initiation and control of immune responses. DCs can capture and process antigens, migrate from the periphery to a lymphoid organ, and present the antigens to resting T cells in a major histocompatibility complex (MHC)-restricted fashion. In some embodiments, the infectious agent is a virus that specifically can target DCs to deliver nucleic acids encoding the immunomodulatory polypeptides for expression in DCs. In some embodiments, the virus is a lentivirus or a variant or derivative thereof, such as an integration-deficient lentiviral vector . In some embodiments, the virus is a lentivirus that is pseudotyped to efficiently bind to and productively infect cells expressing the cell surface marker dendritic cell-specific intercellular adhesion molecule-3-grabbing non-integrin (DC-SIGN), such as DCs. In some embodiments, the virus is a lentivirus pseudotyped with a Sindbis virus E2 glycoprotein or modified form thereof, such as those described in WO 2013/149167. In some embodiments, the virus allows for delivery and expression of a sequence of interest (e.g., a nucleic acid encoding any of the immunomodulatory polypeptides described herein) to a DC. In some embodiments, the virus includes those described in WO 2008/011636, US 2011/0064763, Tareen et al. (2014) Mol. Ther., 22:575-587, or variants thereof. Exemplary of a dendritic cell-tropic vector platform is ZVex™.

### B. Bacteria

In some embodiments, the infectious agent is a bacterium. For example, in some embodiments, the bacteria can deliver nucleic acids encoding any of the immunomodulatory polypeptides described herein to a target cell in the subject, such as a tumor cell, an immune cell, an antigen-presenting cell and/or a phagocytic cell. In some embodiments, the bacterium can be preferentially targeted to a specific environment within a subject, such as a tumor microenvironment (TME), for expression and/or secretion of the immunomodulatory polypeptides and/or to target specific cells in the environment for expression of the variant immunomodulatory polypeptides.

In some embodiments, the bacterium delivers the nucleic acids to the cells via bacterial-mediated transfer of plasmid DNA to mammalian cells (also referred to as "bactofection"). For example, in some embodiments, delivery of genetic material is achieved through entry of the entire bacterium into target cells. In some embodiments, spontaneous or induced bacterial lysis can lead to the release of plasmid for subsequent eukaryotic cell expression. In some embodiments, the bacterium can deliver nucleic acids to non-phagocytic mammalian cells (e.g., tumor cells) and/or to phagocytic cells, e.g., certain immune cells and/or APCs. In some embodiments, the nucleic acids delivered by the bacterium can be transferred to the nucleus of the cell in the subject for expression. In some embodiments, the nucleic acids also include appropriate nucleic acid sequences necessary for the expression of the operably linked sequences encoding the variant immunomodulatory polypeptides in a particular host cell, e.g., regulatory elements such as promoters or enhancers. In some embodiments, the infectious agent that is a bacterium can deliver nucleic acids encoding the immunomodulatory proteins in the form of an RNA, such as a pre-made translation-competent RNA delivered to the cytoplasm of the target cell for translation by the target cell's machinery.

In some embodiments, the bacterium can replicate and lyse the target cells, e.g,. tumor cells. In some embodiments, the bacterium can contain and/or release nucleic acid sequences and/or gene products in the cytoplasm of the target cells, thereby killing the target cell, e.g., tumor cell. In some embodiments, the infectious agent is bacterium that can replicate specifically in a particular environment in the subject, e.g., tumor microenvironment (TME). For example, in some embodiments, the bacterium can replicate specifically in anaerobic or hypoxic microenvironments. In some embodiments, conditions or factors present in particular environments, e.g., aspartate, serine, citrate, ribose or galactose produced by cells in the TME, can act as chemoattractants to attract the bacterium to the environment. In some embodiments, the bacterium can express and/or secrete the immunomodulatory proteins described herein in the environment, e.g., TME

In some embodiments, the infectious agent is a bacterium that is a Listeria sp., a Bifidobacterium sp., an Escherichia sp., a Clostridium sp., a Salmonella sp., a Shigella sp., a Vibrio sp. or a Yersinia sp. In some embodiments, the bacterium is selected from among one or more of *Listeria monocytogenes, Salmonella typhimurium, Salmonella choleraesuis, Escherichia coli, Vibrio cholera, Clostridium perfringens, Clostridium butyricum, Clostridium novyi, Clostridium acetobutylicum, Bifidobacterium infantis, Bifidobacterium longum* and *Bifidobacterium adolescentis.* In some embodiments, the bacterium is an engineered bacterium. In some embodiments, the bacterium is an engineered bacterium such as those described in, e.g., Seow and Wood (2009) Molecular Therapy 17(5):767-777; Baban et al. (2010) Bioengineered Bugs 1:6, 385-394; Patyar et al. (2010) J Biomed Sci 17:21; Tangney et al. (2010) Bioengineered Bugs 1:4, 284-287; van Pijkeren et al. (2010) Hum Gene Ther. 21(4):405-416; WO 2012/149364; WO 2014/198002; US 9103831; US 9453227; US 2014/0186401; US 2004/0146488; US 2011/0293705; US 2015/0359909 and EP 3020816. The bacterium can be modified to deliver nucleic acid sequences encoding any of the immunomodulatory polypeptides, conjugates and/or fusions provided herein, and/or to express such immunomodulatory polypeptides in the subject.

### VIII. COMPOSITIONS, METHODS, AND THERAPEUTIC APPLICATIONS

Provided herein are compositions and methods relating to the provided immunomodulatory proteins, engineered cells and infectious agents described herein for use in modulating immunological activity of a mammalian cell. The compositions can be used in associated methods to, for example, modulate immunological activity in an immunotherapy approach to the treatment of, for example, a mammalian cancer or, in other embodiments the treatment of autoimmune disorders. In some embodiments, the method comprises contacting an immunomodulatory protein (which may be secreted by an engineered cell) of the present invention with a mammalian cell under conditions that are permissive to specific binding of the affinity-modified IgSF domain and modulation of the immunological activity of the mammalian cell. The methods can be employed ex vivo or in vivo.

In some embodiments, the method of modulating immunological activity is achieved by expression and secretion of an immunomodulatory protein of the present invention by an immune cell, such as a lymphocyte (e.g., a T-cell or TIL) or NK cell engineered or infected to express and secrete the immunomodulatory protein. In some embodiments, the method of modulating immunological activity is achieved by expression and surface expression of an immunomodulatory protein of the present invention by an immune cell, such as a lymphocyte (e.g., a T-cell or TIL) or NK cell engineered or infected to express on their surface a transmembrane immunomodulatory protein. In some embodiments, a tumor cell can be infected, e.g. with an oncolytic virus, to express a secretable immunomodulatory protein or transmembrane immunomodulatory protein modulation of immune cells in the tumor environment. In some embodiments, the cell expressing and secreting the immunomodulatory protein is contacted with a mammalian cell such as an APC, a second lymphocyte or tumor cell under conditions that are permissive of specific binding of the affinity modified IgSF domain to a cognate binding partner on the mammalian cell such that immunological activity can be modulated in the mammalian cell.

In some embodiments, the method is conducted by adoptive cell transfer of engineered cells expressing and secreting the immunomodulatory protein (e.g., a T-cell) are infused back into the patient. In some embodiments, the method is conducted by adoptive cell transfer of engineered cells (e.g. T cells) expressing on their surface a transmembrane immunomodulatory protein.

Provided herein are methods of administering an effective amount of engineered cells configured to express and secrete or surface express an immunomodulatory proteins to a subject in need (for example a subject having a disease or disorder). The pharmaceutical compositions described herein can be used in a variety of therapeutic applications, such as the treatment of a disease. For example, in some embodiments the pharmaceutical composition is used to treat inflammatory or autoimmune disorders, cancer, organ transplantation, viral infections, and/or bacterial infections in a mammal. The pharmaceutical composition can modulate an immune response to treat the disease. For example, in some embodiments, the pharmaceutical composition stimulates the immune response, which can be useful, for example, in the treatment of cancer, viral infections, or bacterial infections. In some embodiments, the pharmaceutical composition suppresses an immune response, which can be useful in the treatment of inflammatory or autoimmune disorders, or organ transplantation.

The provided methods are believed to have utility in a variety of applications, including, but not limited to, e.g., in prophylactic or therapeutic methods for treating a variety of immune system diseases or conditions in a mammal in which modulation or regulation of the immune system and immune system responses is beneficial. For example, suppressing an immune response can be beneficial in prophylactic and/or therapeutic methods for inhibiting rejection of a tissue, cell, or organ transplant from a donor by a recipient. In a therapeutic context, the mammalian subject is typically one with an immune system disease or condition, and administration is conducted to prevent further progression of the disease or condition.

Cell compositions engineered to express and secrete immunomodulatory proteins of the present invention and associated methods can be used in immunotherapy applications. In some embodiments, cells isolated from a mammal, such as a mouse or human, and can be engineered to express and secrete or surface express an immunomodulatory protein. In some embodiments, the mammalian cell serving as a host cell for expression and secretion or surface expression of an immunomodulatory protein is a lymphocyte such as a tumor infiltrating lymphocyte (TIL), a natural killer (NK) cell, or a T-cell such as a CD8+ cytotoxic T lymphocyte or a CD4+ helper T lymphocyte. In some embodiments, the cells are autologous cells. In aspects of the provided method, the engineered cells are contacted, generally under physiological conditions, with a mammalian cell in which modulation of immunological activity is desired. For example, the mammalian cell can be a murine or human cell such as an antigen presenting cell or tumor cell. In some embodiments, the engineered cells are autologous cells. In other embodiments, the cells are allogeneic. Cells can be contacted in vivo or ex vivo. In some embodiments, the engineered cells are administered to the subject, such as by infusion. Thus, composition and methods can be used in adoptive cell transfer immunotherapy.

In some embodiments, the method is conducted by administration of a pharmaceutical compositions containing infectious agent containing a nucleic acid molecule encoding the immunomodulatory protein, either secretable immunomodulatory protein or transmembrane immunomodulatory protein. In some embodiments, the pharmaceutical composition contains a dose of infectious agents suitable for administration to a subject that is suitable for treatment. In some embodiments, the pharmaceutical composition contains an infectious agent that is a virus, at a single or multiple dosage amount, of between about between or between about 1×10⁵ and about 1×10¹² plaque-forming units (pfu), 1×10⁶ and 1×10¹⁰ pfu, or 1×10⁷ and 1×10¹⁰ pfu, each inclusive, such as at least or at least about or at about 1×10⁶, 1×10⁷, 1×10⁸, 1×10⁹, 2×10⁹, 3×10⁹, 4×10⁹, 5×10⁹ pfu or about 1×10¹⁰ pfu. In some embodiments, the pharmaceutical composition can contain a virus concentration of from or from about 10⁵ to about 10¹⁰ pfu/mL, for example, 5×10⁶ to 5×10⁹ or 1×10⁷ to 1×10⁹ pfu/mL, such as at least or at least about or at about 10⁶ pfu/mL, 10⁷ pfu/mL, 10⁸ pfu/mL or 10⁹ pfu/mL. In some embodiments, the pharmaceutical composition contains an infectious agent that is a bacterium, at a single or multiple dosage amount, of between about between or between about 1×10³ and about 1×10⁹ colony-forming units (cfu), 1×10⁴ and 1×10⁹ cfu, or 1×10⁵ and 1×10⁷ cfu, each inclusive, such as at least or at least about or at about 1×10⁴, 1×10⁵, 1×10⁶, 1×10⁷, 1×10⁸ or 1×10⁹ cfu. In some embodiments, the pharmaceutical composition can contain a bacterial concentration of from or from about 10³ to about 10⁸ cfu/mL, for example, 5×10⁵ to 5×10⁷ or 1×10⁶ to 1×10⁷ cfu/mL, such as at least or at least about or at about 10⁵ cfu/mL, 10⁶ cfu/mL, 10⁷ cfu/mL or 10⁸ cfu/mL

In some embodiments, an effective amount of a pharmaceutical composition is administered to inhibit, halt, or reverse progression of cancers that are sensitive to modulation of immunological activity by immunomodulatory proteins of the present invention. In some embodiments, the methods of the invention are used in the treatment of a mammalian patient of cancers such as lymphoma, lymphoid leukemia, myeloid leukemia, cervical cancer, neuroblastoma, or multiple myeloma. Other cancers which can be treated by the methods of the invention include, but are not limited to, melanoma, bladder cancer, hematological malignancies (leukemia, lymphoma, myeloma), liver cancer, brain cancer, renal cancer, breast cancer, pancreatic cancer (adenocarcinoma), colorectal cancer, lung cancer (small cell lung cancer and non-small-cell lung cancer), spleen cancer, cancer of the thymus or blood cells (i.e., leukemia), prostate cancer, testicular cancer, ovarian cancer, uterine cancer, gastric carcinoma, or Ewing's sarcoma.

Human cancer cells can be treated in vivo, or ex vivo. In ex vivo treatment of a human patient, tissue or fluids containing cancer cells are treated outside the body and then the tissue or fluids are reintroduced back into the patient. In some embodiments, the cancer is treated in a human patient in vivo by administration of the therapeutic composition into the patient. Thus, the present invention provides ex vivo and in vivo methods to inhibit, halt, or reverse progression of the tumor, or otherwise result in a statistically significant increase in progression-free survival (i.e., the length of time during and after treatment in which a patient is living with cancer that does not get worse), or overall survival (also called "survival rate;" i.e., the percentage of people in a study or treatment group who are alive for a certain period of time after they were diagnosed with or treated for cancer) relative to treatment with a control.

In some embodiments, a pharmaceutical composition of the invention can also be used to inhibit growth of mammalian, particularly human, cancer cells as a monotherapy (i.e., as a single agent), in combination with at least one chemotherapeutic agent (i.e., a combination therapy), in combination with a cancer vaccine, in combination with an immune checkpoint inhibitor and/or in combination with radiation therapy. In some aspects of the present disclosure, the immune checkpoint inhibitor is nivolumab, tremelimumab, pembrolizumab, ipilimumab, or the like.

In some embodiments, the provided compositions can attenuate an immune response, such as, for example, where the immunomodulatory protein comprises an affinity modified IgSF domain of an inhibitory ligand. In some embodiments, the compositions can be used to treat an autoimmune disease. In some embodiments, the administration of a therapeutic composition of the invention to a subject suffering from an immune system disease (e.g., autoimmune disease) can result in suppression or inhibition of such immune system attack or biological responses associated therewith. By suppressing this immune system attack on healthy body tissues, the resulting physical symptoms (e.g., pain, joint inflammation, joint swelling or tenderness) resulting from or associated with such attack on healthy tissues can be decreased or alleviated, and the biological and physical damage resulting from or associated with the immune system attack can be decreased, retarded, or stopped. In a prophylactic context, the subject may be one with, susceptible to, or believed to present an immune system disease, disorder or condition, and administration is typically conducted to prevent progression of the disease, disorder or condition, inhibit or alleviate symptoms, signs, or biological responses associated therewith, prevent bodily damage potentially resulting therefrom, and/or maintain or improve the subject's physical functioning.

In some embodiments, the inflammatory or autoimmune disorder is antineutrophil cytoplasmic antibodies (ANCA)-associated vasculitis, a vasculitis, an autoimmune skin disease, transplantation, a Rheumatic disease, an inflammatory gastrointestinal disease, an inflammatory eye disease, an inflammatory neurological disease, an inflammatory pulmonary disease, an inflammatory endocrine disease, or an autoimmune hematological disease.

In some embodiments, the pharmaceutical compositions comprising cells engineered to express and secrete immunomodulatory proteins can be used to treat one or more other immune disease or disorder in the subject. The immune system disease or disorder of the patient may be or involve, e.g., but is not limited to, Addison's Disease, Allergy, Alopecia Areata, Alzheimer's, Antineutrophil cytoplasmic antibodies (ANCA)-associated vasculitis, Ankylosing Spondylitis, Antiphospholipid Syndrome (Hughes Syndrome), arthritis, Asthma, Atherosclerosis, Atherosclerotic plaque, autoimmune disease (e.g., lupus, RA, MS, Graves' disease, etc.), Autoimmune Hemolytic Anemia, Autoimmune Hepatitis, Autoimmune inner ear disease, Autoimmune Lymphoproliferative syndrome, Autoimmune Myocarditis, Autoimmune Oophoritis, Autoimmune Orchitis, Azoospermia, Behcet's Disease, Berger's Disease, Bullous Pemphigoid, Cardiomyopathy, Cardiovascular disease, Celiac Sprue/Coeliac disease, Chronic Fatigue Immune Dysfunction Syndrome (CFIDS), Chronic idiopathic polyneuritis, Chronic Inflammatory Demyelinating, Polyradicalneuropathy (CIPD), Chronic relapsing polyneuropathy (Guillain-Barré syndrome), Churg-Strauss Syndrome (CSS), Cicatricial Pemphigoid, Cold Agglutinin Disease (CAD), COPD, CREST syndrome, Crohn's disease, Dermatitis, Herpetiformus, Dermatomyositis, diabetes, Discoid Lupus, Eczema, Epidermolysis bullosa acquisita, Essential Mixed Cryoglobulinemia, Evan's Syndrome, Exopthalmos, Fibromyalgia, Goodpasture's Syndrome, graft-related disease or disorder, Graves'Disease, GVHD, Hashimoto's Thyroiditis, Idiopathic Pulmonary Fibrosis, Idiopathic Thrombocytopenia Purpura (ITP), IgA Nephropathy, immunoproliferative disease or disorder (e.g., psoriasis), Inflammatory bowel disease (IBD), Insulin Dependent Diabetes Mellitus (IDDM), Interstitial lung disease, juvenile diabetes, Juvenile Arthritis, juvenile idiopathic arthritis (JIA), Kawasaki's Disease, Lambert-Eaton Myasthenic Syndrome, Lichen Planus, lupus, Lupus Nephritis, Lymphoscytic Lypophisitis, Ménière's Disease, Miller Fish Syndrome/acute disseminated encephalomyeloradiculopathy, Mixed Connective Tissue Disease, Multiple Sclerosis (MS), muscular rheumatism, Myalgic encephalomyelitis (ME), Myasthenia Gravis, Ocular Inflammation, Pemphigus Foliaceus, Pemphigus Vulgaris, Pernicious Anaemia, Polyarteritis Nodosa, Polychondritis, Polyglandular Syndromes (Whitaker's syndrome), Polymyalgia Rheumatica, Polymyositis, Primary Agammaglobulinemia, Primary Biliary Cirrhosis/Autoimmune cholangiopathy, Psoriasis, Psoriatic arthritis, Raynaud's Phenomenon, Reiter's Syndrome/Reactive arthritis, Restenosis, Rheumatic Fever, rheumatic disease, Rheumatoid Arthritis, Sarcoidosis, Schmidt's syndrome, Scleroderma, Sjörgen's Syndrome, Solid-organ transplant rejection (kidney, heart, liver, lung, etc.), Stiff-Man Syndrome, Systemic Lupus Erythematosus (SLE), systemic scleroderma, Takayasu Arteritis, Temporal Arteritis/Giant Cell Arteritis, Thyroiditis, Type 1 diabetes, Type 2 diabetes, Ulcerative colitis, Uveitis, Vasculitis, Vitiligo, Wegener's Granulomatosis, and preventing or suppressing an immune response associated with rejection of a donor tissue, cell, graft, or organ transplant by a recipient subject. Graft-related diseases or disorders include graft versus host disease (GVDH), such as associated with bone marrow transplantation, and immune disorders resulting from or associated with rejection of organ, tissue, or cell graft transplantation (e.g., tissue or cell allografts or xenografts), including, e.g., grafts of skin, muscle, neurons, islets, organs, parenchymal cells of the liver, etc. With regard to a donor tissue, cell, graft or solid organ transplant in a recipient subject, it is believed that a therapeutic composition of the invention disclosed herein may be effective in preventing acute rejection of such transplant in the recipient and/or for long-term maintenance therapy to prevent rejection of such transplant in the recipient (e.g., inhibiting rejection of insulin-producing islet cell transplant from a donor in the subject recipient suffering from diabetes).

In some embodiments, a therapeutic amount of the pharmaceutical composition is administered. Typically, precise amount of the compositions of the present invention to be administered can be determined by a physician with consideration of individual differences in age, weight, tumor size, extent of infection or metastasis, and condition of the patient (subject). It can generally be stated that a pharmaceutical composition comprising engineered cells, e.g. T cells, as described herein may be administered at a dosage of 10⁴ to 10⁹ cells/kg body weight, such as 10⁵ to 10⁶ cells/kg body weight, including all integer values within those ranges. Engineered cell compositions, such as T cell compositions, may also be administered multiple times at these dosages. The cells can be administered by using infusion techniques that are commonly known in immunotherapy (see, e.g., Rosenberg et al, New Eng. J. of Med. 319: 1676, 1988). The optimal dosage and treatment regime for a particular patient can readily be determined by one skilled in the art of medicine by monitoring the patient for signs of disease and adjusting the treatment accordingly.

The administration of the subject compositions may be carried out in any convenient manner, including by aerosol inhalation, injection, ingestion, transfusion, implantation or transplantation. The compositions described herein may be administered to a patient subcutaneously, intradermally, intratumorally, intranodally, intramedullary, intramuscularly, by intravenous (i.v.) injection, or intraperitoneally. In one embodiment, the therapeutic composition is administered to a patient by intradermal or subcutaneous injection. In another embodiment, the therapeutic composition is administered by i.v. injection. In some cases, the cell compositions may be injected directly into a tumor, lymph node, or site of infection.

### A. Pharmaceutical Compositions

Provided are pharmaceutical compositions containing the immunomodulatory protein, engineered cells configured to express and secrete or surface express such immunomodulatory proteins or infectious agents. In some embodiments, the pharmaceutical compositions and formulations include one or more optional pharmaceutically acceptable carrier or excipient.

Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

Such a formulation may, for example, be in a form suitable for intravenous infusion. A pharmaceutically acceptable carrier may be a pharmaceutically acceptable material, composition, or vehicle that is involved in carrying or transporting cells of interest from one tissue, organ, or portion of the body to another tissue, organ, or portion of the body. For example, the carrier may be a liquid or solid filler, diluent, excipient, solvent, or encapsulating material, or some combination thereof. Each component of the carrier is "pharmaceutically acceptable" in that it must be compatible with the other ingredients of the formulation. It also must be suitable for contact with any tissue, organ, or portion of the body that it may encounter, meaning that it must not carry a risk of toxicity, irritation, allergic response, immunogenicity, or any other complication that excessively outweighs its therapeutic benefits.

In some embodiments, the pharmaceutical composition is sterile. In some embodiments, the pharmaceutical composition is free or essentially free of bacteria or viruses. In some embodiments, the pharmaceutical composition is free or essentially free of cells other than the engineered cells described herein.

An effective amount of a pharmaceutical composition to be employed therapeutically will depend, for example, upon the therapeutic context and objectives. One skilled in the art will appreciate that the appropriate dosage levels for treatment will thus vary depending, in part, upon the molecule delivered, the indication for which the binding agent molecule is being used, the route of administration, and the size (body weight, body surface or organ size) and condition (the age and general health) of the patient. Accordingly, the clinician may titer the dosage and modify the route of administration to obtain the optimal therapeutic effect. The pharmaceutical composition of the invention can be administered parentally, subcutaneously, or intravenously, or as described elsewhere herein. The pharmaceutical composition of the invention may be administered in a therapeutically effective amount one, two, three or four times per month, two times per week, biweekly (every two weeks), or bimonthly (every two months). Administration may last for a period of 1, 2, 3, 4, 5, 6, 7, 8, 9, 10, 11, or 12 months or longer (e.g., one, two, three, four or more years, including for the life of the subject).

For any composition, the therapeutically effective dose can be estimated initially either in cell culture assays or in animal models such as mice, rats, rabbits, dogs, pigs, or monkeys. An animal model may also be used to determine the appropriate concentration range and route of administration. Such information can then be used to determine useful doses and routes for administration in humans. The exact dosage will be determined in light of factors related to the subject requiring treatment. Dosage and administration are adjusted to provide sufficient levels of the cell composition or to maintain the desired effect. Factors that may be taken into account include the severity of the disease state, the general health of the subject, the age, weight, and gender of the subject, time and frequency of administration, drug combination(s), reaction sensitivities, and response to therapy. Appropriate dosages may be ascertained through use of appropriate dose-response data. A number of biomarkers or physiological markers for therapeutic effect can be monitored including T cell activation or proliferation, cytokine synthesis or production (e.g., production of TNF-α, IFN-γ, IL-2), induction of various activation markers (e.g., CD25, IL-2 receptor), inflammation, joint swelling or tenderness, serum level of C-reactive protein, anti-collagen antibody production, and/or T cell-dependent antibody response(s).

A variety of means are known for determining if administration of a therapeutic composition of the invention sufficiently modulates immunological activity by eliminating, sequestering, or inactivating immune cells mediating or capable of mediating an undesired immune response; inducing, generating, or turning on immune cells that mediate or are capable of mediating a protective immune response; changing the physical or functional properties of immune cells; or a combination of these effects. Examples of measurements of the modulation of immunological activity include, but are not limited to, examination of the presence or absence of immune cell populations (using flow cytometry, immunohistochemistry, histology, electron microscopy, polymerase chain reaction (PCR)); measurement of the functional capacity of immune cells including ability or resistance to proliferate or divide in response to a signal (such as using T cell proliferation assays and pepscan analysis based on 3H-thymidine incorporation following stimulation with anti-CD3 antibody, anti-T cell receptor antibody, anti-CD28 antibody, calcium ionophores, PMA, antigen presenting cells loaded with a peptide or protein antigen; B cell proliferation assays); measurement of the ability to kill or lyse other cells (such as cytotoxic T cell assays); measurements of the cytokines, chemokines, cell surface molecules, antibodies and other products of the cells (e.g., by flow cytometry, enzyme-linked immunosorbent assays, Western blot analysis, protein microarray analysis, immunoprecipitation analysis); measurement of biochemical markers of activation of immune cells or signaling pathways within immune cells (e.g., Western blot and immunoprecipitation analysis of tyrosine, serine or threonine phosphorylation, polypeptide cleavage, and formation or dissociation of protein complexes; protein array analysis; DNA transcriptional, profiling using DNA arrays or subtractive hybridization); measurements of cell death by apoptosis, necrosis, or other mechanisms (e.g., annexin V staining, TUNEL assays, gel electrophoresis to measure DNA laddering, histology; fluorogenic caspase assays, Western blot analysis of caspase substrates); measurement of the genes, proteins, and other molecules produced by immune cells (e.g., Northern blot analysis, polymerase chain reaction, DNA microarrays, protein microarrays, 2-dimensional gel electrophoresis, Western blot analysis, enzyme linked immunosorbent assays, flow cytometry); and measurement of clinical symptoms or outcomes such as improvement of autoimmune, neurodegenerative, and other diseases involving self proteins or self polypeptides (clinical scores, requirements for use of additional therapies, functional status, imaging studies) for example, by measuring relapse rate or disease severity (using clinical scores known to the ordinarily skilled artisan) in the case of multiple sclerosis, measuring blood glucose in the case of type I diabetes, or joint inflammation in the case of rheumatoid arthritis.

### IX. EXEMPLARY EMBODIMENTS

By way of example, among the provided embodiments are:
1. An immunomodulatory protein comprising at least one non-immunoglobulin affinity-modified immunoglobulin superfamily (IgSF) domain comprising one or more amino acid substitutions in a wild-type IgSF domain, wherein:
   the at least one affinity-modified IgSF domain specifically binds at least one cell surface cognate binding partner of the wild-type IgSF domain;
   the immunomodulatory protein does not comprise a transmembrane domain; and
   the immunomodulatory protein is not conjugated to a half-life extending moiety.
2. The immunomodulatory protein of embodiment 1, wherein the half-life extending moiety is a multimerization domain.
3. The immunomodulatory protein of embodiment 1 or 2, wherein the half-life extending moiety is an Fc domain.
4. The immunomodulatory protein of any one of embodiments 1-3, wherein the immunomodulatory protein further comprises a signal peptide.
5. The immunomodulatory protein of embodiment 4, wherein the signal peptide is a native signal peptide from the corresponding wild-type IgSF member.
6. The immunomodulatory protein of embodiment 4, wherein the signal peptide is a non-native signal peptide.
7. The immunomodulatory protein of embodiment 4 or 6, wherein the signal peptide is an IgG-kappa signal peptide, an IL-2 signal peptide, or a CD33 signal peptide.
8. The immunomodulatory protein of any one of embodiments 1-7, wherein the at least one cell surface cognate binding partner is expressed on a mammalian cell.
9. The immunomodulatory protein of embodiment 8, wherein the mammalian cell is an antigen presenting cell (APC), a tumor cell, or a lymphocyte.
10. The immunomodulatory protein of embodiment 8 or 9, wherein the mammalian cell is a T-cell.
11. The immunomodulatory protein of any of embodiments 8-10, wherein the mammalian cell is a mouse, rat, cynomolgus monkey, or human cell.
12. The immunomodulatory protein of any of embodiments 1-11, wherein the at least one affinity modified IgSF domain has increased binding affinity to the at least one cell surface cognate binding partner compared with the wild-type IgSF domain.
13. The immunomodulatory protein of any of embodiments 8-12, wherein specific binding of the immunomodulatory protein comprising the at least one affinity-modified IgSF domain modulates immunological activity of the mammalian cell compared to the wild-type IgSF domain.
14. The immunomodulatory protein of any of embodiments 8-13, wherein specific binding of the immunomodulatory protein comprising the at least one affinity-modified IgSF domain increases immunological activity of the mammalian cell compared to the wild-type IgSF domain.
15. The immunomodulatory protein of any of embodiments 8-13, wherein specific binding of the immunomodulatory protein attenuates immunological activity of the mammalian cell compared to the wild-type IgSF domain.
16. The immunomodulatory protein of any of embodiments 1-15, wherein the wild-type IgSF domain is from an IgSF family member of a family selected from the group consisting of Signal-Regulatory Protein (SIRP) Family, Triggering Receptor Expressed On Myeloid Cells Like (TREML) Family, Carcinoembryonic Antigen-related Cell Adhesion Molecule (CEACAM) Family, Sialic Acid Binding Ig-Like Lectin (SIGLEC) Family, Butyrophilin Family, B7 family, CD28 family, V-set and Immunoglobulin Domain Containing (VSIG) family, V-set transmembrane Domain (VSTM) family, Major Histocompatibility Complex (MHC) family, Signaling lymphocytic activation molecule (SLAM) family, Leukocyte immunoglobulin-like receptor (LIR), Nectin (Nec) family, Nectin-like (NECL) family, Poliovirus receptor related (PVR) family, Natural cytotoxicity triggering receptor (NCR) family, T cell immunoglobulin and mucin (TIM) family, and Killer-cell immunoglobulin-like receptors (KIR) family.
17. The immunomodulatory protein of any of embodiments 1-16, wherein the wild-type IgSF domain is from an IgSF member selected from the group consisting of CD80, CD86, PD-L1, PD-L2, ICOS Ligand, B7-H3, B7-H4, CD28, CTLA4, PD-1, ICOS, BTLA, CD4, CD8-alpha, CD8-beta, LAG3, TIM-3, CEACAM1, TIGIT, PVR, PVRL2, CD226, CD2, CD160, CD200, CD200R, NKp30, VISTA, VSIG3, and VSIG8.
18. The immunomodulatory protein of any of embodiments 1-17, wherein the wild-type IgSF domain is a human IgSF domain.
19. The immunomodulatory protein of any of embodiments 1-18, wherein the at least one affinity modified IgSF domain has at least 90% sequence identity to a wild-type IgSF domain or a specific binding fragment thereof contained in the sequence of amino acids set forth in any of SEQ ID NOS: 1-27 and 408.
20. The immunomodulatory protein of any of embodiments 1-19, wherein the immunomodulatory protein has at least 90% sequence identity to the amino acid sequence selected from any of SEQ ID NOS:28-54 and 410 or to a specific binding fragment thereof containing an IgSF domain.
21. The immunomodulatory protein of any of embodiments 1-20, wherein the wild-type IgSF domain is a member of the B7 family.
22. The immunomodulatory protein of any of embodiments 1-21, wherein the wild-type IgSF domain is a domain of CD80, CD86 or ICOSL.
23. The immunomodulatory protein of any of embodiments 1-22, wherein the at least one cell surface cognate binding partner is a stimulatory receptor expressed on a T-cell, and the at least one affinity-modified IgSF domain has increased binding affinity to the stimulatory receptor compared to the binding affinity of the wild-type IgSF domain to the stimulatory receptor.
24. The immunomodulatory protein of embodiment 23, wherein binding of the affinity-modified IgSF domain to the stimulatory receptor increases immunological activity of the T-cell.
25. The immunomodulatory protein of embodiment 23 or 24, wherein the stimulatory receptor is CD28, ICOS, or CD226.
26. The immunomodulatory protein of any one of embodiments 23-25, wherein the at least one affinity-modified IgSF domain is an affinity-modified ICOSL IgSF domain that has increased binding affinity to at least one of: ICOS and CD28.
27. The immunomodulatory protein of any one of embodiments 23-26, wherein the at least one affinity-modified IgSF domain is an affinity modified ICOSL IgSF domain and the stimulatory receptor is ICOS.
28. The immunomodulatory protein of any one of embodiments 23-26, wherein the at least one affinity-modified IgSF domain is an affinity modified ICOSL IgSF domain and the stimulatory receptor is CD28.
29. The immunomodulatory protein of any one of embodiments 23-25, wherein the at least one affinity-modified IgSF domain is an affinity modified CD80 IgSF domain and the stimulatory receptor is CD28.
30. The immunomodulatory protein of any one of embodiments 23-29, wherein the affinity-modified IgSF domain does not substantially specifically bind to CTLA-4 or exhibits decreased binding affinity to CTLA-4 compared to the binding affinity of wild-type IgSF domain to CTLA-4.
31. The immunomodulatory protein of any of embodiments 1-30, wherein the at least one affinity-modified IgSF domain specifically binds to no more than one cell surface cognate binding partner.
32. The immunomodulatory protein of any of embodiments 1-31, wherein the immunomodulatory protein specifically binds to no more than one cell surface cognate binding partner.
33. The immunomodulatory protein of any of embodiments 1-30, wherein the at least one affinity-modified domain specifically binds to at least two cell surface cognate binding partners.
34. The immunomodulatory protein of embodiment 33, wherein:
   the first cell surface cognate binding partner is a stimulatory receptor expressed on a T cell; and
   the second cell surface cognate binding partner is an inhibitory ligand of an inhibitory receptor, wherein the inhibitory receptor is expressed on a T-cell.
35. The immunomodulatory protein of embodiment 34, wherein binding of the affinity-modified domain to the inhibitory ligand competitively inhibits binding of the inhibitory ligand to the inhibitory receptor.
36. The immunomodulatory protein of embodiment 34 or embodiment 35, wherein:
   the inhibitory receptor is PD-1, CTLA-4, LAG-3, TIGIT, CD96, CD112R, BTLA, CD160, TIM-3, VSIG3, or VSIG8; or
   the ligand of the inhibitory receptor is PD-L1, PD-L2, B7-1, B7-2, HVEM, MHC class II, PVR, CEACAM-1, GAL9, or VISTA.
37. The immunomodulatory protein of any one of embodiments 34-36, wherein the affinity modified IgSF domain is an affinity modified CD80 domain and the stimulatory receptor is CD28.
38. The immunomodulatory protein of embodiment 37, wherein the inhibitory ligand is PD-L1 and the inhibitory receptor is PD-1.
39. The immunomodulatory protein of embodiment 37 or embodiment 38, wherein the affinity-modified IgSF domain exhibits decreased binding affinity to CTLA-4 compared to the wild-type IgSF domain.
40. The immunomodulatory protein of any one of embodiments 37-39, wherein the affinity-modified IgSF domain does not substantially specifically bind to CTLA-4.
41. The immunomodulatory protein of any of embodiments 1-20, wherein the affinity modified IgSF domain is an affinity modified CD155 IgSF domain or an affinity modified CD112 IgSF domain and the at least one cell surface cognate binding partner is CD226, TIGIT or CD112R.
42. The immunomodulatory protein of embodiment 41, wherein the affinity-modified IgSF domain exhibits decreased binding affinity to CD226 compared to the binding affinity of the wild-type IgSF domain to CD226 and, optionally, retains or exhibits increased binding to TIGIT (T-cell immunoreceptor with Ig and ITIM domains) or CD112R compared to the binding affinity of the wild-type IgSF domain.
43. The immunomodulatory protein of any of embodiments 1-20, wherein the at least one affinity-modified IgSF domain specifically binds to a cell surface cognate binding partner that is a tumor specific antigen.
44. The immunomodulatory protein of embodiment 43, wherein the tumor specific antigen is B7-H6.
45. The immunomodulatory protein of embodiment 43 or 44, wherein the affinity-modified IgSF domain is an affinity modified NKp30 IgSF domain.
46. The immunomodulatory protein of any one of embodiments 1-45, wherein the at least one affinity-modified IgSF domain comprises a first affinity-modified IgSF domain and a second affinity-modified IgSF domain.
47. The immunomodulatory protein of embodiment 46, wherein the first affinity-modified IgSF domain and the second affinity-modified IgSF domain are different.
48. The immunomodulatory protein of embodiment 46 or 47, wherein the first affinity-modified IgSF domain and the second affinity-modified IgSF domain each comprise one or more different amino acid substitutions in the same wild-type IgSF domain.
49. The immunomodulatory protein of embodiment 46 or 47, wherein the first affinity-modified IgSF domain and the second affinity-modified IgSF domain each comprise one or more amino acid substitutions in a different wild-type IgSF domain.
50. The immunomodulatory protein of any of embodiments 1-20, wherein the wild-type IgSF domain is from an IgSF member that is a ligand of an inhibitory receptor, the inhibitory receptor comprising an ITIM signaling domain.
51. The immunomodulatory protein of embodiment 50, wherein:
   the inhibitory receptor is PD-1, CTLA-4, LAG3, TIGIT, TIM-3, or BTLA and the at least one affinity-modified IgSF domain is an affinity-modified IgSF domain of a ligand of PD-1, CTLA-4, LAG3, TIGIT, TIM-3, BTLA, VSIG3, or VSIG8, respectively; or
   the ligand of the inhibitory receptor is PD-L1, PD-L2, B7-1, B7-2, MHC class II, PVR, CEACAM-1, GAL9 or VISTA and the at least one affinity-modified IgSF domain is an affinity-modified IgSF domain of a ligand of PD-L1, PD-L2, B7-1, B7-2, MHC class II, PVR, CEACAM-1, GAL9 or VISTA, respectively.
52. The immunomodulatory protein of embodiment 50 or 51, wherein the inhibitory receptor is PD-1 and the at least one affinity-modified IgSF domain is an affinity-modified IgSF of PD-1.
53. The immunomodulatory protein of any of embodiments 50-52, wherein the affinity-modified IgSF domain has increased binding affinity for a trans surface cognate binding partner compared to the wildtype IgSF domain, whereby the increased binding affinity competitively inhibits binding of the trans surface cognate binding partner to the inhibitory receptor.
54. The immunomodulatory protein of any of embodiments 1-53, wherein the affinity modified IgSF domain differs by no more than ten amino acid substitutions from the wildtype IgSF domain.
55. The immunomodulatory protein of any of embodiments 1-54, wherein the affinity modified IgSF domain differs by no more than five amino acid substitutions from the wildtype IgSF domain.
56. The immunomodulatory protein of any of embodiments 1-55, wherein the one or more affinity-modified IgSF domain is or comprises an affinity modified IgV domain, affinity modified IgC1 domain, or an affinity modified IgC2 domain, or is a specific binding fragment thereof comprising the one or more amino acid substitutions.
57. The immunomodulatory protein of any of embodiments 1-56, wherein the immunomodulatory protein further comprises one or more non-affinity modified IgSF domains.
58. The immunomodulatory protein of any one of embodiments 1-56, wherein the immunomodulatory protein has been secreted from an engineered cell.
59. The immunomodulatory protein of embodiment 58, wherein the engineered cell is an immune cell.
60. The immunomodulatory protein of embodiment 58 or 59, wherein the engineered cell is a primary cell.
61. A recombinant nucleic acid encoding the immunomodulatory protein of any of embodiments 1-60.
62. The recombinant nucleic acid of embodiment 61, wherein the nucleic acid molecule further comprises at least one promoter operably linked to control expression of the immunomodulatory protein.
63. The recombinant nucleic acid of embodiment 62, wherein the promoter is a constitutively active promoter.
64. The recombinant nucleic acid of embodiment 62, wherein the promoter is an inducible promoter.
65. The recombinant nucleic acid of embodiment 64, wherein the promoter is responsive to an element responsive to T-cell activation signaling.
66. The recombinant nucleic acid of embodiment 64, or 65, wherein the promoter comprises a binding site for NFAT or a binding site for NF-κB
67. A recombinant expression vector comprising the nucleic acid of any of embodiments 61-66.
68. A recombinant expression vector comprising a nucleic acid encoding an immunomodulatory protein under the operable control of a signal sequence for secretion, wherein:
   the immunomodulatory protein comprises at least one non-immunoglobulin affinity-modified immunoglobulin superfamily (IgSF) domain comprising one or more amino acid substitutions in a wild-type IgSF domain, wherein the at least one affinity-modified IgSF domain specifically binds at least one cell surface cognate binding partner of the wild-type IgSF domain; and
   the encoded immunomodulatory protein is secreted when expressed from a cell.
69. The expression vector of embodiment 68, wherein the immunomodulatory protein does not comprise a transmembrane domain.
70. The expression vector of embodiment 68 or 69, wherein the immunomodulatory protein is not conjugated to a half-life extending moiety.
71. The expression vector of any one of embodiments 68-70, wherein the half-life extending moiety is a multimerization domain.
72. The expression vector of any one of embodiments 68-71, wherein the half-life extending moiety is an Fc domain.
73. The expression vector of any one of embodiments 68-72, wherein the signal sequence for secretion encodes a secretory signal peptide.
74. The expression vector of embodiment 73, wherein the signal peptide is a native signal peptide from the corresponding wild-type IgSF member.
75. The expression vector of embodiment 73, wherein the signal peptide is a non-native signal peptide.
76. The expression vector of embodiment 73 or 75, wherein the signal peptide is an IgG-kappa signal peptide, an IL-2 signal peptide, or a CD33 signal peptide.
77. The expression vector of any of embodiments 68-76, wherein the nucleic acid molecule further comprises at least one promoter operably linked to control expression of the immunomodulatory protein.
78. The expression vector of embodiment 77, wherein the promoter is a constitutively active promoter.
79. The expression vector of embodiment 77, wherein the promoter is an inducible promoter.
80. The expression vector of embodiment 79 wherein the promoter is responsive to an element responsive to T-cell activation signaling.
81. The expression vector of embodiment 79 or 80, wherein the promoter comprises a binding site for NFAT or a binding site for NF-κB
82. The expression vector of any of embodiments 68-81, wherein the vector is a viral vector.
83. The expression vector of embodiment 82, wherein the viral vector is a retroviral vector.
84. The expression vector of embodiment 82 or 83, wherein the viral vector is a lentiviral vector or a gammaretroviral vector.
85. The expression vector of any one of embodiments 68-84, wherein the at least one affinity-modified IgSF domain has increased binding affinity to the at least one cell surface cognate binding partner compared with the wild-type IgSF domain.
86. The expression vector of any one of embodiments 68-85, wherein the at least one cell surface cognate binding partner is expressed on a mammalian cell.
87. The expression vector of embodiment 86, wherein the mammalian cell is an antigen presenting cell (APC), a tumor cell, or a lymphocyte.
88. The expression vector of embodiment 86 or 87, wherein the mammalian cell is a T-cell.
89. The expression vector of any one of embodiments 86-88, wherein the mammalian cell is a mouse, rat, cynomolgus monkey, or human cell.
90. The expression vector of any one of embodiments 86-89, wherein specific binding of the immunomodulatory protein comprising the at least one affinity-modified IgSF domain modulates immunological activity of the mammalian cell compared to the wild-type IgSF domain.
91. The expression vector of any one of embodiments 86-90, wherein specific binding of the immunomodulatory protein comprising the at least one affinity-modified IgSF domain increases immunological activity of the mammalian cell compared to the wild-type IgSF domain.
92. The expression vector of any one of embodiments 86-910, wherein specific binding of the immunomodulatory protein attenuates immunological activity of the mammalian cell compared to the wild-type IgSF domain.
93. The expression vector of any one of embodiments 68-92, wherein the wild-type IgSF domain is from an IgSF family member of a family selected from the group consisting of Signal-Regulatory Protein (SIRP) Family, Triggering Receptor Expressed On Myeloid Cells Like (TREML) Family, Carcinoembryonic Antigen-related Cell Adhesion Molecule (CEACAM) Family, Sialic Acid Binding Ig-Like Lectin (SIGLEC) Family, Butyrophilin Family, B7 family, CD28 family, V-set and Immunoglobulin Domain Containing (VSIG) family, V-set transmembrane Domain (VSTM) family, Major Histocompatibility Complex (MHC) family, Signaling lymphocytic activation molecule (SLAM) family, Leukocyte immunoglobulin-like receptor (LIR), Nectin (Nec) family, Nectin-like (NECL) family, Poliovirus receptor related (PVR) family, Natural cytotoxicity triggering receptor (NCR) family, T cell immunoglobulin and mucin (TIM) family, and Killer-cell immunoglobulin-like receptors (KIR) family.
94. The expression vector of any one of embodiments 68-93, wherein the wild-type IgSF domain is from an IgSF member selected from the group consisting of CD80, CD86, PD-L1, PD-L2, ICOS Ligand, B7-H3, B7-H4, CD28, CTLA4, PD-1, ICOS, BTLA, CD4, CD8-alpha, CD8-beta, LAG3, TIM-3, CEACAM1, TIGIT, PVR, PVRL2, CD226, CD2, CD160, CD200, CD200R, NKp30, VISTA, VSIG3, and VSIG8.
95. The expression vector of any one of embodiments 68-94, wherein the wild-type IgSF domain is a human IgSF domain.
96. The expression vector of any one of embodiments 68-95, wherein the at least one affinity modified IgSF domain has at least 90% sequence identity to a wild-type IgSF domain or a specific binding fragment thereof contained in the sequence of amino acids set forth in any of SEQ ID NOS: 1-27 and 408.
97. The expression vector of any one of embodiments 68-96, wherein the immunomodulatory protein has at least 90% sequence identity to the amino acid sequence selected from any of SEQ ID NOS: 28-54 and 410.
98. The expression vector of any of embodiments 68-97, wherein the wild-type IgSF domain is a member of the B7 family.
99. The expression vector of any of embodiments 68-98, wherein the wild-type IgSF domain is a domain of CD80, CD86 or ICOSL.
100. The expression vector of any one of embodiments 68-99, wherein the at least one cell surface cognate binding partner is a stimulatory receptor expressed on a T-cell and the at least one affinity-modified IgSF domain has increased binding affinity to the stimulatory receptor compared to the binding affinity of the wild-type IgSF domain to the stimulatory receptor.
101. The expression vector of embodiment 100, wherein binding of the affinity-modified IgSF domain to the stimulatory receptor increases immunological activity of the T-cell.
102. The expression vector of embodiment 100 or 101, wherein the stimulatory receptor is CD28, ICOS, or CD226.
103. The expression vector of any one of embodiments 100-102, wherein the at least one affinity-modified IgSF domain is an affinity-modified ICOSL IgSF domain that has increased binding affinity to at least one of: ICOS and CD28.
104. The expression vector of any one of embodiments 100-103, wherein the at least one affinity-modified IgSF domain is an affinity modified ICOSL IgSF domain and the stimulatory receptor is ICOS.
105. The expression vector of any one of embodiments 100-103, wherein the at least one affinity-modified IgSF domain is an affinity modified ICOSL IgSF domain and the stimulatory receptor is CD28.
106. The expression vector of any one of embodiments 100-102, wherein the at least one affinity-modified IgSF domain is an affinity modified CD80 IgSF domain and the stimulatory receptor is CD28.
107. The expression vector of any one of embodiments 100-106, wherein the affinity-modified IgSF domain does not substantially specifically bind to CTLA-4 or exhibits decreased binding affinity to CTLA-4 compared to the binding affinity of wild-type IgSF domain to CTLA-4.
108. The expression vector of any one of embodiments 68-107, wherein the at least one affinity-modified IgSF domain specifically binds to no more than one cell surface cognate binding partner.
109. The expression vector of any one of embodiments 68-108, wherein the immunomodulatory protein specifically binds to no more than one cell surface cognate binding partner.
110. The expression vector of any one of embodiments 68-107, wherein the at least one affinity-modified domain specifically binds to at least two cell surface cognate binding partners.
111. The expression vector of embodiment 110, wherein:
   the first cell surface cognate binding partner is a stimulatory receptor expressed on a T cell; and
   the second cell surface cognate binding partner is an inhibitory ligand of an inhibitory receptor, wherein the inhibitory receptor is expressed on a T-cell.
112. The expression vector of embodiment 111, wherein binding of the affinity-modified IgSF domain to the inhibitory ligand competitively inhibits binding of the inhibitory ligand to the inhibitory receptor.
113. The expression vector of embodiment 111 or 112, wherein:
   the inhibitory receptor is PD-1, CTLA-4, LAG-3, TIGIT, CD96, CD112R, BTLA, CD160, TIM-3, VSIG3, or VSIG8; or
   the ligand of the inhibitory receptor is PD-L1, PD-L2, B7-1, B7-2, HVEM, MHC class II, PVR, CEACAM-1, GAL9 or VISTA.
114. The expression vector of any one of embodiments 111-113, wherein the affinity modified IgSF domain is an affinity modified CD80 domain and the stimulatory receptor is CD28.
115. The expression vector of embodiment 114, wherein the inhibitory ligand is PD-L1 and the inhibitory receptor is PD-1.
116. The expression vector of embodiment 114 or 115, wherein the affinity-modified IgSF domain exhibits decreased binding affinity to CTLA-4 compared to the wild-type IgSF domain.
117. The expression vector of any one of embodiments 114-116, wherein the affinity-modified IgSF domain does not substantially specifically bind to CTLA-4.
118. The expression vector of any of embodiments 68-97, wherein the affinity-modified IgSF domain is an affinity modified CD155 IgSF domain or an affinity modified CD112 IgSF domain and the at least one cell surface cognate binding partner is CD226, TIGIT or CD112R.
119. The expression vector of embodiment 118, wherein the affinity-modified IgSF domain exhibits decreased binding affinity to CD226 compared to the binding affinity of the wild-type IgSF domain to CD226 and, optionally, retains or exhibits increased binding to TIGIT (T-cell immunoreceptor with Ig and ITIM domains) or CD112R compared to the binding affinity of the wild-type IgSF domain.
120. The expression vector of any of embodiments 68-97, wherein the at least one affinity-modified IgSF domain specifically binds to a cell surface cognate binding partner that is a tumor specific antigen.
121. The expression vector of embodiment 120, wherein the tumor specific antigen is B7-H6.
122. The expression vector of embodiment 120 or 121, wherein the affinity-modified IgSF domain is an affinity modified NKp30 IgSF domain.
123. The expression vector of any one of embodiments 68-122, wherein the at least one affinity-modified IgSF domain comprises a first affinity-modified IgSF domain and a second affinity-modified IgSF domain.
124. The expression vector of embodiment 123, wherein the first affinity-modified IgSF domain and the second affinity-modified IgSF domain are different.
125. The expression vector of embodiment 123 or 124, wherein the first affinity-modified IgSF domain and the second affinity-modified IgSF domain each comprise one or more different amino acid substitutions in the same wild-type IgSF domain.
126. The expression vector of embodiment 123 or 124, wherein the first affinity-modified IgSF domain and the second affinity-modified IgSF domain each comprise one or more amino acid substitutions in a different wild-type IgSF domain.
127. The expression vector of any of embodiments 68-97, wherein the wild-type IgSF domain is from an IgSF member that is a ligand of an inhibitory receptor, the inhibitory receptor comprising an ITIM signaling domain.
128. The expression vector of embodiment 127, wherein:
   the inhibitory receptor is PD-1, CTLA-4, LAG3, TIGIT, TIM-3, or BTLA and the at least one affinity-modified IgSF domain is an affinity-modified IgSF domain of PD-1, CTLA-4, LAG3, TIGIT, TIM-3, BTLA, VSIG3, or VSIG8, respectively; or
   the ligand of the inhibitory receptor is PD-L1, PD-L2, B7-1, B7-2, MHC class II, PVR, CEACAM-1, GAL9 or VISTA and the at least one affinity-modified IgSF domain is an affinity-modified IgSF domain of a ligand of PD-L1, PD-L2, B7-1, B7-2, MHC class II, PVR, CEACAM-1, GAL9 or VISTA, respectively.
129. The expression vector of embodiment 127 or 128, wherein the inhibitory receptor is PD-1 and the at least one affinity-modified IgSF domain is an affinity-modified IgSF of PD-1.
130. The expression vector of any of embodiments 127-129, wherein the affinity-modified IgSF domain has increased binding affinity for a trans surface cognate binding partner compared to the wildtype IgSF domain, whereby the increased binding affinity competitively inhibits binding of the trans surface cognate binding partner to the inhibitory receptor.
131. The expression vector of any of embodiments 68-130, wherein the affinity modified IgSF domain differs by no more than ten amino acid substitutions from the wildtype IgSF domain.
132. The expression vector of any of embodiments 68-131, wherein the affinity modified IgSF domain differs by no more than five amino acid substitutions from the wildtype IgSF domain.
133. The expression vector of any of embodiments 68-132, wherein the one or more affinity-modified IgSF domain is or comprises an affinity modified IgV domain, affinity modified IgC1 domain, or an affinity modified IgC2 domain, or is a specific binding fragment thereof comprising the one or more amino acid substitutions.
134. The expression vector of any of embodiments 68-133, wherein the immunomodulatory protein further comprises one or more non-affinity modified IgSF domains.
135. An engineered cell comprising the nucleic acid of any one of embodiments 61-66 or the expression vector of any one of embodiments 67-134.
136. An engineered cell comprising the immunomodulatory protein of any one of embodiments 1-60.
137. An engineered cell that secretes the immunomodulatory protein of any one of embodiments 1-60.
138. The engineered cell of any of embodiments 135-137, wherein the cell is an immune cell.
139. An engineered immune cell comprising a nucleic acid molecule that encodes an immunomodulatory protein, wherein:
   the immunomodulatory protein comprises at least one non-immunoglobulin affinity-modified immunoglobulin superfamily (IgSF) domain comprising one or more amino acid substitutions in a wild-type IgSF domain, wherein the at least one affinity-modified IgSF domain specifically binds at least one cell surface cognate binding partner of the wild-type IgSF domain; and
   the engineered cell expresses and secretes the immunomodulatory protein.
140. The engineered immune cell of embodiment 139, wherein the immunomodulatory protein does not comprise a transmembrane domain.
141. The engineered immune cell of embodiment 139 or 140, wherein the immunomodulatory protein is not conjugated to a half-life extending moiety.
142. The engineered immune cell of embodiment 141, wherein the half-life extending moiety is a multimerization domain.
143. The engineered immune cell of embodiment 141 or 142, wherein the half-life extending moiety is an Fc domain.
144. The engineered immune cell of any one of embodiments 139-143, wherein the nucleic acid molecule comprises a sequence encoding a secretory signal peptide operably linked to the sequence encoding the immunomodulatory protein.
145. The engineered immune cell of embodiment 144, wherein the signal peptide is the native signal peptide from the corresponding wild-type IgSF member.
146. The engineered immune cell of embodiment 144, wherein the signal peptide is a non-native signal sequence.
147. The engineered immune cell of embodiment 144 or 146, wherein the signal peptide is an IgG-kappa signal peptide, an IL-2 signal peptide, or a CD33 signal peptide.
148. The engineered immune cell of any one of embodiments 139-148, wherein the nucleic acid molecule further comprises at least one promoter operably linked to control expression of the immunomodulatory protein.
149. The engineered immune cell of embodiment 148, wherein the promoter is a constitutively active promoter.
150. The engineered immune cell of embodiment 148, wherein the promoter is an inducible promoter.
151. The engineered immune cell of embodiment 148 or 150, wherein the promoter is responsive to an element responsive to T-cell activation signaling.
152. The engineered immune cell of any one of embodiments 148, 150, and 151, wherein the promoter comprises a binding site for NFAT or a binding site for NF-κB
153. The engineered cell of embodiments 135-152, wherein the immunomodulatory protein is expressed and secreted by the engineered cell after the engineered cell is contacted with an inducing agent or after induction of T cell activation signaling, which optionally is induced upon binding of an antigen to a chimeric antigen receptor (CAR) or engineered T-cell receptor (TCR) expressed by the engineered cell.
154. The engineered cell of any one of embodiments 135-153, wherein the cell is a lymphocyte.
155. The engineered cell of embodiment 154, wherein the lymphocyte is a T cell, a B cell or an NK cell.
156. The engineered cell of any one of embodiments 135-155, wherein the cell is a T cell.
157. The engineered cell of embodiment 156, wherein the T cells is CD4+ or CD8+.
158. The engineered cell of any one of embodiments 135-154, wherein the cell is an antigen presenting cell.
159. The engineered cell of any one of embodiments 135-158, wherein the cell is a primary cell obtained from a subject.
160. The engineered cell of embodiment 159, wherein the subject is a human subject.
161. The engineered cell of any one of embodiments 135-160, wherein the at least one affinity modified IgSF domain has increased binding affinity to the at least one cell surface cognate binding partner compared with the wild-type IgSF domain.
162. The engineered cell of any one of embodiments 135-161, wherein the at least one cell surface cognate binding partner is expressed on a mammalian cell.
163. The engineered cell of embodiment 162, wherein the mammalian cell is an antigen presenting cell (APC), a tumor cell, or a lymphocyte.
164. The engineered cell of embodiment 162 or 163, wherein the mammalian cell is a T-cell.
165. The engineered cell of any one of embodiments 162-164, wherein the mammalian cell is a mouse, rat, cynomolgus monkey, or human cell.
166. The engineered cell of any one of embodiments 162-165, wherein specific binding of the immunomodulatory protein comprising the at least one affinity-modified IgSF domain modulates immunological activity of the mammalian cell compared to the wild-type IgSF domain.
167. The engineered cell of any one of embodiments 162-166, wherein specific binding of the immunomodulatory protein comprising the at least one affinity-modified IgSF domain increases immunological activity of the mammalian cell compared to the wild-type IgSF domain.
168. The engineered cell of any one of embodiments 162-166, wherein specific binding of the immunomodulatory protein attenuates immunological activity of the mammalian cell compared to the wild-type IgSF domain.
169. The engineered cell of any one of embodiments 135-168, wherein the wild-type IgSF domain is from an IgSF family member of a family selected from the group consisting of Signal-Regulatory Protein (SIRP) Family, Triggering Receptor Expressed On Myeloid Cells Like (TREML) Family, Carcinoembryonic Antigen-related Cell Adhesion Molecule (CEACAM) Family, Sialic Acid Binding Ig-Like Lectin (SIGLEC) Family, Butyrophilin Family, B7 family, CD28 family, V-set and Immunoglobulin Domain Containing (VSIG) family, V-set transmembrane Domain (VSTM) family, Major Histocompatibility Complex (MHC) family, Signaling lymphocytic activation molecule (SLAM) family, Leukocyte immunoglobulin-like receptor (LIR), Nectin (Nec) family, Nectin-like (NECL) family, Poliovirus receptor related (PVR) family, Natural cytotoxicity triggering receptor (NCR) family, T cell immunoglobulin and mucin (TIM) family, and Killer-cell immunoglobulin-like receptors (KIR) family.
170. The engineered cell of any one of embodiments 135-169, wherein the wild-type IgSF domain is from an IgSF member selected from the group consisting of CD80, CD86, PD-L1, PD-L2, ICOS Ligand, B7-H3, B7-H4, CD28, CTLA4, PD-1, ICOS, BTLA, CD4, CD8-alpha, CD8-beta, LAG3, TIM-3, CEACAM1, TIGIT, PVR, PVRL2, CD226, CD2, CD160, CD200, CD200R, NKp30, VISTA, VSIG3, and VSIG8.
171. The engineered cell of any one of embodiments 135-170, wherein the wild-type IgSF domain is a human IgSF domain.
172. The engineered cell of any one of embodiments 135-171, wherein the at least one affinity modified IgSF domain has at least 90% sequence identity to a wild-type IgSF domain or a specific binding fragment thereof contained in the sequence of amino acids set forth in any of SEQ ID NOS: 1-27 and 408.
173. The engineered cell of any one of embodiments 135-172, wherein the immunomodulatory protein has at least 90% sequence identity to the amino acid sequence selected from any of SEQ ID NOS: 28-54 and 410.
174. The engineered cell of any one of embodiments 135-173, wherein the at least one cell surface cognate binding partner is a stimulatory receptor expressed on a T-cell and the at least one affinity-modified IgSF domain has increased binding affinity to the stimulatory receptor compared to the binding affinity of the wild-type IgSF domain to the stimulatory receptor.
175. The engineered cell of embodiment 174, wherein binding of the affinity-modified IgSF domain to the stimulatory receptor increases immunological activity of the T-cell.
176. The engineered cell of embodiment 174 or 175, wherein the stimulatory receptor is CD28, ICOS, or CD226.
177. The engineered cell of any one of embodiments 174-176, wherein the at least one affinity-modified IgSF domain is an affinity-modified ICOSL IgSF domain that has increased binding affinity to at least one of: ICOS and CD28.
178. The engineered cell of any one of embodiments 174-177, wherein the at least one affinity-modified IgSF domain is an affinity modified ICOSL IgSF domain and the stimulatory receptor is ICOS.
179. The engineered cell of any one of embodiments 174-177, wherein the at least one affinity-modified IgSF domain is an affinity modified ICOSL IgSF domain and the stimulatory receptor is CD28.
180. The engineered cell of any one of embodiments 174-176, wherein the at least one affinity-modified IgSF domain is an affinity modified CD80 IgSF domain and the stimulatory receptor is CD28.
181. The engineered cell of any one of embodiments 174-180, wherein the affinity-modified IgSF domain does not substantially specifically bind to CTLA-4 or exhibits decreased binding affinity to CTLA-4 compared to the binding affinity of wild-type IgSF domain to CTLA-4.
182. The engineered cell of any one of embodiments 135-181, wherein the at least one affinity-modified IgSF domain specifically binds to no more than one cell surface cognate binding partner.
183. The engineered cell of any one of embodiments 135-182, wherein the immunomodulatory protein specifically binds to no more than one cell surface cognate binding partner.
184. The engineered cell of any one of embodiments 135-181, wherein the at least one affinity-modified domain specifically binds to at least two cell surface cognate binding partners.
185. The engineered cell of embodiment 184, wherein:
   the first cell surface cognate binding partner is a stimulatory receptor expressed on a T cell; and
   the second cell surface cognate binding partner is an inhibitory ligand of an inhibitory receptor, wherein the inhibitory receptor is expressed on a T-cell.
186. The engineered cell of embodiment 185, wherein binding of the affinity-modified domain to the inhibitory ligand competitively inhibits binding of the inhibitory ligand to the inhibitory receptor.
187. The engineered cell of embodiment 185 or 186, wherein:
   the inhibitory receptor is PD-1, CTLA-4, LAG-3, TIGIT, CD96, CD112R, BTLA, CD160,TIM-3, VSIG3, or VSIG8; or
   the ligand of the inhibitory receptor is PD-L1, PD-L2, B7-1, B7-2, HVEM, MHC class II, PVR, CEACAM-1, GAL9 or VISTA.
188. The engineered cell of any one of embodiments 185-187, wherein the affinity modified IgSF domain is an affinity modified CD80 domain and the stimulatory receptor is CD28.
189. The engineered cell of embodiment 188, wherein the inhibitory ligand is PD-L1 and the inhibitory receptor is PD-1.
190. The engineered cell of embodiment 188 or 189, wherein the affinity-modified IgSF domain exhibits decreased binding affinity to CTLA-4 compared to the wild-type IgSF domain.
191. The engineered cell of any one of embodiments 188-190, wherein the affinity-modified IgSF domain does not substantially specifically bind to CTLA-4.
192. The engineered cell of any of embodiments 135-173, wherein the affinity modified IgSF domain is an affinity modified CD155 IgSF domain or an affinity modified CD112 IgSF domain and the at least one cell surface cognate binding partner is CD226, TIGIT or CD112R.
193. The engineered cell of embodiment 192, wherein the affinity-modified IgSF domain exhibits decreased binding affinity to CD226 compared to the binding affinity of the wild-type IgSF domain to CD226 and, optionally, retains or exhibits increased binding to TIGIT (T-cell immunoreceptor with Ig and ITIM domains) or CD112R compared to the binding affinity of the wild-type IgSF domain.
194. The engineered cell of any of embodiments 135-173, wherein the at least one affinity-modified IgSF domain specifically binds to a cell surface cognate binding partner that is a tumor specific antigen.
195. The engineered cell of embodiment 194, wherein the tumor specific antigen is B7-H6.
196. The engineered cell of embodiment 194 or 195, wherein the affinity-modified IgSF domain is an affinity modified NKp30 IgSF domain.
197. The engineered cell of any one of embodiments 135-173, wherein the at least one affinity-modified IgSF domain comprises a first affinity-modified IgSF domain and a second affinity-modified IgSF domain.
198. The engineered cell of embodiment 197, wherein the first affinity-modified IgSF domain and the second affinity-modified IgSF domain are different.
199. The engineered cell of embodiment 197 or 198, wherein the first affinity-modified IgSF domain and the second affinity-modified IgSF domain each comprises one or more different amino acid substitutions in the same wild-type IgSF domain.
200. The engineered cell of embodiment 197 or 198, wherein the first affinity-modified IgSF domain and the second affinity-modified IgSF domain each comprise one or more amino acid substitutions in a different wild-type IgSF domain.
201. The engineered cell of any of embodiments 135-173, wherein the wild-type IgSF domain is from an IgSF member that is a ligand of an inhibitory receptor, the inhibitory receptor comprising an ITIM signaling domain.
202. The engineered cell of embodiment 201, wherein:
   the inhibitory receptor is PD-1, CTLA-4, LAG3, TIGIT, TIM-3, or BTLA and the at least one affinity-modified IgSF domain is an affinity-modified IgSF domain of a ligand of PD-1, CTLA-4, LAG3, TIGIT, TIM-3, BTLA, VSIG3, or VSIG8, respectively; or
   the ligand of the inhibitory receptor is PD-L1, PD-L2, B7-1, B7-2, MHC class II, PVR, CEACAM-1, GAL9 or VISTA and the at least one affinity-modified IgSF domain is an affinity-modified IgSF domain of a ligand of PD-L1, PD-L2, B7-1, B7-2, MHC class II, PVR, CEACAM-1, GAL9 or VISTA, respectively.
203. The engineered cell of embodiment 201 or 202, wherein the inhibitory receptor is PD-1 and the at least one affinity-modified IgSF domain is an affinity-modified IgSF of PD-1.
204. The engineered cell of any of embodiments 201-203, wherein the affinity-modified IgSF domain has increased binding affinity for a trans surface cognate binding partner compared to the wildtype IgSF domain, whereby the increased binding affinity competitively inhibits binding of the trans surface cognate binding partner to the inhibitory receptor.
205. The engineered cell of any of embodiments 135-204, wherein the affinity modified IgSF domain differs by no more than ten amino acid substitutions from the wildtype IgSF domain.
206. The engineered cell of any of embodiments 135-205, wherein the affinity modified IgSF domain differs by no more than five amino acid substitutions from the wildtype IgSF domain.
207. The engineered cell of any of embodiments 135-206, wherein the one or more affinity-modified IgSF domain is or comprises an affinity modified IgV domain, affinity modified IgC1 domain, or an affinity modified IgC2 domain, or is a specific binding fragment thereof comprising the one or more amino acid substitutions.
208. The engineered cell of any of embodiments 135-207, wherein the immunomodulatory protein further comprises one or more non-affinity modified IgSF domains.
209. The engineered cell of any of embodiments 135-208, further comprising a chimeric antigen receptor (CAR) or an engineered T-cell receptor (TCR).
210. A pharmaceutical composition comprising the cell of any of embodiments 135-209 or the infectious agent of any of embodiments 230-252 and a pharmaceutically acceptable carrier.
211. The pharmaceutical composition of embodiment 210 that is sterile.
212. A method of introducing an immunomodulatory protein into a subject, comprising administering an engineered cell of any one of embodiments 135-209, the infectious agent of any of embodiments 230-252 or a pharmaceutical composition of embodiment 210 or 211 to the subject.
213. A method of modulating an immune response in a subject, comprising administering the cell of any one of embodiments 135-209, an infectious agent of any of embodiments 230-252 or a pharmaceutical composition of embodiment 210 or 211 to the subject.
214. The method of embodiment 213, wherein modulating the immune response treats a disease or disorder in the subject.
215. The method of embodiment 213 or 214, wherein the modulated immune response is increased.
216. The method of embodiment 214 or 215, wherein the disease or disorder is a tumor.
217. The method of any one of embodiments 214-216, wherein the disease or disorder is a cancer.
218. The method of any one of embodiments 214-217, wherein the disease or disorder is melanoma, lung cancer, bladder cancer, or a hematological malignancy.
219. The method of embodiment 213 or 214, wherein the modulated immune response is decreased.
220. The method of embodiment 214 or 219, wherein the disease or disorder is an inflammatory disease or condition.
221. The method of any one of embodiments 214, 219, and 220, wherein the disease or condition is Crohn's disease, ulcerative colitis, multiple sclerosis, asthma, rheumatoid arthritis, or psoriasis.
222. The method of any one of embodiments 212-221, wherein the subject is human.
223. The method of any of embodiments 212-222, wherein the cell is autologous to the subject.
224. The method of any of embodiments 212-222, wherein the cell is allogenic to the subject.
225. The method of any one of embodiments 212-224 wherein the engineered cell expresses and secretes the immunomodulatory protein.
226. The method of any one of embodiments 212-225, wherein the immunomodulatory protein is constitutively expressed by the engineered cell.
227. The method of any one of embodiments 212-225, wherein the immunomodulatory protein is expressed and secreted by the engineered cell after the engineered cell is contacted with an inducing agent.
228. The method of any one of embodiments 212-227, wherein the immunomodulatory protein is expressed and secreted by the engineered cell upon T cell activation signaling.
229. The method of embodiment 228, wherein the engineered cell expresses a chimeric antigen receptor (CAR) or an engineered T-cell receptor (TCR) and T cell activation signaling is induced upon binding of an antigen by the CAR or TCR.
230. An infectious agent, comprising a nucleic acid molecule encoding the immunomodulatory protein of any one of embodiments 1-60, a nucleic acid molecule of any of embodiments 61-66 or the expression vector of any one of embodiments 67-134.
231. An infectious agent, comprising a nucleic acid molecule encoding a transmembrane immunomodulatory protein (TIP) comprising:
   (i) an ectodomain comprising at least one non-immunoglobulin affinity-modified immunoglobulin superfamily (IgSF) domain comprising one or more amino acid substitution(s) in a wild-type IgSF domain, wherein the at least one affinity-modified IgSF domain specifically binds at least one cell surface cognate binding partner of the wild-type IgSF domain; and
   (ii) a transmembrane domain.
232. The infectious agent of embodiment 231, wherein the at least one affinity modified IgSF domain has increased binding affinity to the at least one cell surface cognate binding partner compared with the reference wild-type IgSF domain.
233. The infectious agent of embodiment 231 or embodiment 232, wherein the wild-type IgSF domain is from an IgSF family member of a family selected from Signal-Regulatory Protein (SIRP) Family, Triggering Receptor Expressed On Myeloid Cells Like (TREML) Family, Carcinoembryonic Antigen-related Cell Adhesion Molecule (CEACAM) Family, Sialic Acid Binding Ig-Like Lectin (SIGLEC) Family, Butyrophilin Family, B7 family, CD28 family, V-set and Immunoglobulin Domain Containing (VSIG) family, V-set transmembrane Domain (VSTM) family, Major Histocompatibility Complex (MHC) family, Signaling lymphocytic activation molecule (SLAM) family, Leukocyte immunoglobulin-like receptor (LIR), Nectin (Nec) family, Nectin-like (NECL) family, Poliovirus receptor related (PVR) family, Natural cytotoxicity triggering receptor (NCR) family, T cell immunoglobulin and mucin (TIM) family or Killer-cell immunoglobulin-like receptors (KIR) family.
234. The infectious agent of any of embodiments 231-233, wherein the wild-type IgSF domain is from an IgSF member selected from CD80, CD86, PD-L1, PD-L2, ICOS Ligand, B7-H3, B7-H4, CD28, CTLA4, PD-1, ICOS, BTLA, CD4, CD8-alpha, CD8-beta, LAG3, TIM-3, CEACAM1, TIGIT, PVR, PVRL2, CD226, CD2, CD160, CD200, CD200R or NKp30.
235. The infectious agent of any of embodiments 231-234, wherein the wild-type IgSF domain is a human IgSF member.
236. The infectious agent of any of embodiments 231-235, wherein the transmembrane immunomodulatory protein has at least 90% sequence identity to the amino acid sequence selected from any of SEQ ID NOS: 381-407 and 409 or to a contiguous portion thereof containing the affinity-modified IgSF domain and a transmembrane domain.
237. The infectious agent of any of embodiments 231-236, wherein the transmembrane immunomodulatory protein is a chimeric receptor, wherein the endodomain is not the endodomain from the wild-type IgSF member comprising the wild-type IgSF domain.
238. The infectious agent of embodiment 237, wherein the endodomain comprises at least one ITAM (immunoreceptor tyrosine-based activation motif)-containing signaling domain.
239. The infectious agent of embodiment 238, wherein the endodomain comprises a CD3-zeta signaling domain.
240. The infectious agent of embodiment 238 or embodiment 239, wherein the endodomain further comprises at least one of: a CD28 costimulatory domain, an ICOS signaling domain, an OX40 signaling domain, and a 41BB signaling domain.
241. The infectious agent of any of embodiments 231-240, wherein the affinity modified IgSF domain differs by no more than ten amino acid substitutions or no more than five amino acid substitutions from the wildtype IgSF domain.
242. The infectious agent of any of embodiments 231-241, wherein the affinity-modified IgSF domain is or comprises an affinity modified IgV domain, affinity modified IgC1 domain or an affinity modified IgC2 domain or is a specific binding fragment thereof comprising the one or more amino acid substitutions.
243. The infectious agent of any of embodiments 231-242, wherein the transmembrane domain is the native transmembrane domain from the corresponding wild-type IgSF member.
244. The infectious agent of any of embodiments 231-243, wherein the transmembrane domain is not the native transmembrane domain from the corresponding wild-type IgSF member.
245. The infectious agent of embodiment 244, wherein the transmembrane protein is a transmembrane protein derived from CD8.
246. The infectious agent of any of embodiments 230-245, wherein the infectious agent is a bacteria or a virus.
247. The infectious agent of embodiment 246, wherein the virus is an oncolytic virus.
248. The infectious agent of embodiment 247, wherein the oncolytic virus is an adenovirus, adeno-associated virus, herpes virus, Herpes Simplex Virus, Vesticular Stomatic virus, Reovirus, Newcastle Disease virus, parvovirus, measles virus, vesticular stomatitis virus (VSV), Coxsackie virus or a Vaccinia virus.
249. The infectious agent of embodiment 246, wherein the virus specifically targets dendritic cells (DCs) and/or is dendritic cell-tropic.
250. The infectious agent of embodiment 249, wherein the virus is a lentiviral vector that is pseudotyped with a modified Sindbis virus envelope product.
251. The infectious agent of any of embodiments 230-250, further comprising a nucleic acid molecule encoding a further gene product that results in death of a target cell or that can augment or boost an immune response.
252. The infectious agent of embodiment 251, wherein the further gene product is selected from an anticancer agent, anti-metastatic agent, an antiangiogenic agent, an immunomodulatory molecule, an immune checkpoint inhibitor, an antibody, a cytokine, a growth factor, an antigen, a cytotoxic gene product, a pro-apoptotic gene product, an anti-apoptotic gene product, a cell matrix degradative gene, genes for tissue regeneration or a reprogramming human somatic cells to pluripotency.

### X. EXAMPLES

The following examples are included for illustrative purposes only and are not intended to limit the scope of the invention.

Examples 1-12 describe the design, creation, and screening of exemplary affinity modified CD80 (B7-1), CD86 (B7-2), ICOSL, and NKp30 immunomodulatory proteins, which are components of the immune synapse (IS) that have a demonstrated dual role in both immune activation and inhibition. These examples demonstrate that affinity modification of IgSF domains yields proteins that can act to both increase and decrease immunological activity. This work also describes the various combinations of those domains fused in pairs (i.e., stacked) to form a Type II immunomodulatory protein to achieve immunomodulatory activity. The Examples also describe the generation of exemplary secretable immunomodulatory proteins containing such affinity-modified immunomodulatory proteins.

### Example 1

### Generation of Mutant DNA Constructs of IgSF Domains

Example 1 describes the generation of mutant DNA constructs of human CD80, CD86, ICOSL and NKp30 IgSF domains for translation and expression on the surface of yeast as yeast display libraries. The below examples exemplify binding and activity of affinity-modified domains of the exemplary IgSF proteins in an Fc-fusion format; such affinity-modified domains are contemplated in connection with a secretable immunomodulatory protein or transmembrane immunomodulatory protein as described.

### A. Degenerate Libraries

For libraries that target specific residues of target protein for complete or partial randomization with degenerate codons, such as specific mixed base sets to code for various amino acid substitutions, the coding DNAs for the extracellular domains (ECD) of human CD80 (SEQ ID NO:28), ICOSL (SEQ ID NO:32), and NKp30 (SEQ ID NO:54) were ordered from Integrated DNA Technologies (Coralville, IA) as a set of overlapping oligonucleotides of up to 80 base pairs (bp) in length. To generate a library of diverse variants of each ECD, the oligonucleotides contained desired degenerate codons at desired amino acid positions. Degenerate codons were generated using an algorithm at the URL: rosettadesign.med.unc.edu/SwiftLib/.

In general, positions to mutate and degenerate codons were chosen from crystal structures (CD80, NKp30) or homology models (ICOSL) of the target-ligand pairs of interest were used to identify ligand contact residues as well as residues that are at the protein interaction interface. This analysis was performed using a structure viewer available at the URL:spdbv.vital-it.ch). For example, a crystal structure for CD80 bound to CTLA4 is publicly available at the URL:www.rcsb.org/pdb/explore/explore.do?structureId=1I8L and a targeted library was designed based on the CD80::CTLA4 interface for selection of improved binders to CTLA4. However, there are no CD80 structures available with ligands CD28 and PD-L1, so the same library was also used to select for binders of CD28 (binds the same region on CD80 as CTLA4) and PD-L1 (not known if PD-L1 binds the same site as CTLA4). The next step in library design was the alignment of human, mouse, rat and monkey CD80, ICOSL or NKp30 sequences to identify conserved residues. Based on this analysis, conserved target residues were mutated with degenerate codons that only specified conservative amino acid changes plus the wild-type residue. Residues that were not conserved were mutated more aggressively, but also included the wild-type residue. Degenerate codons that also encoded the wild-type residue were deployed to avoid excessive mutagenesis of target protein. For the same reason, only up to 20 positions were targeted for mutagenesis at a time. These residues were a combination of contact residues and non-contact interface residues.

The oligonucleotides were dissolved in sterile water, mixed in equimolar ratios, heated to 95°C for five minutes and slowly cooled to room temperature for annealing. ECD-specific oligonucleotide primers that anneal to the start and end of the ECDs, respectively, were then used to generate PCR product. The ECD-specific oligonucleotides which overlap by 40-50bp with a modified version of pBYDS03 cloning vector (Life Technologies USA), beyond and including the BamHI and Kpnl cloning sites, were then used to amplify 100ng of PCR product from the prior step to generate a total of at least 12 µg of DNA for every electroporation. Both PCR's were by polymerase chain reaction (PCR) using OneTaq 2x PCR master mix (New England Biolabs, USA). The second PCR products were purified using a PCR purification kit (Qiagen, Germany) and resuspended in sterile deionized water. To prepare for library insertion, a modified yeast display version of vector pBYDS03 was digested with BamHI and Kpnl restriction enzymes (New England Biolabs, USA) and the large vector fragment was gel-purified and dissolved in sterile, deionized water. Electroporation-ready DNA for the next step was generated by mixing 12 µg of library DNA for every electroporation with 4 µg of linearized vector in a total volume of 50 µL deionized and sterile water. An alternative way to generate targeted libraries was to carry out site-directed mutagenesis (Multisite kit, Agilent, USA) of target ECDs with oligonucleotides containing degenerate codons. This approach was used to generate sublibraries that only target specific stretches of the DNA for mutagenesis. In these cases, sublibraries were mixed before proceeding to the selection steps. In general, library sizes were in the range of 10×10⁷ to 10×10⁸ clones, except that sublibraries were only in the range of 10×10⁴ to 10×10⁵. Large libraries and sublibraries were generated for CD80, ICOSL, CD86 and NKp30. Sublibraries were generated for CD80, ICOSL and NKp30.

### B. Random Libraries

Random libraries were also constructed to identify variants of the ECD of CD80 (SEQ ID NO:28), CD86 (SEQ ID NO: 29), ICOSL (SEQ ID NO:32) and NKp30 (SEQ ID NO:54. DNA encoding wild-type ECDs was cloned between the BamHI and Kpnl restriction sites of modified yeast display vector pBYDS03 and in some cases, the DNA was released using the same restriction enzymes. The released DNA or undigested plasmid was then mutagenized with the Genemorph II Kit (Agilent, USA) so as to generate an average of three to five amino acid changes per library variant. Mutagenized DNA was then amplified by the two-step PCR and further processed as described above for targeted libraries.

### Example 2

### Introduction of DNA Libraries into Yeast

Example 2 describes the introduction of CD80, CD86, ICOSL and NKp30 DNA libraries into yeast.

To introduce degenerate and random library DNA into yeast, electroporation-competent cells of yeast strain BJ5464 (ATCC.org; ATCC number 208288) were prepared and electroporated on a Gene Pulser II (Biorad, USA) with the electroporation-ready DNA from the step above essentially as described (Colby, D.W. et al. 2004 Methods Enzymology 388, 348-358). The only exception is that transformed cells were grown in non-inducing minimal selective SCD-Leu medium to accommodate the LEU2 selective marker carried by modified plasmid pBYDS03.

Library size was determined by plating serial dilutions of freshly recovered cells on SCD-Leu agar plates and then extrapolating library size from the number of single colonies from plating that generated at least 50 colonies per plate. The remainder of the electroporated culture was grown to saturation and cells from this culture were subcultured into the same medium once more to minimize the fraction of untransformed cells. To maintain library diversity, this subculturing step was carried out using an inoculum that contained at least 10x more cells than the calculated library size. Cells from the second saturated culture were resuspended in fresh medium containing sterile 25% (weight/volume) glycerol to a density of 10×10¹⁰ per mL and frozen and stored at -80°C (frozen library stock).

One liter of SCD-Leu media consists of 14.7 grams of sodium citrate dihydrate, 4.29 grams of citric acid monohydrate, 20 grams of dextrose, 6.7 grams of Difco brand yeast nitrogen base, and 1.6 grams yeast synthetic drop-out media supplement without leucine. Media was filtered sterilized before use using a 0.2 µM vacuum filter device.

Library size was determined by plating dilutions of freshly recovered cells on SCD-Leu agar plates and then extrapolating library size from the number of single colonies from a plating that generate at least 50 colonies per plate.

To segregate plasmid from cells that contain two or more different library clones, a number of cells corresponding to 10 times the library size, were taken from the overnight SCD-Leu culture and subcultured 1/100 into fresh SCD-Leu medium and grown overnight. Cells from this overnight culture were resuspended in sterile 25% (weight/volume) glycerol to a density of 10E10/mL and frozen and stored at -80°C (frozen library stock).

### Example 3

### Yeast Selection

Example 3 describes the selection of yeast expressing affinity modified variants of CD80, CD86, ICOSL and NKp30.

A number of cells equal to at least 10 times the library size were thawed from individual library stocks, suspended to 0.1 x 10E6 cells/mL in non-inducing SCD-Leu medium, and grown overnight. The next day, a number of cells equal to 10 times the library size were centrifuged at 2000 RPM for two minutes and resuspended to 0.5 x 10E6 cells/mL in inducing SCDG-Leu media. One liter of the SCDG-Leu induction media consists of 5.4 grams Na₂HPO₄, 8.56 grams of NaH₂PO₄^{∗}H₂0, 20 grams galactose, 2.0 grams dextrose, 6.7 grams Difco yeast nitrogen base, and 1.6 grams of yeast synthetic drop out media supplement without leucine dissolved in water and sterilized through a 0.22 µm membrane filter device. The culture was grown for two days at 20°C to induce expression of library proteins on the yeast cell surface.

Cells were processed with magnetic beads to reduce non-binders and enrich for all CD80, CD86, ICOSL or NKp30 variants with the ability to bind their exogenous recombinant counter-structure proteins (cognate binding partners). For example, yeast displayed targeted or random CD80 libraries were selected against each of CD28, CTLA-4, PD-L1. ICOSL libraries were selected against ICOS and CD28 and NKp30 libraries were selected against B7-H6. This was then followed by two to three rounds of fluorescence activated cell sorting (FACS) using exogenous cognate binding partner protein staining to enrich the fraction of yeast cells that displays improved binders. Magnetic bead enrichment and selections by flow cytometry are essentially as described in Keith D. Miller, 1 Noah B. Pefaur,2 and Cheryl L. Baird1 Current Protocols in Cytometry 4.7.1-4.7.30, July 2008.

With CD80, CD86, ICOSL, and NKp30 libraries, target ligand proteins were sourced from R&D Systems (USA) as follows: human rCD28.Fc (i.e., recombinant CD28-Fc fusion protein), rPDL1.Fc, rCTLA4.Fc, rICOS.Fc, and rB7H6.Fc. Magnetic streptavidin beads were obtained from New England Biolabs, USA. For biotinylation of cognate binding partner protein, biotinylation kit cat# 21955, Life Technologies, USA, was used. For two-color, flow cytometric sorting, a Becton Dickinson FACS Aria II sorter was used. CD80, CD86, ICOSL, or NKp30 display levels were monitored with an anti-hemagglutinin tag antibody labeled with Alexafluor 488 (Life Technologies, USA). Ligand binding Fc fusion proteins rCD28.Fc, rCTLA4.Fc, rPDL1.Fc, rICOS.Fc, or rB7-H6.Fc were detected with PE conjugated human Ig specific goat Fab (Jackson ImmunoResearch, USA). Doublet yeast were gated out using forward scatter (FSC) / side scatter (SSC) parameters, and sort gates were based upon higher ligand binding detected in FL2 that possessed more limited HA tag expression binding in FL1.

Yeast outputs from the flow cytometric sorts were assayed for higher specific binding affinity. Sort output yeast were expanded and re-induced to express the particular IgSF affinity modified domain variants they encode. This population then can be compared to the parental, wild-type yeast strain, or any other selected outputs, such as the bead output yeast population, by flow cytometry.

For ICOSL, the second sort outputs (F2) were compared to parental ICOSL yeast for binding of each rICOS.Fc, rCD28.Fc, and rCTLA4.Fc by double staining each population with anti-HA (hemagglutinin) tag expression and the anti-human Fc secondary to detect ligand binding.

In the case of ICOSL yeast variants selected for binding to ICOS, the F2 sort outputs gave Mean Fluorescence Intensity (MFI) values of 997, when stained with 5.6 nM rICOS.Fc, whereas the parental ICOSL strain MFI was measured at 397 when stained with the same concentration of rICOS.Fc. This represents a roughly three-fold improvement of the average binding in this F2 selected pool of clones, and it is predicted that individual clones from that pool will have much better improved MFI/affinity when individually tested.

In the case of ICOSL yeast variants selected for binding to CD28, the F2 sort outputs gave MFI values of 640 when stained with 100nM rCD28.Fc, whereas the parental ICOSL strain MFI was measured at 29 when stained with the same concentration of rCD28.Fc (22-fold improvement). In the case of ICOSL yeast variants selected for binding to CTLA4, the F2 sort outputs gave MFI values of 949 when stained with 100nM rCTLA4.Fc, whereas the parental ICOSL strain MFI was measured at 29 when stained with the same concentration of rCTLA4.Fc (32-fold improvement).

In the case of NKp30 yeast variants selected for binding to B7-H6, the F2 sort outputs gave MFI values of 533 when stained with 16.6nM rB7H6.Fc, whereas the parental NKp30 strain MFI was measured at 90 when stained with the same concentration of rB7H6.Fc (6-fold improvement).

Among the NKp30 variants that were identified, was a variant that contained mutations L30V/A60V/S64P/S86G with reference to positions in the NKp30 extracellular domain corresponding to positions set forth in SEQ ID NO:54. Among the CD86 variants that were identified, was a variant that contained mutations Q35H/H90L/Q102H with reference to positions in the CD86 extracellular domain corresponding to positions set forth in SEQ ID NO:29.

Importantly, the MFIs of all F2 outputs described above when measured with the anti-HA tag antibody on FL1 did not increase and sometimes decreased compared to wild-type strains, indicating that increased binding was not a function of increased expression of the selected variants on the surface of yeast, and validated gating strategies of only selecting mid to low expressors with high ligand binding.

### Example 4

### Reformatting Selection Outputs as Fc-Fusions and in Various Immunomodulatory Protein Types

Example 4 describes reformatting of selection outputs as immunomodulatory proteins containing an affinity modified (variant) extracellular domain (ECD) of CD80 or ICOSL fused to an Fc molecule (variant ECD-Fc fusion molecules).

Output cells from final flow cytometric CD80 and ICOSL sorts were grown to terminal density in SCD-Leu medium. Plasmid DNA from each output was isolated using a yeast plasmid DNA isolation kit (Zymo Research, USA). For Fc fusions, PCR primers with added restriction sites suitable for cloning into the Fc fusion vector of choice were used to batch-amplify from the plasmid DNA preps the coding DNA for the mutant target's ECD. After restriction digestion, the PCR products were ligated into an appropriate Fc fusion vector followed by chemical transformation into strain *E. coli* strain XL1 Blue *E. coli* (Agilent, USA) or NEB5alpha (New England Biolabs, USA) as directed by supplier. Exemplary of an Fc fusion vector is pFUSE-hIgG1-Fc2 (InvivoGen, USA).

Dilutions of transformation reactions were plated on LB-agar containing 100 µg/mL carbenicillin (Teknova, USA) to generate single colonies. Up to 96 colonies from each transformation were then grown in 96 well plates to saturation overnight at 37°C in LB-broth (Teknova cat # L8112) and a small aliquot from each well was submitted for DNA sequencing of the ECD insert in order to identify mutation(s) in all clones. Sample preparation for DNA sequencing was carried out using protocols provided by the service provider (Genewiz; South Plainfield, NJ). After removal of sample for DNA sequencing, glycerol was then added to the remaining cultures for a final glycerol content of 25% and plates were stored at -20°C for future use as master plates (see below). Alternatively, samples for DNA sequencing were generated by replica plating from grown liquid cultures to solid agar plates using a disposable 96 well replicator (VWR, USA). These plates were incubated overnight to generate growth patches and the plates were submitted to Genewiz as specified by Genewiz.

After identification of clones of interest from analysis of Genewiz-generated DNA sequencing data, clones of interest were recovered from master plates and individually grown to density in 5 mL liquid LB-broth containing 100 µg/mL carbenicillin (Teknova, USA) and 2 mL of each culture were then used for preparation of approximately 10 µg of miniprep plasmid DNA of each clone using a standard kit such as the Pureyield kit (Promega). Identification of clones of interest generally involved the following steps. First, DNA sequence data files were downloaded from the Genewiz website. All sequences were then manually curated so that they start at the beginning of the ECD coding region. The curated sequences were then batch-translated using a suitable program available at the URL www.ebi.ac.uk/Tools/st/emboss_transeq/. The translated sequences were then aligned using a suitable program available at the URL:multalin.toulouse.inra.fr/multalin/multalin.html. Alternatively, Genewiz sequenced were processed to generate alignments using Ugene software (http://ugene.net).

Clones of interest were then identified using the following criteria: 1.) identical clone occurs at least two times in the alignment and 2.) a mutation occurs at least two times in the alignment and preferably in distinct clones. Clones that meet at least one of these criteria were clones that have been enriched by our sorting process most likely due to improved binding.

The methods generated immunomodulatory proteins containing an ECD of CD80 or ICOSL with at least one affinity-modified domain in which the encoding DNA was generated to encode a protein designed as follows: signal peptide followed by variant (mutant) ECD followed by a linker of three alanines (AAA) followed by a human IgG1 Fc set forth in SEQ ID NO:2084 containing the mutation N297G (N82G with reference to wild-type human IgG1 Fc set forth in SEQ ID NO: 226). The human IgG1 Fc also contained the mutations R292C and V302C (corresponding to R77C and V87C with reference to wild-type human IgG1 Fc set forth in SEQ ID NO: 226). Since the construct does not include any antibody light chains that can form a covalent bond with a cysteine, the human IgG1 Fc also contained replacement of the cysteine residues to a serine residue at position 220 (C220S) by EU numbering (corresponding to position 5 (C5S) with reference 5 (C5S) compared to the wild-type or unmodified Fc set forth in SEQ ID NO: 226.

In addition, Example 8 below describes further immunomodulatory proteins that were generated as stack constructs containing at least two different affinity modified domains from identified variant CD80, CD86, ICOSL, and NKp30 molecules linked together and fused to an Fc.

### Example 5

### Expression and Purification of Fc-Fusions

Example 5 describes the high throughput expression and purification of Fc-fusion proteins containing variant ECD CD80, CD86, ICOSL, and NKp30 as described in the above Examples.

Recombinant variant Fc fusion proteins were produced from suspension-adapted human embryonic kidney (HEK) 293 cells using the Expi293 expression system (Invitrogen, USA). 4µg of each plasmid DNA from the previous step was added to 200µL Opti-MEM (Invitrogen, USA) at the same time as 10.8µL ExpiFectamine was separately added to another 200µL Opti-MEM. After 5 minutes, the 200µL of plasmid DNA was mixed with the 200µL of ExpiFectamine and was further incubated for an additional 20 minutes before adding this mixture to cells. Ten million Expi293 cells were dispensed into separate wells of a sterile 10mL, conical bottom, deep 24 well growth plate (Thomson Instrument Company, USA) in a volume 3.4mL Expi293 media (Invitrogen, USA). Plates were shaken for 5 days at 120 RPM in a mammalian cell culture incubator set to 95% humidity and 8% CO2. Following a 5 day incubation, cells were pelleted and culture supernatants were retained.

Proteins were purified from supernatants using a high throughput 96 well Protein A purification kit using the manufacturer's protocol (Catalog number 45202, Life Technologies, USA). Resulting elution fractions were buffer exchanged into PBS using Zeba 96 well spin desalting plate (Catalog number 89807, Life Technologies, USA) using the manufacturer's protocol. Purified protein was quantitated using 280nm absorbance measured by Nanodrop instrument (Thermo Fisher Scientific, USA), and protein purity was assessed by loading 5 µg of protein on NUPAGE pre-cast, polyacrylamide gels (Life Technologies, USA) under denaturing and reducing conditions and subsequent gel electrophoresis. Proteins were visualized in gel using standard Coomassie staining.

### Example 6

### Assessment of Binding and Activity of Affinity-Matured IgSF Domain-Containing Molecules

### A. Binding to Cell Surface-Expressed Cognate Binding Partners

This Example describes Fc-fusion binding studies of purified proteins from the above Examples to assess specificity and affinity of CD80 and ICOSL domain variant immunomodulatory proteins for cognate binding partners.

To produce cells expressing cognate binding partners, full-length mammalian surface expression constructs for each of human CD28, CTLA4, PD-L1, ICOS and B7-H6 were designed in pcDNA3.1 expression vector (Life Technologies) and sourced from Genscript, USA. Binding studies were carried out using the Expi293F transient transfection system (Life Technologies, USA) described above. The number of cells needed for the experiment was determined, and the appropriate 30 mL scale of transfection was performed using the manufacturer's suggested protocol. For each CD28, CTLA-4, PD-L1, ICOS, B7-H6, or mock 30 mL transfection, 75 million Expi293F cells were incubated with 30 µg expression construct DNA and 1.5mL diluted ExpiFectamine 293 reagent for 48 hours, at which point cells were harvested for staining.

For staining by flow cytometry, 200,000 cells of appropriate transient transfection or negative control were plated in 96 well round bottom plates. Cells were spun down and resuspended in staining buffer (PBS (phosphate buffered saline), 1% BSA (bovine serum albumin), and 0.1% sodium azide) for 20 minutes to block non-specific binding. Afterwards, cells were centrifuged again and resuspended in staining buffer containing 100nM to 1nM variant immunomodulatory protein, depending on the experiment of each candidate CD80 variant Fc, ICOSL variant Fc, or stacked IgSF variant Fc fusion protein in 50 µl. Primary staining was performed on ice for 45 minutes, before washing cells in staining buffer twice. PE-conjugated anti-human Fc (Jackson ImmunoResearch, USA) was diluted 1:150 in 50 µl staining buffer and added to cells and incubated another 30 minutes on ice. Secondary antibody was washed out twice, cells were fixed in 4% formaldehyde/PBS, and samples were analyzed on FACScan flow cytometer (Becton Dickinson, USA).

Mean Fluorescence Intensity (MFI) was calculated for each transfectant and negative parental line with Cell Quest Pro software (Becton Dickinson, USA).

### B.Bioactivity Characterization

This Example further describes Fc-fusion variant protein bioactivity characterization in human primary T cell in vitro assays.

### 1. Mixed Lymphocyte Reaction (MLR)

Soluble rICOSL.Fc or rCD80.Fc bioactivity was tested in a human Mixed Lymphocyte Reaction (MLR). Human primary dendritic cells (DC) were generated by culturing monocytes isolated from PBMC (BenTech Bio, USA) in vitro for 7 days with 500U/mL rIL-4 (R&D Systems, USA) and 250U/mL rGM-CSF (R&D Systems, USA) in Ex-Vivo 15 media (Lonza, Switzerland). 10,000 matured DC and 100,000 purified allogeneic CD4+ T cells (BenTech Bio, USA) were co-cultured with ICOSL variant fusion protein, CD80 variant Fc fusion protein, or controls in 96 well round bottom plates in 200µl final volume of Ex-Vivo 15 media. On day 5, IFN-gamma secretion in culture supernatants was analyzed using the Human IFN-gamma Duoset ELISA kit (R&D Systems, USA). Optical density was measured by VMax ELISA Microplate Reader (Molecular Devices, USA) and quantitated against titrated rIFN-gamma standard included in the IFN-gamma Duo-set kit (R&D Systems, USA).

### 2. Anti-CD3 Coimmobilization Assay

Costimulatory bioactivity of ICOSL fusion variants and CD80 Fc fusion variants was determined in anti-CD3 coimmobilization assays. InM or 4nM mouse anti-human CD3 (OKT3, Biolegends, USA) was diluted in PBS with 1nM to80nM rICOSL.Fc or rCD80.Fc variant proteins. This mixture was added to tissue culture treated flat bottom 96 well plates (Corning, USA) overnight to facilitate adherence of the stimulatory proteins to the wells of the plate. The next day, unbound protein was washed off the plates and 100,000 purified human pan T cells (BenTech Bio, US) or human T cell clone BC3 (Astarte Biologics, USA) were added to each well in a final volume of 200µL of Ex-Vivo 15 media (Lonza, Switzerland). Cells were cultured 3 days before harvesting culture supernatants and measuring human IFN-gamma levels with Duoset ELISA kit (R&D Systems, USA) as mentioned above.

### C.Results

Results for the binding and activity studies for exemplary tested variants are shown in Tables 12-15. In particular, Table 12 indicates exemplary IgSF domain amino acid substitutions (replacements) in the ECD of CD80 selected in the screen for affinity-maturation against the respective cognate structure CD28. Table 13 indicates exemplary IgSF domain amino acid substitutions (replacements) in the ECD of CD80 selected in the screen for affinity-maturation against the respective cognate structure PD-L1. Table 14 indicates exemplary IgSF domain amino acid substitutions (replacements) in the ECD of ICOSL selected in the screen for affinity-maturation against the respective cognate structures ICOS and CD28. For each Table, the exemplary amino acid substitutions are designated by amino acid position number corresponding to the respective reference unmodified ECD sequence as follows. For example, the reference unmodified ECD sequence in Tables 12 and 13 is the unmodified CD80 ECD sequence set forth in SEQ ID NO:28 and the reference unmodified ECD sequence in Table 14 is the unmodified ICOSL ECD sequence (SEQ ID NO:32). The amino acid position is indicated in the middle, with the corresponding unmodified (e.g. wild-type) amino acid listed before the number and the identified variant amino acid substitution listed after the number. Column 2 sets forth the SEQ ID NO identifier for the variant ECD for each variant ECD-Fc fusion molecule.

Also shown is the binding activity as measured by the Mean Fluorescence Intensity (MFI) value for binding of each variant Fc-fusion molecule to cells engineered to express the cognate binding partner ligand and the ratio of the MFI compared to the binding of the corresponding unmodified ECD-Fc fusion molecule not containing the amino acid substitution(s) to the same cell-expressed cognate binding partner ligand. The functional activity of the variant Fc-fusion molecules to modulate the activity of T cells also is shown based on the calculated levels of IFN-gamma in culture supernatants (pg/mL) generated either i) with the indicated variant ECD-Fc fusion molecule coimmoblized with anti-CD3 or ii) with the indicated variant ECD-Fc fusion molecule in an MLR assay. The Tables also depict the ratio of IFN-gamma produced by each variant ECD-Fc compared to the corresponding unmodified ECD-Fc in both functional assays.

As shown, the selections resulted in the identification of a number of CD80 or ICOSL IgSF domain variants that were affinity-modified to exhibit increased binding for at least one, and in some cases more than one, cognate binding partner ligand. In addition, the results showed that affinity modification of the variant molecules also exhibited improved activities to both increase and decrease immunological activity depending on the format of the molecule. For example, coimmobilization of the ligand likely provides a multivalent interaction with the cell to cluster or increase the avidity to favor agonist activity and increase T cell activation compared to the unmodified (e.g. wildtype) ECD-Fc molecule not containing the amino acid replacement(s). However, when the molecule is provided as a bivalent Fc molecule in solution, the same IgSF domain variants exhibited an antagonist activity to decrease T cell activation compared to the unmodified (e.g. wildtype) ECD-Fv molecule not containing the amino acid replacement(s).

| **Table 12: CD80 variants selected against CD28. Molecule sequences, binding data, and costimulatory bioactivity data.** | | | | | | |
|---|---|---|---|---|---|---|
| **CD80 mutation(s)** | **SEQ ID NO (ECD)** | **Binding** | | | **Coimmobilization with anti-CD3** | **MLR** |
| | | **CD28 MFI (parental ratio)** | **CTLA-4 MFI (parental ratio)** | **PD-L1 MFI (parental ratio)** | **IFN-gamma pg/mL (parental ratio)** | **IFN-gamma levels pg/mL (parental ratio)** |
| L70Q/A91G | 55 | 125 (1.31) | 283 (1.36) | 6 (0.08) | 93 (1.12) | 716 (0.83) |
| L70Q/A91G/T130A | 56 | 96 (1.01) | 234 (1.13) | 7 (0.10) | 99 (1.19) | 752 (0.87) |
| L70Q/A91G/I118A/T120S/T130A | 57 | 123 (1.29) | 226 (1.09) | 7 (0.10) | 86 (1.03) | 741 (0.86) |
| V4M/L70Q/A91G/T120S/T130A | 58 | 89 (0.94) | 263 (1.26) | 6 (0.09) | 139 (1.67) | 991 (1.14) |
| L70Q/A91G/T120S/T130A | 59 | 106 (1.12) | 263 (1.26) | 6 (0.09) | 104 (1.25) | 741 (0.86) |
| V20L/L70Q/A91S/T120S/T130A | 60 | 105 (1.11) | 200 (0.96) | 9 (0.13) | 195 (2.34) | 710 (0.82) |
| S44P/L70Q/A91G/T130A | 61 | 88 (0.92) | 134 (0.64) | 5 (0.07) | 142 (1.71) | 854 (0.99) |
| L70Q/A91G/E117G/T120S/T130A | 62 | 120 (1.27) | 193 (0.93) | 6 (0.08) | 98 (1.05) | 736 (0.85) |
| A91G/T120S/T130A | 63 | 84 (0.89) | 231 (1.11) | 44 (0.62) | 276 (3.33) | 714 (0.82) |
| L70R/A91G/T120S/T130A | 64 | 125 (1.32) | 227 (1.09) | 6 (0.09) | 105 (1.26) | 702 (0.81) |
| L70Q/E81A/A91G/T120S/II27T/T130A | 65 | 140 (1.48) | 185 (0.89) | 18 (0.25) | 98 (1.18) | 772 (0.89) |
| L70Q/Y87N/A91G/T130A | 66 | 108 (1.13) | 181 (0.87) | 6 (0.08) | 136 (1.63) | 769 (0.89) |
| T28S/L70Q/A91G/E95K/T120S/T130A | 67 | 32 (0.34) | 65 (0.31) | 6 (0.08) | 120 (1.44) | 834 (0.96) |
| N63S/L70Q/A91G/T120S/T130A | 68 | 124 (1.30) | 165 (0.79) | 6 (0.08) | 116 (1.39) | 705 (0.81) |
| K36E/I67T/L70Q/A91G/TI20S/T130A/N152T | 69 | 8 (0.09) | 21 (0.10) | 5 (0.08) | 53 (0.63) | 852 (0.98) |
| E52G/L70Q/A91G/T120S/T130A | 70 | 113 (1.19) | 245 (1.18) | 6 (0.08) | 94 (1.13) | 874 (1.01) |
| K37E/F59S/L70Q/A91G/T120S/T130A | 71 | 20 (0.21) | 74 (0.36) | 6 (0.08) | 109 (1.31) | 863 (1.00) |
| A91G/S103P | 72 | 39 (0.41) | 56 (0.27) | 9 (0.13) | 124 (1.49) | 670 (0.77) |
| K89E/T130A | 73 | 90 (0.95) | 148 (0.71) | 75 (1.07) | 204 (2.45) | 761 (0.88) |
| A91G | 74 | 96 (1.01) | 200 (0.96) | 85 (1.21) | 220 (2.65) | 877 (1.01) |
| D60V/A91G/T120S/T130A | 75 | 111 (1.17) | 222 (1.07) | 12 (0.18) | 120 (1.44) | 744 (0.86) |
| K54M/A91G/T120S | 76 | 68 (0.71) | 131 (0.63) | 5 (0.08) | 152 (1.83) | 685 (0.79) |
| M38T/L70Q/E77G/A91G/T120S/T130A/N152T | 77 | 61 (0.64) | 102 (0.49) | 5 (0.07) | 119 (1.43) | 796 (0.92) |
| R29H/E52G/L70R/E88G/A91G/T130A | 78 | 100 (1.05) | 119 (0.57) | 5 (0.08) | 200 (2.41) | 740 (0.85) |
| Y31H/T41G/L70Q/A91G/T120S/T130A | 79 | 85 (0.89) | 85 (0.41) | 6 (0.08) | 288 (3.47) | 782 (0.90) |
| V68A/T110A | 80 | 103 (1.08) | 233 (1.12) | 48 (0.68) | 163 (1,96) | 861 (0.99) |
| S66H/D90G/T110A/F116L | 81 | 33 (0.35) | 121 (0.58) | 11 (0.15) | 129 (1.55) | 758 (0.88) |
| R29H/E52G/T120S/T130A | 82 | 66 (0.69) | 141 (0.68) | 11 (0.15) | 124 (1.49) | 800 (0.92) |
| A91G/L102S | 83 | 6 (0.06) | 6 (0.03) | 5 (0.08) | 75 (0.90) | 698 (0.81) |
| I67T/L70Q/A91G/T120S | 84 | 98 (1.03) | 160 (0.77) | 5 (0.08) | 1751 (21.1) | 794 (0.92) |
| L70Q/A91G/T110A/T120S/T130A | 85 | 8 (0.09) | 14 (0.07) | 5 (0.07) | 77 (0.93) | 656 (0.76) |
| M38V/T41D/M43I/W50G/D76GN83A/K89E/T120S/T130A | 86 | 5 (0.06) | 8 (0.04) | 8 (0.11) | 82 (0.99) | 671 (0.78) |
| V22A/L70Q/S121P | 87 | 5 (0.06) | 7 (0.04) | 5 (0.07) | 105 (1.27) | 976 (1.13) |
| A12V/S15F/Y31H/T41G/T130A/P137L/N152T | 88 | 6 (0.06) | 6 (0.03) | 5 (0.08) | 104 (1.25) | 711 (0.82) |
| I67F/L70R/E88G/A91G/T120S/T130A | 89 | 5 (0.05) | 6 (0.03) | 6 (0.08) | 62 (0.74) | 1003 (1.16) |
| E24G/L25P/L70Q/T120S | 90 | 26 (0.27) | 38 (0.18) | 8 (0.11) | 101 (1.21) | 969 (1.12) |
| A91G/F92L/F108L/T120S | 91 | 50 (0.53) | 128 (0.61) | 16 (0.11) | 59 (0.71) | 665 (0.77) |
| WT CD80 | 28 | 95 (1.00) | 208 (1.00) | 70 (1.00) | 83 (1.00) | 866 (1.00) |

| **Table 13: CD80 variants selected against PD-L1. Molecule sequences, binding data, and costimulatory bioactivity data.** | | | | | | |
|---|---|---|---|---|---|---|
| **CD80 mutation(s)** | **SEQ ID NO (ECD)** | **Binding** | | | **Coimmobilization with anti-CD3** | **MLR** |
| | | **CD28 MFI (parental ratio)** | **CTLA-4 MFI (parental ratio)** | **PD-L1 MFI (parental ratio)** | **IFN-gamma pg/mL (parental ratio)** | **IFN-gamma levels pg/mL (parental ratio)** |
| R29D/Y31L/Q33H/ K36G/M38I/ T41A/ M43R/M47T/E81V/ L85R/K89N/A91T/ F92P/K93V/ R94L/ I118T/N149S | 92 | 1071 (0.08) | 1089 (0.02) | 37245 (2.09) | 387 (0.76) | 5028 (0.26) |
| R29D/Y31L/Q33H/ K36G/M38I/T41A/ M43R/M47T/E81V/ L85R/K89N/A91T/ F92P/K93V/R94L/ N144S/N149S | 93 | 1065 (0.08) | 956 (0.02) | 30713 (1.72) | 400 (0.79) | 7943 (0.41) |
| R29D/Y31L/Q33H/ K36G/M38I/T41A/ M42T/M43R/M47T/ E81V/L85R/K89N/ A91 T/F92P/K93V / R94L/L148S/N149S | 94 | 926 (0.07) | 954 (0.02) | 47072 (2.64) | 464 (0.91) | 17387 (0.91) |
| E24G/R29D/Y31L/ Q33H/K36G/M38I/ T41A/M43R/M47T/ F59L/E81V/L85R/ K89N/A91 T/F92P/ K93V /R94 L/H96R | 95 | 1074 (0.08) | 1022 (0.02) | 1121 (0.06) | 406 (0.80) | 13146 (0.69) |
| R29D/Y31L/Q33H/ K36G/M38I/T41A/ M43R/M47T/E81V/ L85R/K89N/A91T/ F92P/K93V/R94L/ N149S | 96 | 1018 (0.08) | 974 (0.02) | 25434 (1.43) | 405 (0.80) | 24029 (1.25) |
| R29V /M43Q/E81R/ L85I/K89R/D90L/ A91E/F92N/K93Q/ R94G | 97 | 1029 (0.08) | 996 (0.02) | 1575 (0.09) | 342 (0.67) | 11695 (0.61) |
| T41I/A91G | 98 | 17890 (1.35) | 50624 (1.01) | 12562 (0.70) | 433 (0.85) | 26052 (1.36) |
| K89R/D90K/A91 G/ F92Y/K93R/N122S/ N178S | 99 | 41687 (3.15) | 49429 (0.99) | 20140 (1.13) | 773 (1.52) | 6345 (0.33) |
| K89R/D90K/A91G/ F92Y/K93R | 100 | 51663 (3.91) | 72214 (1.44) | 26405 (1.48) | 1125 (2.21) | 9356 (0.49) |
| K36G/K37Q/M38I/ F59L/E81V/L85R/ K89N/A91T/F92P/ K93V/R94L/E99G/ T130A/N149S | 101 | 1298 (0.10) | 1271 (0.03) | 3126 (0.18) | 507 (1.00) | 3095 (0.16) |
| AE88D/K89R/D90K/ A91G/F92Y/K93R | 102 | 31535 (2.38) | 50868 (1.02) | 29077 (1.63) | 944 (1.85) | 5922 (0.31) |
| K36G/K37Q/M38I/ L40M | 103 | 1170 (0.09) | 1405 (0.03) | 959 (0.05) | 427 (0.84) | 811 (0.04) |
| K36G | 104 | 29766 (2.25) | 58889 (1.18) | 20143 (1.13) | 699 (1.37) | 30558 (1.59) |
| WTCD80 | 28 | 13224 (1.00) | 50101 (1.00) | 17846 (1.00) | 509 (1.00) | 19211 (1.00) |

| **Table 14: ICOSL variants selected against CD28 or ICOS. Molecule sequences, binding data, and costimulatory bioactivity data.** | | | | | |
|---|---|---|---|---|---|
| **ICOSL mutation(s)** | **SEQ ID NO (ECD)** | **Binding** | | **Coimmobilization with anti-CD3** | **MLR** |
| | | **ICOS OD (parental ratio)** | **CD28 MFI (parental ratio)** | **IFN-gamma pg/mL (parental ratio)** | **IFN-gamma levels pg/mL (parental ratio)** |
| N52S | 109 | 1.33 (1.55) | 162 (9.00) | 1334 (1.93) | 300 (0.44) |
| N52H | 110 | 1.30 (1.51) | 368 (20.44) | 1268 (1.83) | 39 (0.06) |
| N52D | 111 | 1.59 (1.85) | 130 (7.22) | 1943 (2.80) | 190 (0.28) |
| N52Y/N57Y/ F138L/L203P | 112 | 1.02 (1.19) | 398 (22.11) | 510* (1.47*) | 18 (0.03) |
| N52H/N57Y/ Q100P | 113 | 1.57 (1.83) | 447 (24.83) | 2199 (3.18) | 25 (0.04) |
| N52S/Y146C/ Y152C | 114 | 1.26 (1.47) | 39 (2.17) | 1647 (2.38) | 152 (0.22) |
| N52H/C198R | 115 | 1.16 (1.35) | 363 (20.17) | 744* (2.15*) | ND (ND) |
| N52H/C140del/ T225A | 1563 | ND (ND) | 154 (8.56) | 522* (1.51*) | ND (ND) |
| N52H/C198R/ T225A | 117 | 1.41 (1.64) | 344 (19.11) | 778* (2.25*) | 0 (0) |
| N52H/K92R | 118 | 1.48 | 347 | 288* | 89 |
| | | (1.72) | (19.28) | (0.83*) | (0.13) |
| N52H/S99G | 119 | 0.09 (0.10) | 29 (1.61) | 184* (0.53*) | 421 (0.61) |
| N52Y | 120 | 0.08 (0.09) | 18 (1.00) | 184* (0.53*) | 568 (0.83) |
| N57Y | 121 | 1.40 (1.63) | 101 (5.61) | 580* (1.68*) | 176 (0.26) |
| N57Y/Q100P | 122 | 0.62 (0.72) | 285 (15.83) | 301* (0.87*) | 177 (0.26) |
| N52S/S130G/ Y152C | 123 | 0.16 (0.19) | 24 (1.33) | 266* (0.77*) | 1617 (2.35) |
| N52S/Y152C | 124 | 0.18 (0.21) | 29 (1.61) | 238* (0.69*) | 363 (0.53) |
| N52S/C198R | 125 | 1.80 (2.09) | 82 (4.56) | 1427 (2.06) | 201 (0.29) |
| N52Y/N57Y/Y152C | 126 | 0.08 (0.09) | 56 (3.11) | 377* (1.09*) | 439 (0.64) |
| N52Y/N57Y/ H129P/C198R | 127 | ND (ND) | 449 (24.94) | 1192 (1.72) | ND (ND) |
| N52H/L161P/ C198R | 128 | 0.18 (0.21) | 343 (19.05) | 643* (1.86*) | 447 (0.65) |
| N52S/T113E | 129 | 1.51 (1.76) | 54 (3.00) | 451* (1.30*) | 345 (0.50) |
| S54A | 130 | 1.62 (1.88) | 48 (2.67) | 386* (1.12*) | 771 (1.12) |
| N52S/S54P | 1564 | 1.50 (1.74) | 38 (2.11) | 476* (1.38*) | 227 (0.33) |
| N52K/L208P | 132 | 1.91 (2.22) | 291 (16.17) | 1509 (2.18) | 137 (0.20) |
| N52S/Y152H | 133 | 0.85 (0.99) | 68 (3.78) | 2158 (3.12) | 221 (0.32) |
| N52D/V151A | 134 | 0.90 (1.05) | 19 (1.06) | 341* (0.99*) | 450 (0.66) |
| N52H/I143T | 135 | 1.83 (2.13) | 350 (19.44) | 2216 (3.20) | 112 (0.16) |
| N52S/L80P | 136 | 0.09 (0.10) | 22 (1.22) | 192* (0.55*) | 340 (0.49) |
| F120S/Y152H/ N201S | 137 | 0.63 (0.73) | 16 (0.89) | 351* (1.01*) | 712 (1.04) |
| N52S/R75Q/L203P | 138 | 1.71 (1.99) | 12 (0.67) | 1996 (2.88) | 136 (0.20) |
| N52S/D158G | 139 | 1.33 (1.55) | 39 (2.17) | 325* (0.94*) | 277 (0.40) |
| N52D/Q133H | 140 | 1.53 (1.78) | 104 (5.78) | 365* (1.05*) | 178 (0.26) |
| WT ICOSL | 32 | 0.86 (1.00) | 18 (1.00) | 692 / 346* (1.00) | 687 (1.00) |

| | | | | | |
|---|---|---|---|---|---|
| *: Parental ratio calculated using 346 pg/mL IFN-gamma for WT ICOSL | | | | | |

### Example 7

### Ligand Binding Competition Assay

As shown in Example 6, several CD80 variant molecules exhibited improved binding to one or both of CD28 and PD-L1. To further assess the binding activity of CD80 to ligands CD28 and PD-L1, this Example describes a ligand competition assay assessing the non-competitive nature of exemplary CD80 variants to bind both CD28 and PD-L1.

An ELISA based binding assay was set up incorporating plate-bound CD80 variant A91G ECD-Fc to assess the ability of CD80 to simultaneously bind CD28 and PD-L1. Maxisorp 96 well ELISA plates (Nunc, USA) were coated overnight with 100nM human recombinant CD80 variant A91G ECD-Fc fusion protein in PBS. The following day unbound protein was washed out, and the plate was blocked with 1% bovine serum albumin (Millipore, USA)/PBS for 1 hour at room temperature. This blocking reagent was washed out three times with PBS/ 0.05% Tween, which included a two minute incubation on a platform shaker for each wash.

In one arm of the competition assay, CD80 was incubated with CD28, and then CD28-bound CD80 was then assessed for competitive binding in the presence of either the other known CD80 cognate binding partner PD-L1 or CTLA-4 or negative control ligand PD-L2. Specifically, biotinylated recombinant human CD28 Fc fusion protein (rCD28.Fc; R&D Systems) was titrated into the wells starting at 10nM, diluting out for eight points with 1:2 dilutions in 25 µL volume. Immediately after adding the biotinylated rCD28.Fc, unlabeled competitive binders, recombinant human PD-L1 monomeric his- tagged protein, recombinant human CTLA-4 monomeric his-tagged protein, or a negative control human recombinant PD-L2 Fc fusion protein (R&D Systems) were added to wells at 2000/1000/500nM respectively in 25 µL volume for a final volume of 50 µL. These proteins were incubated together for one hour before repeating the three wash steps as described above.

After washing, 2.5 ng per well of HRP-conjugated streptavidin (Jackson Immunoresearch, USA) was diluted in 1%BSA/PBS and added to wells to detect bound biotinylated rCD28.Fc. After one hour incubation, wells were washed again three times as described above. To detect signal, 50 µL of TMB substrate (Pierce, USA) was added to wells following wash and incubated for 7 minutes, before adding 50ul 2M sulfuric acid stop solution. Optical density was determined on an Emax Plus microplate reader (Molecular Devices, USA). Optical density values were graphed in Prism (Graphpad, USA).

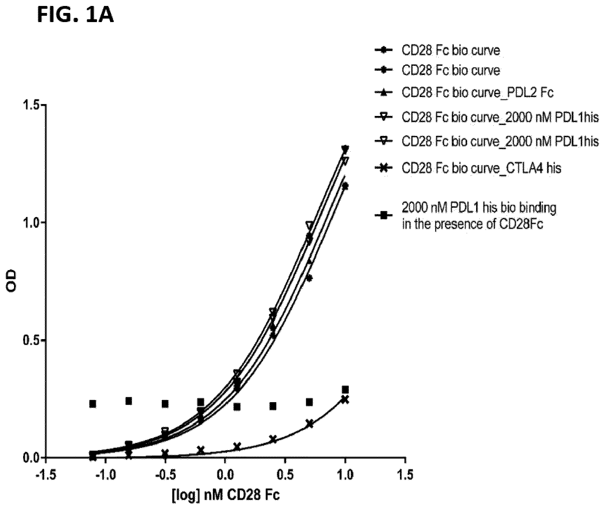

The results are set forth in FIG. 1A. The results showed decreased binding of biotinylated rCD28.Fc to the CD80 variant A91G ECD-Fc fusion protein with titration of the rCD28.Fc. When rCD28.Fc binding was performed in the presence of non-competitive control protein, rPDL2, there was no decrease in CD28 binding for CD80 (solid triangle). In contrast, a competitive control protein, rCTLA-4, when incubated with the CD28.Fc, did result in decreased CD28 binding for CD80 as expected (x line). When recombinant PD-L1 was incubated with CD28.Fc, no decrease in CD28 binding to CD80 was observed, which demonstrated that the epitopes of CD28 and PD-L1 for CD80 are non-competitive. Binding of the recombinant PD-L1 protein used in the CD28 competition assay to CD80 was confirmed by incubating the biotinylated PD-L1in the presence of non-biotinylated rCD28.Fc (square).

The reverse competition also was set up in which CD80 was incubated with PD-L1, and then PD-L1-bound CD80 was then assessed for competitive binding in the presence of either the other known CD80 cognate binding partners CD28 or CTLA-4 or negative control ligand PD-L2. Specifically, the assay was performed by titrating biotinylated recombinant human PD-L1-his monomeric protein into wells containing the recombinant CD80 variant. Because binding is weaker with this ligand, titrations started at 5000nM with similar 1:2 dilutions over eight points in 25 µL. When the rPD-L1-his was used to detect binding, the competitive ligands human rCD28.Fc, human rCTLA-4.Fc, or human rPD-L2.Fc control were added at 2.5nM final concentration in 25 µL for a total volume of 50 µL. The subsequent washes, detection, and OD measurements were the same as described above.

The results are set forth in FIG. IB. Titrated PD-L1-his binding alone confirmed that PD-L1 bound to the CD80 variant A91G ECD-Fc fusion molecule immobilized on the plate (square). When PD-L1-his binding was performed in the presence of non-competitive control protein, rPDL2, there was no decrease in PD-L1 binding for CD80 (triangle). The CD28-competitive control protein, rCTLA-4, when incubated with the PD-L1-his, did not result in decreased PD-L1 binding for CD80 (x line), even though CTLA-4 is competitive for CD28. This result further demonstrated that lack of competition between CD28 and PD-L1 for CD80 binding. Finally, when PD-L1-his was incubated with CD28.Fc, no decrease in PD-L1 binding to CD80 was observed, which demonstrated that the epitopes of CD28 and PD-L1 for CD80 are non-competitive.

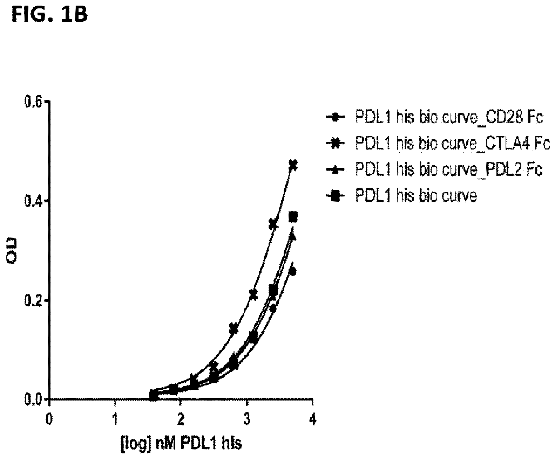

Thus, the results showed that CTLA-4, but not PD-L1 or the negative control PD-L2, competed for binding of CD28 to CD80 (FIG. 1A) and that CD28, CTLA-4, and PD-L2 did not compete for binding of PD-L1 to CD80 (FIG. 1B). Thus, these results demonstrated that CD28 and PD-L1 are non-competitive binders of CD80, and that this non-competitive binding can be demonstrated independently of which ligand is being detected in the ELISA.

### Example 8

### Generation and Assessment of Stacked Molecules Containing Different Affinity-Modified Domains

Selected variant molecules described above that were affinity-modified for one or more cognate binding partners were used to generate "stack" molecule (i.e., Type II immunomodulatory protein) containing two or more affinity-modified IgSF domains. Stack constructs were obtained as geneblocks (Integrated DNA Technologies, Coralville, IA) that encode the stack in a format that enables its fusion to Fc by standard Gibson assembly using a Gibson assembly kit (New England Biolabs). As above, this example exemplifies binding and activity of a molecule containing the affinity-modified domains in an Fc-fusion format; such stack molecules containing two or more affinity-modified domains are contemplated in connection with a secretable immunomodulatory protein or transmembrane immunomodulatory protein as described.

The encoding nucleic acid molecule of all stacks was generated to encode a protein designed as follows: Signal peptide, followed by the first variant IgV of interest, followed by a 15 amino acid linker which is composed of three GGGGS(G4S) motifs (SEQ ID NO:228), followed by the second IgV of interest, followed by two GGGGS linkers (SEQ ID NO: 229) followed by three alanines (AAA), followed by a human IgG1 Fc as described above. To maximize the chance for correct folding of the IgV domains in each stack, the first IgV was preceded by all residues that normally occur in the wild-type protein between this IgV and the signal peptide (leading sequence). Similarly, the first IgV was followed by all residues that normally connect it in the wild-type protein to either the next Ig domain (typically an IgC domain) or if such a second IgV domain is absent, the residues that connect it to the transmembrane domain (trailing sequence). The same design principle was applied to the second IgV domain except that when both IgV domains were derived from same parental protein (e.g. a CD80 IgV stacked with another CD80 IgV), the linker between both was not duplicated.

Table 15 sets forth the design for exemplary stacked constructs. The exemplary stack molecules shown in Table 15 contain the IgV domains as indicated and additionally leading or trailing sequences as described above. In the Table, the following components are present in order: signal peptide (SP; SEQ ID NO:225), IgV domain 1 (IgV1), trailing sequence 1 (TS1), linker 1 (LR1; SEQ ID NO:228), IgV domain 2(IgV2), trailing sequence 2 (TS2), linker 2 (LR2; SEQ ID NO:230) and Fc domain (SEQ ID NO:226 containing C5S/N82G amino acid substitution). In some cases, a leading sequence 1(LS1) is present between the signal peptide and IgV1 and in some cases a leading sequence 2 (LS2) is present between the linker and IgV2.

| **Table 15: Amino acid sequence (SEQ ID NO) of components of exemplary stacked constructs** | | | | | | | | | | |
|---|---|---|---|---|---|---|---|---|---|---|
| | **SP** | **First domain** | | | **LR1** | **Second domain** | | | **LR2** | **Fc** |
| | | **LS1** | **IgV1** | **TS1** | | **LS2** | **IgV2** | **TS2** | | |
| NKp30 WT ICOSL WT | + | - | SEQ ID NO: 214 | SEQ ID NO: 235 | + | - | SEQ ID NO: 196 | SEQ ID NO: 233 | + | + |
| NKp30 L30V/A60V/S64P/ S86G ICOSL N52S/N57Y/H94D /L96F/L98F/Q100 R | + | - | SEQ ID NO: 215 | SEQ ID NO: 235 | + | - | SEQ ID NO: 212 | SEQ ID NO: 233 | + | + |
| NKp30 L30V/A60V/S64P/ S86G) ICOSL N52D | + | - | SEQ ID NO: 215 | SEQ ID NO: 235 | + | - | SEQ ID NO: 199 | SEQ ID NO: 233 | + | + |
| NKp30 L30V/A60V/S64P/ S86G ICOSL N52H/N57Y/Q100 P | + | - | SEQ ID NO: 215 | SEQ ID NO: 235 | + | - | SEQ ID NO: 201 | SEQ ID NO: 233 | + | + |
| ICOSL WT Nkp30 WT | + | - | SEQ ID NO: 196 | SEQ ID NO: 233 | + | - | SEQ ID NO: 214 | SEQ ID NO: 235 | + | + |
| ICOSL N52D NKp30 L30V/A60V/S64P/ S86G | + | - | SEQ ID NO: 199 | SEQ ID NO: 233 | + | - | SEQ ID NO: 215 | SEQ ID NO: 235 | + | + |
| ICOSL N52H/N57Y/Q100 P NKp30 L30V/A60V/S64P/ S86G | + | - | SEQ ID NO: 201 | SEQ ID NO: 233 | + | - | SEQ ID NO: 215 | SEQ ID NO: 235 | + | + |
| Domain 1: NKp30 WT Domain 2 : CD80 WT | + | - | SEQ ID NO: 214 | SEQ ID NO: 235 | + | - | SEQ ID NO: 152 | SEQ ID NO: 231 | + | + |
| Domain 1: NKp30 WT Domain 2 : CD86 WT | + | - | SEQ ID NO: 214 | SEQ ID NO: 235 | + | SEQ ID NO: 236 | SEQ ID NO: 220 | SEQ ID NO: 237 | + | + |
| Domain 1: NKp30 L30V/A60V/S64P/ S86G Domain 2: CD80 R29H/Y31H/T41G /Y87N/E88G/K89E /D90N/A91G/P109 S | + | - | SEQ ID NO: 215 | SEQ ID NO: 235 | + | - | SEQ ID NO: 192 | SEQ ID NO: 231 | + | + |
| Domain 1: NKp30 L30V/A60V/S64P/ S86G Domain 2: CD80 I67T/L70Q/A91G/ T120S | + | - | SEQ ID NO: 215 | SEQ ID NO: 235 | + | - | SEQ ID NO: 175 | SEQ ID NO: 231 | + | + |
| Domain 1: NKp30 L30V/A60V/S64P/ S86G Domain 2: CD86 Q35H/H90L/Q102 H | + | - | SEQ ID NO: 215 | SEQ ID NO: 235 | + | SEQ ID NO: 236 | SEQ ID NO: 221 | SEQ ID NO: 237 | + | + |
| Domain 1: CD80 WT Domain 2: Nkp30 WT | + | - | SEQ ID NO: 152 | SEQ ID NO: 231 | + | - | SEQ ID NO: 214 | SEQ ID NO: 235 | + | + |
| Domain 1: CD86 WT Domain 2: Nkp30 WT | + | SEQ ID NO: 236 | SEQ ID NO: 220 | SEQ ID NO: 237 | + | - | SEQ ID NO: 214 | SEQ ID NO: 235 | + | + |
| Domain 1: CD80 R29H/Y31H/T41G /Y87N/E88G/K89E /D90N/A91G/P 109 S Domain 2: NKp30 L30V/A60V/S64P/ S86G | + | - | SEQ ID NO: 192 | SEQ ID NO: 231 | + | - | SEQ ID NO: 215 | SEQ ID NO: 235 | + | + |
| Domain 1: CD80 I67T/L70Q/A91G/ | + | - | SEQ ID NO: 175 | SEQ ID NO: 231 | + | - | SEQ ID NO: 215 | SEQ ID NO: 235 | + | + |
| T120S Domain 2: NKp30 L30V/A60V/S64P/ S86G | | | | | | | | | | |
| Domain 1: CD86 Q35H/H90L/Q102 H Domain 2: NKp30 L30V/A60V/S64P/ S86G | + | SEQ ID NO: 236 | SEQ ID NO: 221 | SEQ ID NO: 237 | + | - | SEQ ID NO: 215 | SEQ ID NO: 235 | + | + |
| | | | | | | | | | | |
| Domain 1: CD80 WT Domain 2: ICOSL WT | + | - | SEQ ID NO: 152 | SEQ ID NO: 231 | + | - | SEQ ID NO: 196 | SEQ ID NO: 233 | + | + |
| Domain 1: CD80 WT Domain 2 : CD86 WT | + | - | SEQ ID NO: 152 | SEQ ID NO: 231 | + | SEQ ID NO: 236 | SEQ ID NO: 220 | SEQ ID NO: 237 | + | + |
| Domain 1: CD80 WT Domain 2: CD80 WT | + | - | SEQ ID NO: 152 | SEQ ID NO: 231 | + | - | SEQ ID NO: 152 | SEQ ID NO: 231 | + | + |
| Domain 1: CD80 E88D/K89R/D90K /A91G/F92Y/K93R Domain 2: CD80 R29H/Y31H/T41G /Y87N/E88G/K89E /D90N/A91G/P 109 S | + | - | SEQ ID NO: 189 | SEQ ID NO: 231 | + | - | SEQ ID NO: 192 | SEQ ID NO: 231 | + | + |
| Domain 1: CD80 A12T/H18L/M43V /F59L/E77K/P109S /I118T Domain 2: CD80 R29H/Y31H/T41G /Y87N/E88G/K89E /D90N/A91G/P 109 S | + | - | SEQ ID NO: 193 | SEQ ID NO: 231 | + | - | SEQ ID NO: 192 | SEQ ID NO: 231 | + | + |
| Domain 1: CD80 A12T/H18L/M43V /F59L/E77K/P109S /I118T Domain 2 : CD80 I67T/L70Q/A91G/T120S | + | - | SEQ ID NO: 193 | SEQ ID NO: 231 | + | - | SEQ ID NO: 175 | SEQ ID NO: 231 | + | + |
| Domain 1: CD80 E88D/K89R/D90K /A91G/F92Y/K93R Domain 2 : CD86 Q35H/H90L/Q102 H | + | - | SEQ ID NO: 189 | SEQ ID NO: 231 | + | SEQ ID NO: 236 | SEQ ID NO: 221 | SEQ ID NO: 237 | + | + |
| Domain 1: CD80 A12T/H18L/M43V /F59L/E77K/P109S /I118T Domain 2 : CD86 Q35H/H90L/Q102 H | + | - | SEQ ID NO: 193 | SEQ ID NO: 231 | + | SEQ ID NO: 236 | SEQ ID NO: 221 | SEQ ID NO: 237 | + | + |
| Domain 1: CD80 E88D/K89R/D90K /A91G/F92Y/K93R Domain 2: ICOSL N52S/N57Y/H94D /L96F/L98F/Q100 R/G103E/ F120S | + | - | SEQ ID NO: 189 | SEQ ID NO: 231 | + | - | SEQ ID NO: 213 | SEQ ID NO: 233 | + | + |
| Domain 1: CD80 A12T/H18L/M43V /F59L/E77K/P109S /I118T Domain 2: ICOSL N52S/N57Y/H94D /L96F/L98F/Q100 R/G103E/ F120S | + | - | SEQ ID NO: 193 | SEQ ID NO: 231 | + | - | SEQ ID NO: 213 | SEQ ID NO: 233 | + | + |
| Domain 1: CD80 A12T/H18L/M43V /F59L/E77K/P109S /I118T Domain 2: ICOSL N52D | + | - | SEQ ID NO: 193 | SEQ ID NO: 231 | + | - | SEQ ID NO: 199 | SEQ ID NO: 233 | + | + |
| Domain 1: CD80 E88D/K89R/D90K /A91G/F92Y/K93R Domain 2: ICOSL N52H/N57Y/Q100 P | + | - | SEQ ID NO: 189 | SEQ ID NO: 231 | + | - | SEQ ID NO: 201 | SEQ ID NO: 233 | + | + |
| Domain 1: CD80 A12T/H18L/M43V /F59L/E77K/P109S /I118T Domain 2: ICOSL | + | - | SEQ ID NO: 193 | SEQ ID NO: 231 | + | - | SEQ ID NO: 201 | SEQ ID NO: 233 | + | + |
| N52H/N57Y/Q100 P | | | | | | | | | | |
| Domain 1: ICOSL WT Domain 2 : CD80 WT | + | - | SEQ ID NO: 196 | SEQ ID NO: 233 | + | - | SEQ ID NO: 152 | SEQ ID NO: 231 | + | + |
| Domain 1: CD86 WT Domain 2 : CD80 WT | + | SEQ ID NO: 236 | SEQ ID NO: 220 | SEQ ID NO: 237 | + | - | SEQ ID NO: 152 | SEQ ID NO: 231 | + | + |
| Domain 1: CD80 R29H/Y31H/T41G /Y87N/E88G/K89E /D90N/A91G/P 109 S Domain 2: CD80 E88D/K89R/D90K /A91G/F92Y/K93R | + | | SEQ ID NO: 192 | SEQ ID NO: 231 | + | - | SEQ ID NO: 189 | SEQ ID NO: 231 | + | + |
| Domain 1: CD80 R29H/Y31H/T41G /Y87N/E88G/K89E /D90N/A91G/P 109 S Domain 2 : CD80 A12T/H18L/M43V /F59L/E77K/P109S /I118T | + | - | SEQ ID NO: 192 | SEQ ID NO: 231 | + | - | SEQ ID NO: 193 | SEQ ID NO: 231 | + | + |
| Domain 1: CD80 I67T/L70Q/A91G/ T120S Domain 2 : CD80 E88D/K89R/D90K /A91G/F92Y/K93R | + | - | SEQ ID NO: 175 | SEQ ID NO: 231 | + | - | SEQ ID NO: 189 | SEQ ID NO: 231 | + | + |
| Domain 1: CD80 I67T/L70Q/A91G/ T120S Domain 2 : CD80 A12T/H18L/M43V /F59L/E77K/P109S /I118T | + | - | SEQ ID NO: 175 | SEQ ID NO: 231 | + | - | SEQ ID NO: 193 | SEQ ID NO: 231 | + | + |
| Domain 1: CD86 Q35H/H90L/Q102 H Domain 2: CD80 E88D/K89R/D90K /A91G/F92Y/K93R | + | SEQ ID NO: 236 | SEQ ID NO: 221 | SEQ ID NO: 237 | + | - | SEQ ID NO: 189 | SEQ ID NO: 231 | + | + |
| Domain 1: CD86 | + | SEQ | SEQ ID | SEQ ID | + | - | SEQ ID | SEQ ID | + | + |
| Q35H/H90L/Q102 H Domain 2 : CD80 A12T/H18L/M43V /F59L/E77K/P109S /I118T | | ID NO: 236 | NO: 221 | NO: 237 | | | NO: 193 | NO: 231 | | |
| Domain 1: ICOSL N52S/N57Y/H94D /L96F/L98F/Q100 R/G103E/ F120S Domain 2 : CD80 E88D/K89R/D90K /A91G/F92Y/K93R | + | - | SEQ ID NO: 213 | SEQ ID NO: 233 | + | - | SEQ ID NO: 189 | SEQ ID NO: 231 | + | + |
| Domain 1: ICOSL N52S/N57Y/H94D /L96F/L98F/Q100 R/G103E/ F120S Domain 2: CD80 A12T/H18L/M43V /F59L/E77K/P109S /I118T | + | - | SEQ ID NO: 213 | SEQ ID NO: 233 | + | - | SEQ ID NO: 193 | SEQ ID NO: 231 | + | + |
| Domain 1: ICOSL N52D Domain 2 : CD80 E88D/K89R/D90K /A91G/F92Y/K93R | + | - | SEQ ID NO: 199 | SEQ ID NO: 233 | + | - | SEQ ID NO: 189 | SEQ ID NO: 231 | + | + |
| Domain 1: ICOSL N52D Domain 2 : CD80 A12T/H18L/M43V /F59L/E77K/P109S /I118T | + | - | SEQ ID NO: 199 | SEQ ID NO: 233 | + | - | SEQ ID NO: 193 | SEQ ID NO: 231 | + | + |
| Domain 1: ICOSL N52H/N57Y/Q100 P Domain 2 : CD80 E88D/K89R/D90K /A91G/F92Y/K93R | + | - | SEQ ID NO: 201 | SEQ ID NO: 233 | + | - | SEQ ID NO: 189 | SEQ ID NO: 231 | + | + |
| Domain 1: ICOSL N52H/N57Y/Q100 P Domain 2 : CD80 A12T/H18L/M43V /F59L/E77K/P109S /I118T | + | - | SEQ ID NO: 201 | SEQ ID NO: 233 | + | - | SEQ ID NO: 193 | SEQ ID NO: 231 | + | + |
| Domain 1: CD80 V68M/L70P/L72P/ K86E Domain 2: CD80 E88D/K89R/D90K /A91G/F92Y/K93R | + | - | SEQ ID NO: 195 | SEQ ID NO: 231 | + | - | SEQ ID NO: 189 | SEQ ID NO: 231 | + | + |
| Domain 1: CD80 R29V/Y31F/K36G/ M38L/M43Q/E81R /V83I/L85I/K89R/ D90L/A91E/F92N/ K93Q/R94G Domain 2 : CD80 E88D/K89R/D90K /A91G/F92Y/K93R | + | - | SEQ ID NO: 194 | SEQ ID NO: 231 | + | - | SEQ ID NO: 189 | SEQ ID NO: 231 | + | + |
| Domain 1: CD80 V68M/L70P/L72P/ K86E Domain 2 : CD80 A12T/H18L/M43V /F59L/E77K/P109S /I118T | + | - | SEQ ID NO: 195 | SEQ ID NO: 231 | + | - | SEQ ID NO: 193 | SEQ ID NO: 231 | + | + |
| Domain 1: CD80 R29V/Y31F/K36G/ M38L/M43Q/E81R /V83I/L85I/K89R/ D90L/A91E/F92N/ K93Q/R94G Domain 2 : CD80 A12T/H18L/M43V /F59L/E77K/P109S /I118T | + | - | SEQ ID NO: 194 | SEQ ID NO: 231 | + | - | SEQ ID NO: 193 | SEQ ID NO: 231 | + | + |
| Domain 1: CD80 E88D/K89R/D90K /A91G/F92Y/K93R Domain 2 : CD80 V68M/L70P/L72P/ K86E | + | - | SEQ ID NO: 189 | SEQ ID NO: 231 | + | - | SEQ ID NO: 195 | SEQ ID NO: 231 | + | + |
| Domain 1: CD80 E88D/K89R/D90K /A91G/F92Y/K93R Domain 2 : CD80 R29V/Y31F/K36G/ M38L/M43Q/E81R /V83I/L85I/K89R/ D90L/A91E/F92N/ K93Q/R94G | + | - | SEQ ID NO: 189 | SEQ ID NO: 231 | + | - | SEQ ID NO: 194 | SEQ ID NO: 231 | + | + |
| Domain 1: CD80 A12T/H18L/M43V | + | - | SEQ ID NO: 193 | SEQ ID NO: 231 | + | - | SEQ ID NO: 195 | SEQ ID NO: 231 | + | + |
| /F59L/E77K/P109S /I118T Domain 2 : CD80 V68M/L70P/L72P/ K86E | | | | | | | | | | |
| Domain 1: CD80 A12T/H18L/M43V /F59L/E77K/P109S /I118T Domain 2 : CD80 R29V/Y31F/K36G/ M38L/M43Q/E81R /V83I/ L85I/K89R/D90L/ A91E/F92N/K93Q/ R94G | + | - | SEQ ID NO: 193 | SEQ ID NO: 231 | + | - | SEQ ID NO: 194 | SEQ ID NO: 231 | + | + |
| | | | | | | | | | | |
| Domain 1: CD86 WT Domain 2: ICOSL WT | + | SEQ ID NO: 236 | SEQ ID NO: 220 | SEQ ID NO: 237 | + | - | SEQ ID NO: 196 | SEQ ID NO: 233 | + | + |
| Domain 1: CD80 R29H/Y31H/T41G /Y87N/E88G/K89E /D90N/A91G/P 109 S Domain 2: ICOSL N52S/N57Y/H94D /L96F/L98F/Q100 R/G103E/ F120S | + | - | SEQ ID NO: 192 | SEQ ID NO: 231 | + | - | SEQ ID NO: 213 | SEQ ID NO: 233 | + | + |
| Domain 1: CD80 I67T/L70Q/A91G/ T120S Domain 2: ICOSL N52S/N57Y/H94D /L96F/L98F/Q100 R/G103E/ F120S | + | - | SEQ ID NO: 175 | SEQ ID NO: 231 | + | - | SEQ ID NO: 213 | SEQ ID NO: 233 | + | + |
| Domain 1: CD80 R29H/Y31H/T41G /Y87N/E88G/K89E /D90N/A91G/P109 S Domain 2: ICOSL N52D | + | - | SEQ ID NO: 192 | SEQ ID NO: 231 | + | - | SEQ ID NO: 199 | SEQ ID NO: 233 | + | + |
| Domain 1: CD80 I67T/L70Q/A91G/ T120S Domain 2: ICOSL N52D | + | - | SEQ ID NO: 175 | SEQ ID NO: 231 | + | - | SEQ ID NO: 199 | SEQ ID NO: 233 | + | + |
| Domain 1: CD80 R29H/Y31H/T41G /Y87N/E88G/K89E /D90N/A91G/P 109 S Domain 2: ICOSL N52H/N57Y/Q100 P | + | | SEQ ID NO: 192 | SEQ ID NO: 231 | + | - | SEQ ID NO: 201 | SEQ ID NO: 233 | + | + |
| Domain 1: CD80 I67T/L70Q/A91G/ T120S Domain 2: ICOSL N52H/N57Y/Q100 P | + | - | SEQ ID NO: 175 | SEQ ID NO: 231 | + | - | SEQ ID NO: 201 | SEQ ID NO: 233 | + | + |
| Domain 1: CD86 Q35H/H90L/Q102 H Domain 2: ICOSL N52S/N57Y/H94D /L96F/L98F/Q100 R/G103E/ F120S | + | SEQ ID NO: 236 | SEQ ID NO: 221 | SEQ ID NO: 237 | + | - | SEQ ID NO: 213 | SEQ ID NO: 233 | + | + |
| Domain 1: CD86 Q35H/H90L/Q102 H Domain 2: ICOSL N52D | + | SEQ ID NO: 236 | SEQ ID NO: 221 | SEQ ID NO: 237 | + | - | SEQ ID NO: 199 | SEQ ID NO: 233 | + | + |
| Domain 1: CD86 Q35H/H90L/Q102 H Domain 2: ICOSL N52H/N57Y/Q100 P | + | SEQ ID NO: 236 | SEQ ID NO: 221 | SEQ ID NO: 237 | + | - | SEQ ID NO: 201 | SEQ ID NO: 233 | + | + |
| Domain 1: ICOSL WT Domain 2 : CD86 WT | + | - | SEQ ID NO: 196 | SEQ ID NO: 233 | + | SEQ ID NO: 236 | SEQ ID NO: 220 | SEQ ID NO: 237 | + | + |
| Domain 1: ICOSL N52S/N57Y/H94D /L96F/L98F/Q100 R/G103E/ F120S Domain 2 : CD80 R29H/Y31H/T41G /Y87N/E88G/K89E/D90N/A91G/P 109 S | + | - | SEQ ID NO: 213 | SEQ ID NO: 233 | + | - | SEQ ID NO: 192 | SEQ ID NO: 231 | + | + |
| Domain 1: ICOSL N52S/N57Y/H94D /L96F/L98F/Q100 R/G103E/ F120S Domain 2 : CD80 I67T/L70Q/A91G/ T120S | + | - | SEQ ID NO: 213 | SEQ ID NO: 233 | + | - | SEQ ID NO: 175 | SEQ ID NO: 231 | + | + |
| Domain 1: ICOSL N52D Domain 2: CD80 R29H/Y31H/T41G /Y87N/E88G/K89E /D90N/A91G/P 109 S | + | - | SEQ ID NO: 199 | SEQ ID NO: 233 | + | - | SEQ ID NO: 192 | SEQ ID NO: 231 | + | + |
| Domain 1: ICOSL N52D Domain 2 : CD80 I67T/L70Q/A91G/ T120S | + | - | SEQ ID NO: 199 | SEQ ID NO: 233 | + | - | SEQ ID NO: 175 | SEQ ID NO: 231 | + | + |
| Domain 1: ICOSL N52H/N57Y/Q100 P Domain 2 : CD80 R29H/Y31H/T41G /Y87N/E88G/K89E /D90N/A91G/P 109 S | + | - | SEQ ID NO: 201 | SEQ ID NO: 233 | + | - | SEQ ID NO: 192 | SEQ ID NO: 231 | + | + |
| Domain 1: ICOSL N52S/N57Y/H94D /L96F/L98F/Q100 R/G103E/ F120S Domain 2: CD86 Q35H/H90L/Q102 H | + | - | SEQ ID NO: 213 | SEQ ID NO: 233 | + | SEQ ID NO: 236 | SEQ ID NO: 221 | SEQ ID NO: 237 | + | + |
| Domain 1: ICOSL N52D Domain 2: CD86 Q35H/H90L/Q102 H | + | - | SEQ ID NO: 199 | SEQ ID NO: 233 | + | SEQ ID NO: 236 | SEQ ID NO: 221 | SEQ ID NO: 237 | + | + |
| Domain 1: ICOSL N52H/N57Y/Q100 P Domain 2: CD86 Q35H/H90L/Q102H | + | - | SEQ ID NO: 201 | SEQ ID NO: 233 | + | SEQ ID NO: 236 | SEQ ID NO: 221 | SEQ ID NO: 237 | + | + |

High throughput expression and purification of the variant IgV-stacked-Fc fusion molecules containing various combinations of variant IgV domains from CD80, CD86, ICOSL or NKp30 containing at least one affinity-modified IgV domain were generated as described in Example 5. Binding of the variant IgV-stacked-Fc fusion molecules to respective cognate binding partners and functional activity by anti-CD3 coimmobilization assay also were assessed as described in Example 6. For example, costimulatory bioactivy of the stacked IgSF Fc fusion proteins was determined in a similar immobilized anti-CD3 assay as above. In this case, 4nM of anti-CD3 (OKT3, Biolegend, USA) was coimmobilized with 4nM to 120nM of human rB7-H6.Fc (R&D Systems, USA) or human rPD-L1.Fc (R&D Systems, USA) overnight on tissue-culture treated 96 well plates (Corning, USA). The following day unbound protein was washed off with PBS and 100,000 purified pan T cells were added to each well in 100ul Ex-Vivo 15 media (Lonza, Switzerland). The stacked IgSF domains were subsequently added at concentrations ranging from 8nM to 40nM in a volume of 100ul for 200ul volume total. Cells were cultured 3 days before harvesting culture supernatants and measuring human IFN-gamma levels with Duoset ELISA kit (R&D Systems, USA) as mentioned above.

The results are set forth in Tables 16-20. Specifically, Table 16 sets forth binding and functional activity results for variant IgV-stacked-Fc fusion molecules containing an NKp30 IgV domain and an ICOSL IgV domain. Table 17 sets forth binding and functional activity results for variant IgV-stacked-Fv fusion molecules containing an NKp30 IgV domain and a CD80 or CD86 IgV domain. Table 18 sets forth binding and functional activity results for variant IgV-stacked-Fc fusion molecules containing a variant CD80 IgV domain and a CD80, CD86 or ICOSL IgV domain. Table 19 sets forth binding and functional activity results for variant IgV-stacked-Fc fusion molecules containing two variant CD80 IgV domains. Table 20 sets forth results for variant IgV-stacked Fc fusion molecules containing a variant CD80 or CD86 IgV domain and a variant ICOSL IgV domain.

For each of Tables 16-20, Column 1 indicates the structural organization and orientation of the stacked, affinity modified or wild-type (WT) domains beginning with the amino terminal (N terminal) domain, followed by the middle WT or affinity modified domain located before the C terminal human IgG1 Fc domains. Column 2 sets forth the SEQ ID NO identifier for the sequence of each IgV domain contained in a respective "stack" molecule. Column 3 shows the binding partners which the indicated affinity modified stacked domains from column 1 were selected against.

Also shown is the binding activity as measured by the Mean Fluorescence Intensity (MFI) value for binding of each stack molecule to cells engineered to express various cognate binding partners and the ratio of the MFI compared to the binding of the corresponding stack molecule containing unmodified IgV domains not containing the amino acid substitution(s) to the same cell-expressed cognate binding partner. The functional activity of the variant stack molecules to modulate the activity of T cells also is shown based on the calculated levels of IFN-gamma in culture supernatants (pg/mL) generated with the indicated variant stack molecule in solution and the appropriate ligand coimmoblized with anti-CD3 as described in Example 6. The Tables also depict the ratio of IFN-gamma produced by each variant stack molecule compared to the corresponding unmodified stack molecule in the coimmobilization assay.

As shown, the results showed that it was possible to generate stack molecules containing at least one variant IgSF domains that exhibited affinity-modified activity of increased binding for at least one cognate binding partner compared to a corresponding stack molecule containing the respective unmodified (e.g. wild-type) IgV domain. In some cases, the stack molecule, either from one or a combination of both variant IgSF domains in the molecule, exhibited increased binding for more than one cognate binding partner. The results also showed that the order of the IgV domains in the stacked molecules could, in some cases, alter the degree of improved binding activity. In some cases, functional T cell activity also was altered when assessed in the targeted coimmobilization assay.

| **TABLE 16: Stacked variant IgV Fc fusion proteins containing an NKp30 IgV domain and an ICOSL IgV domain** | | | | | | |
|---|---|---|---|---|---|---|
| **Domain Structure N terminal to C terminal: domain 1/domain 2/Fc** | **SEQ ID NO (IgV)** | **cognate binding partner selected against** | **Binding Activity** | | | **Anti-CD3 coimmobilization assay pg/mL IFN-gamma (WT parental IFN-gamma ratio)** |
| | | | **B7H6 MFI (WT parental MFI ratio)** | **ICOS MFI (WT parental MFI ratio)** | **CD28 MFI (WT parental MFI ratio)** | |
| Domain 1: NKp30 WT | 214 | - | 64538 (1.00) | 26235 (1.00) | 6337 (1.00) | 235 (1.00) |
| Domain 2: ICOSL WT | 196 | | | | | |
| Domain 1: NKp30 (L30V A60V S64P S86G) | 215 | B7-H6 | 59684 (0.92) | 12762 (0.49) | 9775 (1.54) | 214 (0.91) |
| Domain 2: ICOSL (N52S N57Y H94D L96F L98F Q100R) | 212 | ICOS-CD28 | | | | |
| Domain 1: NKp30 (L30V A60V S64P S86G) | 215 | B7-H6 | 65470 (1.01) | 30272 (1.15) | 9505 (1.50) | 219 (0.93) |
| Domain 2: ICOSL (N52D) | 199 | ICOS-CD28 | | | | |
| Domain 1: NKp30 (L30V A60V S64P S86G)/ | 215 | B7-H6 | 38153 (0.59) | 27903 (1.06) | 11300 (1.78) | 189 (0.80) |
| Domain 2: ICOSL (N52H N57Y Q100P) | 201 | ICOS-CD28 | | | | |
| Domain 1: ICOSL WT | 196 | | 117853 (1.0) | 70320 (1.0) | 7916 (1.0) | 231 (1.0) |
| Domain 2: Nkp30 WT | 214 | - | | | | |
| Domain 1:ICOSL (N52D) | 199 | ICOS-CD28 B7-H6 | 100396 (0.85) | 83912 (1.19) | 20778 (2.62) | 228 (0.98) |
| Domain 2: NKp30 (L30V A60V S64P S86G) | 215 | | | | | |
| Domain 1: ICOSL (N52H N57Y Q100P) | 201 | ICOS-CD28 | 82792 (0.70) | 68874 (0.98) | 72269 (9.12) | 561 (2.43) |
| Domain 2: NKp30 (L30V A60V S64P S86G) | 215 | B7-H6 | | | | |

| **TABLE 17: Stacked variant IgV Fc fusion proteins containing an NKp30 IgV domain and a CD80 or CD86 IgV domain** | | | | | |
|---|---|---|---|---|---|
| **Domain Structure** N **terminal to C terminal: domain 1/domain 2/Fc** | **SEQ ID NO (IgV)** | **cognate binding partner selected against** | **Binding Activity** | | **Anti-CD3 coimmobilization assay pg/mL IFN-gamma (WT parental IFN-gamma ratio)** |
| | | | **B7H6 MFI (WT parental MFI ratio)** | **CD28 MFI (WT parental MFI ratio)** | |
| Domain 1: NKp30 WT | 214 | | 88823 (1.00) | 7022 (1.00) | 68 (1.00) |
| Domain 2: CD80 WT | 152 | - | | | |
| Domain 1: NKp30 WT | 214 | | 14052 (1.00) | 1690 (1.00) | 92 (1.00) |
| Domain 2: CD86 WT | 220 | - | | | |
| Domain 1: NKp30 (L30V A60V S64P S86G) | 215 | B7-H6 | 53279 (0.60) | 9027 (1.29) | 94 (1.38) |
| Domain 2: CD80 R29H/Y31H/T41G/Y87N/E8 8G/K89E/D90N/A91G/P109 S | 192 | CD28 | | | |
| Domain 1: NKp30 (L30V (A60V/S64P/S86G) | 215 | B7-H6 | 41370 (0.47) | 11240 (1.60) | 60 (0.88) |
| Domain 2: CD80 (I67T/L70Q/A91G/T120S) | 175 | CD28 | | | |
| Domain 1: NKp30 (L30V | 215 | B7-H6 | 68480 | 9115 | 110 |
| (A60V/S64P/S86G) | | CD28 | (4.87) | (5.39) | (1.19) |
| Domain 2: CD86 (Q35H/H90L/Q102H) | 221 | | | | |
| Domain 1: CD80 WT | 152 | - | 110461 (1.00) | 13654 (1.00) | 288 (1.00) |
| Domain 2: Nkp30 WT | 214 | | | | |
| Domain 1: CD86 WT | 220 | CD28 | 128899 (1.00) | 26467 (1.00) | 213 (1.00) |
| Domain 2: Nkp30 WT | 214 | B7-H6 | | | |
| Domain 1: CD80 (R29H/Y31H/T41G/Y87N/E 88G/K89E/D90N/A91G/P10 9S) | 192 | CD28 | 55727 (0.50) | 4342 (0.32) | 100 (0.35) |
| Domain 2: NKp30 (L30V/A60V/S64P. S86G) | 215 | B7-H6 | | | |
| Domain 1: CD80 (I67T/L70Q/A91G/T120S) | 175 | CD28 | 40412 (0.37) | 7094 (0.52) | 84 (0.29) |
| Domain 2: NKp30 (L30V/A60V/S64P/S86G) | 215 | B7-H6 | | | |
| Domain 1: CD86 (Q35H/H90L/Q102H) | 221 | CD28 | 220836 (1.71) | 11590 (0.44) | 113 (0.53) |
| Domain 2: NKp30 (L30V/A60V/S64P/S86G) | 215 | B7-H6 | | | |

| **TABLE 18: Stacked variant IgV Fc fusion proteins containing a CD80 IgV domain and a CD80, CD86, or ICOSL IgV domain** | | | | | | |
|---|---|---|---|---|---|---|
| **Domain Structure** N **terminal to C terminal: domain 1/domain 2/Fc** | **SEQ ID NO (IgV)** | **cognate binding partner selected against** | **Binding Activity** | | | **Anti-CD3 coimmobilization assay pg/mL IFN-gamma (WT parental IFN-gamma ratio)** |
| | | | **CD28 MFI (WT parental MFI ratio)** | **PD-L1 MFI (WT parental MFI ratio)** | **ICOS MFI (WT parental MFI ratio)** | |
| Domain 1: CD80 WT | 152 | | 1230 (1.00) | 2657 (1.00) | 11122 (1.00) | 69 (1.00) |
| Domain 2: ICOSL WT | 196 | | | | | |
| Domain 1: CD80 WT | 152 | | 60278 (1.00) | 2085 (1.00) | | 59 (1.00) |
| Domain 2: CD86 WT | 220 | | | | | |
| Domain 1: CD80 WT | 152 | | 1634 (1.00) | 6297 (1.00) | | 98 (1.00) |
| Domain 2: CD80 WT | 152 | | | | | |
| Domain 1: CD80 E88D/K89R/D 90K/A91G/F9 2Y/K93R | 189 | PD-L1 | 4308 (2.64) | 4234 (0.67) | | 214 (2.18) |
| Domain 2: CD80 R29H/Y31H/T41 G/Y87N/E8 8 G/K89E/D90N/A91G/P109 S | 192 | CD28 | | | | |
| Domain 1: CD80 A12T/H18L/M43V/F59L/E7 7K/P109S/I118T | 193 | PD-L1 | 7613 (4.66) | 2030 (0.32) | | 137 (1.40) |
| Domain 2: CD80 R29H/Y31H/T41G/Y87N/E8 8 G/K89E/D90N/A91G/P109 S | 192 | CD28 | | | | |
| Domain 1: CD80 A12T/H18L/M43V/F59L/E7 7K/P109S/I118T | 193 | PD-L1 | 3851 (2.36) | 3657 (0.58) | | 81 (0.83) |
| Domain 2: CD80 I67T/L70Q/A91G/T120S | 175 | CD28 | | | | |
| Domain 1:CD80 E88D/K89R/D 90K/A91G/F9 2Y/K93R | 189 | PD-L1 | 4117 (0.07) | 2914 (1.40) | | 96 (1.63) |
| Domain 2:CD86 Q35H/H90L/Q102H | 221 | CD28 | | | | |
| Domain 1:CD80 A12T/H18L/M43V/F59L/E7 7K/P109S/I118T | 193 | PD-L1 | 2868 (0.05) | 3611 (1.73) | | 94 (1.60) |
| Domain 2:CD86 Q35H/H90L/Q102H | 221 | CD28 | | | | |
| Domain 1: CD80 E88D/K89R/D90K/A91G/F9 2Y/K93R | 189 | PD-L1 | 3383 (2.75) | 4515 (1.70) | 5158 (0.46) | 90 (1.30) |
| Domain 2: ICOSL N52S/N57Y/H94D/L96F/L9 8F/Q100R/G103E/ F120S | 213 | ICOS/CD 28 | | | | |
| Domain 1: CD80 A12T/H18L/M43V/F59L/E7 7K/P109S/I118T | 193 | PD-L1 | 2230 (1.81) | 2148 (0.81) | 3860 (0.35) | 112 (1.62) |
| Domain 2: ICOSL N52S/N57Y/H94D/L96F/L9 8F/Q100R/G103E/ F120S | 213 | ICOS/CD 28 | | | | |
| Domain 1: CD80 A12T/H18L/M43V/F59L/E7 7K/P109S/I118T | 193 | PD-L1 ICOS/CD 28 | 5665 (4.61) | 6446 (2.43) | 15730 (1.41) | 126 (1.83) |
| Domain 2: ICOSL N52D | 199 | | | | | |
| Domain 1: CD80 E88D/K89R/D 90K/A91G/F9 2Y/K93R | 189 | PD-L1 | 6260 (5.09) | 4543 (1.71) | 11995 (1.08) | 269 (3.90) |
| Domain 2: ICOSL N52H/N57Y/Q100P | 201 | ICOS/CD 28 | | | | |
| Domain 1: CD80 A12T/H18L/M43V/F59L/E7 7K/P109S/I118T | 193 | PD-L1 | 3359 (2.73) | 3874 (1.46) | 8541 (0.77) | 97 (1.41) |
| Domain 2: ICOSL N52H/N57Y/Q100P | 201 | ICOS/CD 28 | | | | |
| Domain 1: ICOSL WT | 196 | | 3000 (1.00) | 2966 (1.00) | 14366 (1.00) | 101 (1.00) |
| Domain 2: CD80 WT | 152 | | | | | |
| Domain 1: CD86 WT | 220 | | 4946 (1.00) | 1517 (1.00) | | 125 (1.00) |
| Domain 2: CD80 WT | 152 | | | | | |
| Domain 1: CD80 R29H/Y31H/T41G/Y87N/E8 8G/K89E/D90N/A91G/P109 S | 192 | CD28 | 2832 (1.73) | 3672 (0.58) | | 114 (1.16) |
| Domain 2: CD80 E88D/K89R/D 90K/A91G/F9 2Y/K93R | 189 | PD-L1 | | | | |
| Domain 1: CD80 R29H/Y31H/T41G/Y87N/E8 8G/K89E/D90N/A91G/P109 S | 192 | CD28 | 4542 (2.78) | 2878 (0.45) | | 142 (1.45) |
| Domain 2: CD80 A12T/H18L/M43V/F59L/E7 7K/P109S/I118T | 193 | PD-L1 | | | | |
| Domain 1: CD80 I67T/L70Q/A91G/T120S | 175 | CD28 | 938 (0.57) | 995 (0.16) | | 102 (1.04) |
| Domain 2: CD80 E88D/K89R/D 90K/A91G/F9 2Y/K93R | 189 | PD-L1 | | | | |
| Domain 1: CD80 I67T/L70Q/A91G/T120S | 175 | CD28 | 4153 (2.54) | 2827 (0.45) | | 108 (1.10) |
| Domain 2: CD80 A12T/H18L/M43V/F59L/E7 7K/P109S/I118T | 193 | PD-L1 | | | | |
| Domain 1: CD86 Q3 5H/H90L/Q102H | 221 | CD28 | 14608 (2.95) | 2535 (1.67) | | 257 (2.06) |
| Domain 2: CD80 E88D/K89R/D 90K/A91G/F9 2Y/K93R | 189 | PD-L1 | | | | |
| Domain 1: CD86 Q35H/H90L/Q102H | 221 | CD28 | 2088 (0.42) | 2110 (1.39) | | 101 (0.81) |
| Domain 2: CD80 A12T/H18L/M43V/F59L/E7 7K/P109S/I118T | 193 | PD-L1 | | | | |
| Domain 1: ICOSL N52S/N57Y/H94D/L96F/L9 8F/Q100R/G103E/ F120S | 213 | ICOS/CD 28 | 3634 (1.21) | 4893 (1.65) | 6403 (0.45) | 123 (1.22) |
| Domain 2: CD80 E88D/K89R/D90K/A91G/F9 2Y/K93R | 189 | PD-L1 | | | | |
| Domain 1: ICOSL N52S/N57Y/H94D/L96F/L9 | 213 | ICOS/CD 28 | 1095 (0.37) | 5929 (2.0) | 7923 (0.55) | 127 (1.26) |
| 8F/Q100R/G103E/ F120S | 193 | | | | | |
| Domain 2: CD80 A12T/H18L/M43V/F59L/E7 7K/P109S/I118T | | PD-L1 | | | | |
| Domain 1: ICOSL N52D | 199 | ICOSL/C D28 | 2023 (0.67) | 5093 (1.72) | 16987 (1.18) | 125 (1.24) |
| Domain 2: CD80 E88D/K89R/D 90K/A91G/F9 2Y/K93R | 189 | PD-L1 | | | | |
| Domain 1: ICOSL N52D | 199 | ICOS/CD 28 | 3441 (1.15) | 3414 (1.15) | 20889 (1.45) | 165 (1.63) |
| Domain 2: CD80 A12T/H18L/M43V/F59L/E7 7K/P109S/I118T | 193 | PD-L1 | | | | |
| Domain 1: ICOSL N52H/N57Y/Q100P | 201 | ICOS/CD 28 | 7835 (2.61) | 6634 (2.24) | 20779 (1.45) | 95 (0.94) |
| Domain 2: CD80 E88D/K89R/D 90K/A91G/F9 2Y/K93R | 189 | PD-L1 | | | | |
| Domain 1: ICOSL N52H/N57Y/Q100P | 201 | ICOS/CD 28 | 8472 (2.82) | 3789 (1.28) | 13974 (0.97) | 106 (1.05) |
| Domain 2: CD80 A12T/H18L/M43V/F59L/E7 7K/P109S/I118T | 193 | PD-L1 | | | | |

| **TABLE 19: Stacked variant IgV Fc fusion proteins containing two CD80 IgV domains** | | | | | |
|---|---|---|---|---|---|
| **Domain Structure N terminal to C terminal: domain 1/domain 2/Fc** | **SEQ ID NO (IgV)** | **Cognate binding partner selected against** | **Binding Activity** | | **Functional Activity MLR IFN-gamma pg/mL** |
| | | | **PD-L1 MFI (WT parental MFI ratio)** | **CTLA-4 MFI (WT parental MFI ratio)** | |
| Domain 1: CD80 WT | 152 | | 6297 (1.00) | 4698 (1.00) | 35166 (1.00) |
| Domain 2: CD80 WT | 152 | | | | |
| Domain 1: CD80 V68M/L70P/L72P/K86E | 195 | CTLA-4 | 2464 (0.39) | 4955 (1.05) | 5705 (0.16) |
| Domain 2: CD80 E88D/K89R/D 90K/A91G/F9 2Y/K93R | 189 | PD-L1 | | | |
| Domain 1: CD80 R29V/Y31F/K36G/M38L/M 43Q/E81R/V83I/L85I/K89R/ D90L/A91E/F92N/K93Q/R9 4G | 194 | CTLA-4 | 1928 (0.31 ) | 1992 (0.42) | 1560 (0.04) |
| Domain 2: CD80 E88D/K89R/D90K/A91G/F9 2Y/K93R | 189 | PD-L1 | | | |
| Domain 1: CD80 V68M/L70P/L72P/K86E | 195 | CTLA-4 | 1215 (0.19) | 1382 (0.29) | 2171 (0.06) |
| Domain 2: CD80 A12T/H18L/M43V/F59L/E7 7K/P109S/I118T | 193 | PD-L1 | | | |
| Domain 1: CD80 R29V/Y31F/K36G/M38L/M 43Q/E81R/V83I/L85I/K89R/ D90L/A91E/F92N/K93Q/R9 4G | 194 | CTLA-4 | 1592 (0.25) | 1962 (0.42) | 1512 (0.04) |
| Domain 2: CD80 A12T/H18L/M43V/F59L/E7 7K/P109S/I118T | 193 | PD-L1 | | | |
| Domain 1: CD80 E88D/K89R/D 90K/A91G/F9 2Y/K93R | 189 | PD-L1 | 1747 (0.28) | 2057 (0.44) | 9739 (0.28) |
| Domain 2: CD80 V68M/L70P/L72P/K86E | 195 | CTLA-4 | | | |
| Domain 1: CD80 E88D/K89R/D 90K/A91 G/F9 2Y/K93R | 189 | PD-L1 | 1752 (0.28) | 1772 (0.38) | 5412 (0.15) |
| Domain 2: CD80 R29V/Y31F/K36G/M38L/M 43Q/E81R/V83I/L85I/K89R/ D90L/A91E/F92N/K93Q/R9 4G | 194 | CTLA-4 | | | |
| Domain 1: CD80 A12T/H18L/M43V/F59L/E7 7K/P109S/I118T | 193 | PD-L1 | 1636 (0.26) | 1887 (0.40) | 7608 (0.22) |
| Domain 2: CD80 V68M/L70P/L72P/K86E | 195 | CTLA-4 | | | |
| Domain 1: CD80 A12T/H18L/M43V/F59L/E7 7K/P109S/I118T | 193 | PD-L1 | 2037 (0.32) | 4822 (1.03) | 11158 (0.32) |
| Domain 2: CD80 R29V/Y31F/K36G/M38L/M 43Q/E81R/V83I/ L85I/K89R/D90L/A91E/F92 N/K93Q/R94G | 194 | CTLA-4 | | | |

| **TABLE 20: Stacked variant IgV Fc fusion proteins containing a CD80 or CD86 IgV domain and an ICOSL IgV domain** | | | | | |
|---|---|---|---|---|---|
| **Domain Structure N terminal to C terminal: domain 1/domain 2/Fc** | **SEQ ID NO (IgV)** | **Cognate binding partner selected against** | **Binding Activity** | | **Functional Activity MLR IFN-gamma pg/mL** |
| | | | **PD-L1 MFI (WT parental MFI ratio)** | **CTLA-4 MFI (WT parental MFI ratio)** | |
| Domain 1: CD80 WT | 152 | | 1230 (1.00) | 11122 (1.00) | 1756 (1.00) |
| Domain 2: ICOSL WT | 196 | | | | |
| Domain 1: CD86 WT | 220 | | 29343 (1.00) | 55193 (1.00) | 6305 (1.00) |
| Domain 2: ICOSL WT | 196 | | | | |
| Domain 1: CD80 R29H/Y31H/T41G/Y87N/E8 8G/K89E/D90N/A91G/P109 S | 192 | CD28 | 2280 (1.85) | 3181 (0.29) | 2281 (1.30) |
| Domain 2: ICOSL N52S/N57Y/H94D/L96F/L9 8F/Q100R/G103E/ F120S | 213 | ICOS/CD 28 | | | |
| Domain 1: CD80 I67T/L70Q/A91G/T120S | 175 | CD28 | 2309 (1.88) | 26982 (2.43) | 1561 (0.89) |
| Domain 2: ICOSL N52S/N57Y/H94D/L96F/L9 8F/Q100R/G103E/ F120S | 213 | ICOS/CD 28 | | | |
| Domain 1: CD80 R29H/Y31H/T41G/Y87N/E8 8 G/K89E/D90N/A91G/P109 S | 192 | CD28 | 4285 (3.48) | 22744 (2.04) | 1612 (0.92) |
| Domain 2: ICOSL N52D | 199 | ICOS/CD 28 | | | |
| Domain 1: CD80 I67T/L70Q/A91G/T120S | 175 | CD28 | 3024 (2.46) | 16916 (1.52) | 3857 (2.20) |
| Domain 2: ICOSL N52D | 199 | ICOS/CD 28 | | | |
| Domain 1: CD80 R29H/Y31H/T41G/Y87N/E8 8G/K89E/D90N/A91G/P109 S | 192 | CD28 | 6503 (5.29) | 7240 (0.65) | 6886 (3.92) |
| Domain 2: ICOSL N52H/N57Y/Q100P | 201 | ICOS/CD 28 | | | |
| Domain 1: CD80 I67T/L70Q/A91G/T120S | 175 | CD28 | 3110 (2.53) | 4848 (0.44) | 3393 (1.93) |
| Domain 2: ICOSL N52H/N57Y/Q100P | 201 | ICOS/CD 28 | | | |
| Domain 1: CD86 Q35H/H90L/Q102H | 221 | CD28 | 11662 (0.40) | 21165 (0.38) | 880 (0.14) |
| Domain 2: ICOSL N52S/N57Y/H94D/L96F/L9 8F/Q100R/G103E/ F120S | 213 | ICOS/CD 28 | | | |
| Domain 1: CD86 Q35H/H90L/Q102H | 221 | CD28 | 24230 (0.83) | 73287 (1.33) | 1110 (0.18) |
| Domain 2: ICOSL N52D | 199 | ICOS/CD 28 | | | |
| Domain 1: CD86 Q35H/H90L/Q102H | 221 | CD28 ICOS/CD | 1962 (0.07) | 1630 (0.03) | 587 (0.09) |
| Domain 2: ICOSL N52H/N57Y/Q100P | 201 | 28 | | | |
| Domain 1: ICOSL WT | 196 | | 3000 (1.00) | 14366 (1.00) | 4113 (1.00) |
| Domain 2: CD80 WT | 152 | | | | |
| Domain 1: ICOSL WT | 196 | | 18005 (1.00) | 53602 (1.00) | 18393 (1.00) |
| Domain 2: CD86 WT | 220 | | | | |
| Domain 1: ICOSL N52S/N57Y/H94D/L96F/L9 8F/Q100R/G103E/ F120S | 213 | ICOSL/C D28 | 10426 (3.48) | 51286 (3.57) | 18680 (4.54) |
| Domain 2: CD80 R29H/Y31H/T41 G/Y87N/E8 8G/K89E/D90N/A91G/P109 S | 192 | CD28 | | | |
| Domain 1: ICOSL N52S/N57Y/H94D/L96F/L9 8F/Q100R/G103E/ F120S | 213 | ICOS/CD 28 | 17751 (5.92) | 29790 (2.07) | 10637 (2.59) |
| Domain 2: CD80 I67T/L70Q/A91G/T120S | 175 | CD28 | | | |
| Domain 1: ICOSL N52D | 199 | ICOS/CD 28 | 2788 (0.93) | 25870 (1.80) | 6205 (1.51) |
| Domain 2: CD80 R29H/Y31H/T41G/Y87N/E8 8G/K89E/D90N/A91G/P109 S | 192 | CD28 | | | |
| Domain 1: ICOSL N52D | 199 | ICOS/CD 28 | 2522 (0.84) | 13569 (0.94) | 5447 (1.32) |
| Domain 2 : CD80 I67T/L70Q/A91G/T120S | 175 | CD28 | | | |
| Domain 1: ICOSL N52H/N57Y/Q100P | 201 | ICOS/CD 28 | 9701 (3.23) | 9187 (0.64) | 5690 (1.38) |
| Domain 2: CD80 R29H/Y31H/T41 G/Y87N/E8 8G/K89E/D90N/A91G/P109 S | 192 | CD28 | | | |
| Domain 1: ICOSL N52S/N57Y/H94D/L96F/L9 8F/Q100R/G103E/ F120S | 213 | ICOS/CD 28 | 27050 (1.50) | 21257 (0.40) | 8131 (0.44) |
| Domain 2: CD86 Q35H/H90L/Q102H | 221 | CD28 | | | |
| Domain 1: ICOSL N52D | 199 | ICOS/CD 28 | 34803 (1.93) | 80210 (1.50) | 6747 (0.37) |
| Domain 2: CD86 Q35H/H90L/Q102H | 221 | CD28 | | | |
| Domain 1: ICOSL N52H/N57Y/Q100P | 201 | ICOS/CD 28 | 5948 (0.33) | 4268 (0.08) | 26219 (1.43) |
| Domain 2: CD86 Q35H/H90L/Q102H | 221 | CD28 | | | |

### Example 9

### Generation, Selection and Screening of Affinity-modified IgSF Domain Variants of CD155

Affinity-modified IgSF domain variants of CD155 were generated substantially as described in Examples 1-6 above with some slight modifications. For example, for the generation of CD155 variants, only the IgV domain, and not the other two domains of the ECD, was included in the generated proteins. The example exemplifies binding and activity of the exemplary affinity-modified domains in an Fc-fusion format; such affinity-modified domains are contemplated in connection with a secretable immunomodulatory protein or transmembrane immunomodulatory protein as described.

To generate a library targeting specific residues of CD155 by complete or partial randomization with degenerate codons, the coding DNA for the immunoglobulin-like V-type (IgV) domain of human CD155 (SEQ ID NO:241) was ordered from Integrated DNA Technologies (Coralville, IA) as a set of overlapping oligonucleotides of up to 80 base pairs (bp) in length. In general, to generate a library of diverse variants of the IgV domain, the oligonucleotides contained desired degenerate codons at desired amino acid positions. Degenerate codons were generated using an algorithm at the URL rosettadesign.med.unc.edu/SwiftLib/. In general, positions to mutate and degenerate codons were chosen from crystal structure information (PDB: 3UDW) or homology models built from this structure containing the target-ligand pairs of interest to identify ligand contact residues as well as residues that are at the protein interaction interface. For example, a crystal structure of CD155 bound to TIGIT is publicly available at the URL www.rcsb.org/pdb/explore/explore.do?structureId=3UDW for Protein Data Base code 3UDW. This analysis was performed using a structure viewer available at the URL: spdbv.vital-it.ch).

The oligonucleotides were used for PCR amplification to generate library DNA inserts for insertion into the modified yeast display version of vector pBYDS03 substantially as described in Example 1. Alternatively, Ultramers (Integrated DNA Technologies) of up to 200 bp in length were used in conjunction with megaprimer PCR (URL http://www.ncbi.nlm.nih.gov/pmc/articles/PMC146891/pdf/253371.pdf) to generate larger stretches of degenerate codons that could not be as easily incorporated using multiple small overlapping primers. Following the generation of full length product using megaprimer PCR, the mutant IgV domain library was PCR amplified again using DNA primers containing 40 bp overlap region with the modified pBYDS03 cloning variant for homologous recombination into yeast. The library of DNA inserts were prepared for library insertion substantially as descried in Example 1 and electroporation-ready DNA was prepared.

As alternative approaches, either sublibraries generated by site-directed mutagenesis to target specific residues of the IgV domain of CD155 or random libraries of the IgV domain of CD155 were generated to further identify variants of the IgV domain of CD155 substantially as described in Example 1.

The degenerate or random library DNA was inserted into yeast substantially as described in Example 2. Yeast expressing affinity modified variants of CD155 were selected using the method substantially described in Example 3. For the selection, the following target ligand proteins were employed: human rTIGIT.Fc (i.e., recombinant TIGIT-Fc fusion protein) and rCD226.Fc. Magnetic Protein A beads were obtained from New England Biolabs, USA. For two-color, flow cytometric sorting, a Bio-Rad S3e sorter was used. CD155 display levels were monitored with an anti-hemagglutinin antibody labeled with Alexafluor 488 (Life Technologies, USA). Ligand binding of Fc fusion proteins, rTIGIT.Fc or rCD226.Fc, were detected with PE conjugated human Ig specific goat Fab (Jackson ImmunoResearch, USA). Doublet yeast were gated out using forward scatter (FSC) / side scatter (SSC) parameters, and sort gates were based upon higher ligand binding detected in FL2 that possessed more limited tag expression binding in FL1.

Second sort outputs (F2) were obtained substantially as described in Example 3 by expanding and re-inducing expression of sort outputs that had been assayed for higher specific binding affinity and were used to assess binding compared to the parental, wild-type yeast strain. For CD155, the second FACS outputs (F2) were compared to parental CD155 yeast for binding rTIGIT.Fc or rCD226.Fc by double staining each population with anti-HA (hemagglutinin) tag expression and the anti-human Fc secondary to detect ligand binding.

Selected outputs were reformatted as immunomodulatory proteins containing an affinity modified (variant) immunoglobulin-like V-type (IgV) domain of CD155 fused to an Fc molecule (variant IgV domain -Fc fusion molecules) substantially as described in Example 4, except including only the IgV domain and not the full ECD domain. In some alternative methods for the CD155 outputs, DNA from the outputs were PCR amplified with primers containing 40 bp overlap regions on either end with an Fc fusion vector to carry out in vitro recombination using Gibson Assembly Mastermix (New England Biolabs), which was subsequently used in heat shock transformation into E. Coli strain NEB5alpha. Exemplary of an Fc fusion vector is pFUSE-hIgG1-Fc2 (Invivogen, USA).

After transformation, samples were prepared for DNA sequencing substantially as described in Example 4. The sequences were then manually curated as described in Example 4, except that they were manually curated so that they start at the beginning of the IgV coding region. The curated sequences were batch-translated and aligned as described in Example 4. Clones of interest were identified using the criteria as described in Example 4. Table 21 sets forth the identified variant CD155 affinity-modified molecules, including the amino acid substitutions contained in each variant.

The methods generated immunomodulatory proteins containing an affinity-modified IgV domain of CD155 in which the encoding DNA was generated to encode a protein as follows: signal peptide followed by variant (mutant) IgV domain followed by a linker of three alanines (AAA) followed by a human IgG1 Fc containing the mutation N297G (N82G with reference to wild-type human IgG1 Fc set forth in SEQ ID NO: 226). The human IgG1 Fc also contained the mutations R292C and V302C by EU numbering (corresponding to R77C and V87C with reference to wild-type human IgG1 Fc set forth in SEQ ID NO: 226). Since the construct does not include any antibody light chains that can form a covalent bond with a cysteine, the human IgG1 Fc also contained replacement of the cysteine residues to a serine residue at position 220 (C220S) by EU numbering (corresponding to position 5 (C5S) with reference to the wild-type or unmodified Fc set forth in SEQ ID NO: 226.

Recombinant variant CD155 Fc fusion proteins were expressed and purified substantially as described in Example 5. Binding and activity of the affinity-modified variant CD155 Fc fusion proteins was assessed substantially as described in Example 6, except that cells expressing full length human CD226 and TIGIT cognate binding partners were generated. Cells were stained by flow cytometry with CD155 Fc variant and mean fluorescence intensity (MFI) was determined as described in Example 5. For bioactivity characterization, recombinant CD155 Fc variants were assessed in an anti-CD3 coimmobilization assay substantially as described in Example 5.

The results for the binding and activity studies are set forth in Table 21. The Table indicates exemplary IgSF domain amino acid substitutions (replacements) in the IgV domain of CD155 selected in the screen for affinity-maturation against the respective cognate structures TIGIT and CD226. The exemplary amino acid substitutions are designated by amino acid position number corresponding to position of the unmodified sequence set forth in SEQ ID NO: 241 (IgV). The amino acid position is indicated in the middle, with the corresponding unmodified (e.g. wild-type) amino acid listed before the number and the identified variant amino acid substitution listed after the number. Column 2 sets forth the SEQ ID NO identifier for the variant for each variant ECD-Fc fusion molecule.

Also shown is the binding activity as measured by the Mean Fluorescence Intensity (MFI) value for binding of each variant Fc-fusion molecule to cells engineered to express the cognate binding partner as the ratio of the MFI compared to the binding of the corresponding unmodified ECD-Fc fusion molecule not containing the amino acid substitution(s) to the same cell-expressed cognate binding partner. The functional activity of the variant Fc-fusion molecules to modulate the activity of T cells also is shown based on the calculated levels of IFN-gamma in culture supernatants (pg/mL) generated with the indicated variant Fc fusion molecule coimmoblized with anti-CD3 as a ratio of IFN-gamma produced by each variant CD155 IgV-Fc compared to the corresponding unmodified CD155 IgV-Fc in both functional assays.

| **TABLE 21: CD155 variants selected against cognate binding partners. Molecule sequences, binding data, and costimulatory bioactivity data.** | | | | | | |
|---|---|---|---|---|---|---|
| **CD155 mutations** | **SEQ ID NO (IgV)** | **CD226 tfxn MFI (CD226 MFI parental ratio)** | **TIGIT tfxn MFI (TIGIT MFI parental ratio)** | **CD96 MFI (CD96 MFI parental ratio)** | **Mock Expi293 MFI (Mock MFI parental ratio)** | **Anti-CD3 IFN-gamma (pg/mL) (Anti-CD3 IFN-gamma parental ratio)** |
| P18S, P64S, F91S | 653 | 497825 (133.7) | 247219 (91.1) | 140065 (45.4) | 3528 (1.2) | 270.1 (0.7) |
| P18S, F91S, L104P | 654 | 26210 (7.0) | 75176 (27.7) | 10867 (3.5) | 2130 (0.7) | 364.2 (0.9) |
| L44P | 655 | 581289 (156.1) | 261931 (96.5) | 152252 (49.4) | 3414 (1.2) | 277.6 (0.7) |
| A56V | 656 | 455297 (122.3) | 280265 (103.2) | 161162 (52.2) | 2601 (0.9) | 548.2 (1.4) |
| P18L, L79V, F91S | 657 | 5135 (1.4) | 4073 (1.5) | 3279 (1.1) | 2719 (0.9) | 1241.5 (3.2) |
| P18S, F91S | 658 | 408623 (109.8) | 284190 (104.7) | 147463 (47.8) | 3348 (1.1) | 760.6 (2.0) |
| P18T, F91S | 659 | 401283 (107.8) | 223985 (82.5) | 157644 (51.1) | 3065 (1.1) | 814.7 (2.1) |
| P18T, S42P, F91S | 660 | 554105 (148.8) | 223887 (82.5) | 135395 (43.9) | 3796 (1.3) | 539.7 (1.4) |
| G7E, P18T, Y30C, F91S | 661 | 12903 (3.5) | 12984 (4.8) | 7906 (2.6) | 2671 (0.9) | 275.9 (0.7) |
| P18T, F91S, G111D | 662 | 438327 | 287315 | 167583 | 4012 | 307.2 |
| | | (117.7) | (105.8) | (54.3) | (1.4) | (0.8) |
| P18S, F91P | 663 | 4154 (1.1) | 3220 (1.2) | 2678 (0.9) | 2816 (1.0) | 365.7 (0.9) |
| P18T, F91S, F108L | 664 | 394546 (106.0) | 298680 (110.0) | 193122 (62.6) | 2926 (1.0) | 775.4 (2.0) |
| P18T, T45A, F91S | 665 | 435847 (117.1) | 222044 (81.8) | 191026 (61.9) | 2948 (1.0) | 1546.8 (4.0) |
| P18T, F91S, R94H | 666 | 3589 (1.0) | 2942 (1.1) | 2509 (0.8) | 2390 (0.8) | 1273.2 (3.3) |
| P18S, Y30C, F91S | 667 | 382352 (102.7) | 276358 (101.8) | 56934 (18.5) | 3540 (1.2) | 426.5 (1.1) |
| A81V, L83P | 668 | 4169 (1.1) | 2912 (1.1) | 2616 (0.8) | 2993 (1.0) | 339.7 (0.9) |
| L88P | 669 | 65120 (17.5) | 74845 (27.6) | 35280 (11.4) | 2140 (0.7) | 969.2 (2.5) |
| Wild type | 652 | 3723 (1.0) | 2715 (1.0) | 3085 (1.0) | 2913 (1.0) | 389.6 (1.0) |
| R94H | 670 | 18905 (5.1) | 104013 (38.3) | 11727 (3.8) | 1663 (0.6) | 372.6 (1.0) |
| A13E, P18S, A56V, F91S | 671 | 357808 (96.1) | 179060 (66.0) | 118570 (38.4) | 2844 (1.0) | 349.2 (0.9) |
| P18T, F91S, V115A | 672 | 38487 (10.3) | 46313 (17.1) | 22718 (7.4) | 2070 (0.7) | 1574.5 (4.0) |
| P18T, Q60K | 673 | 238266 (64.0) | 173730 (64.0) | 154448 (50.1) | 4778 (1.6) | 427.2 (1.1) |

### Example 10

### Generation, Selection and Screening of Affinity-modified IgSF Domain Variants of CD112

Affinity-modified IgSF domain variants of CD112 were generated substantially as described in Examples 1-6 above with some slight modifications. For example, for the generation of CD112 variants, only the IgV domain, and not the other two domains of the ECD, was included in the generated proteins. The example exemplifies binding and activity of the exemplary affinity-modified domains in an Fc-fusion format; such affinity-modified domains are contemplated in connection with a secretable immunomodulatory protein or transmembrane immunomodulatory protein as described.

To generate a library targeting specific residues of CD155 by complete or partial randomization with degenerate codons, the coding DNA for the immunoglobulin-like V-type (IgV) domain of human CD112 (SEQ ID NO:286) was ordered from Integrated DNA Technologies (Coralville, IA) as a set of overlapping oligonucleotides of up to 80 base pairs (bp) in length. In general, to generate a library of diverse variants of the IgV domain, the oligonucleotides contained desired degenerate codons at desired amino acid positions. Degenerate codons were generated using an algorithm at the URL rosettadesign.med.unc.edu/SwiftLib/. In general, positions to mutate and degenerate codons were chosen from crystal structure information (PDB: 3UDW) or homology models built from this structure containing the target-ligand pairs of interest to identify ligand contact residues as well as residues that are at the protein interaction interface.

The oligonucleotides were used for PCR amplification to generate library DNA inserts for insertion into the modified yeast display version of vector pBYDS03 substantially as described in Example 1. Alternatively, Ultramers (Integrated DNA Technologies) of up to 200 bp in length were used in conjunction with megaprimer PCR (URL http://www.ncbi.nlm.nih.gov/pmc/articles/PMC146891/pdf/253371.pdf) to generate larger stretches of degenerate codons that could not be as easily incorporated using multiple small overlapping primers. Following the generation of full length product using megaprimer PCR, the mutant IgV domain library was PCR amplified again using DNA primers containing 40 bp overlap region with the modified pBYDS03 cloning variant for homologous recombination into yeast. The library of DNA inserts were prepared for library insertion substantially as descried in Example 1 and electroporation-ready DNA was prepared.

As alternative approaches, either sublibraries generated by site-directed mutagenesis to target specific residues of the IgV domain of CD112 or random libraries of the IgV domain of CD112 were generated to further identify variants of the IgV domain of CD112 substantially as described in Example 1.

The degenerate or random library DNA was inserted into yeast substantially as described in Example 2. Yeast expressing affinity modified variants of CD112 were selected using the method substantially described in Example 3. For the selection, the following target ligand proteins were employed: human rTIGIT.Fc (i.e., recombinant TIGIT-Fc fusion protein), rCD226.Fc and rCD112R.Fc. Magnetic Protein A beads were obtained from New England Biolabs, USA. For two-color, flow cytometric sorting, a Bio-Rad S3e sorter was used. CD112 display levels were monitored with an anti-hemagglutinin antibody labeled with Alexafluor 488 (Life Technologies, USA). Ligand binding of Fc fusion proteins, rTIGIT.Fc, rCD226.Fc, or rCD112R.Fc, were detected with PE conjugated human Ig specific goat Fab (Jackson ImmunoResearch, USA). Doublet yeast were gated out using forward scatter (FSC) / side scatter (SSC) parameters, and sort gates were based upon higher ligand binding detected in FL2 that possessed more limited tag expression binding in FL1.

Second sort outputs (F2) were obtained substantially as described in Example 3 by expanding and re-inducing expression of sort outputs that had been assayed for higher specific binding affinity and were used to assess binding compared to the parental, wild-type yeast strain. For CD112, the second FACS outputs (F2) were compared to parental CD112 yeast for binding of each rTIGIT.Fc, rCD226.Fc, and rCD112R by double staining each population with anti-HA (hemagglutinin) tag expression and the anti-human Fc secondary to detect ligand binding.

Selected outputs were reformatted as immunomodulatory proteins containing an affinity modified (variant) immunoglobulin-like V-type (IgV) domain of CD112 fused to an Fc molecule (variant IgV domain -Fc fusion molecules) substantially as described in Example 4, except including only the IgV domain and not the full ECD domain. In some alternative methods for the CD112 outputs, DNA from the outputs were PCR amplified with primers containing 40 bp overlap regions on either end with an Fc fusion vector to carry out in vitro recombination using Gibson Assembly Mastermix (New England Biolabs), which was subsequently used in heat shock transformation into E. Coli strain NEB5alpha. Exemplary of an Fc fusion vector is pFUSE-hIgG1-Fc2 (Invivogen, USA).

After transformation, samples were prepared for DNA sequencing substantially as described in Example 4. The sequences were then manually curated as described in Example 4, except that they were manually curated so that they start at the beginning of the IgV coding region. The curated sequences were batch-translated and aligned as described in Example 4. Clones of interest were identified using the criteria as described in Example 4. Table 22 sets forth the identified variant CD112 affinity-modified molecules, including the amino acid substitutions contained in each variant.

The methods generated immunomodulatory proteins containing an affinity-modified IgV domain of CD112 in which the encoding DNA was generated to encode a protein as follows: signal peptide followed by variant (mutant) IgV domain followed by a linker of three alanines (AAA) followed by a human IgG1 Fc containing the mutation N297G (N82G with reference to wild-type human IgG1 Fc set forth in SEQ ID NO: 226). The human IgG1 Fc also contained the mutations R292C and V302C (corresponding to R77C and V87C with reference to wild-type human IgG1 Fc set forth in SEQ ID NO: 226). Since the construct does not include any antibody light chains that can form a covalent bond with a cysteine, the human IgG1 Fc also contained replacement of the cysteine residues to a serine residue at position 5 (C5S) compared to the wild-type or unmodified Fc set forth in SEQ ID NO: 226.

Recombinant variant CD112 Fc fusion proteins were expressed and purified substantially as described in Example 5. Binding and activity of the affinity-modified variant CD112 Fc fusion proteins was assessed substantially as described in Example 6, except that cells expressing full length human CD226, TIGIT and CD112R cognate binding partners were generated. Cells were stained by flow cytometry with CD112 Fc variant and mean fluorescence intensity (MFI) was determined as described in Example 5. For bioactivity characterization, recombinant CD112 Fc variants were assessed in an anti-CD3 coimmobilization assay substantially as described in Example 5.

The results for the binding and activity studies are set forth in Table 22. The Table indicates exemplary IgSF domain amino acid substitutions (replacements) in the IgV domain of CD112 selected in the screen for affinity-maturation against the respective cognate structures TIGIT, CD226 and CD112R. The exemplary amino acid substitutions are designated by amino acid position number corresponding to position of the unmodified sequence set forth in SEQ ID NO: 286. The amino acid position is indicated in the middle, with the corresponding unmodified (e.g. wild-type) amino acid listed before the number and the identified variant amino acid substitution listed after the number. Column 2 sets forth the SEQ ID NO identifier for the variant ECD for each variant IgV-Fc fusion molecule.

Also shown is the binding activity as measured by the Mean Fluorescence Intensity (MFI) value for binding of each variant Fc-fusion molecule to cells engineered to express the cognate binding partner as the ratio of the MFI compared to the binding of the corresponding unmodified IgV-Fc fusion molecule not containing the amino acid substitution(s) to the same cell-expressed cognate binding partner. The functional activity of the variant Fc-fusion molecules to modulate the activity of T cells also is shown based on the calculated levels of IFN-gamma in culture supernatants (pg/ml) generated with the indicated variant Fc fusion molecule coimmoblized with anti-CD3 as a ratio of IFN-gamma produced by each variant CD112 IgV-Fc compared to the corresponding unmodified CD112 IgV-Fc in both functional assays.

| **TABLE 22: CD112 variants selected against cognate binding partners. Molecule sequences, binding data, and costimulatory bioactivity data.** | | | | | | |
|---|---|---|---|---|---|---|
| **CD112 mutations** | **SEQ ID NO (IgV)** | **TIGIT tfxn MFI (TIGIT MFI parental ratio)** | **CD112R tfxn MFI (CD112R MFI parental ratio)** | **CD226 MFI (CD226 MFI parental ratio)** | **Mock Expi293 MFI (Mock MFI parental ratio)** | **Anti-CD3 IFN-gamma (pg/mL) (Anti-CD3 IFN-gamma parental ratio)** |
| WT CD112 | 965 | 210829 (1.00) | 1452 (1.00) | 265392 (1.00) | 1112 (1.00) | 676.6 (1.00) |
| Y33H, A112V, G117D | 966 | 12948 (0.06) | 1552 (1.07) | 1368 (0.01) | 1241 (1.12) | 164.8 (0.24) |
| V19A, Y33H, S64G, S80G, G98S, N106Y, A112V | 967 | 48356 (0.23) | 1709 (1.18) | 2831 (0.01) | 1098 (0.99) | |
| L32P, A112V | 968 | 191432 (0.91) | 1557 (1.07) | 11095 (0.04) | 1259 (1.13) | 390.4 (0.58) |
| A95V, A112I | 969 | 238418 (1.13) | 1706 (1.17) | 51944 (0.20) | 1215 (1.09) | 282.5 (0.42) |
| P28S, A112V | 970 | 251116 (1.19) | 1985 (1.37) | 153382 (0.58) | 1189 (1.07) | 503.4 (0.74) |
| P27A, T38N, V101A, A112V | 971 | 255803 (1.21) | 2138 (1.47) | 222822 (0.84) | 1399 (1.26) | 240.7 (0.36) |
| S118F | 972 | 11356 (0.05) | 5857 (4.03) | 6938 (0.03) | 1270 (1.14) | 271.7 (0.40) |
| R12W, H48Y, F54S, S118F | 973 | 10940 (0.05) | 3474 (2.39) | 5161 (0.02) | 1069 (0.96) | |
| R12W, Q79R, S118F | 974 | 2339 (0.01) | 7370 (5.08) | 1880 (0.01) | 1338 (1.20) | 447.4 (0.66) |
| T113S, S118Y | 975 | 6212 (0.03) | 6823 (4.70) | 1554 (0.01) | 1214 (1.09) | 225.1 (0.33) |
| S118Y | 976 | 2921 (0.01) | 6535 (4.50) | 2003 (0.01) | 1463 (1.32) | 190.4 (0.28) |
| N106I, S118Y | 977 | 2750 (0.01) | 7729 (5.32) | 1815 (0.01) | 1222 (1.10) | 265.8 (0.39) |
| N106I, S118F | 978 | 1841 (0.01) | 9944 (6.85) | 1529 (0.01) | 1308 (1.18) | 437.9 (0.65) |
| A95T, L96P, S118Y | 979 | 2352 (0.01) | 4493 (3.09) | 1412 (0.01) | 1329 (1.19) | 292.4 (0.43) |
| Y33H, P67S, N106Y, A112V | 980 | 225015 (1.07) | 3259 (2.24) | 204434 (0.77) | 1296 (1.17) | 618.8 (0.91) |
| N106Y, A112V | 981 | 6036 (0.03) | 1974 (1.36) | 15334 (0.06) | 1108 (1.00) | 409.9 (0.61) |
| T18S, Y33H, A112V | 982 | 252647 (1.20) | 1347 (0.93) | 183181 (0.69) | 1412 (1.27) | 601.8 (0.89) |
| P9S, Y33H, N47S, A112V | 983 | 240467 (1.14) | 1418 (0.98) | 203608 (0.77) | 1361 (1.22) | 449.1 (0.66) |
| P42S, P67H, A112V | 984 | 204484 (0.97) | 1610 (1.11) | 188647 (0.71) | 1174 (1.06) | 530.6 (0.78) |
| P27L, L32P, P42S, A112V | 985 | 219883 (1.04) | 1963 (1.35) | 84319 (0.32) | 1900 (1.71) | 251.6 (0.37) |
| G98D, A112V | 986 | 4879 (0.02) | 2369 (1.63) | 6100 (0.02) | 1729 (1.55) | 387.0 (0.57) |
| Y33H, S35P, N106Y, A112V | 987 | 250724 (1.19) | 1715 (1.18) | 94373 (0.36) | 1495 (1.34) | 516.2 (0.76) |
| L32P, P42S, T100A, A112V | 988 | 242675 (1.15) | 1742 (1.20) | 202567 (0.76) | 1748 (1.57) | 435.3 (0.64) |
| P27S, P45S, N106I, A112V | 989 | 223557 (1.06) | 1799 (1.24) | 84836 (0.32) | 1574 (1.42) | 277.5 (0.41) |
| Y33H, N47K, A112V | 990 | 251339 (1.19) | 1525 (1.05) | 199601 (0.75) | 1325 (1.19) | 483.2 (0.71) |
| Y33H, N106Y, A112V | 991 | 297169 (1.41) | 1782 (1.23) | 258315 (0.97) | 1440 (1.30) | 485.4 (0.72) |
| K78R, D84G, A112V, F114S | 992 | 236662 (1.12) | 1638 (1.13) | 24850 (0.09) | 1345 (1.21) | 142.5 (0.21) |
| Y33H, N47K, F54L, A112V | 993 | 14483 (0.07) | 1617 (1.11) | 2371 (0.01) | 1353 (1.22) | 352.8 (0.52) |
| Y33H, A112V | 994 | 98954 (0.47) | 1216 (0.84) | 1726 (0.01) | 1298 (1.17) | |
| A95V, A112V | 995 | 168521 (0.80) | 2021 (1.39) | 200789 (0.76) | 1459 (1.31) | 412.9 (0.61) |
| R12W, A112V | 996 | 135635 (0.64) | 1582 (1.09) | 23378 (0.09) | 1412 (1.27) | 165.8 (0.24) |
| A112V | 1002 | 213576 (1.01) | 1986 (1.37) | 151900 (0.57) | 1409 (1.27) | 211.4 (0.31) |
| Y33H, A112V | 994 | 250667 (1.19) | 1628 (1.12) | 230578 (0.87) | 1216 (1.09) | 612.7 (0.91) |
| R12W, P27S, A112V | 997 | 3653 (0.02) | 1308 (0.90) | 9105 (0.03) | 1051 (0.94) | |
| Y33H, V51M, A112V | 998 | 218698 (1.04) | 1384 (0.95) | 195450 (0.74) | 1170 (1.05) | 709.4 (1.05) |
| Y33H, A112V, S118T | 999 | 219384 (1.04) | 1566 (1.08) | 192645 (0.73) | 1313 (1.18) | 396.3 (0.59) |
| Y33H, V101A, A112V, P115S | 1000 | 5605 (0.03) | 1582 (1.09) | 5079 (0.02) | 1197 (1.08) | |
| H24R, T38N, D43G, A112V | 1001 | 227095 (1.08) | 1537 (1.06) | 229311 (0.86) | 1336 (1.20) | 858.6 (1.27) |
| A112V | 1002 | 4056 (0.02) | 1356 (0.93) | 10365 (0.04) | 986 (0.89) | |
| P27A, A112V | 1003 | 193537 (0.92) | 1531 (1.05) | 230708 (0.87) | 3084 (2.77) | 355.1 (0.52) |
| A112V, S118T | 1004 | 233173 (1.11) | 1659 (1.14) | 121817 (0.46) | 845 (0.76) | 533.3 (0.79) |
| R12W, A112V, M122I | 1005 | 235935 (1.12) | 1463 (1.01) | 217748 (0.82) | 1350 (1.21) | 528.0 (0.78) |
| Q83K, N106Y, A112V | 1006 | 205948 (0.98) | 2042 (1.41) | 234958 (0.89) | 1551 (1.39) | 481.4 (0.71) |
| R12W, P27S, A112V, S118T | 1007 | 11985 (0.06) | 2667 (1.84) | 12756 (0.05) | 1257 (1.13) | 334.4 (0.49) |
| P28S, Y33H, A112V | 1008 | 4711 (0.02) | 1412 (0.97) | 3968 (0.01) | 955 (0.86) | |
| P27S, Q90R, A112V | 1009 | 3295 (0.02) | 1338 (0.92) | 6755 (0.03) | 1048 (0.94) | |
| L15V, P27A, A112V, S118T | 1010 | 209888 (1.00) | 1489 (1.03) | 84224 (0.32) | 1251 (1.13) | 512.3 0.76) |
| Y33H, N106Y, T108I, A112V | 1011 | Not tested | | | | |
| Y33H, P56L, V75M, V101M, A112V | 1012 | Not tested | | | | |

### Example 11

### Additional Affinity Modified IgSF Domains

This examples describe the design, creation, and screening of additional affinity modified CD80, CD155, CD112, PD-L1, PD-L2, CD86 (B7-2) immunomodulatory proteins, which are other components of the immune synapse (IS) that have a demonstrated dual role in both immune activation and inhibition. Also described are generation and screening of Nkp30 variants. These examples demonstrate that affinity modification of IgSF domains yields proteins that can act to both increase and decrease immunological activity. Various combinations of those domains fused in pairs (i.e., stacked) with a variant affinity modified CD80 to form a Type II immunomodulatory protein to achieve immunomodulatory activity. The example exemplifies binding and activity of the exemplary affinity-modified domains in an Fc-fusion format; such affinity-modified domains are contemplated in connection with a secretable immunomodulatory protein or transmembrane immunomodulatory protein as described.

Mutant DNA constructs of encoding a variant of the IgV domain of human CD80, CD155, CD112, PD-L1, PD-L2, CD86 (B7-2), and NKp30 for translation and expression as yeast display libraries were generated substantially as described in Example 1. For target libraries that target specific residues for complete or partial randomization with degenerate codons and/or random libraries were constructed to identify variants of the IgV of CD80 (SEQ ID NO:578), IgV of CD112 (SEQ ID NO: 965), CD155(SEQ ID NO: 652), PD-L1 (SEQ ID NO:1332), and variants of the IgV of PD-L2 (SEQ ID NO:1393) substantially as described in Example 1. Similar methods also were used to generate libraries of the IgC-like domain of NKp30 (SEQ ID NO:1566).

The degenerate or random library DNA was introduced into yeast substantially as described in Example 2 to generate yeast libraries. The libraries were used to select yeast expressing affinity modified variants of CD80, CD155, CD112, PD-L1, PD-L2, CD86 (B7-2), and NKp30 substantially as described in Example 3. Cells were processed to reduce non-binders and to enrich for CD80, CD155, CD112, PD-L1 or PD-L2 variants with the ability to bind their exogenous recombinant counter-structure proteins substantially as described in Example 3.

With CD80, CD86 and NKp30 libraries, target ligand proteins were sourced from R&D Systems (USA) as follows: human rCD28.Fc (i.e., recombinant CD28-Fc fusion protein), rPDL1.Fc, rCTLA4.Fc, and rB7H6.Fc. Two-color flow cytometry was performed substantially as described in Example 3. Yeast outputs from the flow cytometric sorts were assayed for higher specific binding affinity. Sort output yeast were expanded and re-induced to express the particular IgSF affinity modified domain variants they encode. This population then can be compared to the parental, wild-type yeast strain, or any other selected outputs, such as the bead output yeast population, by flow cytometry.

In the case of NKp30 yeast variants selected for binding to B7-H6, the F2 sort outputs gave MFI values of 533 when stained with 16.6nM rB7H6.Fc, whereas the parental NKp30 strain MFI was measured at 90 when stained with the same concentration of rB7H6.Fc (6-fold improvement).

Among the NKp30 variants that were identified, was a variant that contained mutations L30V/A60V/S64P/S86G with reference to positions in the NKp30 extracellular domain corresponding to positions set forth in SEQ ID NO:54.

For CD155 variants provided in Table 23A-E, selection involved two positive selections with the desired counter structures TIGIT and CD96 followed by one negative selection with the counter structure CD226 to select away from CD226 and improve binding specificity of the variant CD155. Selection was performed essentially as described in Example 3 above except the concentrations of the counter structures (TIGIT/CD96) and selection stringency of the positive sorts were varied to optimize lead identification. The concentration of CD226 for the negative selection was kept at 100 nM.

For additional CD112 variants provided in Table 24A-B, selection involved two positive selections with the desired counter structures TIGIT and CD112R followed by one negative selection with the counter structure CD226 to select away from CD226 and improve binding specificity of the variant CD112. Selection was performed essentially as described in Example 3 above except the concentrations of the counter structures (TIGIT/CD112R) and selection stringency of the positive sorts were varied to optimize lead identification. The concentration of CD226 for the negative selection was kept at 100 nM.

For PD-L1 and PD-L2 variants provided in Table 25 and 26A-B, yeast display targeted or random PD-L1 or PD-L2 libraries were selected against PD-1. This was then followed by two to three rounds of flow cytometry sorting using exogenous counter-structure protein staining to enrich the fraction of yeast cells that displays improved binders. Alternatively, for PD-L1, selections were performed with human rCD80.Fc (i.e., human recombinant CD80 Fc fusion protein from R&D Systems, USA). Selections were carried out largely as described for PD-1 above. Magnetic bead enrichment and selections by flow cytometry are essentially as described in Miller K. D. et al., Current Protocols in Cytometry 4.7.1-4.7.30, July 2008.

For CD80 variants provided in Tables 27A-B, CD80 libraries consisted of positive selection with the desired counter structure CTLA4 and negative selection with the counter structure CD28.

Exemplary selection outputs were reformatted as immunomodulatory proteins containing an affinity modified (variant) IgV of CD80, variant IgV of CD 155, variant IgV of CD112, variant IgV of PD-L1, variant IgV of PD-L2, each fused to an Fc molecule (variant IgV-Fc fusion molecules) substantially as described in Example 4 and the Fc-fusion protein was expressed and purified substantially as described in Example 5. In some cases, Fc-fusion proteins were generated containing: affinity-modified IgSF domain followed by a GSGGGGS linker followed by a human IgG1 Fc containing the mutations L234A, L235E, G237A, E356D and M358L by EU numbering (SEQ ID NO:2153).

Binding of exemplary IgSF domain variants to cell-expressed counter structures (cognate binding partners) was then assessed substantially as described in Example 6. Cells expressing cognate binding partners were produced and binding studies and flow cytometry were carried out substantially as described in Example 6. In addition, the bioactivity of the Fc-fusion variant protein was characterized by either mixed lymphocyte reaction (MLR) or anti-CD3 coimmobilization assay substantially as described in Example 6.

As above, for each Table, the exemplary amino acid substitutions are designated by amino acid position number corresponding to the respective reference unmodified ECD s (see Table 1). The amino acid position is indicated in the middle, with the corresponding unmodified (e.g. wild-type) amino acid listed before the number and the identified variant amino acid substitution listed (or inserted designated by a) after the number.

Also shown is the binding activity as measured by the Mean Fluorescence Intensity (MFI) value for binding of each variant Fc-fusion molecule to cells engineered to express the cognate counter structure ligand and the ratio of the MFI compared to the binding of the corresponding unmodified Fc fusion molecule not containing the amino acid substitution(s) to the same cell-expressed counter structure ligand. The functional activity of the PD-L2 variant Fc-fusion molecules to modulate the activity of T cells also is shown based on the calculated levels of IFN-gamma in culture supernatants (pg/mL) generated with the indicated variant Fc fusion molecule in an MLR assay. Table 26B also depicts the ratio of IFN-gamma produced by each variant IgV-Fc compared to the corresponding unmodified IgV-Fc in an MLR assay.

As shown, the selections resulted in the identification of a number of CD80, CD155, CD112, PD-L1, and PD-L2 IgSF domain variants that were affinity-modified to exhibit increased binding for at least one, and in some cases more than one, cognate counter structure ligand. In addition, the results showed that affinity modification of the variant molecules also exhibited improved activities to both increase and decrease immunological activity.

| **TABLE 23A: Additional CD155 Variants and Binding Data.** | | | | | | | | | |
|---|---|---|---|---|---|---|---|---|---|
| **CD155 Mutation(s)** | **SEQ ID NO (IgV)** | **TIGIT** | | **CD226** | | **CD112R** | | **CD96** | |
| | | **MFI at 100nM** | **Fold ↑ to WT ECD** | **MFI at 100nM** | **Fold ↑ to WT ECD** | **MFI at 100nM** | **Fold ↑ to WT ECD** | **MFI at 100nM** | **Fold ↑ to WT ECD** |
| S52M | 868 | 1865.3 | 0.00 | 1901.0 | 0.01 | 1553.4 | 0.87 | 1609.8 | 0.02 |
| T45Q, S52L, L104E, G111R | 869 | 2287.0 | 0.01 | 2390.4 | 0.01 | 1735.1 | 0.97 | 1575.1 | 0.02 |
| S42G | 879 | 4837.5 | 0.01 | 2448.1 | 0.01 | 1815.4 | 1.02 | 1699.6 | 0.02 |
| Q62F | 871 | 2209.5 | 0.01 | 2572.1 | 0.01 | 2706.5 | 1.52 | 2760.7 | 0.03 |
| S52Q | 872 | 2288.1 | 0.01 | 2022.3 | 0.01 | 1790.1 | 1.00 | 1822.3 | 0.02 |
| S42A, L104Q, G111R | 873 | 1923.7 | 0.00 | 1901.7 | 0.01 | 1815.1 | 1.02 | 1703.8 | 0.02 |
| S42A, S52Q, L104Q, G111R | 874 | 1807.5 | 0.00 | 2157.2 | 0.01 | 1894.4 | 1.06 | 1644.0 | 0.02 |
| S52W, L104E | 875 | 1938.2 | 0.00 | 1905.6 | 0.01 | 2070.6 | 1.16 | 1629.5 | 0.02 |
| S42C | 876 | 1914.0 | 0.00 | 2096.1 | 0.01 | 1685.0 | 0.95 | 1592.4 | 0.02 |
| S52W | 877 | 1991.6 | 0.00 | 2037.3 | 0.01 | 1612.8 | 0.90 | 1712.9 | 0.02 |
| S52M, L104Q | 878 | 2666.6 | 0.01 | 2252.2 | 0.01 | 1706.0 | 0.96 | 1633.1 | 0.02 |
| S42L, S52L, Q62F, L104Q | 879 | 2021.4 | 0.00 | 2643.8 | 0.02 | 1730.1 | 0.97 | 2318.7 | 0.02 |
| S42W | 880 | 2434.5 | 0.01 | 2133.4 | 0.01 | 2325.7 | 1.30 | 2555.4 | 0.03 |
| S42Q | 881 | 2073.5 | 0.00 | 2225.9 | 0.01 | 1905.1 | 1.07 | 2143.1 | 0.02 |
| S52L | 882 | 2224.8 | 0.01 | 2676.3 | 0.02 | 2038.6 | 1.14 | 2043.2 | 0.02 |
| S52R | 883 | 4395.4 | 0.01 | 3964.4 | 0.02 | 2741.7 | 1.54 | 4846.9 | 0.05 |
| L104E | 884 | 3135.4 | 0.01 | 2264.2 | 0.01 | 1803.5 | 1.01 | 1556.7 | 0.02 |
| G111R | 885 | 2082.7 | 0.00 | 2791.3 | 0.02 | 2470.9 | 1.39 | 3317.1 | 0.03 |
| S52E | 886 | 2655.4 | 0.01 | 2599.8 | 0.02 | 1904.9 | 1.07 | 1799.0 | 0.02 |
| Q62Y | 887 | 2528.6 | 0.01 | 2621.4 | 0.02 | 1918.4 | 1.08 | 1827.5 | 0.02 |
| T45Q, S52M, L104E | 888 | 79498. 2 | 0.19 | 143238 .5 | 0.83 | 2600.6 | 1.46 | 6310.4 | 0.06 |
| S42N, L104Q, G111R | 889 | 2432.1 | 0.01 | 2311.3 | 0.01 | 1847.4 | 1.04 | 1958.3 | 0.02 |
| S52M, V57L | 890 | 1760.7 | 0.00 | 2431.6 | 0.01 | 2006.9 | 1.13 | 1858.7 | 0.02 |
| S42N, S52Q, Q62F | 891 | 2402.7 | 0.01 | 2152.0 | 0.01 | 1855.0 | 1.04 | 1737.6 | 0.02 |
| S42A, S52L, L104E, G111R | 892 | 2262.7 | 0.01 | 1889.4 | 0.01 | 1783.2 | 1.00 | 1606.2 | 0.02 |
| S42W, S52Q, V57L, Q62Y | 893 | 1961.4 | 0.00 | 2138.3 | 0.01 | 1844.9 | 1.03 | 1699.6 | 0.02 |
| L104Q | 894 | 10314. 4 | 0.02 | 3791.4 | 0.02 | 2119.9 | 1.19 | 1542.6 | 0.02 |
| S42L, S52Q, L104E | 895 | 1946.9 | 0.00 | 6474.3 | 0.04 | 1749.0 | 0.98 | 1702.2 | 0.02 |
| S42C, S52L | 896 | 1762.5 | 0.00 | 2147.3 | 0.01 | 1663.4 | 0.93 | 1484.7 | 0.01 |
| S42W, S52R, Q62Y, L104Q | 897 | 1918.8 | 0.00 | 2300.1 | 0.01 | 1824.6 | 1.02 | 1756.0 | 0.02 |
| T45Q, S52R, L104E | 898 | 121636 .9 | 0.29 | 142381 .2 | 0.82 | 2617.9 | 1.47 | 3748.2 | 0.04 |
| S52R, Q62F, L104Q, G111R | 899 | 2969.2 | 0.01 | 3171.6 | 0.02 | 1725.4 | 0.97 | 2362.3 | 0.02 |
| T45Q, S52L, V57L, L104E | 900 | 2857.7 | 0.01 | 5943.5 | 0.03 | 1496.8 | 0.84 | 1533.3 | 0.02 |
| S52M, Q62Y | 901 | 1926.6 | 0.00 | 2000.3 | 0.01 | 1771.6 | 0.99 | 1651.1 | 0.02 |
| Q62F, L104E, G111R | 902 | 1966.4 | 0.00 | 2043.5 | 0.01 | 1701.9 | 0.95 | 1524.8 | 0.02 |
| T45Q, S52Q | 903 | 4812.8 | 0.01 | 5787.5 | 0.03 | 1765.6 | 0.99 | 2451.3 | 0.02 |
| S52L, L104E | 904 | 4317.8 | 0.01 | 2213.9 | 0.01 | 1756.9 | 0.99 | 1829.3 | 0.02 |
| S42V, S52E | 905 | 2055.0 | 0.00 | 2272.6 | 0.01 | 1808.0 | 1.01 | 2530.2 | 0.03 |
| T45Q, S52R, G111R | 906 | 4092.3 | 0.01 | 2075.2 | 0.01 | 1793.6 | 1.01 | 2336.6 | 0.02 |
| S42G, S52Q, L104E, G111R | 907 | 2010.1 | 0.00 | 2019.2 | 0.01 | 1706.4 | 0.96 | 1707.6 | 0.02 |
| S42N, S52E, V57L, | 908 | 1784.2 | 0.00 | 1743.6 | 0.01 | 1690.1 | 0.95 | 1538.7 | 0.02 |
| L104E | | | | | | | | | |
| Wildtype | 652 | 1964.7 | 0.00 | 2317.1 | 0.01 | 2169.6 | 1.22 | 1893.4 | 0.02 |
| S42C, S52M, Q62F | 909 | 1861.0 | 0.00 | 2084.2 | 0.01 | 1592.3 | 0.89 | 1481.3 | 0.01 |
| S42L | 910 | 1930.4 | 0.00 | 2187.2 | 0.01 | 1743.2 | 0.98 | 1618.4 | 0.02 |
| Wildtype | 652 | 2182.6 | 0.01 | 2374.5 | 0.01 | 1743.1 | 0.98 | 1680.4 | 0.02 |
| S42A | 911 | 1929.2 | 0.00 | 2188.6 | 0.01 | 1733.7 | 0.97 | 1623.6 | 0.02 |
| S42G, S52L, Q62F, L104Q | 912 | 1924.3 | 0.00 | 2157.6 | 0.01 | 1661.3 | 0.93 | 1642.1 | 0.02 |
| S42N | 913 | 1817.4 | 0.00 | 1910.9 | 0.01 | 1699.7 | 0.95 | 1691.5 | 0.02 |
| CD155 IgV Fc | 652 (IgV) | 4690 | 0.01 | 4690 | 0.03 | 2941 | 1.65 | 3272 | 0.03 |
| Wildtype CD155 ECD-Fc | 47 (ECD) | 423797 | 1.00 | 172839 | 1.00 | 1783 | 1.00 | 99037 | 1.00 |
| Anti-human Fc PE | | 1506.3 | 0.00 | 3774 | 0.02 | 1587 | 0.89 | 1618 | 0.02 |

| **TABLE 23B: Additional CD155 Variants and Binding Data.** | | | | | | | |
|---|---|---|---|---|---|---|---|
| **CD155 Mutation(s)** | **SEQ ID NO (IgV)** | **TIGIT** | | **CD226** | | **CD96** | |
| | | **MFI at 100nM** | **Fold Increase to WT ECD** | **MFI at 100nM** | **Fold Increase to WT ECD** | **MFI at 100nM** | **Fold Increase to WT ECD** |
| P18T, S65A, S67V, F91S | 914 | 297843 | 1.99 | 351195 | 3.22 | 128180 | 1.68 |
| P18F, T39A, T45Q, T61R, S65N, S67L, E73G, R78G | 915 | Little to no protein produced | | | | | |
| P18T, T45Q, T61R, S65N, S67L | 916 | 224682 | 1.50 | 270175 | 2.48 | 22820 | 0.30 |
| P18F, S65A, S67V, F91S | 917 | 534106 | 3.57 | 350410 | 3.21 | 144069 | 1.89 |
| P18F, T45Q, T61R, S65N, S67L, F91S, L104P | 918 | Little to no protein produced | | | | | |
| P18S, L79P, L104M | 919 | 342549 | 2.29 | 320823 | 2.94 | 107532 | 1.41 |
| P18S, L104M | 920 | 449066 | 3.00 | 295126 | 2.70 | 121266 | 1.59 |
| L79P, L104M | 921 | 3210 | 0.02 | 8323 | 0.08 | 2894 | 0.04 |
| P18T, T45Q, L79P | 922 | 542878 | 3.63 | 371498 | 3.40 | 193719 | 2.55 |
| P18T, T45Q, T61R, S65H, S67H | 923 | 312337 | 2.09 | 225439 | 2.07 | 152903 | 2.01 |
| P18T, A81E | 924 | Little to no protein produced | | | | | |
| P18S, D23Y, E37P, S52G, Q62M, G80S, A81P, G99Y, S112N | 925 | Little to no protein produced | | | | | |
| A13R, D23Y, E37P, S42P, Q62Y, A81E | 926 | 4161 | 0.03 | 11673 | 0.11 | 5762 | 0.08 |
| A13R, D23Y, E37P, G99Y, S112N | 927 | Little to no protein produced | | | | | |
| A13R, D23Y, E37P, Q62M, A77V, G80S, A81P, G99Y | 928 | Little to no protein produced | | | | | |
| P18L, E37S, Q62M, G80S, A81P, G99Y, S112N | 929 | 5900 | 0.04 | 14642 | 0.13 | 3345 | 0.04 |
| P18S, L104T | 930 | 321741 | 2.15 | 367470 | 3.37 | 108569 | 1.43 |
| P18S, Q62H, L79Q, F91S | 931 | 283357 | 1.89 | 324877 | 2.98 | 125541 | 1.65 |
| P18S, F91S | 658 | 222780 | 1.49 | 300049 | 2.75 | 48542 | 0.64 |
| T45Q, S52K, Q62F, L104Q, G111R | 932 | Little to no protein produced | | | | | |
| T45Q, S52Q, Q62Y, L104Q, G111R | 933 | Little to no protein produced | | | | | |
| T45Q, S52Q, Q62Y, L104E, G111R | 934 | Little to no protein produced | | | | | |
| V57A, T61M, S65W, S67A, E96D, L104T | 935 | Little to no protein produced | | | | | |
| P18L, V57T, T61S, S65Y, S67A, L104T | 936 | 278178 | 1.86 | 276870 | 2.54 | 121499 | 1.60 |
| P18T, T45Q | 937 | 326769 | 2.18 | 357515 | 3.28 | 92389 | 1.21 |
| P18L, V57A, T61M, S65W, S67A, L104T | 938 | Little to no protein produced | | | | | |
| T61M, S65W, S67A, L104T | 939 | 360915 | 2.41 | 417897 | 3.83 | 148954 | 1.96 |
| P18S, V41A, S42G, T45G, L104N | 940 | 3821 | 0.03 | 11449 | 0.10 | 3087 | 0.04 |
| P18H, S42G, T45I, S52T, G53R, S54H, V57L, H59E, T61S, S65D, E68G, L104N | 941 | 5066 | 0.03 | 177351 | 1.63 | 3700 | 0.05 |
| P18S, S42G, T45V, F58L, S67W, L104N | 942 | 14137 | 0.09 | 15175 | 0.14 | 15324 | 0.20 |
| P18S, T45I, L104N | 943 | 141745 | 0.95 | 298011 | 2.73 | 97246 | 1.28 |
| P18S, S42G, T45G, L104N, V106A | 944 | 29387 | 0.20 | 117965 | 1.08 | 15884 | 0.21 |
| P18H, H40R, S42G, T45I, S52T, G53R, S54H, V57L, H59E, T61S, S65D, E68G, L104Y, V106L, F108H | 945 | 12335 | 0.08 | 14657 | 0.13 | 15779 | 0.21 |
| E37V, S42G, T45G, L104N | 946 | Little to no protein produced | | | | | |
| P18S, T45Q, L79P, L104T | 947 | 206674 | 1.38 | 285512 | 2.62 | 87790 | 1.15 |
| P18L, Q62R | 948 | 66939 | 0.45 | 25063 | 0.23 | 10928 | 0.14 |
| A13R, D23Y, E37P, S42L, S52G, Q62Y, A81E | 949 | Little to no protein produced | | | | | |
| P18L, H49R, L104T, D116N | 950 | 167980 | 1.12 | 214677 | 1.97 | 62451 | 0.82 |
| A13R, D23Y, E37P, Q62M, G80S, A81P, L104T | 951 | Little to no protein produced | | | | | |
| S65T, L104T | 952 | 205942 | 1.38 | 187147 | 1.71 | 65207 | 0.86 |
| A13R, D23Y, E37P, S52G, V57A, Q62M, K70E, L104T | 953 | Little to no protein produced | | | | | |
| P18L, A47V, Q62Y, E73D, L104T | 954 | 146142 | 0.98 | 248926 | 2.28 | 73956 | 0.97 |
| H40T, V41M, A47V, S52Q, Q62L, S65T, E73R, D97G, E98S, L104T, D116N | 955 | Little to no protein produced | | | | | |
| P18L, S42P, T45Q, T61G, S65H, S67E, L104T, D116N | 956 | 153536 | 1.03 | 402503 | 3.69 | 53044 | 0.70 |
| P18S, H40T, V41M, A47V, S52Q, Q62L, S65T, E73R, L104M, V106A | 957 | Little to no protein produced | | | | | |
| H40T, V41M, A47V, S52Q, Q62L, S65T, E68G, E73R, D97G, E98S, L104T | 958 | Little to no protein produced | | | | | |
| T45Q, S52E, L104E | 959 | Little to no protein produced | | | | | |
| T45Q, S52E, Q62F, L104E | 960 | 132850 | 0.89 | 276434 | 2.53 | 14558 | 0.19 |
| Wildtype CD155 ECD-Fc | 47 (ECD) | 149692 | 1.00 | 109137 | 1.00 | 76083 | 1.00 |
| Anti-human Fc PE | - | 2287 | 0.02 | 4799 | 0.04 | 2061 | 0.03 |

| **TABLE 23C: Additional CD155 Variants and Binding Data.** | | | | | | | |
|---|---|---|---|---|---|---|---|
| **CD155 Mutations** | **SEQID NO (IgV)** | **TIGIT** | | **CD226** | | **CD96** | |
| | | **MFI at 100nM** | **Fold Increase to WT IgV** | **MFI at 100nM** | **Fold Increase to , WT IgV** | **MFI at 100nM** | **Fold Increase to WT IgV** |
| P18F, T26M, L44V, Q62K, L79P, F91S, L104M, G111D | 961 | 117327 | 1.2 | 1613 | 0.1 | 1629 | 0.1 |
| P18S, T45S, T61K, S65W, S67A, F91S, G111R | 962 | 124936 | 1.3 | 2114 | 0.1 | 2223 | 0.1 |
| P18S, L79P, L104M, T107M | 963 | 110512 | 1.1 | 18337 | 0.9 | 22793 | 1.3 |
| P18S, S65W, S67A, M90V, V95A, L104Q, G111R | 964 | 101726 | 1.0 | 1605 | 0.1 | 2571 | 0.1 |
| Wildtype CD155-ECD | 47 (ECD) | 98935 | 1.0 | 20029 | 1.0 | 17410 | 1.0 |

| **TABLE 23D: Additional CD155 Variants and Binding Data.** | | | | | | | |
|---|---|---|---|---|---|---|---|
| **CD155 Mutations** | **SEQ ID NO (IgV)** | **TIGIT** | | **CD226** | | **CD96** | |
| | | MFI at 11.1nM | Fold Change from CD155-ECD | MFI at 11.1nM | Fold Change from CD155-ECD | MFI at 11.1nM | Fold Change from CD155-ECD |
| P18S, A47G, L79P, F91S, L104M, T107A, R113W | 1678 | 56,409 | 1.19 | 1,191 | 0.08 | 25,362 | 1.49 |
| P18T, D23G, S24A, N35D, H49L, L79P, F91S, L104M, G111R | 1679 | 128,536 | 2.72 | 987 | 0.06 | 3,497 | 0.20 |
| V9L, P18S, Q60R, V75L, L79P, R89K, F91S, L104E, G111R | 1680 | 125,329 | 2.65 | 986 | 0.06 | 959 | 0.06 |
| P18S, H49R, E73D, L79P, N85D, F91S, V95A, L104M, G111R | 1681 | Little to no protein produced | | | | | |
| V11A, P18S, L79P, F91S, L104M, G111R | 1682 | 48,246 | 1.02 | 974 | 0.06 | 923 | 0.05 |
| V11A, P18S, S54R, Q60P, Q62K, L79P, N85D, F91S, T107M | 1683 | 190,392 | 4.02 | 1,019 | 0.07 | 1,129 | 0.07 |
| P18T, S52P, S65A, S67V, L79P, F91S, L104M, G111R | 1684 | 121,611 | 2.57 | 986 | 0.06 | 16,507 | 0.97 |
| P18T, M36T , L79P, F91S, G111R | 1685 | 150,015 | 3.17 | 1,029 | 0.07 | 2,514 | 0.15 |
| D8G, P18S, M36I, V38A, H49Q, A76E, F91S, L104M, T107A, R113W | 1686 | 79,333 | 1.68 | 1,026 | 0.07 | 2,313 | 0.14 |
| P18S, S52P, S65A, S67V, L79P, F91S, L104M, T107S, R113W | 1687 | 23,766 | 0.50 | 1,004 | 0.07 | 1,080 | 0.06 |
| T15I, P18T, L79P, F91S, L104M, G111R | 1688 | 55,498 | 1.17 | 1,516 | 0.10 | 1,030 | 0.06 |
| P18F, T26M, L44V, Q62K, L79P, E82D, F91S, L104M, G111D | 1689 | 213,640 | 4.51 | 991 | 0.06 | 1,276 | 0.07 |
| P18T, E37G, G53R, Q62K, L79P, F91S, E98D, L104M, T107M | 1690 | 251,288 | 5.31 | 2,001 | 0.13 | 45,878 | 2.69 |
| P18L, K70E, L79P, F91S, V95A, G111R | 1691 | 62,608 | 1.32 | 1,117 | 0.07 | 973 | 0.06 |
| V9I, Q12K, P18F, S65A, S67V, L79P, L104T, G111R, S112I | 1692 | 81,932 | 1.73 | 803 | 0.05 | 68,295 | 4.00 |
| P18F, S65A, S67V, F91S, L104M, G111R | 1693 | 30,661 | 0.65 | 901 | 0.06 | 3,193 | 0.19 |
| V9I, V10I, P18S, F20S, T45A, L79P, F91S, L104M, F108Y, G111R, S112V | 1694 | 151,489 | 3.20 | 973 | 0.06 | 974 | 0.06 |
| V9L, P18L, L79P, M90I, F91S, T102S, L104M, G111R | 1695 | 155,279 | 3.28 | 910 | 0.06 | 10,568 | 0.62 |
| P18C, T26M, L44V, M55I, Q62K, L79P, F91S, L104M, T107M | 1696 | 137,521 | 2.91 | 973 | 0.06 | 111,085 | 6.51 |
| V9I, P18T, D23G, L79P, F91S, G111R | 1697 | 151,426 | 3.20 | 897 | 0.06 | 2,725 | 0.16 |
| P18F, L79P, M90L, F91S, V95A, L104M, G111R | 1698 | 125,639 | 2.66 | 917 | 0.06 | 3,939 | 0.23 |
| P18F, L79P, M90L, F91S, V95A, L104M, G111R | 1698 | 115,156 | 2.43 | 1,073 | 0.07 | 2,464 | 0.14 |
| P18T, M36T, S65A, S67E, L79Q, A81T, F91S, G111R | 1699 | 10,616 | 0.22 | 1,130 | 0.07 | 963 | 0.06 |
| V9L, P18T, Q62R, L79P, F91S, L104M, G111R | 1700 | 195,111 | 4.12 | 835 | 0.05 | 1,497 | 0.09 |
| CD 155-ECD-Fc | 47 (ECD) | 47,319 | 1.00 | 15,421 | 1.00 | 17,067 | 1.00 |
| Fc Control | - | 2,298 | 0.05 | 1,133 | 0.07 | 996 | 0.06 |

| **TABLE 23E: Additional CD155 Variants and Binding Data.** | | | | | | | | | |
|---|---|---|---|---|---|---|---|---|---|
| **CD155 Mutations** | **SEQ ID NO (IgV)** | **TIGIT** | | **CD226** | | **CD112R** | | **CD96** | |
| | | MFI at 25nM | Fold Change from CD155 -ECD | MFI at 25nM | Fold Change from CD155 -ECD | MFI at 25nM | Fold Change from CD155 -ECD | MFI at 25nM | Fold Change from CD155 -ECD |
| P18T, G19D, M36T, S54N, L79P, L83Q, F91S, T107M, F108Y | 1819 | 905 | 0.02 | 748 | 0.02 | 1276 | 1.56 | 726 | 0.01 |
| V9L, P18L, M55V, S69L, L79P, A81E, F91S, T107M | 1820 | 58656 | 1.34 | 11166 | 0.29 | 920 | 1.13 | 67364 | 1.39 |
| P18F, H40Q, T61K, Q62K, L79P, F91S, L104M, T107V | 1821 | 108441 | 2.48 | 853 | 0.02 | 918 | 1.13 | 8035 | 0.17 |
| P18S, Q32R, Q62K, R78G, L79P, F91S, T107A, R113W | 1822 | 5772 | 0.13 | 701 | 0.02 | 843 | 1.03 | 831 | 0.02 |
| Q12H, P18T, L21S, G22S, V57A, Q62R, L79P, F91S, T107M | 1823 | 1084 | 0.02 | 687 | 0.02 | 876 | 1.07 | 818 | 0.02 |
| V9I, P18S, S24P, H49Q, F58Y, Q60R, Q62K, L79P, F91S, T107M | 1824 | 69926 | 1.60 | 1089 | 0.03 | 1026 | 1.26 | 43856 | 0.90 |
| P18T, W46C, H49R, S65A, S67V, A76T, L79P, S87T, L104M | 1825 | 918 | 0.02 | 640 | 0.02 | 803 | 0.98 | 717 | 0.01 |
| P18S, S42T, E51G , L79P, F91S, G92W, T107M | 1826 | 12630 | 0.29 | 707 | 0.02 | 857 | 1.05 | 1050 | 0.02 |
| P18S, S42T , E51G , L79P, F91S, G92W, T107M | 1826 | 7476 | 0.17 | 851 | 0.02 | 935 | 1.15 | 924 | 0.02 |
| V10F, T15S, P18L, R48Q, L79P, F91S, T107M, V115M | 1827 | 1168 | 0.03 | 792 | 0.02 | 901 | 1.10 | 998 | 0.02 |
| P18S, L21M, Y30F, N35D, R84W, F91S, T107M, D116G | 1828 | 1377 | 0.03 | 743 | 0.02 | 946 | 1.16 | 1033 | 0.02 |
| P18F, E51V, S54G, Q60R, L79Q, E82G, S87T, M90I, F91S, G92R, T107M | 1829 | 46090 | 1.05 | 15701 | 0.41 | 1012 | 1.24 | 61814 | 1.27 |
| Q16H, P18F, F91S, T107M | 1830 | Little to no protein produced | | | | | | | |
| P18T, D23G, Q60R, S67L, L79P, F91S, T107M, V115A | 1831 | 64091 | 1.47 | 30931 | 0.81 | 874 | 1.07 | 108875 | 2.24 |
| D8G, V9I, V11A, P18T, T26M, S52P, L79P, F91S, G92A, T107L, V115A | 1832 | 52508 | 1.20 | 9483 | 0.25 | 817 | 1.00 | 97770 | 2.01 |
| V9I, P18F, A47E, G50S, E68G, L79P, F91S, T107M | 1833 | 55167 | 1.26 | 54341 | 1.43 | 752 | 0.92 | 102115 | 2.10 |
| P18S, M55I, Q62K, S69P, L79P, F91S, T107M | 1834 | Little to no protein produced | | | | | | | |
| P18T, T39S, S52P, S54R, L79P, F91S, T107M | 1835 | 45927 | 1.05 | 744 | 0.02 | 1038 | 1.27 | 1225 | 0.03 |
| P18S, D23N, L79P, F91S, T107M, S114N | 1836 | Little to no protein produced | | | | | | | |
| P18S, P34S , E51V, L79P, F91S, G111R | 1837 | 7917 | 0.18 | 769 | 0.02 | 853 | 1.04 | 892 | 0.02 |
| P18S, H59N, V75A, L79P, A81T, F91S, L104M, T107M | 1838 | 800 | 0.02 | 676 | 0.02 | 915 | 1.12 | 759 | 0.02 |
| P18S, W46R, E68D, L79P, F91S, T107M, R113G | 1839 | 1359 | 0.03 | 717 | 0.02 | 798 | 0.98 | 737 | 0.02 |
| V9L, P18F, T45A, S65A, S67V, R78K, L79V, F91S, T107M, S114T | 1840 | 130274 | 2.98 | 153569 | 4.04 | 812 | 1.00 | 85605 | 1.76 |
| P18T, M55L , T61R , L79P, F91S, V106I, T107M | 1841 | 133399 | 3.05 | 1906 | 0.05 | 827 | 1.01 | 57927 | 1.19 |
| T15I, P18S, V33M, N35F, T39S, M55L, R78S, L79P, F91S, T107M | 1842 | 7550 | 0.17 | 1015 | 0.03 | 789 | 0.97 | 2709 | 0.06 |
| P18S, Q62K, K70E, L79P, F91S, G92E, R113W | 1843 | 11173 | 0.26 | 691 | 0.02 | 735 | 0.90 | 1951 | 0.04 |
| P18F, F20I, T26M, A47V, E51K, L79P, F91S | 1844 | 136088 | 3.11 | 54026 | 1.42 | 1401 | 1.72 | 96629 | 1.99 |
| P18T, D23A, Q60H, L79P, M90V, F91S, T107M | 1845 | 43795 | 1.00 | 98241 | 2.58 | 888 | 1.09 | 70891 | 1.46 |
| P18S, D23G, C29R, N35D, E37G, M55I, Q62K, S65A, S67G, R78G, L79P, F91S, L104M, T107M, Q110R | 1846 | 1599 | 0.04 | 1030 | 0.03 | 1115 | 1.37 | 1944 | 0.04 |
| A13E, P18S, M36R, Q62K, S67T, L79P, N85D, F91S, T107M | 1847 | Little to no protein produced | | | | | | | |
| V9I, P18T, H49R, L79P, N85D, F91S, L104T, T107M | 1848 | 46375 | 1.06 | 76851 | 2.02 | 794 | 0.97 | 80210 | 1.65 |
| V9A, P18F, T61S, Q62L, L79P, F91S, G111R | 1849 | 26109 | 0.60 | 891 | 0.02 | 825 | 1.01 | 2633 | 0.05 |
| D8E, P18T, T61A, L79P, F91S, T107M | 1850 | Little to no protein produced | | | | | | | |
| P18S, V41A, H49R, S54C, L79S, N85Y, L88P, F91S, L104M, T107M | 1851 | 1098 | 0.03 | 830 | 0.02 | 876 | 1.07 | 1678 | 0.03 |
| V11E, P18H, F20Y, V25E, N35S, H49R, L79P, F91S, T107M, G111R | 1852 | 979 | 0.02 | 846 | 0.02 | 844 | 1.03 | 928 | 0.02 |
| V11A, P18F, D23A, L79P, | 1853 | 45249 | 1.04 | 913 | 0.02 | 830 | 1.02 | 33883 | 0.70 |
| G80D, V95A, T107M | | | | | | | | | |
| P18S, K70R, L79P, F91S, G111R | 1854 | 16180 | 0.37 | 793 | 0.02 | 854 | 1.05 | 1182 | 0.02 |
| P18T, D23A, Q60H, L79P, M90V, F91S, T107M | 1845 | 175673 | 4.02 | 161958 | 4.26 | 879 | 1.08 | 50981 | 1.05 |
| V9L, V11M, P18S, N35S, S54G, Q62K, L79P, L104M, T107M, V115M | 1855 | 2999 | 0.07 | 2315 | 0.06 | 893 | 1.09 | 925 | 0.02 |
| V9L, P18Y, V25A, V38G, M55V, A77T, L79P, M90I, F91S, L104M | 1856 | 138011 | 3.16 | 26015 | 0.68 | 919 | 1.13 | 17970 | 0.37 |
| V10G, P18T, L72Q, L79P, F91S, T107M | 1857 | 4253 | 0.10 | 1584 | 0.04 | 863 | 1.06 | 3643 | 0.07 |
| P18S, H59R, A76G, R78S, L79P | 1858 | 130622 | 2.99 | 79435 | 2.09 | 1009 | 1.24 | 44493 | 0.91 |
| V9A, P18S, M36T, S65G, L79P, F91S, L104T, G111R, S112I | 1859 | 92503 | 2.12 | 989 | 0.03 | 886 | 1.09 | 7850 | 0.16 |
| P18T, S52A, V57A, Q60R, Q62K, S65C, L79P, F91T, N100Y, T107M | 1860 | 187338 | 4.29 | 10579 | 0.28 | 908 | 1.11 | 3791 | 0.08 |
| V11A, P18F, N35D, A47E, Q62K, L79P, F91S, G99D, T107M, S114N | 1861 | Little to no protein produced | | | | | | | |
| V11A, P18T, N35S, L79P, S87T, F91S | 1862 | 218660 | 5.00 | 273825 | 7.20 | 1269 | 1.56 | 69871 | 1.44 |
| V9D, V11M, Q12L, P18S, E37V, M55I, Q60R, K70Q, L79P, F91S, L104M, T107M | 1863 | 8693 | 0.20 | 790 | 0.02 | 852 | 1.04 | 1991 | 0.04 |
| T15S, P18S, Y30H, Q32L, Q62R, L79P, F91S, T107M | 1864 | 16213 | 0.37 | 2092 | 0.06 | 1056 | 1.29 | 6994 | 0.14 |
| CD155-ECD-Fc | 47 (ECD) | 43704 | 1.00 | 38032 | 1.00 | 816 | 1.00 | 48638 | 1.00 |
| CD 112-IgV | 965 | 1289 | | 824 | | 17819 | | 1172 | 0.02 |

| **TABLE 24A: Additional CD112 Variants and Binding Data.** | | | | | | | | | |
|---|---|---|---|---|---|---|---|---|---|
| **CD112 Mutation(s)** | **SEQ ID NO (IgV)** | **TIGIT** | | **CD226** | | **CD112R** | | **CD96** | |
| | | **MFI 100nM** | **Fold Increase to WT IgV** | **MFI at 100nM** | **Fold Increase to WT IgV** | **MFI at 100nM** | **Fold Increase to WT IgV** | **MFI at 100nM** | **Fold Increase to WT IgV** |
| S118F | 972 | 1763 | 0.02 | 1645 | 0.08 | 2974 | 0.61 | 1659 | 0.19 |
| N47K, Q79R, S118F | 1095 | 1738 | 0.02 | 1689 | 0.09 | 2637 | 0.54 | 1647 | 0.19 |
| Q40R, P60T, A112V, S118T | 1096 | 4980 | 0.06 | 1608 | 0.08 | 2399 | 0.50 | 2724 | 0.32 |
| F114Y, S118F | 1097 | 110506 | 1.34 | 7325 | 0.37 | 1502 | 0.31 | 1553 | 0.18 |
| N106I, S118Y | 977 | 1981 | 0.02 | 1700 | 0.09 | 2394 | 0.49 | 1582 | 0.19 |
| S118Y | 976 | 101296 | 1.23 | 9990 | 0.50 | 1429 | 0.30 | 1551 | 0.18 |
| Y33H, K78R, S118Y | 1098 | 2276 | 0.03 | 2115 | 0.11 | 3429 | 0.71 | 2082 | 0.24 |
| N106I, S118F | 978 | 1875 | 0.02 | 1675 | 0.08 | 2365 | 0.49 | 1662 | 0.19 |
| R12W, A46T, K66M, Q79R, N106I, T113A, S118F | 1099 | 3357 | 0.04 | 1808 | 0.09 | 1664 | 0.34 | 4057 | 0.48 |
| Y33H, A112V, S118F | 1100 | 3376 | 0.04 | 2886 | 0.15 | 3574 | 0.74 | 3685 | 0.43 |
| R12W, Y33H, N106I, S118F | 1101 | 100624 | 1.22 | 24513 | 1.24 | 1490 | 0.31 | 2060 | 0.24 |
| L15V, Q90R, S118F | 1102 | 5791 | 0.07 | 4169 | 0.21 | 2752 | 0.57 | 4458 | 0.52 |
| N47K, D84G, N106I, S118Y | 1103 | 3334 | 0.04 | 2819 | 0.14 | 2528 | 0.52 | 3498 | 0.41 |
| L32P, S118F | 1104 | 3881 | 0.05 | 2506 | 0.13 | 2659 | 0.55 | 2518 | 0.29 |
| Y33H, Q79R, A112V, S118Y | 1105 | | | | | | | | |
| T18A, N106I, S118T | 1106 | 84035 | 1.02 | 10208 | 0.52 | 1585 | 0.33 | 1590 | 0.19 |
| L15V, Y33H, N106Y, A112V, S118F | 1107 | | | | | | | | |
| V37M, S118F | 1108 | 96986 | 1.18 | 2523 | 0.13 | 1985 | 0.41 | 1849 | 0.22 |
| N47K, A112V, S118Y | 1109 | 1980 | 0.02 | 1859 | 0.09 | 2733 | 0.56 | 1825 | 0.21 |
| A46T, A112V | 1110 | 4224 | 0.05 | 4685 | 0.24 | 3288 | 0.68 | 4273 | 0.50 |
| P28S, Y33H, N106I, S118Y | 1111 | 6094 | 0.07 | 2181 | 0.11 | 1891 | 0.39 | 3021 | 0.35 |
| P30S, Y33H, N47K, V75M, Q79R, N106I, S118Y | 1112 | 2247 | 0.03 | 2044 | 0.10 | 1796 | 0.37 | 2658 | 0.31 |
| V19A, N47K, N106Y, K116E, S118Y | 1113 | 2504 | 0.03 | 2395 | 0.12 | 2174 | 0.45 | 2852 | 0.33 |
| Q79R, T85A, A112V, S118Y | 1114 | 2192 | 0.03 | 1741 | 0.09 | 2367 | 0.49 | 1620 | 0.19 |
| Y33H, A112V | 994 | 20646 | 0.25 | 1465 | 0.07 | 1794 | 0.37 | 2589 | 0.30 |
| V101M, N106I, S118Y | 1115 | 55274 | 0.67 | 6625 | 0.33 | 1357 | 0.28 | 1494 | 0.17 |
| Y33H, Q79R, N106I, A112V, S118T | 1116 | 6095 | 0.07 | 1760 | 0.09 | 2393 | 0.49 | 3033 | 0.36 |
| Q79R, A112V | 1117 | 1571 | 0.02 | 1490 | 0.08 | 2284 | 0.47 | 1326 | 0.16 |
| Y33H, A46T, Q79R, N106I, S118F | 1118 | 90813 | 1.10 | 15626 | 0.79 | 1298 | 0.27 | 3571 | 0.42 |
| A112V, G121S | 1119 | 95674 | 1.16 | 19992 | 1.01 | 1252 | 0.26 | 4005 | 0.47 |
| Y33H, Q79R, N106I, S118Y | 1120 | 36246 | 0.44 | 2118 | 0.11 | 1970 | 0.41 | 3250 | 0.38 |
| Y33H, N106I, A112V | 1121 | 47352 | 0.57 | 4217 | 0.21 | 2641 | 0.55 | 1488 | 0.17 |
| Y33H, A46T, V101M, A112V, S118T | 1122 | 14413 | 0.17 | 1596 | 0.08 | 2335 | 0.48 | 1441 | 0.17 |
| L32P, L99M, N106I, S118F | 1123 | 3056 | 0.04 | 1791 | 0.09 | 2210 | 0.46 | 2000 | 0.23 |
| L32P, T108A, S118F | 1124 | 104685 | 1.27 | 4531 | 0.23 | 2308 | 0.48 | 1518 | 0.18 |
| A112V | 1002 | 4937 | 0.06 | 1903 | 0.10 | 1646 | 0.34 | 3011 | 0.35 |
| R12W, Q79R, A112V | 1125 | 55539 | 0.67 | 6918 | 0.35 | 1386 | 0.29 | 1740 | 0.20 |
| Y33H, N106Y, E110G, A112V | 1126 | 2786 | 0.03 | 2517 | 0.13 | 1787 | 0.37 | 2023 | 0.24 |
| Y33H, N106I, S118Y | 1127 | 1967 | 0.02 | 1579 | 0.08 | 2601 | 0.54 | 1517 | 0.18 |
| Q79R, S118F | 1128 | 82055 | 1.00 | 7582 | 0.38 | 1298 | 0.27 | 1970 | 0.23 |
| Y33H, Q79R, G98D, V101M, A112V | 1129 | 21940 | 0.27 | 1632 | 0.08 | 1141 | 0.24 | 18423 | 2.16 |
| N47K, T81S, V101M, A112V, S118F | 1130 | 6889 | 0.08 | 1311 | 0.07 | 1303 | 0.27 | 1145 | 0.13 |
| G82S, S118Y | 1131 | 4267 | 0.05 | 1938 | 0.10 | 2140 | 0.44 | 2812 | 0.33 |
| Y33H, A112V, S118Y | 1132 | 14450 | 0.18 | 1532 | 0.08 | 2353 | 0.49 | 3004 | 0.35 |
| Y33H, N47K, Q79R, N106Y, A112V | 1133 | 70440 | 0.85 | 3557 | 0.18 | 1447 | 0.30 | 1679 | 0.20 |
| Y33H, S118T | 1134 | 113896 | 1.38 | 17724 | 0.89 | 1252 | 0.26 | 5001 | 0.59 |
| R12W, Y33H, Q79R, V101M, A112V | 1135 | 3376 | 0.04 | 2727 | 0.14 | 2047 | 0.42 | 2339 | 0.27 |
| S118F | 972 | 2685 | 0.03 | 1864 | 0.09 | 2520 | 0.52 | 1566 | 0.18 |
| Wildtype CD112-IgV Fc | 965 (IgV) | 82414 | 1.00 | 19803 | 1.00 | 4842 | 1.00 | 8541 | 1.00 |
| CD112 ECD-Fc | 48 | 29157 | 0.35 | 8755 | 0.44 | 1107 | 0.23 | 1103 | 0.13 |
| | (ECD) | | | | | | | | |
| Anti-hFc PE | - | 1383 | 0.02 | 1461 | 0.07 | 1358 | 0.28 | 1468 | 0.17 |

| **TABLE 24B: Additional CD112 Variants and Binding Data.** | | | | | | | | | |
|---|---|---|---|---|---|---|---|---|---|
| **Mutation(s)** | **SEQ ID NO (IgV)** | **TIGIT** | | **CD226** | | **CD112R** | | **CD96** | |
| | | **MFI 20nM** | **Fold Increase to WT IgV** | **MFI at 20nM** | **Fold Increase to WT IgV** | **MFI at 20nM** | **Fold Increase to WT IgV** | **MFI at 20nM** | **Fold Increa se to WT IgV** |
| N106I, S118Y | 977 | 1288 | 0.04 | 1334 | 0.12 | 6920 | 4.16 | 1102 | 0.44 |
| Y33H, Q83K, A112V, S118T | 1631 | 115690 | 3.31 | 10046 | 0.93 | 1128 | 0.68 | 2053 | 0.82 |
| R12W, Q79R, S118F | 974 | 1436 | 0.04 | 1296 | 0.12 | 6546 | 3.93 | 1046 | 0.42 |
| V29M, Y33H, N106I, S118F | 1632 | Not tested | | | | | | | |
| Y33H, A46T, A112V | 1633 | 111256 | 3.18 | 14974 | 1.39 | 1148 | 0.69 | 3333 | 1.34 |
| Y33H, Q79R, S118F | 1634 | 1483 | 0.04 | 1326 | 0.12 | 7425 | 4.46 | 1138 | 0.46 |
| Y33H, N47K, F74L, S118F | 1635 | 1338 | 0.04 | 1159 | 0.11 | 1516 | 0.91 | 1140 | 0.46 |
| R12W, V101M, N106I, S118Y | 1636 | 1378 | 0.04 | 1249 | 0.12 | 5980 | 3.59 | 1182 | 0.47 |
| A46T, V101A, N106I, S118Y | 1637 | 1359 | 0.04 | 1199 | 0.11 | 6729 | 4.04 | 1173 | 0.47 |
| Y33H, N106Y, A112V | 991 | 113580 | 3.25 | 17771 | 1.65 | 1207 | 0.72 | 2476 | 0.99 |
| N106Y, A112V, S118T | 1638 | Not tested | | | | | | | |
| S76P, T81I, V101M, N106Y, A112V, S118F | 1639 | Not tested | | | | | | | |
| N106Y, A112V | 981 | 29015 | 0.83 | 2760 | 0.26 | 1159 | 0.70 | 1639 | 0.66 |
| P9R, L21V, P22L, I34M, S69F, F74L, A87V, A112V, L125A | 1640 | 1920 | 0.05 | 1218 | 0.11 | 1107 | 0.66 | 1074 | 0.43 |
| Y33H, V101M, A112V | 1641 | 126266 | 3.61 | 24408 | 2.27 | 1150 | 0.69 | 4535 | 1.82 |
| N106I, S118F | 978 | 1776 | 0.05 | 1385 | 0.13 | 9058 | 5.44 | 1370 | 0.55 |
| V29A, L32P, S118F | 1642 | 1265 | 0.04 | 1148 | 0.11 | 5057 | 3.04 | 1194 | 0.48 |
| A112V | 1002 | 69673 | 1.99 | 6387 | 0.59 | 1140 | 0.68 | 1214 | 0.49 |
| Y33H, V101M, A112V | 1641 | 133815 | 3.83 | 24992 | 2.32 | 1184 | 0.71 | 6338 | 2.54 |
| P28S, Y33H, N106I, S118Y | 1111 | 2745 | 0.08 | 1689 | 0.16 | 6625 | 3.98 | 1978 | 0.79 |
| Y33H, V101M, N106I, A112V | 1643 | 118654 | 3.40 | 21828 | 2.03 | 1253 | 0.75 | 3871 | 1.55 |
| R12W, Y33H, N47K, Q79R, S118Y | 1644 | 171390 | 4.91 | 5077 | 0.47 | 1124 | 0.68 | 2636 | 1.06 |
| A112V, S118T | 1004 | 103203 | 2.95 | 15076 | 1.40 | 1155 | 0.69 | 1426 | 0.57 |
| Y33H, A46T, A112V, S118T | 1645 | 141859 | 4.06 | 29436 | 2.74 | 1184 | 0.71 | 5760 | 2.31 |
| Y33H, A112V, F114L, S118T | 1646 | 5161 | 0.15 | 1734 | 0.16 | 1184 | 0.71 | 1249 | 0.50 |
| A112V | 1002 | 78902 | 2.26 | 6224 | 0.58 | 1114 | 0.67 | 1181 | 0.47 |
| Y33H, T38A, A46T, V101M, A112V | 1647 | 111293 | 3.19 | 25702 | 2.39 | 1192 | 0.72 | 99015 | 39.69 |
| Q79R, A112V | 1117 | 96674 | 2.77 | 7264 | 0.67 | 1130 | 0.68 | 1216 | 0.49 |
| Y33H, N106I, S118Y | 1127 | 5720 | 0.16 | 1453 | 0.14 | 6543 | 3.93 | 1248 | 0.50 |
| P28S, Y33H, S69P, N106I, A112V, S118Y | 1648 | 22393 | 0.64 | 1378 | 0.13 | 1550 | 0.93 | 19174 | 7.68 |
| Y33H, P42L, N47K, V101M, A112V | 1649 | 214116 | 6.13 | 13878 | 1.29 | 1315 | 0.79 | 4753 | 1.91 |
| Y33H, N47K, F74S, Q83K, N106I, F111L, A112V, S118T | 1650 | 6719 | 0.19 | 1319 | 0.12 | 1305 | 0.78 | 1278 | 0.51 |
| Y33H, A112V, S118T, V119A | 1651 | 184794 | 5.29 | 10204 | 0.95 | 1269 | 0.76 | 4321 | 1.73 |
| Y33H, N106I, A112V, S118F | 1652 | 6872 | 0.20 | 1591 | 0.15 | 2308 | 1.39 | 2796 | 1.12 |
| Y33H, K66M, S118F, W124L | 1653 | 1724 | 0.05 | 1259 | 0.12 | 6782 | 4.07 | 1197 | 0.48 |
| S118F | 972 | 1325 | 0.04 | 1213 | 0.11 | 7029 | 4.22 | 1135 | 0.46 |
| N106I, A112V | 1654 | 111342 | 3.19 | 4241 | 0.39 | 1546 | 0.93 | 1178 | 0.47 |
| Y33H, A112V | 994 | 177926 | 5.09 | 13761 | 1.28 | 1152 | 0.69 | 3117 | 1.25 |
| WT CD112 IgV | 965 | 34932 | 1.00 | 10762 | 1.00 | 1665 | 1.00 | 2495 | 1.00 |
| WT CD112-Fc ECD | 48 (ECD) | 28277 | 0.81 | 8023 | 0.75 | 1253 | 0.75 | 1064 | 0.43 |
| Anti-huFc PE | - | 1138 | 0.03 | 1006 | 0.09 | 1010 | 0.61 | 1062 | 0.43 |

| **TABLE 25A: Selected PD-L1 variants and binding data.** | | | |
|---|---|---|---|
| **PD-L1 Mutation(s)** | **SEQ ID NO (IgV)** | **Binding to Jurkat/PD-1 Cells** | |
| | | **MFI at 50nM** | **Fold increase over wildtype PD-L1 IgV-Fc** |
| K28N, M41V, N45T, H51N, K57E | 1267 | 12585 | 2.4 |
| I20L, I36T, N45D, I47T | 1268 | 3119 | 0.6 |
| I20L, M41K, K44E | 1269 | 9206 | 1.8 |
| P6S, N45T, N78I, I83T | 1270 | 419 | 0.1 |
| N78I | 1271 | 2249 | 0.4 |
| M41K, N78I | 1272 | Little or no protein produced | |
| I20L, I36T, N45D | 1277 | Little or no protein produced | |
| N17D, N45T, V50A, D72G | 1278 | Little or no protein produced | |
| I20L, F49S | 1279 | Little or no protein produced | |
| N45T, V50A | 1280 | 23887 | 4.6 |
| I20L, N45T, N78I | 1281 | 29104 | 5.6 |
| N45T, N78I | 1273 | 24865 | 4.7 |
| I20L, N45T | 1274 | 24279 | 4.6 |
| I20L, N45T, V50A | 1282 | 34158 | 6.5 |
| N45T | 1275 | 6687 | 1.3 |
| M41K | 1276 | 5079 | 1.0 |
| M41V, N45T | 1283 | Little or no protein produced | |
| M41K, N45T | 1284 | Little or no protein produced | |
| A33D, S75P, D85E | 1285 | 685 | 0.1 |
| M18I, M41K, D43G, H51R, N78I | 1286 | 20731 | 4.0 |
| V11E, I20L, I36T, N45D, H60R, S75P | 1287 | 3313 | 0.6 |
| A33D, V50A | 1288 | Little or no protein produced | |
| S16G, A33D, K71E, S75P | 1289 | Little or no protein produced | |
| E27G, N45T, M97I | 1290 | 881 | 0.2 |
| E27G, N45T, K57R | 1291 | 5022 | 1.0 |
| A33D, E53V | 1292 | 650 | 0.1 |
| D43G, N45D, V58A | 1293 | 63960 | 12.2 |
| E40G, D43V, N45T, V50A | 1294 | 809 | 0.2 |
| Y14S, K28E, N45T | 1295 | 16232 | 3.1 |
| A33D, N78S | 1296 | 1725 | 0.3 |
| A33D, N78I | 1297 | 8482 | 1.6 |
| A33D, N45T | 1298 | 17220 | 3.3 |
| A33D,N45T, N78I | 1299 | | |
| E27G, N45T, V50A | 1300 | 25267 | 4.8 |
| N45T, V50A, N78S | 1301 | 28572 | 5.4 |
| N45T, V50A | 1280 | 18717 | 3.6 |
| I20L, N45T, V110M | 1302 | 464 | 0.1 |
| I20L, I36T, N45T, V50A | 1303 | 7658 | 1.5 |
| N45T, L74P, S75P | 1304 | 5251 | 1.0 |
| N45T, S75P | 1305 | 12200 | 2.3 |
| S75P, K106R | 1306 | 388 | 0.1 |
| S75P | 1307 | 1230 | 0.2 |
| A33D, S75P | 1308 | 306 | 0.1 |
| A33D, S75P, D104G | 1309 | 251 | 0.0 |
| A33D, S75P | 1310 | 1786 | 0.3 |
| I20L, E27G, N45T, V50A | 1311 | 29843 | 5.7 |
| I20L, E27G, D43G, N45D, V58A, N78I | 1312 | 69486 | 13.3 |
| I20L, D43G, N45D, V58A, N78I | 1313 | 72738 | 13.9 |
| I20L, A33D, D43G, N45D, V58A, N78I | 1314 | 80205 | 15.3 |
| I20L, D43G, N45D, N78I | 1315 | 67018 | 12.8 |
| E27G, N45T, V50A, N78I | 1316 | 30677 | 5.9 |
| N45T, V50A, N78I | 1317 | 32165 | 6.1 |
| V11A, I20L, E27G, D43G, N45D, H51Y, S99G | 1318 | 73727 | 14.1 |
| I20L, E27G, D43G, N45T, V50A | 1319 | 36739 | 7.0 |
| I20L, K28E, D43G, N45D, V58A, Q89R, G101G-ins | 1320 | 80549 | 15.4 |
| I20L, I36T, N45D | 1321 | 16870 | 3.2 |
| I20L, K28E, D43G, N45D, E53G, V58A, N78I | 1322 | 139 | 0.0 |
| A33D, D43G, N45D, V58A, S75P | 1323 | 58484 | 11.2 |
| K23R, D43G, N45D | 1324 | 67559 | 12.9 |
| I20L, D43G, N45D, V58A, N78I, D90G, G101D | 1325 | 259 | 0.0 |
| D43G, N45D, L56Q, V58A, G101Gins (G101GG) | 1326 | 88277 | 16.8 |
| I20L, K23E, D43G, N45D, V58A, N78I | 1327 | 89608 | 17.1 |
| I20L, K23E, D43G, N45D, V50A, N78I | 1328 | 88829 | 16.9 |
| T19I, E27G, N45I, V50A, N78I, M97K | 1329 | 25496 | 4.9 |
| I20L, M41K, D43G, N45D | 1330 | 599 | 0.1 |
| K23R, N45T, N78I | 1331 | 84980 | 16.2 |
| Full length PD-L1 Fc | - | 18465 | 3.5 |
| Wild type PD-L1 IgV | 1332 | 5243 | 1.0 |
| Anti-PD-1 monoclonal antibody (nivolumab) | - | 79787 | 15.2 |
| Human IgG | - | 198 | 0.0 |

| **Table 25B: Flow Binding to Cells Expressing PD-1 or CD80** | | | | | |
|---|---|---|---|---|---|
| **PD-L1 Mutation(s)** | **SEQ ID NO (ECD)** | **PD-1** | | **CD80** | |
| | | **MFI at 20nM** | **Fold Change Compared to WT PD-L1** | **MFI at 20nM** | **Fold Change Compared to WT PD-L1** |
| K57R, S99G | 1875 | 2953 | 0.9 | 16253 | 121.3 |
| K57R, S99G, F189L | 1876 | 1930 | 0.6 | 12906 | 96.3 |
| M18V, M97L, F193S, R195G, E200K, H202Q | 1877 | 69 | 0.0 | 241 | 1.8 |
| I36S, M41K, M97L, K144Q, R195G, E200K, H202Q, L206F | 1878 | 3498 | 1.1 | 68715 | 512.8 |
| C22R, Q65L, L124S, K144Q, R195G, E200N, H202Q, T221L | 1879 | Little or no protein produced | | | |
| M18V, I98L, L124S, P198T, L206F | 1880 | 2187 | 0.7 | 143 | 1.1 |
| S99G, N117S, I148V, K171R, R180S | 1881 | Little or no protein produced | | | |
| I36T, M97L, A103V, Q155H | 1882 | 120 | 0.0 | 128 | 1.0 |
| K28I, S99G | 1883 | 830 | 0.3 | 693 | 5.2 |
| R195S | 1884 | 3191 | 1.0 | 138 | 1.0 |
| A79T, S99G, T185A, R195G, E200K, H202Q, L206F | 1885 | 1963 | 0.6 | 643 | 4.8 |
| K57R, S99G, L124S, K144Q | 1886 | 2081 | 0.7 | 14106 | 105.3 |
| K57R, S99G, R195G | 1887 | 2479 | 0.8 | 10955 | 81.8 |
| D55V, M97L, S99G | 1888 | 11907 | 3.8 | 71242 | 531.7 |
| E27G, 136T, D55N, M97L, K111E | 1889 | 1904 | 0.6 | 88724 | 662.1 |
| E54G, M97L, S99G | 1890 | 8414 | 2.7 | 51905 | 387.4 |
| G15A, I36T, M97L, K111E, H202Q | 1891 | 112 | 0.0 | 13530 | 101.0 |
| G15A, I36T, V129D | 1892 | 114 | 0.0 | 136 | 1.0 |
| G15A, I36T, V129D, R195G | 1893 | 125 | 0.0 | 134 | 1.0 |
| G15A, V129D | 1894 | 2075 | 0.7 | 128 | 1.0 |
| I36S, M97L | 1895 | 3459 | 1.1 | 44551 | 332.5 |
| I36T, D55N, M97L, K111E, A204T | 1896 | 265 | 0.1 | 62697 | 467.9 |
| I36T, D55N, M97L, K111E, V129A, F173L | 1897 | 393 | 0.1 | 72641 | 542.1 |
| I36T, D55S, M97L, K111E, I148V, R180S | 1898 | 94 | 0.0 | 30704 | 229.1 |
| I36T, G52R, M97L, V112A, K144E, V175A, P198T | 1899 | 81 | 0.0 | 149 | 1.1 |
| I36T, I46V, D55G, M97L, K106E, K144E, T185A, R195G | 1900 | 69 | 0.0 | 190 | 1.4 |
| I36T, I83T, M97L, K144E, P198T | 1901 | 62 | 0.0 | 6216 | 46.4 |
| I36T, M97L, K111E | 1902 | Little or no protein produced | | | |
| I36T, M97L, K144E, P198T | 1903 | 197 | 0.1 | 40989 | 305.9 |
| I36T, M97L, Q155H, F193S, N201Y | 1904 | 69 | 0.0 | 1251 | 9.3 |
| I36T, M97L, V129D | 1905 | 523 | 0.2 | 50905 | 379.9 |
| L35P, I36S, M97L, K111E | 1906 | 190 | 0.1 | 155 | 1.2 |
| M18I, I36T, E53G, M97L, K144E, E199G, V207A | 1907 | 104 | 0.0 | 47358 | 353.4 |
| M18T, I36T, D55N, M97L, K111E | 1908 | 138 | 0.0 | 71440 | 533.1 |
| M18V, M97L, T176N, R195G | 1909 | 1301 | 0.4 | 45300 | 338.1 |
| M97L, S99G | 1910 | 12906 | 4.1 | 81630 | 609.2 |
| N17D, M97L, S99G | 1911 | 10079 | 3.2 | 73249 | 546.6 |
| S99G, T185A, R195G, P198T | 1912 | 2606 | 0.8 | 22062 | 164.6 |
| V129D, H202Q | 1913 | 2001 | 0.6 | 219 | 1.6 |
| V129D, P198T | 1914 | 3245 | 1.0 | 152 | 1.1 |
| V129D, T150A | 1915 | 1941 | 0.6 | 142 | 1.1 |
| V93E, V129D | 1916 | 1221 | 0.4 | 150 | 1.1 |
| Y10F, M18V, S99G, Q138R, T203A | 1917 | 70 | 0.0 | 412 | 3.1 |
| WT **PD-L1** (IgV+IgC) Fc | - | 3121 | 1.0 | 134 | 1.0 |
| CTLA4-Fc | - | 59 | N/A | 199670 | N/A |
| Anti-PD1 mAb | - | 31482 | N/A | 134 | N/A |
| Fc Control | - | 59 | N/A | 132 | N/A |

| **TABLE 25C. Additional Affinity-Matured IgSF Domain-Containing Molecules** | | | |
|---|---|---|---|
| **PD-L1 Mutation(s)** | **SEQ ID NO (ECD)** | **PD-L1 Mutation(s)** | **SEQ ID NO (ECD)** |
| N45D | 1918 | N45D, G102D, R194W, R195G | 1943 |
| K160M, R195G | 1919 | N45D, G52V, Q121L, P198S | 1943 |
| N45D, K144E | 1920 | N45D, I148V, R195G, N201D | 1944 |
| N45D, P198S | 1921 | N45D, K111T, T183A, I188V | 1945 |
| N45D, P198T | 1922 | N45D, Q89R, F189S, P198S | 1946 |
| N45D, R195G | 1923 | N45D, S99G, C137R, V207A | 1947 |
| N45D, R195S | 1924 | N45D, T163I, K167R, R195G | 1948 |
| N45D, S131F | 1925 | N45D, T183A, T192S, R194G | 1949 |
| N45D, V58D | 1926 | N45D, V50A, I119T, K144E | 1950 |
| V129D, R195S | 1927 | T19A, N45D, K144E, R195G | 1951 |
| I98T, F173Y, L196S | 1928 | V11E, N45D, T130A, P198T | 1952 |
| N45D, E134G, L213P | 1929 | V26A, N45D, T163I, T185A | 1953 |
| N45D, F173I, S177C | 1930 | K23N, N45D, L124S, K167T, R195G | 1954 |
| N45D, I148V, R195G | 1931 | K23N, N45D, Q73R, T163I | 1955 |
| N45D, K111T, R195G | 1932 | K28E, N45D, W149R, S158G, P198T | 1956 |
| N45D, N113Y, R195S | 1933 | K28R, N45D, K57E, I98V, R195S | 1957 |
| N45D, N165Y, E170G | 1934 | K28R, N45D, V129D, T163N, R195T | 1958 |
| N45D, Q89R, I98V | 1935 | M41K, D43G, N45D, R64S, R195G | 1959 |
| N45D, S131F, P198S | 1936 | M41K, D43G, N45D, R64S, S99G | 1960 |
| N45D, S75P, P198S | 1937 | N45D, R68L, F173L, D197G, P198S | 1961 |
| N45D, V50A, R195T | 1938 | N45D, V50A, I148V, R195G, N201D | 1962 |
| E27D, N45D, T183A, I188V | 1939 | M41K, D43G, K44E, N45D, R195G, N201D | 1963 |
| F173Y, T183I, L196S, T203A | 1940 | N45D, V50A, L124S, K144E, L179P, R195G | 1964 |
| K23N, N45D, S75P, N120S | 1941 | | |

| **TABLE 26A: Variant PD-L2 selected against PD-1. Molecule sequence and binding data.** | | | | |
|---|---|---|---|---|
| **PD-L2 mutation(s)** | **SEQ ID NO (IgV)** | **Binding to Jurkat/PD-1 Cells** | | **Fortebio binding to PD-1-Fc Response Units** |
| | | **MFI at 50nM** | **Fold increase over wildtype PD-L2 IgV-Fc** | |
| H15Q | 1487 | 15998 | 1.63 | 0.007 |
| N24D | 1488 | 1414 | 0.14 | -0.039 |
| E44D | 1489 | 2928 | 0.3 | -0.006 |
| V89D | 1490 | 3361 | 0.34 | 0.005 |
| Q82R,V89D | 1491 | 44977 | 4.57 | 1.111 |
| E59G,Q82R | 1492 | 12667 | 1.29 | -0.028 |
| S39I,V89D | 1493 | 26130 | 2.65 | 0.26 |
| S67L,V89D | 1494 | 15991 | 1.62 | 0.608 |
| S67L,I85F | 1495 | 529 | 0.05 | -0.005 |
| S67L,I86T | 1496 | 6833 | 0.69 | 0.141 |
| H15Q,K65R | 1497 | 13497 | 1.37 | -0.001 |
| H15Q,Q72H,V89D | 1498 | 12629 | 1.28 | 0.718 |
| H15Q,S67L,R76G | 1499 | 47201 | 4.8 | 0.418 |
| H15Q,R76G,I85F | 1500 | 2941 | 0.3 | -0.038 |
| H15Q,T47A,Q82R | 1501 | 65174 | 6.62 | 0.194 |
| H15Q,Q82R,V89D | 1502 | 49652 | 5.04 | 1.198 |
| H15Q,C23S,I86T | 1503 | 830 | 0.08 | -0.026 |
| H15Q,S39I,I86T | 1504 | 1027 | 0.1 | 0.309 |
| H15Q,R76G,I85F | 1505 | 1894 | 0.19 | -0.006 |
| E44D,V89D,W91R | 1506 | 614 | 0.06 | -0.048 |
| I13V,S67L,V89D | 1507 | 26200 | 2.66 | 1.42 |
| H15Q,S67L,I86T | 1508 | 15952 | 1.62 | 0.988 |
| I13V,H15Q,S67L,I86T | 1509 | 21570 | 2.19 | 1.391 |
| I13V,H15Q,E44D,V89D | 1510 | 23958 | 2.43 | 1.399 |
| I13V,S39I,E44D,Q82R,V89D | 1511 | 71423 | 7.26 | 0.697 |
| I13V,E44D,Q82R,V89D | 1512 | 45191 | 4.59 | 1.283 |
| I13V,Q72H,R76G,I86T | 1513 | 10429 | 1.06 | 0.733 |
| I13V,H15Q,R76G,I85F | 1514 | 4736 | 0.48 | -0.04 |
| H15Q,S67L,R76G,I85F | 1516 | 2869 | 0.29 | 0.025 |
| H15Q,S39I,R76G,V89D | 1515 | Little or no protein produced | | |
| H15Q,T47A,Q72H,R76G,I86T | 1517 | 32103 | 3.26 | 0.512 |
| H15Q,T47A,Q72H,R76G | 1518 | 16500 | 1.68 | 0.327 |
| I13V,H15Q,T47A,Q72H,R76G | 1519 | 73412 | 7.46 | 0.896 |
| H15Q,E44D,R76G,I85F | 1520 | 2885 | 0.29 | -0.013 |
| H15Q,S39I,S67L,V89D | 1521 | 45502 | 4.62 | 1.174 |
| H15Q,N32D,S67L,V89D | 1522 | 25880 | 2.63 | 1.407 |
| N32D,S67L,V89D | 1523 | 31753 | 3.23 | 1.155 |
| H15Q,S67L,Q72H,R 76G,V89D | 1524 | 40180 | 4.08 | 1.464 |
| H15Q,Q72H,Q74R,R76G,I86T | 1525 | 4049 | 0.41 | 0.093 |
| G28V,Q72H,R76G,I86T | 1526 | 5563 | 0.57 | 0.003 |
| I13V,H15Q,S39I,E44D,S67L | 1527 | 63508 | 6.45 | 0.889 |
| E44D,S67L,Q72H,Q82R,V89D | 1528 | 51467 | 5.23 | 1.061 |
| H15Q,V89D | 1529 | 17672 | 1.8 | 0.31 |
| H15Q,T47A | 1530 | 26578 | 2.7 | 0.016 |
| I13V,H15Q,Q82R | 1531 | 76146 | 7.74 | 0.655 |
| I13V,H15Q,V89D | 1532 | 28745 | 2.92 | 1.331 |
| I13V,S67L,Q82R,V89D | 1533 | 58992 | 5.99 | 1.391 |
| I13V,H15Q,Q82R,V89D | 1534 | 49523 | 5.03 | 1.419 |
| H15Q,V31M,S67L,Q82R,V89D | 1535 | 67401 | 6.85 | 1.37 |
| I13V,H15Q,T47A,Q82R | 1536 | 89126 | 9.05 | 0.652 |
| I13V,H15Q,V31A,N45S,Q82R,V89D | 1537 | 68016 | 6.91 | 1.327 |
| I13V,T20A,T47A,K65X,Q82R,V89D | 1538 | Not tested | | |
| H15Q,T47A,H69L,Q82R,V89D | 1539 | 65598 | 6.66 | 1.44 |
| I13V,H15Q,T47A,H69L,R76G,V89D | 1540 | 54340 | 5.52 | 1.719 |
| I12V,I13V,H15Q,T47A,Q82R,V89D | 1541 | 61207 | 6.22 | 1.453 |
| I13V,H15Q,R76G,D77N,Q82R,V89D | 1542 | 33079 | 3.36 | 0.065 |
| I13V,H15Q,T47A,R76G,V89D | 1543 | 53668 | 5.45 | 1.596 |
| I13V,H15Q,T47A,Q82R,V89D | 1544 | 63320 | 6.43 | 1.418 |
| I13V,H15Q,T47A,Q82R,V89D | 1545 | 60980 | 6.2 | 1.448 |
| I13V,H15Q,I36V,T47A,S67L,V89D | 1546 | 52835 | 5.37 | 1.627 |
| H15Q,T47A,K65R,S67L,Q82R,V89D | 1547 | 79692 | 8.1 | 1.453 |
| H15Q,L33P,T47A,S67L,P71S,V89D | 1548 | 45726 | 4.65 | 1.467 |
| I13V,H15Q,Q72H,R76G,I86T | 1549 | 24450 | 2.48 | 1.355 |
| H15Q,T47A,S67L,Q82R,V89D | 1550 | 67962 | 6.9 | 1.479 |
| F2L,H15Q,D46E,T47A,Q72H,R76G,Q82R,V89D | 1551 | 23039 | 2.34 | 1.045 |
| I13V,H15Q,L33F,T47A,Q82R,V89D | 1552 | 62254 | 6.32 | 1.379 |
| I13V,H15Q,T47A,E58G,S67L,Q82R,V89D | 1543 | Not tested | | |
| H15Q,N24S,T47A,Q72H,R76G,V89D | 1554 | 32077 | 3.26 | 0.4 |
| I13V,H15Q,E44V,T47A,Q82R,V89D | 1555 | 61005 | 6.2 | 1.329 |
| H15Q,N18D,T47A,Q72H,V73A,R76G,I86T,V89D | 1556 | 48317 | 4.91 | 0.475 |
| I13V,H15Q,T37A,E44D,S48C,S67L,Q82R,V89D | 1557 | 47605 | 4.84 | 1.255 |
| H15Q,L33H,S67L,R76G,Q82R,V89D | 1558 | 62326 | 6.33 | 1.507 |
| I13V,H15Q,T47A,Q72H,R76G,I86T | 1559 | 49016 | 4.98 | 1.477 |
| H15Q,S39I,E44D,Q72H,V75G,R76G,Q82R,V89D | 1560 | 43713 | 4.44 | 0.646 |
| H15Q,T47A,S67L,R76G,Q82R,V89D | 1561 | 71897 | 7.3 | 1.539 |
| I13V,H15Q,T47A,S67L,Q72H,R76G,Q82R,V89D | 1562 | 71755 | 7.29 | 1.536 |
| Wild Type PD-L2 IgV | 1393 | 9843 | 1 | -0.024 |
| Full length ECD of PD-L2 | 31 (ECD) | 2145 | 0.22 | 0.071 |
| Full length ECD of PD-L1 (R&D Systems) | - | 23769 | 2.41 | 1.263 |
| Anti-PD-1 monoclonal antibody (nivolumab) | - | 87002 | 8.84 | 0.899 |

| **TABLE 26B: Bioactivity Data of PD-L2 variants selected against PD-1 in MLR.** | | | |
|---|---|---|---|
| **PD-L2 mutation(s)** | **SEQ ID NO (IgV)** | **IFN gamma levels pg/mL** | **Fold increase over wildtype PD-L2 IgV-Fc** |
| H15Q | 1487 | 1817.1 | 1.32 |
| N24D | 1488 | 1976.3 | 1.44 |
| E44D | 1489 | 1499.4 | 1.09 |
| V89D | 1490 | 1168.1 | 0.85 |
| Q82R,V89D | 1491 | 1617 | 1.17 |
| E59G,Q82R | 1492 | 1511.3 | 1.1 |
| S39I,V89D | 1493 | 1314.5 | 0.95 |
| S67L,V89D | 1494 | 1230.1 | 0.89 |
| S67L,I85F | 1495 | 1281.9 | 0.93 |
| S67L,I86T | 1496 | 1020.4 | 0.74 |
| H15Q,K65R | 1497 | 1510.8 | 1.1 |
| H15Q,Q72H,V89D | 1498 | 1272.2 | 0.92 |
| H15Q,S67L,R76G | 1499 | 1426.2 | 1.04 |
| H15Q,R76G,I85F | 1500 | 1725.7 | 1.25 |
| H15Q,T47A,Q82R | 1501 | 1317.9 | 0.96 |
| H15Q,Q82R,V89D | 1502 | 1081.2 | 0.79 |
| H15Q,C23S,I86T | 1503 | 1847.2 | 1.34 |
| H15Q,S39I,I86T | 1504 | 1415.2 | 1.03 |
| H15Q,R76G,I85F | 1505 | 1437.8 | 1.04 |
| E44D,V89D,W91R | 1506 | 1560.1 | 1.13 |
| I13V,S67L,V89D | 1507 | 867.5 | 0.63 |
| H15Q,S67L,I86T | 1508 | 1034.2 | 0.75 |
| I13V,H15Q,S67L,I86T | 1509 | 1014.4 | 0.74 |
| I13V,H15Q,E44D,V89D | 1510 | 1384.2 | 1.01 |
| I13V,S39I,E44D,Q82R,V89D | 1511 | 935.6 | 0.68 |
| I13V,E44D,Q82R,V89D | 1512 | 1009.5 | 0.73 |
| I13V,Q72H,R76G,I86T | 1513 | 1953 | 1.42 |
| I13V,H15Q,R76G,I85F | 1514 | 1528.5 | 1.11 |
| H15Q,S67L,R76G,I85F | 1516 | 1318.7 | 0.96 |
| H15Q,T47A,Q72H,R76G,I86T | 1517 | 1599.6 | 1.16 |
| H15Q,T47A,Q72H,R76G | 1518 | 1462.5 | 1.06 |
| I13V,H15Q,T47A,Q72H,R76G | 1519 | 1469.8 | 1.07 |
| H15Q,E44D,R76G,I85F | 1520 | 1391.6 | 1.01 |
| H15Q,S39I,S67L,V89D | 1521 | 1227 | 0.89 |
| H15Q,N32D,S67L,V89D | 1522 | 1285.7 | 0.93 |
| N32D,S67L,V89D | 1523 | 1194 | 0.87 |
| H15Q,S67L,Q72H,R 76G,V89D | 1524 | 1061.2 | 0.77 |
| H15Q,Q72H,Q74R,R76G,I86T | 1525 | 933.8 | 0.68 |
| G28V,Q72H,R76G,I86T | 1526 | 1781.6 | 1.29 |
| I13V,H15Q,S39I,E44D,S67L | 1527 | 1256.9 | 0.91 |
| E44D,S67L,Q72H,Q82R,V89D | 1528 | 1281.4 | 0.93 |
| H15Q,V89D | 1529 | 1495.4 | 1.09 |
| H15Q,T47A | 1530 | 1637.2 | 1.19 |
| I13V,H15Q,Q82R | 1531 | 1432.9 | 1.04 |
| I13V,H15Q,V89D | 1532 | 1123 | 0.82 |
| I13V,S67L,Q82R,V89D | 1533 | 1372.8 | 1 |
| I13V,H15Q,Q82R,V89D | 1534 | 1596.6 | 1.16 |
| H15Q,V31M,S67L,Q82R,V89D | 1535 | 1206.5 | 0.88 |
| I13V,H15Q,T47A,Q82R | 1536 | 1703.3 | 1.24 |
| I13V,H15Q,V31A,N45S,Q82R,V89D | 1537 | 1723.1 | 1.25 |
| H15Q,T47A,H69L,Q82R,V89D | 1539 | 1732.5 | 1.26 |
| I13V,H15Q,T47A,H69L,R76G,V89D | 1540 | 1075.5 | 0.78 |
| I12V,I13V,H15Q,T47A,Q82R,V89D | 1541 | 1533.2 | 1.11 |
| I13V,H15Q,R76G,D77N,Q82R,V89D | 1542 | 1187.9 | 0.86 |
| I13V,H15Q,T47A,R76G,V89D | 1543 | 1253.7 | 0.91 |
| I13V,H15Q,T47A,Q82R,V89D | 1544 | 1445.5 | 1.05 |
| I13V,H15Q,T47A,Q82R,V89D | 1545 | 1737 | 1.26 |
| I13V,H15Q,I36V,T47A,S67L,V89D | 1546 | 1357.4 | 0.99 |
| H15Q,T47A,K65R,S67L,Q82R,V89D | 1547 | 1335.3 | 0.97 |
| H15Q,L33P,T47A,S67L,P71S,V89D | 1548 | 1289.1 | 0.94 |
| I13V,H15Q,Q72H,R76G,I86T | 1549 | 1221 | 0.89 |
| H15Q,T47A,S67L,Q82R,V89D | 1550 | 1197.1 | 0.87 |
| F2L,H15Q,D46E,T47A,Q72H,R76G,Q82R,V89D | 1551 | 1170.7 | 0.85 |
| I13V,H15Q,L33F,T47A,Q82R,V89D | 1552 | 1468.4 | 1.07 |
| I13V,H15Q,T47A,E58G,S67L,Q82R,V89D | 1543 | 836.1 | 0.61 |
| H15Q,N24S,T47A,Q72H,R76G,V89D | 1554 | 1091.8 | 0.79 |
| I13V,H15Q,E44V,T47A,Q82R,V89D | 1555 | 1270.5 | 0.92 |
| H15Q,N18D,T47A,Q72H,V73A,R76G,I86T,V89D | 1556 | 1065.8 | 0.77 |
| I13V,H15Q,T37A,E44D,S48C,S67L,Q82R,V89D | 1557 | 1751.7 | 1.27 |
| H15Q,L33H,S67L,R76G,Q82R,V89D | 1558 | 1502 | 1.09 |
| I13V,H15Q,T47A,Q72H,R76G,I86T | 1559 | 1088.1 | 0.79 |
| H15Q,S39I,E44D,Q72H,V75G,R76G,Q82R,V89D | 1560 | 940.9 | 0.68 |
| H15Q,T47A,S67L,R76G,Q82R,V89D | 1561 | 1097.8 | 0.8 |
| I13V,H15Q,T47A,S67L,Q72H,R76G,Q82R,V89D | 1562 | 1559.6 | 1.13 |
| Wild Type PD-L2 IgV | 1393 | 1376.8 | 1 |
| Full length ECD of PD-L2 | 31 (ECD) | 1173.2 | 0.85 |
| Full length ECD of **PD-L1** | 2118 (ECD) | 2190.9 | 1.59 |
| Nivolumab (anti-PD-1) | - | 418.9 | 0.3 |

| **TABLE 27A: Variant CD80 Binding to HEK293 Cells Transfected with CTLA4, CD28 or PD-L1** | | | | | | | | |
|---|---|---|---|---|---|---|---|---|
| **CD80 mutation(s)** | **SEQ ID NO (IgV)** | **CTLA4** | | **CD28** | | **PD-L1** | | |
| | | **MFI at 66.6 nM** | **Fold change to WT** | **MFI at 66.6 nM** | **Fold change to WT** | **MFI at 22.2 nM** | **Fold change to WT** | **Ratio of CTLA 4:CD2 8** |
| L70P | 579 | Not tested | | | | | | |
| I30F/L70P | 580 | Not tested | | | | | | |
| Q27H/T41S/A71D | 581 | 368176 | 2.3 | 25051 | 1.01 | 24181 | N/A | 14.7 |
| I30T/L 70R | 582 | 2234 | 0.0 | 2596 | 0.10 | 5163 | N/A | 0.9 |
| T13R/C16R/L70Q/A71D | 583 | 197357 | 1.2 | 16082 | 0.65 | 9516 | N/A | 12.3 |
| T57I | 584 | 393810 | 2.4 | 23569 | 0.95 | 3375 | N/A | 16.7 |
| M43I/C82R | 585 | 3638 | 0.0 | 3078 | 0.12 | 7405 | N/A | 1.2 |
| V22L/M38V/M47T/A71D/ L85M | 586 | 175235 | 1.1 | 3027 | 0.12 | 6144 | N/A | 57.9 |
| 130V/T571/L70P/A71D/ A91T | 587 | 116085 | 0.7 | 10129 | 0.41 | 5886 | N/A | 11.5 |
| V22I/L70M/A71D | 588 | 163825 | 1.0 | 22843 | 0.92 | 33404 | N/A | 7.2 |
| N55D/L70P/E77G | 589 | Not tested | | | | | | |
| T57A/I69T | 590 | Not tested | | | | | | |
| N55D/K86M | 591 | 3539 | 0.0 | 3119 | 0.13 | 5091 | N/A | 1.1 |
| L72P/T79I | 592 | 50176 | 0.3 | 3397 | 0.14 | 6023 | N/A | 14.8 |
| L70P/F92S | 593 | 4035 | 0.0 | 2948 | 0.12 | 6173 | N/A | 1.4 |
| T79P | 594 | 2005 | 0.0 | 2665 | 0.11 | 4412 | N/A | 0.8 |
| E35D/M47I/L65P/D90N | 595 | 4411 | 0.0 | 2526 | 0.10 | 4034 | N/A | 1.7 |
| L25S/E35D/M47I/D90N | 596 | 61265 | 0.4 | 4845 | 0.20 | 20902 | N/A | 12.6 |
| Q27X*/S44P/I67T/P74S/ E81G/E95D | 597 | 195637 | 1.2 | 17524 | 0.71 | 17509 | N/A | 11.2 |
| A71D | 598 | 220090 | 1.4 | 16785 | 0.68 | 29642 | N/A | 13.1 |
| T13A/Q27X*/I61N/A71D | 599 | 195061 | 1.2 | 17519 | 0.71 | 21717 | N/A | 11.1 |
| E81K/A91S | 600 | 98467 | 0.6 | 3309 | 0.13 | 44557 | N/A | 29.8 |
| A12V/M47V/L70M | 601 | 81616 | 0.5 | 7400 | 0.30 | 31077 | N/A | 11.0 |
| K34E/T41A/L72V | 602 | 88982 | 0.6 | 3755 | 0.15 | 35293 | N/A | 23.7 |
| T41S/A71D/V84A | 603 | 103010 | 0.6 | 5573 | 0.22 | 83541 | N/A | 18.5 |
| E35D/A71D | 604 | 106069 | 0.7 | 18206 | 0.73 | 40151 | N/A | 5.8 |
| E35D/M47I | 605 | 353590 | 2.2 | 14350 | 0.58 | 149916 | N/A | 24.6 |
| K36R/G78A | 606 | 11937 | 0.1 | 2611 | 0.11 | 5715 | N/A | 4.6 |
| Q33E/T41A | 607 | 8292 | 0.1 | 2442 | 0.10 | 3958 | N/A | 3.4 |
| M47V/N48H | 608 | 207012 | 1.3 | 14623 | 0.59 | 145529 | N/A | 14.2 |
| M47L/V68A | 609 | 74238 | 0.5 | 13259 | 0.53 | 11223 | N/A | 5.6 |
| S44P/A71D | 610 | 8839 | 0.1 | 2744 | 0.11 | 6309 | N/A | 3.2 |
| Q27H/M43I/A71D/R73 S | 611 | 136251 | 0.8 | 12391 | 0.50 | 8242 | N/A | 11.0 |
| E35D/T57I/L70Q/A71D | 613 | 121901 | 0.8 | 21284 | 0.86 | 2419 | N/A | 5.7 |
| M47I/E88D | 614 | 105192 | 0.7 | 7337 | 0.30 | 97695 | N/A | 14.3 |
| M421/161V/A71D | 615 | 54478 | 0.3 | 6074 | 0.24 | 4226 | N/A | 9.0 |
| P51A/A71D | 616 | 67256 | 0.4 | 4262 | 0.17 | 5532 | N/A | 15.8 |
| H18Y/M47I/T57I/A71G | 617 | 136455 | 0.8 | 20081 | 0.81 | 13749 | N/A | 6.8 |
| V20I/M47V/T57I/V84I | 618 | 183516 | 1.1 | 26922 | 1.08 | 3583 | N/A | 6.8 |
| WT | 578 | 161423 | 1.0 | 24836 | 1.00 | Not tested | N/A | 6.5 |

| | | | | | | | | |
|---|---|---|---|---|---|---|---|---|
| *Stop codon at indicated position | | | | | | | | |

| **TABLE 27B: Variant CD80 Binding to HEK293 Cells Transfected with CTLA4, CD28 or PD-L1** | | | | | | | | |
|---|---|---|---|---|---|---|---|---|
| **CD80 mutation(s)** | **SEQ ID** NO **(IgV)** | **CTLA4** | | **CD28** | | **PD-L1** | | |
| | | **MFI at 66.6 nM** | **Fold change to WT** | **MFI at 66.6 nM** | **Fold change to WT** | **MFI at 22.2 nM** | **Fold change to WT** | **Ratio of CTLA4: CD28** |
| V20I/M47V/A71D | 619 | 149937 | 7.23 | 15090 | 9.33 | 9710 | 5.48 | 9.9 |
| A71D/L72V/E95K | 620 | 140306 | 6.77 | 6314 | 3.90 | 8417 | 4.75 | 22.2 |
| V22L/E35G/A71D/L72P | 621 | 152588 | 7.36 | 8150 | 5.04 | 1403 | 0.79 | 18.7 |
| E35D/A71D | 622 | 150330 | 7.25 | 14982 | 9.26 | 13781 | 7.77 | 10.0 |
| E35D/I67L/A71D | 623 | 146087 | 7.04 | 11175 | 6.91 | 9354 | 5.28 | 13.1 |
| T13R/M42V/M47I/A71D | 625 | 108900 | 5.25 | 16713 | 10.33 | 1869 | 1.05 | 6.5 |
| E35D | 626 | 116494 | 5.62 | 3453 | 2.13 | 25492 | 14.38 | 33.7 |
| E35D/M47I/L70M | 627 | 116531 | 5.62 | 14395 | 8.90 | 49131 | 27.71 | 8.1 |
| E35D/A71/L72V | 628 | 134252 | 6.47 | 11634 | 7.19 | 13125 | 7.40 | 11.5 |
| E35D/M43L/L70M | 629 | 102499 | 4.94 | 3112 | 1.92 | 40632 | 22.92 | 32.9 |
| A26P/E35D/M43I/L85Q/ E88D | 630 | 83139 | 4.01 | 5406 | 3.34 | 9506 | 5.36 | 15.4 |
| E35D/D46V/L85Q | 631 | 85989 | 4.15 | 7510 | 4.64 | 38133 | 21.51 | 11.4 |
| Q27L/E35D/M47I/T57I/ L70Q/E88D | 632 | 59793 | 2.88 | 14011 | 8.66 | 1050 | 0.59 | 4.3 |
| Q27H/E35G/A71D/L72P/ T79I | 624 | 85117 | 4.10 | 10317 | 6.38 | 1452 | 0.82 | 8.3 |
| M47V/I69F/A71D/V83I | 633 | 76944 | 3.71 | 15906 | 9.83 | 3399 | 1.92 | 4.8 |
| E35D/T57A/A71D/L85Q | 634 | 85724 | 4.13 | 3383 | 2.09 | 1764 | 0.99 | 25.3 |
| H18Y/A26T/E35D/A71D/ L85Q | 635 | 70878 | 3.42 | 6487 | 4.01 | 8026 | 4.53 | 10.9 |
| E35D/M47L | 636 | 82410 | 3.97 | 11508 | 7.11 | 58645 | 33.08 | 7.2 |
| E23D/M42V/M43I/I58V/ L70R | 637 | 37331 | 1.80 | 10910 | 6.74 | 2251 | 1.27 | 3.4 |
| V68M/L70M/A71D/E95K | 638 | 56479 | 2.72 | 10541 | 6.51 | 38182 | 21.53 | 5.4 |
| N55I/T57I/I69F | 639 | 2855 | 0.14 | 1901 | 1.17 | 14759 | 8.32 | 1.5 |
| E35D/M43I/A71D | 640 | 63789 | 3.08 | 6369 | 3.94 | 27290 | 15.39 | 10.0 |
| T41SAT57I/L70R | 641 | 59844 | 2.89 | 4902 | 3.03 | 19527 | 11.01 | 12.2 |
| H18Y/A71D/L72P/E88V | 642 | 68391 | 3.30 | 8862 | 5.48 | 1085 | 0.61 | 7.7 |
| V20I/A71D | 643 | 60323 | 2.91 | 10500 | 6.49 | 3551 | 2.00 | 5.7 |
| E23G/A26S/E35D/T62N/ A71D/L72V/L85M | 644 | 59025 | 2.85 | 5484 | 3.39 | 10662 | 6.01 | 10.8 |
| A12T/E24D/E35D/D46V/ I61V/L72P/E95V | 645 | 63738 | 3.07 | 7411 | 4.58 | 1221 | 0.69 | 8.6 |
| V22L/E35D/M43L/A71G/ D76H | 646 | 2970 | 0.14 | 1498 | 0.93 | 1851 | 1.04 | 2.0 |
| E35G/K54E/A71D/L72P | 647 | 71899 | 3.47 | 3697 | 2.29 | 1575 | 0.89 | 19.4 |
| L70Q/A71D | 648 | 45012 | 2.17 | 18615 | 11.50 | 1692 | 0.95 | 2.4 |
| A26E/E35D/M47L/L85Q | 649 | 40325 | 1.94 | 2266 | 1.40 | 55548 | 31.33 | 17.8 |
| D46E/A71D | 650 | 69674 | 3.36 | 16770 | 10.36 | 22777 | 12.85 | 4.2 |
| Y31H/E35D/T41S/V68L/ K93R/R94W | 651 | 3379 | 0.16 | 2446 | 1.51 | 18863 | 10.64 | 1.4 |
| CD80 IgV Fc | 578 (IgV) | 20739 | 1.00 | 1618 | 1.00 | 1773 | 1.00 | 12.8 |
| CD80 ECD Fc | 28 (ECD) | 72506 | 3.50 | 3072 | 1.90 | 4418 | 2.49 | 23.6 |

### Example 12

### Generation of Secreted Immunomodulatory Protein

To generate a PD-L2 secreted immunomodulatory protein (SIP), DNA encoding exemplary SIPs was obtained as gene blocks from Integrated DNA Technologies (Coralville, USA) and then cloned by Gibson assembly (New England Biolabs Gibson assembly kit) into a modified version of pRRL vector (Dull et al., (1998) J Virol, 72(11): 8463-8471) between restriction sites downstream of MND promoter to remove GFP. Exemplary SIP constructs were generated to encode a protein set forth in SEQ ID NO: 1571-1573, including the signal peptide. In this exemplary Example, the constructs were generated to additionally include a tag moiety. The gene blocks had the following structure in order: 39 base pair overlap with pRRL prior to first restriction site-first restriction site-GCCGCCACC (Kozak); complete ORF encoding PD-L2 IgV wildtype amino acid sequence set forth in SEQ ID NO:1393 or variant PD-L2 IgV set forth in SEQ ID NO:1535 (H15Q,V31M,S67L,Q82R,V89D), SEQ ID NO:1547 (H15Q,T47A,K65R,S67L,Q82R,V89D) or SEQ ID NO: 1561 (H15Q,T47A,S67L,R76G,Q82R,V89D), also including in all cases the PD-L2 signal peptide MIFLLLMLSLELQLHQIAA as set forth in SEQ ID NO: 1567; DNA encoding Avitag as set forth in SEQ ID NO:1568 (GLNDIFEAQKIEWHE); DNA encoding His tag as set forth in SEQ ID NO: 1569 (HHHHHH); TAA stop codon; second restriction site- 41 base pair overlap with pRRL beyond second restriction site.

To prepare lentiviral vectors, 3x10⁶ HEK293 cells were plated per 100mm dish. On the next day, 4.5µg of P-Mix (3µg of PAX2 and 1.5µg of pMD2G) was added to 6µg of DNA encoding the SIPs constructs in a 5mL polypropylene tube. Diluent buffer (10mM HEPES/150mM NaCl pH7.05/1L TC grade H20) was added to the tube to bring up the total volume of 500µL. To the diluent DNA(PEI:total DNA 4:1), 42µL of PEI (1µg/µL) was added and mixed by vortexing. The mixture was incubated at room temperature for 10 minutes and cells were prepared by aspirating medium from the dish gently without disturbing the adherent cells, then replaced with 6mL of Opti-MEM(1X). DNA/PEI mixture was then added to the dish and incubated at 37°C for 24 hours. After 24 hours, media was aspirated from the dishes and replaced with 10mL of fresh DMEM media and then incubated at 37°C. Viral supernatant was collected after 48 hours using a syringe attached to a 0.45µm filter PES to remove cells and debris from the culture (Thermo Scientific Nalgene Syringe Filter). A separate lentiviral vector stock also was prepared encoding an anti-CD 19 CAR (containing an anti-CD 19 scFv, a hinge and transmembrane domain derived from CD8, and a CD3zeta signaling domain) substantially as described. The exemplary anti-CD19 CAR used is set forth in SEQ ID NO: 2160 (encoded by the sequence in set forth in SEQ ID NO: 2161) containing the scFv set forth in SEQ ID NO: 1576, the CD8-derived hinge and transmembrane domain set forth in SEQ ID NO: 1574, and the CD3zeta set forth in SEQ ID NO:1575.

Pan T-cells were transduced with the viral vectors encoding the PD-L2 SIPs. T-cells were thawed and activated with anti-CD3/anti-CD28 beads (Dynal) at a 1:1 ratio. The T-cells (1 x 10⁶ cells) were mixed with 1 mL total lentiviral vector supernatant containing equal volume (0.5 mL each) of the lentiviral vector supernatant encoding the indicated PD-L2 SIPs and a lentiviral vector supernatant encoding the anti-CD19 CAR. As a control, cells were transduced only with the lentiviral vector encoding the anti-CD 19 CAR or were transduced with mock vector control. Transduction was performed in the presence of 10 µg/mL polybrene and 50 IU/mL IL-2. Cells were spun down at 2500 rpm for 60 min at 30°C. After 24 hours, 3mL of Xvivo15 plus media and IL2 was added to each well. The cells were fed every two days with fresh media and cytokines.

Transduction also was carried out on HEK-293 cells, which were resuspended at 2x10⁵ cells with 1mL of the lentiviral supernatant encoding the indicated PD-L2 SIPs. To the cells, 3mL of DMEM media was added and cells were fed every two days with fresh media.

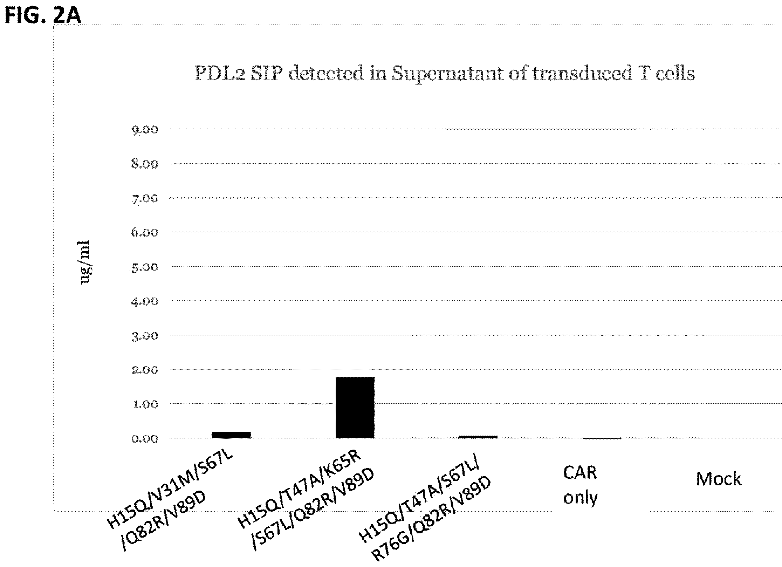

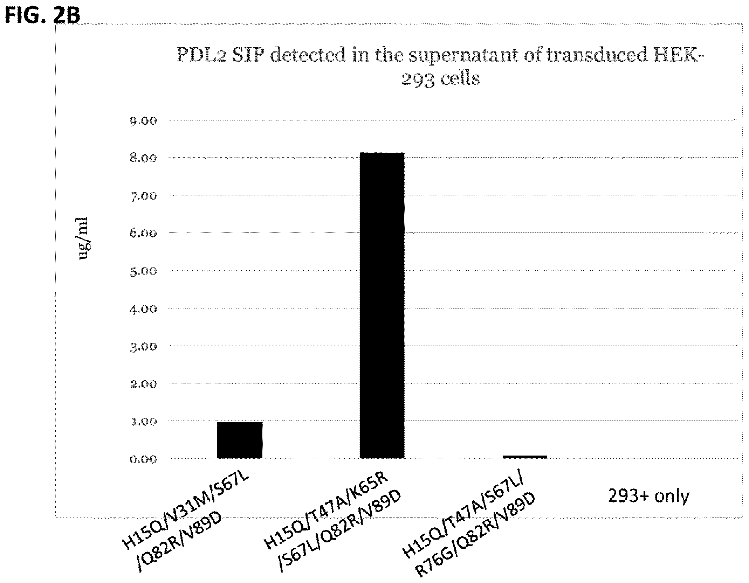

To assess the amount of secreted SIP, a cell-based assay was performed to assess binding of the secretable variant PD-L2 to PD-1. Approximately, 100,000 PD-1+ Jurkat cells were plated per well in the presence of 50µL of culture supernatant containing PD-L2 SIP obtained from transduced cells above and incubated at 4°C for 30 minutes. To generate a standard curve, 50 µL of the respective variant PD-L2 protein was added to PD-1+ Jurkat cells at 10µg/mL, 3 µg/mL, 1µg/mL, 0.3µg/mL, 0.1µg/mL, and 0µg/mL and also incubated at 4°C for 30 minutes. Cells were washed and 50 µL of anti-his-APC were added (1:50) and this was incubated at 4°C for 30 minutes. Surface bound PD-L2 protein was detected by flow cytometry and the concentration of SIP in the supernatant sample was determined by comparison to the standard curve. As shown in FIG. 2A and 2B, SIP proteins were detected in the supernatant of transduced T cells and transduced HEK293 cell, but were not detected from supernatant samples from mock transduced or cells transduced without SIPs.

### Example 13

### Assessment of Proliferation and Bioactivity of Pan T cells transduced with PD-L2 SIP

Pan T-cells were transduced essentially as described in Example 12 with the viral vectors encoding the PD-L2 SIPs. T-cells were thawed and activated with anti-CD3/anti-CD28 beads (Dynal) at a 1:1 ratio. The T-cells (1 x 10⁶ cells) were mixed with 1 mL total lentiviral vector supernatant containing equal volume (0.5 mL each) of the lentiviral vector supernatant encoding the indicated PD-L2 SIPs and a lentiviral vector supernatant encoding the anti-CD 19 CAR. As a control, cells were transduced only with the lentiviral vector encoding the anti-CD 19 CAR or were transduced with mock vector control. Transduction was performed in the presence of 10 µg/mL polybrene and 50 IU/mL IL-2. Cells were spun down at 2500 rpm for 60 min at 30°C. After 24 hours, 3mL of Xvivo15 plus media and IL2 was added to each well. The cells were fed every two days with fresh media and cytokines.

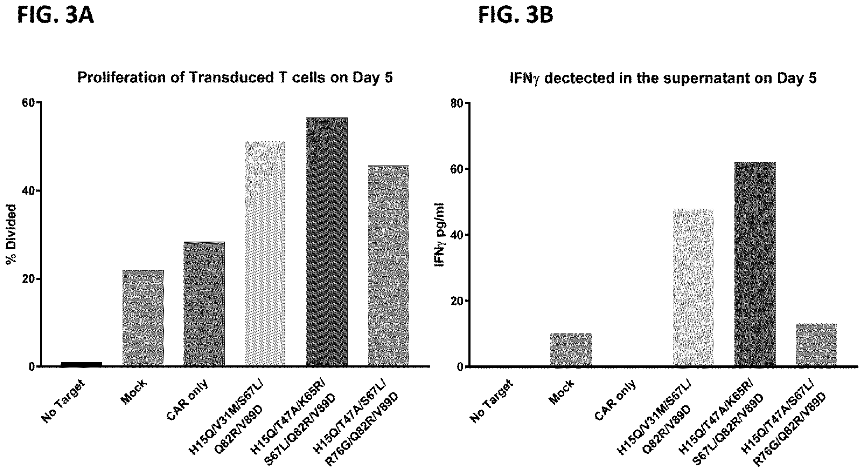

At 14 days after activation, cells were re-stimulated with Nalm6 cells that had been transduced with a lenti-viral vector to provide expression of PD-L1 (Nalm6 PDL1+). Transduced T cells were labeled with Cell Trace Far Red and proliferation was measured at day 5 by determining the fraction of the cells that showed dilution of the dye. Results for the proliferation studies for T cells transduced with exemplary tested variant PD-L2 SIP are shown in FIG. 3A.

Levels of IFN-gamma released into the supernatant were measured by ELISA on day 5 after re-stimulation. Results for the bioactivity studies for T cells transduced with exemplary tested variant PD-L2 SIP are shown in FIG. 3B. The T cells transduced with PD-L2 variant SIP are identified with reference to the amino acid substitutions in the IgV of PD-L2 with reference to positions corresponding to positions of the unmodified (wildtype) PD-L2 ECD sequence set forth in SEQ ID NO:31. As shown in FIG. 3A and 3B, proliferation and improved activities to increase immunological activity was observed.

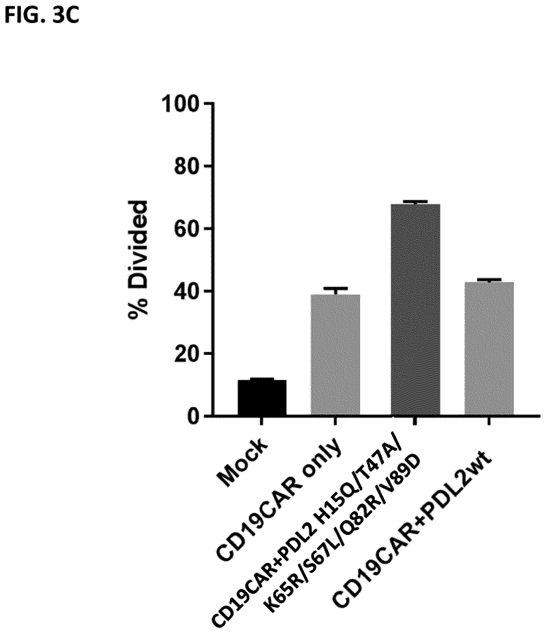

A similar study was carried out except that T cells were co-transduced with the anti-CD19 CAR and a lentiviral vector encoding a SIP, either a variant PD-L2 IgV or wild-type (WT) PD-L2 IgV. Following stimulation of transduced T cells with Nalm6 PDL1+ cells as described above, the proliferation of T cells was measured at day 3 by determining the fraction of the cells that showed dilution of the dye. As shown in FIG. 3C, cells engineered with the variant PD-L2 SIP improved proliferation compared to proliferation of T cells only expressing the CAR, and the improved proliferation was also greater than the proliferation of T cells expressing the wild-type PD-L2 SIP.

### Example 14

### Generation of Secreted Immunomodulatory Protein and Assessment of Proliferation of Pan T cells transduced with PD-L1 SIP

To generate a PD-L1 secreted immunomodulatory protein (SIP), DNA encoding exemplary SIPs was obtained as gene blocks from Integrated DNA Technologies (Coralville, USA) and then cloned by Gibson assembly (New England Biolabs Gibson assembly kit) into a modified version of pRRL vector (Dull et al., (1998) J Virol, 72(11): 8463-8471) between restriction sites downstream of MND promoter to remove GFP. Exemplary PD-L1 SIP constructs were generated to encode a protein set forth in SEQ ID NO: 2155-2156, including the signal peptide. In this exemplary Example, the constructs were generated to additionally include a tag moiety. The gene blocks had the following structure in order: 39 base pair overlap with pRRL prior to first restriction site-first restriction site-GCCGCCACC (Kozak); complete ORF encoding PD-L1 IgV wildtype amino acid sequence set forth in SEQ ID NO: 1332 or variant PD-L1 IgV set forth in SEQ ID NO: 1326 (D43G/N45D/L56Q/V58A/G101G-ins (G101GG), also including in all cases the signal peptide MGSTAILALLLAVLQGVSA as set forth in SEQ ID NO: 2157; DNA encoding Flag-tag as set forth in SEQ ID NO:2154 (DYKDDDDK); DNA encoding His tag as set forth in SEQ ID NO: 1569 (HHHHHH); TAA stop codon; second restriction site- 41 base pair overlap with pRRL beyond second restriction site. For comparison, a SIP encoding wild-type PD-L1 also was assessed.

To prepare lentiviral vectors, 3x10⁶ HEK293 cells were plated per 100mm dish. On the next day, 4.5µg of P-Mix (3µg of PAX2 and 1.5µg of pMD2G) was added to 6µg of DNA encoding the SIPs constructs in a 5mL polypropylene tube. Diluent buffer (10mM HEPES/150mM NaCl pH7.05/1L TC grade H20) was added to the tube to bring up the total volume of 500µL. To the diluent DNA(PEI:total DNA 4:1), 42µL of PEI (1µg/µL) was added and mixed by vortexing. The mixture was incubated at room temperature for 10 minutes and cells were prepared by aspirating medium from the dish gently without disturbing the adherent cells, then replaced with 6mL of Opti-MEM(IX). DNA/PEI mixture was then added to the dish and incubated at 37°C for 24 hours. After 24 hours, media was aspirated from the dishes and replaced with 10mL of fresh DMEM media and then incubated at 37°C. Viral supernatant was collected after 48 hours using a syringe attached to a 0.45µm filter PES to remove cells and debris from the culture (Thermo Scientific Nalgene Syringe Filter). A separate lentiviral vector stock also was prepared encoding an anti-CD 19 CAR (containing an anti-CD 19 scFv, a hinge and transmembrane domain derived from CD8, and a CD3zeta signaling domain) substantially as described. The exemplary anti-CD19 CAR used is set forth in SEQ ID NO: 2160 (encoded by the sequence in set forth in SEQ ID NO: 2161) containing the scFv set forth in SEQ ID NO:1576, the CD8-derived hinge and transmembrane domain set forth in SEQ ID NO: 1574, and the CD3zeta set forth in SEQ ID NO:1575.

T-cells were thawed and activated with anti-CD3/anti-CD28 beads (Dynal) at a 1:1 ratio. The T-cells (1 x 10⁶ cells) were mixed with 1 mL total lentiviral vector supernatant containing equal volume (0.5 mL each) of the lentiviral vector supernatant encoding the indicated PD-L1 SIP (D43G/N45D/L56Q/V58A/G101GG or wildtype) and a lentiviral vector supernatant encoding the anti-CD19 CAR. As a control, cells were transduced only with the lentiviral vector encoding the anti-CD 19 CAR or were transduced with mock vector control. Transduction was performed in the presence of 10 µg/mL polybrene and 50 IU/mL IL-2. Cells were spun down at 2500 rpm for 60 min at 30°C. After 24 hours, 3mL of Xvivo15 plus media and IL2 was added to each well. The cells were fed every two days with fresh media and cytokines.

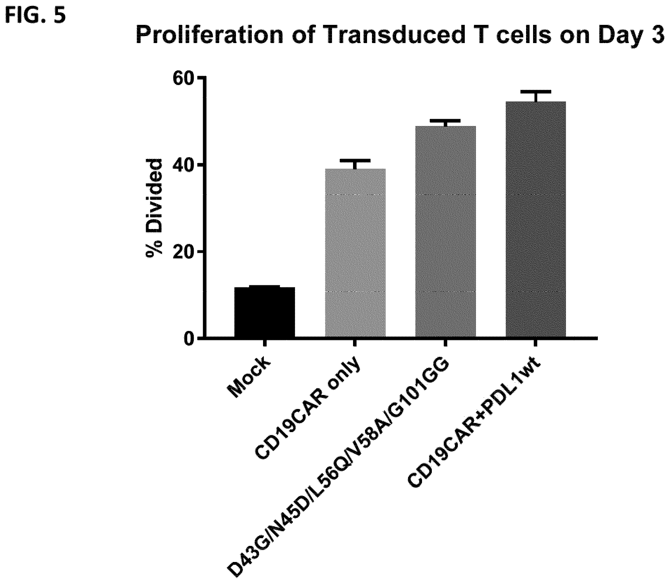

At 14 days after activation, cells were re-stimulated with Nalm6 cells that had been transduced with a lenti-viral vector to provide expression of PD-L1 (Nalm6 PDL1+). Transduced T cells were labeled with Cell Trace Far Red and proliferation was measured at day 3 by determining the fraction of the cells that showed dilution of the dye. Results for the proliferation studies for T cells transduced with exemplary tested variant PD-L1 SIP are shown in FIG. 5.

### Example 15

### Detection of Secreted Immunomodulatory Protein in Supernatant of Transduced Cells

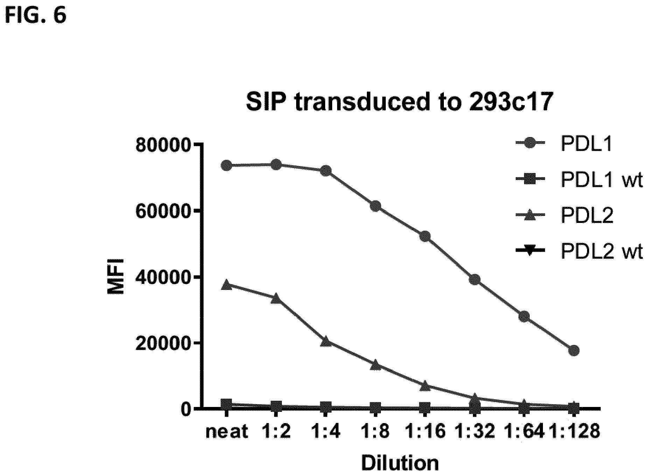

A cell-based assay was employed to detect the presence of SIPs in culture supernatant. HEK-293 cells were transduced with a lentiviral vector encoding exemplary SIPs, variant PD-L1 IgV (D43G/N45D/L56Q/V58A/G101G-ins(G101GG) set forth in SEQ ID NO:1326), wild-type PD-L1 IgV (set forth in SEQ ID NO:1332), variant PD-L2 IgV (HI5Q/T47A/K65R/S67L/Q82R/V89D set forth in SEQ ID NO:1547) or wild-type PD-L2 IgV (set forth in SEQ ID NO:1393), as described in Examples 12 and 14. Four days after transduction, supernatant was collected. Approximately 50 µL of supernatant, diluted 1:2, 1:4, 1:8, 1:16, 1:32, 1:64, 1:128 or neat, was added to 1 x 10⁵ K562 cells transduced to express PD-1 (K562 PD-1+) in a 96-well round bottom plate, and incubated at 4°C for 30 minutes. To generate a standard curve, PD-L2 his tag protein, diluted to 10,000 pg/mL, 1,000 pg/mL, 100 pg/mL, 10 pg/mL, 1 pg/mL and 0.1 pg/mL and also incubated with K562 PD-1+ cells at 4°C for 30 minutes. Cells were washed and 50 µL of anti-his-APC were added (1:50) and this was incubated at 4°C for 30 minutes. Surface bound PD-L2 protein was detected by flow cytometry and the concentration of SIP in the supernatant sample was determined by comparison to the standard curve. As shown in FIG. 6, the variant PD-L1 and variant PD-L2 SIPs, but not the wild-type proteins, were detected in the supernatant of cells.

The cell-based assay described above was used to assess the presence of PD-L2 SIP in supernatant of T cells following culture with antigen-expressing target cells. T cells were activated and transduced with a lentiviral vector encoding an anti-CD 19 CAR and a lentiviral vector encoding either variant PD-L2 H15Q/T47A/K65R/S67L/Q82R/V89D set forth in SEQ ID NO:1547) or wild-type PD-L2 IgV (set forth in SEQ ID NO:1393), as described in Example 9. At 14 days after activation, transduced T cells were cultured with CD19-expressing Nalm6 PD-L1+ cells or Raji cells. At days 3, 6, 9 and 12 after initiation of culture of T cells, supernatant was collected and the presence of PD-L2 was detected as described above. The variant PD-L2 SIP, but not the wild-type PD-L2 SIP, was detected in supernatant following stimulation with target antigen-expressing cells using this assay. This result may be due to the wild-type PD-L2 SIP not having a high enough affinity to bind to the K562/PD-1+ cells. The variant PD-L2 SIP was detected at substantially higher levels in supernatant of T cells stimulated with Raji cells compared to Nalm6 PD-L1 cells, and the level of variant PD-L2 SIP in the supernatant following stimulation with Raji cells was sustained throughout the time course of this study.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. An engineered immune cell comprising a nucleic acid molecule that encodes an immunomodulatory protein, wherein:
wherein the immune cell is a T cell;
the immunomodulatory protein comprises at least one non-immunoglobulin affinity-modified immunoglobulin superfamily (IgSF) domain of CD80 comprising one or more amino acid substitutions in a wild-type IgSF domain of CD80, wherein the at least one IgSF domain of CD80 specifically binds to at least two cell surface cognate binding partners, wherein (i) the first cell surface cognate binding partner is a stimulatory receptor expressed on a T cell, wherein the stimulatory receptor is CD28, and (ii) the second cell surface cognate binding partner is an inhibitory ligand of an inhibitory receptor expressed on a T cell, wherein the inhibitory ligand is PD-L1 and the inhibitory receptor is PD-1;
the engineered immune cell expresses and secretes the immunomodulatory protein;
the at least one affinity-modified IgSF domain of CD80 comprises at least 90% sequence identity with a wild-type IgSF domain as set forth in SEQ ID NO:1.

2. The engineered immune cell of claim 1, wherein binding of the at least one affinity-modified IgSF domain of CD80 to at least one its cognate binding partners is increased compared to the wild-type IgSF domain of CD80, optionally wherein the at least one affinity-modified IgSF domain of CD80 binds with increased affinity to CD28 and with increased affinity to PD-L1.

3. The engineered immune cell of claim 1 or claim 2, wherein the at least one affinity-modified IgSF domain of CD80 binds non-competitively to the at least two cell surface cognate binding partners.

4. The engineered immune cell of any one of claims 1-3, wherein the nucleic acid molecule comprises a sequence encoding a secretory signal peptide operably linked to the sequence encoding the immunomodulatory protein.

5. The engineered immune cell of claim 4, wherein the signal peptide is the native signal peptide from the corresponding wild-type IgSF domain of CD80 or wherein the signal peptide is a non-native signal sequence, optionally wherein the non-native signal sequence is an IgG-kappa signal peptide, an IL-2 signal peptide, or a CD33 signal peptide.

6. The engineered immune cell of any of claims 1-5, wherein the nucleic acid molecule further comprises at least one promoter operably linked to control expression of the immunomodulatory protein, optionally wherein the promoter is a constitutively active promoter or is an inducible promoter.

7. The engineered immune cell of claim 6, wherein the promoter is responsive to an element responsive to T-cell activation signaling, optionally wherein the promoter comprises a binding site for NFAT or a binding site for NF-κB.

8. The engineered immune cell of any of claims 1-7, wherein the T cell is CD4+ or CD8+ and/or wherein the immune cell is a primary cell obtained from a subject, optionally wherein the subject is a human subject.

9. The engineered immune cell of any of claims 1-8, wherein the at least one affinity-modified IgSF domain of CD80 is an affinity-modified IgV domain.

10. The engineered immune cell of any of claims 1-9, wherein the engineered immune cell further comprises a chimeric antigen receptor (CAR) or an engineered T-cell receptor (TCR).

11. A pharmaceutical composition comprising the engineered immune cell of any of claims 1-10 and a pharmaceutically acceptable carrier.

12. A composition for use in the treatment of a disease or disorder in a subject, wherein the composition comprises the engineered immune cell of any of claims 1-10 and a pharmaceutically acceptable carrier.

13. The composition for use of claim 12, wherein the composition for use increases an immune response in the subject.

14. The composition for use of claim 12 or claim 13, wherein the disease or disorder is a cancer.

15. The composition for use of any of claims 12-14, wherein the cell is autologous to the subject or is allogeneic to the subject.
